# EUROPEAN PATENT APPLICATION

(11) **EP 4 564 002 A1**
(43) Date of publication of application: **04.06.2025**
(21) Application number: 23849204.5
(22) Date of filing: 17.07.2023
(51) Int. Cl.: G01N 33/48

(54) **KIT, MEASUREMENT ASSEMBLY, AND BLOOD GAS ANALYSIS DEVICE**

(30) Priority: 31.07.2022 CN 202210912914
(71) Applicant: Edan Instruments, Inc., Shenzhen, Guangdong 518000 (CN)
(72) Inventor: HUANG, Gaoxiang, Shenzhen, Guangdong 518000 (CN); LIN, Cai, Shenzhen, Guangdong 518000 (CN); LI, Yong, Shenzhen, Guangdong 518000 (CN); ZHAO, Zhixiang, Shenzhen, Guangdong 518000 (CN); ZHUANG, Zhenwei, Shenzhen, Guangdong 518000 (CN); LI, Yuanbiao, Shenzhen, Guangdong 518000 (CN); LI, Guoxia, Shenzhen, Guangdong 518000 (CN); ZHANG, Xing, Shenzhen, Guangdong 518000 (CN)
(74) Representative: Herrero & Asociados, S.L.
(86) International application number: PCT/CN2023/107779
(87) International publication number: WO 2024/027496

(57) **Abstract**

The present application provides a blood gas analysis device, comprising a first measurement assembly and a kit. The first measurement assembly is provided with a measurement element, a first liquid path, and a first interface, the first liquid path is communicated with the first interface, and the measurement element is used for performing blood gas measurement on a liquid in the first liquid path. The kit is provided with an accommodating cavity, a second liquid path and a second interface, the accommodating cavity is used for placing a liquid bag, one end of the second liquid path is communicated with the liquid bag, and the other end of the second liquid path is communicated with the second interface. The first measurement assembly and the kit can be in butt joint with each other or separated, so that the first interface and the second interface are communicated or separated. During being communicated, the first liquid path is communicated with the second liquid path, so that a reagent in the liquid bag can reach the first liquid path. When the kit is moved into a mounting bracket of the blood gas analysis device, the first interface is communicated with the second interface. According to the blood gas analysis device provided in embodiments of the present application, the first measurement assembly and the kit are configured to be capable of being in butt joint with each other or separated, so that the first measurement assembly and the kit are detachably assembled.

## Description

The present disclosure claims priority of Chinese Patent Application No. 202210912914.X, in the title of "REAGENT KIT, DETECTION ASSEMBLY, AND BLOOD GAS ANALYSIS DEVICE", filed on July 31, 2022, the entire contents of which are hereby incorporated by reference in their entirety.

### TECHNICAL FIELD

The present disclosure relates to the field of sample analysis technologies, and in particular to a reagent kit, a detection assembly, and a blood gas analysis device.

### BACKGROUND

Blood gas analysis is to apply a blood gas analysis device to detect blood samples, so as to obtain biochemical parameters.

During the blood gas analysis, the blood gas analysis device is required to be cleaned and calibrated to avoid the mutual influence of different blood samples during the analysis process. In addition, liquid packs configured to clean and calibrate the blood gas analysis device and fluid pipes during the blood gas analysis process mostly belong to consumables and need to be replaced after being configured to a certain period of time or a certain number of times. Therefore, a reasonable structure layout of the blood gas analysis device to improve the analysis efficiency of the blood gas analysis device has become an urgent technical problem that needs to be solved.

### SUMMARY OF THE DISCLOSURE

The purpose of the present disclosure is to provide reagent kit, a detection assembly, and a blood gas analysis device to address the technical defect of the blood gas analysis device having an unreasonable structure.

A blood gas analysis device, including: a first detection assembly, including a detection element, a first liquid path, and a first port, where the first liquid path and the first port are in communication, and the detection element is configured to perform a blood gas detection on liquid in the first fluid path; and a reagent kit, including a cavity, a second fluid path, and a second port, where the cavity is configured to hold a liquid pack; an end of the second fluid path is configured to be in communication with the liquid pack, and another end of the second fluid path is configured to be in communication with the second port; where the first detection assembly and the reagent kit are capable of being docked and of being separated, and the first port and the second port are caused to be in communication or separated; in condition of the first detection assembly and the reagent kit being docked with each other, the first liquid path is in communication with the second liquid path, and reagent in the liquid pack is configured to reach the first liquid path; in condition of the reagent kit being moved into a mounting bracket of the blood gas analysis device, the first port and the second port are in communication.

A detection assembly, including: a housing, a detection element and a first liquid path that are disposed in the housing, and a first port and a connection terminal that are disposed on the housing, where the connection terminal is electrically connected to the detection element; where the first liquid path is in communication with the first port, and the detection element is configured to perform a blood gas detection on liquid in the first liquid path; the first port is capable of being docked with and of being separated from a second port of a reagent kit; in condition of the first port being docked with the second port, reagent in the reagent kit is obtainable through the first port; in condition of the first port being docked with the second port, reagent in the reagent kit is obtainable through the first port, the connection terminal is configured to be electrically connected to a processing circuit of a blood gas analysis device.

A reagent kit, including: a cavity, a second liquid path, and a second port; where the cavity is configured to hold a liquid pack; an end of the second liquid path is in communication with the liquid pack, and another end of the second liquid path is in communication with to the second port; the second port is capable of being docked with and of being separated from a first port of a first detection assembly; in condition of the first port being docked with the second port, reagent in the liquid pack is configured to be delivered to the first detection assembly through the second port; the reagent kit is capable of being moved into and of being moved out of a mounting bracket of a blood gas analysis device, and in condition of the reagent kit being moved into the mounting bracket, the first port and the second port are in communication.

A blood gas analysis device, including: a mounting bracket, including a first mounting position and a second mounting position, where the first mounting position is configured to detachably mount a first detection assembly, and the second mounting position is configured to hold a reagent kit; where the first detection assembly and the reagent kit are capable of being docked and of being separated, and the first detection assembly and the reagent kit are caused to be in communication or separated; in condition of the first detection assembly and the reagent kit being docked with each other, reagent in the reagent kit is capable of reaching the first detection assembly.

The blood gas analysis device provided in the embodiments of the present disclosure can achieve a detachable assembly of the first detection assembly and the reagent kit by enabling the first detection assembly and the reagent kit to be docked as well as separated. In particular, when the first detection assembly and the reagent kit are docked, the first liquid path of the first detection assembly can be in communication with the second liquid path of the reagent kit, such that the reagent of the reagent kit can reach the first liquid path for blood gas detection. In addition, the mounting bracket is provided for assembling the reagent kit and the first detection assembly, and when the assembly is complete, the first detection assembly and the reagent kit can be docked as well as separated. When the first detection assembly and the reagent kit are separated, the reagent kit can be removed separately for replacement, which increases the detection efficiency.

A regulating device, applied to a liquid sample detection apparatus and including: a first adjusting component and a second adjusting component; where the first adjusting component is configured to adjust a rotation angle of a sampling element relative to the liquid sample detection apparatus, so as to switch between a first fluid path and a second fluid path for conveying liquid; the second adjusting component is configured to adjust an axial position of a sleeve sleeved on the sampling element, so as to switch between a first position and a second position of the sleeve; where in condition of the sleeve being in the first position, the sampling element is capable of achieving a connection of the first fluid path; in condition of the sleeve being in the second position, the first adjusting component is capable of adjusting the rotation angle of the sampling element, so as to achieve a connection of the second fluid path.

A liquid sample detection apparatus, including: a body, including a first mounting position; and a regulating device, arranged on the first mounting position; where the regulating device includes: a first adjusting component and a second adjusting component; where the first adjusting component is configured to adjust a rotation angle of a sampling element relative to the liquid sample detection apparatus, so as to switch between a first fluid path and a second fluid path for conveying liquid; the second adjusting component is configured to adjust an axial position of a sleeve sleeved on the sampling element, so as to switch between a first position and a second position of the sleeve; where in condition of the sleeve being in the first position, the sampling element is capable of achieving a connection of the first fluid path; in condition of the sleeve being in the second position, the first adjusting component is capable of adjusting the rotation angle of the sampling element, so as to achieve a connection of the second fluid path.

The regulating device provided in the embodiments of the present disclosure can adjust the rotation angle of the sampling element by means of the first adjusting component and the axial position of the sleeve sleeved on the sampling element by means of the second adjusting component, such that the sampling element can be switched between the first fluid path and the second fluid path, and the need to switch between different sampling fluid paths may be easily and reliably met.

A biological tissue detection frame, including: a plurality of plates, that are interconnected to form a cavity and a first mounting position, where the first mounting position is configured to mount a biological tissue measurement platform, and the cavity is configured to contain an auxiliary consumable; where an avoidance passage is arranged between the cavity and the first mounting position, the avoidance passage being configured to avoid mechanical docking operations and consumable delivery between the biological tissue measurement platform and the auxiliary consumable; the consumable delivery is a passage connecting a first pipe of the biological tissue measurement platform and a second pipe of the auxiliary consumable.

A biological tissue detection device, including: a biological tissue detection frame, including a cavity and a first mounting position; a biological tissue measurement platform, detachably arranged on the first mounting position and including a first pipe; and an auxiliary consumable, capable of being moved into and of being moved out of the cavity, and including a second pipe; where an avoidance passage is arranged between the cavity and the first mounting position, the avoidance passage being configured to avoid mechanical docking operations and consumable delivery between the biological tissue measurement platform and the auxiliary consumable; the consumable delivery is a passage connecting the first pipe and the second pipe.

The biological tissue detection frame provided in the embodiments of the present disclosure has a simple structure, as an avoidance passage is provided between the cavity and the first mounting position of the biological tissue detection frame, such that mechanical docking operations and consumable delivery between the auxiliary consumable in the cavity and the biological tissue measurement platform on the first mounting position can be achieved.

A sample analysis apparatus, including: a fixing seat and a mounting seat, where a guide rod is arranged between the fixing seat and the mounting seat; a conveying assembly, disposed between the fixing seat and the mounting seat, where the conveying assembly is sleeved on the guide rod and is slidable along the guide rod; and a testing assembly, disposed between fixing seat and mounting seat, where the testing assembly is sleeved on the guide rod and is slidable along the guide rod; where the testing assembly is arranged on a side of the conveying assembly away from the fixing seat, and a first elastic member is arranged between the conveying assembly and the fixing seat; the conveying assembly and the fixing seat are configured to be separated by the first elastic member; a second elastic member is arranged between the conveying assembly and the testing assembly, and the conveying assembly and the testing assembly are configured to be separated by the second elastic member.

The sample analysis apparatus provided in the embodiments of the present disclosure is arranged with a first elastic member such that the conveying assembly and the fixing seat can be separated by the first elastic member, and a second elastic member such that the conveying assembly and the testing assembly can be separated by the second elastic member. That is, when the sample analysis apparatus completes an analysis operation, no additional drive mechanism is required, and the separation of the conveying assembly and the fixing seat and the separation of the conveying assembly and the testing assembly can be achieved by the first elastic member and the second elastic member, such that the overall structure is simple.

A biological detection device, including: a first base and a second base; a guide member, disposed between the first base and the second base and extending in an arrangement direction of the first base and the second base; a testing assembly, arranged on the guide member and movable along the guide member; and a transmission assembly, arranged on the first base and including a drive member; where the testing assembly is arranged with a supporting member on a side close to the first base, and the drive member is in rolling fit with the supporting member to drive the testing assembly to move along the guide member.

The biological detection device provided in the embodiments of the present disclosure can ensure the stability of the movement of the testing assembly and move the testing assembly to a predetermined position for corresponding testing, by providing the supporting member and the drive member that are in rolling fit on the testing assembly to drive the testing assembly to move along the guide rod.

A blood sample analysis platform, including: a blood sample analyzer, including a non-contact detection element and an avoidance slot; and an auxiliary liquid box, including a cavity and a liquid path, where the cavity is configured to hold an auxiliary liquid and a waste liquid pack; an end of the liquid path is configured to introduce the auxiliary liquid, and another end of the liquid path is configured to communicate with the waste liquid pack; where the liquid path has a portion that is exposed from the auxiliary liquid box, and the non-contact detection element is configured to detect a portion of the liquid path entering the avoidance slot.

A blood sample analyzer, including: a testing seat and a non-contact detection element, where the testing seat defines an avoidance slot, and the non-contact detection element is connected to the testing seat for detecting liquid entering the avoidance slot; where the testing seat is capable of being docked with and of being separated from an auxiliary liquid box; a liquid path of the auxiliary liquid box includes a bent part that protrudes from the auxiliary liquid box; in condition of the testing seat being docked with the liquid box, the bent part enters the avoidance slot to be detected by the non-contact detection element.

An auxiliary liquid box, including: a cavity and a liquid path; where the cavity is configured to hold an auxiliary liquid and a waste liquid pack; an end of the liquid path is configured to introduce the auxiliary liquid, and another end of the liquid path is configured to communicate with the waste liquid pack; the liquid path has a portion that is exposed from the auxiliary liquid box, and in condition of the auxiliary liquid box being docked with a blood sample analyzer, the portion of the liquid path enters an avoidance slot of the blood sample analyzer to be detected by a non-contact detection element of the blood sample analyzer.

In the blood sample analysis platform provided in the embodiments of the present disclosure, a part of the liquid path of the auxiliary liquid box is exposes to the auxiliary liquid box, and the non-contact detection element of the blood sample analyzer can detect the part when the part enters the avoidance slot of the blood sample analyzer, thereby realizing non-contact detection of the liquid in the liquid path.

A fluid detection instrument, including: a valve assembly, including a control element, a first pipe, a plurality of inlets, and an outlet, where the control element is configured to control one of the plurality of inlets to be in communication with one end of the first pipe, and the other end of the first pipe is in communication with the outlet; and a fluid kit, including: a cavity for accommodating a fluid pack, and a plurality of ports, where an end of each port is configured to be in communication with the fluid pack, and the other end of the port is configured to be in communication with a corresponding inlet; where the valve assembly is capable of being docked with and of being separated from the fluid kit, and the inlet and the port are configured to be docked or separated; in condition of the inlet and the port being docked, the first pipe is capable of being in communication with the port under control of the control element, and fluid in the fluid pack is configured to reach the first pipe.

A valve assembly, including: a control element, a first pipe, a plurality of inlets, and an outlet; where the control element is configured to control one of the plurality of inlets to be in communication with one end of the first pipe, and the other end of the first pipe is in communication with the outlet; each inlet is capable of being docked with and of being separated from a port of a fluid kit, to obtain fluid of the fluid kit by controlling the inlet through the control element in condition of the inlet being docked with the port.

A fluid kit, including: a cavity for accommodating a fluid pack, and a plurality of ports; where an end of each port is configured to be in communication with the fluid pack, and the other end of the port is configured to be in communication with a corresponding inlet of a valve assembly; the valve assembly is capable of being docked with and of being separated from the fluid kit, and in condition of the valve assembly and the fluid kit being docked, fluid in the fluid kit is configured to be delivered to the valve assembly through the port.

The fluid detection apparatus provided in the embodiments of the present disclosure can realize the detachable assembly of the fluid kit and valve assembly, by providing the valve assembly and fluid kit that can be docked or separated. In particular, when they are docked, the control element of the valve assembly can control the first pipe of the valve assembly to be in communication with the port of the fluid kit, such that the liquid in the fluid kit can reach the first pipe to complete the corresponding detection. In addition, the control element can control one of multiple inlets to be in communication with the first pipe, such that the liquid flowing into the first pipe can be conveniently selected.

An integrated reagent kit, including: a case, defining a storage space, where the storage space is configured to store a reagent pack and a recovery pack; a sampling apparatus, arranged on the case and disposed outside the storage space; and a first pipe and a second pipe, that are arranged on the case, where an input end of the sampling apparatus is capable of being in communication with the reagent pack through the first pipe, and an output end of the sampling apparatus is in communication with the recovery pack through the second pipe; the sampling apparatus is rotatable relative to the case, and the input end of the sampling apparatus is configured to collect liquid from the reagent pack or an external container.

The embodiments of the present disclosure provide an integrated reagent kit, which is arranged with a sampling apparatus, a first pipe and a second pipe arranged on the case, such that the sampling apparatus can collect the liquid in the reagent pack through the first pipe, and the liquid in the sampling apparatus can reach the recovery pack through the second pipe. In addition, the sampling apparatus can be rotated relative to the case, such that the sampling apparatus can collect liquid from an external container or reagent pack, thereby enhancing the versatility of the sampling apparatus for collecting liquid and the flexibility of the reagent kit.

A reagent storage apparatus, including: a storage box, and a sampling assembly and a docking assembly that are disposed on the storage box and outside the storage box; where an outlet end of the sampling assembly is in communication with a first position in the storage box, the docking assembly defines a sampling groove, and the sampling groove is in communication with a second position in the storage box; the sampling assembly is capable of being connected with and of being separated from the docking assembly; in condition of the sampling assembly and the docking assembly being connected, an inlet end of the sampling assembly is inserted into the sampling groove to sample from the second position in storage box; in condition of the sampling assembly and the docking assembly being separated, the inlet end of sampling assembly is capable of sampling from outside the storage box.

In the reagent storage apparatus provided in the embodiments of the present disclosure, the docking assembly can cooperate with the sampling assembly arranged on the storage box, such that the sampling assembly can sample from the inside or outside of the storage box. Specifically, when the sampling assembly and the docking assembly are docked, the inlet end of the sampling assembly is inserted into the sampling groove of the docking assembly to sample from the inside of the storage box; when the sampling assembly and the docking assembly are separated, the inlet end of the sampling assembly can sample from the outside of the storage box. That is, the sampling position of the sampling assembly can be flexibly rotated by the above structural arrangement of the embodiments of the present disclosure, and the amount and type of liquid in the storage box can be reasonably allocated.

A liquid parameter measurement platform, including: a liquid loading box, including a liquid taking assembly and an adjusting assembly, where the adjusting assembly is configured to be driven under an external force to drive the liquid taking assembly to rotate relative to the liquid loading box; and a rotatable assembly and a movable assembly, where the rotatable assembly is rotatable to drive the movable assembly to rotate synchronously, and the movable assembly is movable relative to the rotatable assembly; where the adjusting assembly is capable of being docked with and of being separated from the rotatable assembly; in condition of the adjusting assembly and the rotatable assembly being docked, the rotatable assembly is capable of driving the adjusting assembly to rotate relative to the liquid loading box, thereby driving the liquid taking assembly to rotate relative to the liquid loading box; the liquid taking assembly and the movable assembly are configured to follow docking or separation of the adjusting assembly and the rotatable assembly to achieve corresponding docking or separation; in condition of the liquid taking assembly and the movable assembly being docked, the movable assembly is configured to adjust an axial position of at least part of the liquid taking assembly, and the rotatable assembly is configured to drive the liquid taking assembly to switch between a first attitude and a second attitude; where the first attitude corresponds to that the liquid taking assembly is limited to collect liquid from inside the liquid loading box, and the second attitude corresponds to that the liquid taking assembly is limited to collect liquid from outside the liquid loading box.

A liquid loading box, applied to a liquid parameter measurement platform and including: a liquid taking assembly and an adjusting assembly, where the adjusting assembly is configured to, under an external force, drive the liquid taking assembly to rotate relative to the liquid loading box; where the adjusting assembly is capable of being docked with and of being separated from a rotatable assembly, and in condition of the adjusting assembly being docked with the rotatable assembly, the rotatable assembly is capable of driving the adjusting assembly to rotate relative to the liquid loading box, thereby driving the liquid taking assembly to rotate relative to the liquid loading box; the liquid taking assembly is capable of being docked with and of being separated from a movable assembly, and the liquid taking assembly and the movable assembly are configured to follow docking or separation of the adjusting assembly and the rotatable assembly to achieve corresponding docking or separation; in condition of the liquid taking assembly and the movable assembly being docked, the movable assembly is configured to adjust an axial position of at least part of the liquid taking assembly, and the rotatable assembly is configured to drive the liquid taking assembly to switch between a first attitude and a second attitude; where the first attitude corresponds to that the liquid taking assembly is limited to collect liquid from inside the liquid loading box, and the second attitude corresponds to that the liquid taking assembly is limited to collect liquid from outside the liquid loading box.

A motion apparatus, applied to a liquid parameter measurement platform and including: a rotatable assembly and a movable assembly, where the rotatable assembly is rotatable to drive the movable assembly to rotate synchronously, and the movable assembly is movable relative to the rotatable assembly; where the rotatable assembly is capable of being docked with and of being separated from an adjusting assembly of a liquid loading box; in condition of the adjusting assembly and the rotatable assembly being docked, the rotatable assembly is capable of driving the adjusting assembly to rotate relative to the liquid loading box, thereby driving a liquid taking assembly of the liquid loading box to rotate relative to the liquid loading box; where the movable assembly is capable of being docked with and of being separated from the liquid taking assembly; the liquid taking assembly and the movable assembly are configured to follow docking or separation of the adjusting assembly and the rotatable assembly to achieve corresponding docking or separation; in condition of the liquid taking assembly and the movable assembly being docked, the movable assembly is configured to adjust an axial position of at least part of the liquid taking assembly, and the rotatable assembly is configured to drive the liquid taking assembly to switch between a first attitude and a second attitude; where the first attitude corresponds to that the liquid taking assembly is limited to collect liquid from inside the liquid loading box, and the second attitude corresponds to that the liquid taking assembly is limited to collect liquid from outside the liquid loading box.

In the liquid parameter measurement platform provided in the embodiments of the present disclosure, the adjusting assembly and the rotatable assembly that can be docked or separated are provided, and when they are docked, the rotatable assembly can drive the adjusting assembly to rotate relative to the liquid loading box, thereby driving the liquid taking assembly to rotate relative to the liquid loading box. The liquid taking assembly and the movable assembly are set to be docked or separated accordingly following the docking and separating of the adjusting assembly and the rotatable assembly, and when they are docked, the movable assembly can adjust the axial position of at least part of the liquid taking assembly such that the rotatable assembly can drive the liquid taking assembly to switch between a first attitude and a second attitude. The first attitude corresponds to that the liquid taking assembly is limited to collect liquid from inside the liquid loading box, and the second attitude corresponds to that the liquid taking assembly is limited to collect liquid from outside the liquid loading box. That is, through the above structural settings, the liquid collection attitude of the liquid taking assembly can be flexibly adjusted, and thus the diversity of liquid parameter measurement platform detection may be enriched.

A biological sample analysis device, including: a drive assembly, including an output shaft; and a reagent storage apparatus, including a receiving cavity, a connecting pipe, and a squeezing assembly, where the receiving cavity is configured to hold a reagent pack and a recovery pack, and the connecting pipe is partially embedded in the squeezing assembly; an end of the connecting pipe is configured to be in communication with the reagent pack, and another end of the connecting pipe is configured to be in communication with the recovery pack; where the drive assembly is capable of being docked with and of being separated from the reagent storage apparatus, causing the output shaft to be docked with or separated from the squeezing assembly; in condition of the drive assembly and the reagent storage apparatus being docked, the output shaft is capable of driving the squeezing assembly to squeeze the connecting pipe to cause liquid in the connecting pipe to flow.

A reagent storage apparatus, including: a receiving cavity, a connecting pipe, and a squeezing assembly, where the receiving cavity is configured to hold a reagent pack and a recovery pack, and the connecting pipe is partially embedded in the squeezing assembly; an end of the connecting pipe is configured to be in communication with the reagent pack, and another end of the connecting pipe is configured to be in communication with the recovery pack; where the squeezing assembly is capable of being docked with and of being separated from an output shaft of a drive assembly; in condition of the squeezing assembly and the output shaft being docked, the output shaft of the drive assembly is configured to drive the squeezing assembly to squeeze the connecting pipe to cause liquid in the connecting pipe to flow.

The biological sample analysis device provided in the embodiments of the present disclosure can realize a detachable assembling of the drive assembly and reagent storage apparatus, by providing the drive assembly and reagent storage apparatus that can be docked or separated. When they are docked, the output shaft of the drive assembly can drive the squeezing assembly on the reagent storage apparatus to squeeze the connecting pipe to allow the liquid in the connecting pipe to flow. Therefore, there is no need to additionally provide a drive apparatus in the reagent storage apparatus to drive the flow of liquid, and the drive assembly and reagent storage apparatus may thus be detachably assembled for ease of replacement.

A liquid drive apparatus, including: a power assembly, a rotatable assembly, and a liquid pipe; where one of the power assembly and the rotatable assembly includes a rotating shaft, and the other of the power assembly and the rotatable assembly includes a mating portion into which the rotating shaft is configured to be inserted; one of the rotating shaft and the mating portion is arranged with a snap fastener, and the other of the rotating shaft and the mating portion defines a snap groove; the liquid pipe is arranged in the rotatable assembly, and the rotatable assembly is rotatable to squeeze the liquid pipe; where the power assembly is capable of being docked with and of being separated from the rotatable assembly, and the rotating shaft is configured to be inserted into the mating portion or pulled out of the mating portion; in condition of the rotating shaft being inserted into the mating portion, the rotating shaft is rotatable to engage the snap fastener and the snap groove to achieve a locking limit.

A reagent kit, including: a cavity, a liquid pipe, and a rotatable assembly; where the cavity is configured to hold a reagent pack and a recovery pack, the liquid pipe is arranged in the rotatable assembly, and the rotatable assembly is rotatable to squeeze the liquid pipe, causing liquid in the reagent pack to pass through the liquid pipe and reach the recovery pack; the rotatable assembly is configured to capable of being docked with and of being separated from a power assembly; one of the power assembly and the rotatable assembly includes a rotating shaft, and the other of the power assembly and the rotatable assembly includes a mating portion into which the rotating shaft is configured to be inserted; one of the rotating shaft and the mating portion is arranged with a snap fastener, and the other of the rotating shaft and the mating portion defines a snap groove; in condition of the rotatable assembly being docked with the power assembly, the rotating shaft is inserted into the mating portion, and the rotating shaft is rotatable to engage the snap fastener and the snap groove to achieve a locking limit.

A sample analysis apparatus, including: a mounting bracket, including a first mounting position and a second mounting position, where the first mounting position is configured to mount a reagent kit, and the second mounting position is configured to mount a power assembly; where the power assembly is capable of being docked with and of being separated from a rotatable assembly of the reagent kit; one of the power assembly and the rotatable assembly includes a rotating shaft, and the other of the power assembly and the rotatable assembly includes a mating portion into which the rotating shaft is configured to be inserted; one of the rotating shaft and the mating portion is arranged with a snap fastener, and the other of the rotating shaft and the mating portion defines a snap groove; in condition of the rotatable assembly being docked with the power assembly, the rotating shaft is inserted into the mating portion, and the rotating shaft is rotatable to engage the snap fastener and the snap groove to achieve a locking limit.

The liquid drive apparatus provided in the embodiments of the present disclosure is arranged with a snap fastener on one of the rotating shaft and the mating portion, and a snap groove on the other. The snap fastener and the snap groove can be engaged to achieve a locking limit by rotating the rotating shaft. In this way, there is no need to align the snap fastener and the snap groove when assembling the rotating shaft and the mating portion, that is, after the rotating shaft and the mating portion are assembled, the rotating shaft can be rotated to achieve the locking limit of the snap fastener and the snap groove, thereby improving the assembly efficiency.

A medical detection device, including: a detection assembly, including a detection element, a first liquid path, and a first port, where the first liquid path is in communication with the first port, and the detection element is configured to detect liquid in the first liquid path to obtain a detection signal; a processing circuit, detachably connected to the detection element of the detection assembly and configured to receive the detection signal in condition of being connected to the detection element; and a reagent kit, including a cavity, a second liquid path, and a second port, where the cavity is configured to hold a liquid pack; an end of the second liquid path is configured to be in communication with the liquid pack, and another end of the second liquid path is in communication with the second port; where the detection assembly and the reagent kit are capable of being docked and of being separated, causing the first port and the second port to be connected or separated; in condition of the first port and the second port being connected, the first liquid path is in communication with the second liquid path, causing reagent in the liquid pack to reach the first liquid path; in condition of the detection assembly and the reagent kit being separated to replace the reagent kit, the processing circuit keeps electrically connected to the detection element of the detection assembly.

A detection assembly, including: a housing, a detection element and a first liquid path that are disposed in the housing, and a first port and a connection terminal that are disposed on the housing, where the connection terminal is electrically connected to the detection element; where the first liquid path is in communication with the first port, and the detection element is configured to perform a blood gas detection on liquid in the first liquid path to obtain a detection signal; the first port is capable of being docked with and of being separated from a second port of a reagent kit for obtaining reagent of the reagent kit through the first port in condition of the first port being docked with the second port; in condition of the detection assembly and the reagent kit being separated to replace the reagent kit, the connection terminal remains electrically connected to a processing circuit of a medical detection device.

A reagent kit, including: a cavity, a second liquid path, and a second port; where the cavity is configured to hold a liquid pack; an end of the second liquid path is configured to be in communication with the liquid pack, and another end of the second liquid path is in communication with the second port; the second port is configured to be docked with a first port of a detection assembly in condition of a detection being performed using the reagent kit, and reagent in the liquid pack is configured to be delivered to the detection assembly through the second port; the second port is separated from the detection assembly before the detection is performed using the reagent kit; the reagent kit is capable of being moved into and of being moved out of a mounting bracket of a medical detection device, and in condition of the reagent kit being moved in, the first port and the second port are in communication.

A medical detection device, including: a mounting bracket, including a first mounting position, a second mounting position, and a third mounting position, where the first mounting position is configured to mount a detection assembly, and the second mounting position is configured to mount a reagent kit; and a processing circuit, arranged on the third mounting position and detachably connected to the detection assembly; where the detection assembly and the reagent kit are capable of being docked and of being separated, and the detection assembly and the reagent kit are caused to be in communication or separated; in condition of the detection assembly and the reagent kit being in communication, reagent in the reagent kit is capable of reaching the detection assembly; in condition of the detection assembly and the reagent kit being separated to replace the reagent kit, the processing circuit remains electrically connected to the detection assembly.

The medical testing device provided in the embodiments of the present disclosure allows the testing assembly and the reagent kit to be docked or separated, such that when the first port and the second port are in communication, the first liquid path is in communication with the second liquid path, allowing the reagent in the liquid pack to reach the first liquid path for detection. Furthermore, when the detection assembly and the reagent kit are separated to replace the reagent kit, the processing circuit remains electrically connected to the detection element of the detection assembly, thereby preventing the power failure between the processing circuit and the detection element from affecting the continuity of subsequent detection and the accuracy of the detection data.

A medical detection device, including: a detection assembly, including a detection element, a first liquid path, and a first port, where the first liquid path is in communication with the first port, and the detection element is configured to detect liquid in the first liquid path to obtain a detection signal; a reagent kit, including a cavity, a second liquid path, and a second port, where the cavity is configured to hold a liquid pack; an end of the second liquid path is configured to be in communication with the liquid pack, and another end of the second liquid path is in communication with the second port; and a processing circuit, detachably connected to the detection element of the detection assembly for receiving the detection signal in condition of being connected to the detection element, where in condition of the processing circuit and the detection element being disassembled, the detection assembly is capable of being disassembled synchronously with the reagent kit; where the detection assembly is detachably arranged on the reagent kit, and the first port and the second port are caused to be in communication or separated; in condition of the first port and the second port being in communication, the first liquid path is in communication with the second liquid path, and reagent in the liquid pack is capable of reaching the first liquid path.

A detection assembly, including: a housing, a detection element and a first liquid path that are disposed in the housing, and a first port and a connection terminal that are disposed on the housing, where the connection terminal is electrically connected to the detection element; where the first liquid path is in communication with the first port, and the detection element is configured to perform a blood gas detection on liquid in the first liquid path to obtain a detection signal; the first port is capable of being docked with and of being separated from a second port of a reagent kit for obtaining reagent of the reagent kit through the first port in condition of the first port being docked with the second port; the detection assembly is configured to be arranged on the reagent kit, and the connection terminal is configured to be electrically connected to a processing circuit of a medical detection device.

A reagent kit, including: a cavity, a second liquid path, and a second port; where the cavity is configured to hold a liquid pack; an end of the second liquid path is configured to be in communication with the liquid pack, and another end of the second liquid path is in communication with the second port; the second port is configured to be docked with a first port of a detection assembly, and reagent in the liquid pack is capable of being delivered to the detection assembly through the second port in condition of the first port and the second port being docked; the detection assembly is detachably arranged on the reagent kit, and in condition of the reagent kit being disassembled, the detection assembly is capable of being disassembled simultaneously with the reagent kit.

A medical detection device, including: a mounting bracket, including a first mounting position for detachably mounting a reagent kit, where the reagent kit is arranged with a second mounting position for detachably mounting a detection assembly; where the detection assembly and the reagent kit are capable of being docked and of being separated, and the detection assembly and the reagent kit are caused to be in communication or separated; in condition of the detection assembly and the reagent kit being in communication, reagent in the reagent kit is capable of reaching the detection assembly; in condition of the reagent kit being disassembled, the detection assembly is capable of being disassembled synchronously with the reagent kit.

The medical detection device provided in the embodiments of the present disclosure is arranged with a detection assembly detachably arranged on the reagent kit, such that when the first port and the second port are connected, the reagent in the liquid pack of the reagent kit can reach the first liquid path of the detection assembly for detection to obtain a detection signal. The processing circuit is further detachably connected to the detection element of the detection assembly for receiving the detection signal when the detection element is connected. In addition, when the processing circuit and the detection element are disassembled, the detection assembly can be disassembled simultaneously with the reagent kit to avoid disassembling the detection assembly and the reagent kit separately, thereby improving the ease of disassembly.

A biological tissue detection apparatus, including: a bracket, including a storage space and a first mounting position, where the first mounting position is configured to mount a sample measuring device, and a reagent kit is capable of being moved into and of being moved out of the storage space; the reagent kit is configured to convey liquid to the sample measuring device to assist the sample measuring device in sample measurement, and the sample measuring device is configured to measure the liquid flowing into the sample measuring device; where the dimension of the receiving space in a moving direction of the reagent kit is larger than an intended placement dimension of the reagent kit, and the bracket is arranged with a limit portion and an elastic portion; the limit portion is configured to position the reagent kit, and the elastic member is configured to provide an elastic force in condition of the reagent kit being moved into the storage space; and the reagent kit is capable of retracting part of a moving travel and being restrained by the limit portion after being moved into the storage space.

A biological tissue detection apparatus, including: a bracket, including a storage space and a first mounting position; a sample measuring device, detachably arranged on the first mounting position and including a first pipe; and a reagent kit, capable of being moved into and of being moved out the storage space, where the reagent kit includes a second pipe; where a dimension of the storage space in a moving direction of the reagent kit is larger than an intended placement dimension of the reagent kit; the bracket is arranged with a limit portion and an elastic member, the limit portion being configured to position the reagent kit, and the elastic member being configured to provide an elastic force for the reagent kit to move into the storage space, causing the reagent kit to retract a part of a movement travel and be restrained by the limit portion after being moved into the storage space.

In the biological tissue detection apparatus provided in the embodiments of the present disclosure, a limit portion is arranged on the bracket to position the reagent kit when it is moved into the storage space of the bracket, and an elastic member is arranged on the bracket to provide an elastic force when the reagent kit is moved into the storage space. The elastic force allows the reagent kit to retract part of the travel after it is moved into the solute space to resist against the limit member, so as to facilitate the positioning of the reagent kit in the storage space.

A sample analysis apparatus, including: a seat body, defining a storage groove and a pick-up and placement opening in communication with the storage groove, where a detection card is capable of being moved into and of being moved out of the storage groove from the pick-up and placement opening; at least one of the storage groove and detection card is arranged with a locking member, the locking member being configured to lock the detection card in the seat body in condition of the detection card being moved into the storage groove; at least one of the seat body and the detection card is arranged with an elastic member; where in condition of the detection card being moved into the storage groove, the detection card squeezes or stretches the elastic member, causing the elastic member to deform and generate an elastic force; in condition of the locking member releasing locking and fixing of the detection card, the detection card pops up from the pick-up and placement opening under an action of the elastic force.

The sample analysis apparatus provided in the embodiments of the present disclosure facilitates the placement of the detection card on the seat body by providing a storage groove on the seat body for moving the detection card in or out. Further, the detection card can be locked to the seat body by a locking member to facilitate the corresponding detection. In addition, an elastic member is provided to eject the detection card when the locking member releases the lock on the detection card, so as to facilitate replacement.

A fluid detection instrument, including: a valve assembly, including a control element, a first pipe, a first inlet, and a first outlet, where the control element is configured to control the first inlet to be in communication with an end of the first pipe, and another end of the first pipe is in communication with the first outlet; and a reagent kit, including a cavity, a second pipe, and a first port, where the cavity is configured to hold a reagent pack and a recovery pack, the first port is configured to connect the reagent pack to the first inlet, and the first outlet is in communication with a docking groove defined on the reagent kit; the docking groove is configured to be docked with a sampling element, an end of the second pipe being in communication with the docking groove, and another end of the second pipe being in communication with the recovery pack; where the valve assembly and the reagent kit are capable of being docked and of being separated, and the first inlet and the first port are caused to be in communication or separated; in condition of the first inlet and the first port being in communication, the first pipe is configured to be in communication with the docking groove under control of the control element, and liquid in the reagent pack is capable of reaching the docking groove, and liquid in the docking groove is capable of reaching the recovery pack through the second pipe.

A valve assembly, including: a control element, a first pipe, a first inlet, and a first outlet; where the control element is configured to control the first inlet to be in communication with an end of the first pipe, and another end of the first pipe is in communication with the first outlet; the first inlet is capable of being docked with and of being separated from a first port of a reagent kit; in condition of the first port being docked with the first inlet, fluid in the reagent kit is capable of being obtained by controlling the first inlet through the control element; the first outlet is configured to be in communication with a docking groove of the reagent kit; in condition of the first outlet being docked with the docking groove, the liquid in the reagent kit is capable of passing through the first outlet to reach the docking groove, and liquid in the docking groove is capable of passing through a second pipe of the reagent kit to reach a recovery pack of the reagent kit.

A reagent kit, including: a cavity, a second pipe, a first port, and a docking groove; where the cavity is configured to hold a reagent pack and a recovery pack; an end of the second pipe is in communication with the docking groove, and another end of the second pipe is in communication with the recovery pack; the first port is capable of being docked with and of being separated from a first inlet of a valve assembly, and the docking groove is configured to be in communication with a first outlet of the valve assembly; in condition of the first port being docked with the first inlet, liquid in the reagent pack is capable of being delivered to the valve assembly through the first port, and liquid in the docking groove is capable of reaching the recovery pack through the second pipe.

In the fluid detection instrument provided in the embodiments of the present disclosure, the valve assembly and the reagent kit that can be docked or separated are provided, such that the reagent kit can be replaced easily. In addition, when the valve assembly is docked with the reagent kit, the first pipe can be in communication with the docking groove through the control element, such that the liquid in the reagent kit can reach the docking groove. In addition, the second pipe of the reagent kit is in communication with the recovery pack in the reagent kit, such that the liquid in the docking groove can reach the recovery pack, thereby forming a liquid conduit, such that the liquid in the reagent pack can reach the recovery pack after reaching the docking groove, and the liquid reaching the docking groove can be configured to clean the sample inlet of the sampling element on the reagent kit, thereby maintaining the cleanliness of the sample inlet and thus extending the service life of the reagent kit.

A liquid pack, including a body and a connector, where the body is configured to contain liquid, and the connector is configured to control the liquid to flow from the liquid pack through the connector or to seal the body; where the connector includes a pipe in communication with an internal space of the body, and a seal disposed in the pipe; the seal is movable in the pipe under an external force to open the pipe, and return to an initial position to seal the pipe in a case where the external force is removed.

The liquid pack provided in the embodiments of the present disclosure includes a pipe connected to the internal space of the liquid pack body and a seal arranged in the pipe, such that the seal can be moved in the pipe to open or seal the pipe, without the need to provide an extra structure on the liquid pack to open or seal the pipe, thereby improving the convenience of using the liquid pack.

An integrated reagent kit, including: a case, and a sampling assembly disposed on the case; where the sampling assembly includes a rotary member rotatably connected to the case and a sampling member connected to the rotary member; the rotary member is capable of changing to a state under an action of a first external force to drive the sampling member to rotate synchronously relative to the case, and of returning to an original state in a case where the first external force is removed; where the sampling member includes a sampling needle and a sleeve sleeved on the sampling needle; the sleeve is capable of changing to a state under an action of a second external force to move relative to the rotary member to expose the sampling needle, and of returning to an original state to cover the sampling needle in a case where the second external force is removed.

The embodiments of the present disclosure provide an integrated reagent kit, in which the sampling member and the rotary member are arranged on the case, and the rotary member can be driven to rotate the sampling member relative to the case under the action of a first external force to adjust the sampling angle of the sampling member, and the sampling member can restore its state when the first external force is removed. **In** addition, the sleeve sleeved on the sampling needle can move relative to the rotary member under the action of a second external force to expose the sampling needle, such that the sampling needle can sample from a liquid container, and the sleeve can cover the sampling needle when the second external force is removed. Therefore, by providing the sampling member and the rotary member with the above structure on the case, the sampling state of the sampling needle may be flexibly adjusted.

A reagent kit that is easy to clean, including: a case, a first pipe, a second pipe, and a docking groove, where a reagent pack and a recovery pack are arranged in the case; an end of the first pipe is in communication with the docking groove, and another end of the first pipe is in communication with the reagent pack; an end of the second pipe is in communication with the docking groove, and another end of the second pipe is in communication with the recovery pack; where the docking groove is capable of being docked with and of being separated from a sampling element; in condition of the docking groove being docked with the sampling element, liquid in the reagent pack is capable of reaching the docking groove through the first pipe for cleaning a sample inlet of the sampling element, and liquid after the cleaning is capable of reaching the recovery pack through the second pipe.

The reagent kit provided in the embodiments of the present disclosure is arranged with a first pipe and a second pipe that are in communication with the docking groove respectively, such that the liquid in the reagent pack can reach the docking groove and clean the sample inlet of the sampling element, and the cleaned liquid can reach the recovery pack. In this way, the cleanliness of the sample inlet of the sampling element of the reagent kit can be maintained during use.

A sample analysis device, including: a reagent kit, including a sampling assembly, a regulating assembly, and a movable assembly, where the regulating assembly is configured to drive the sampling assembly to rotate relative to the reagent kit; the movable assembly is arranged facing the sampling assembly; the movable assembly is capable of being docked with and of being separated from the sampling assembly; where the movable assembly is movable between a first position and a second position relative to the sampling assembly; the movable assembly is docked with the sampling assembly in the first position, and the movable assembly is separated from the sampling assembly in the second position; the first position corresponds to that the sampling assembly is subjected to positional restriction such that the sampling assembly is capable of collecting liquid inside the reagent kit; the second position corresponds to that positional restriction on the sampling assembly is cancelled such that the regulating assembly is capable of driving the sampling assembly to rotate, and the sampling assembly is capable of collecting liquid outside the reagent kit.

The sample analysis device provided in the embodiments of the present disclosure can achieve different sampling states of the sampling assembly by arranging the movable assembly to be able to move between a first position and a second position relative to the sampling assembly. In particular, the sampling assembly can be limited to collect liquid inside the reagent kit when the movable assembly is in the first position, and the limitation of the sampling assembly can be released when the movable assembly is in the second position to enable the regulating assembly to drive the sampling assembly to rotate, such that the sampling assembly can collect liquid outside the reagent kit. That is, the sampling assembly can be switched between different sampling states through the cooperation of the movable assembly and the when the movable assembly is, which may meet the needs of the sample analysis device for changing sampling positions.

A biological sample analysis device, including: a drive assembly, including an output shaft; and a reagent storage apparatus, including a receiving cavity, a first connecting pipe, a second connecting pipe, and a squeezing assembly, where the receiving cavity is configured to hold a reagent pack, a cleaning pack, and a recovery pack; the first connecting pipe and the second connecting pipe are partially embedded in the squeezing assembly; an end of the first connecting pipe is configured to be in communication with the reagent pack, and another end of the first connecting pipe is configured to be in communication with the recovery pack; an end of the second connecting pipe is configured to be in communication with the cleaning pack, and another end of the second connecting pipe is configured to be in communication with the recovery pack; where the drive assembly and the reagent storage apparatus are capable of being docked and of being separated, and the output shaft is caused to be docked with or separated from the squeezing assembly; in condition of the output shaft being docked with the squeezing assembly, the output shaft is capable of driving the squeezing assembly to synchronously squeeze the first connecting pipe and the second connecting pipe, and liquid in the first connecting pipe or the second connecting pipe is capable of flowing.

A reagent storage apparatus, including: a receiving cavity, a first connecting pipe, a second connecting pipe, and a squeezing assembly; where the receiving cavity is configured to hold a reagent pack, a cleaning pack, and a recovery pack; the first connecting pipe and the second connecting pipe are partially embedded in the squeezing assembly; an end of the first connecting pipe is configured to be in communication with the reagent pack, and another end of the first connecting pipe is configured to be in communication with the recovery pack; an end of the second connecting pipe is configured to be in communication with the cleaning pack, and another end of the second connecting pipe is configured to be in communication with the recovery pack; the squeezing assembly is capable of being docked with and of being separated from the output shaft of the drive assembly; in condition of the squeezing assembly being docked with the output shaft, the squeezing assembly is driven by the output shaft to synchronously squeeze the first connecting pipe and the second connecting pipe, and liquid in the first connecting pipe or the second connecting pipe is capable of flowing.

In the biological sample analysis device provided in the embodiments of the present disclosure, the first connecting pipe and the second connecting pipe are partially embedded in the squeezing assembly, such that the squeezing assembly can synchronously squeeze the first connecting pipe and the second connecting pipe to allow the liquid in the first connecting pipe or the second connecting pipe to flow, thereby saving the space occupied by the squeezing assembly of the reagent storage apparatus for each connecting pipe. Further, the drive assembly can be docked with or separated from the reagent storage apparatus, that is, the output shaft of the drive assembly can be docked with or separated from the squeezing assembly, such that when they are docked, the squeezing assembly can be driven to perform the above operations. In this way, the reagent storage apparatus can be separated from the drive assembly to facilitate replacement of the reagent storage apparatus.

A biological parameter analysis device, including: a housing, arranged with a window and a door capable of covering the window; a reagent kit, capable of being moved into and of being moved out of the housing, where the reagent kit includes a sampling assembly and a docking assembly; in condition of the reagent kit being arranged within the housing, the sampling assembly is capable of being unscrewed from the window to collect liquid outside the housing; the docking assembly is capable of being docked with the sampling assembly to enable the sampling assembly to collect liquid inside the reagent kit; and a linkage assembly, coupled to the docking assembly and the door, where the door covers the window in condition of the sampling assembly being docked with the docking assembly, and the door opens the window in condition of the sampling assembly being separated from the docking assembly.

In the biological parameter analysis device provided in the embodiments of the present disclosure, by providing a linkage assembly that can couple with the door on the shell and the docking assembly on the reagent kit, the door can be driven to close the window when the sampling assembly on the reagent kit docks with the docking assembly when collecting the liquid inside the reagent kit, and the door can be driven to close the window when the sampling assembly separates with the docking assembly, in which case the sampling assembly can be unscrewed out of the window to collect liquid outside the reagent kit. By providing the above linkage assembly to link the movement relationship between the docking assembly and the door, an extra drive mechanism that drives the door to open and close may be cancelled, which is beneficial to simplifying the overall structure of the device.

A sample parameter analysis apparatus, including: a housing, arranged with a window and a door that is capable of covering the window, where the housing includes a first side and a second side arranged opposite each other, and the door is arranged on the first side and is rotatably connected to the housing; and a connecting assembly, arranged on the second side of the housing to connect the housing and the door in condition of the door covering the window, where the connecting assembly includes a clamping member, a drive member, and a first elastic member; an end of the first elastic 6402c is connected to a middle portion of the clamping member, and another end of the first elastic member is connected to the housing; an end of the clamping member is connected to the drive member, and another end of the clamping member is configured to limit a position of the door in condition of the door covering the window; where the drive member is configured to drive the clamping member to release a restriction on the door, and the door is rotatable relative to the housing to open the window; the first elastic member deforms to generate an elastic force in condition of the clamping member moving, and the elastic force is configured to act on the clamping member to cause the clamping member to return to an original position in condition of the drive member cancels a drive on the clamping member.

The sample parameter analysis apparatus provided in the embodiments of the present disclosure is arranged with a connecting assembly to fix the door when the door covers the window, and the connecting assembly is capable of releasing the restriction on the door such that the door can move to open the window. In particular, a clamping member is arranged to restrict and fix the door when the door is closed, and a drive member is arranged to drive the clamping member to move so as to release the restriction on the door. Further, a first elastic member is arranged to connect the clamping member and the housing, such that when the clamping member releases the restriction on the door, the first elastic member deforms to generate an elastic force. The elastic force can cause the clamping member to return to its original position without the need to drive the clamping member to return to its original position again through the drive member, which is beneficial to simplifying the overall structure of the device.

A sample analysis box, including: a seat body, including a first cavity, a second cavity, and an isolation member, where the isolation member is configured to cause the first cavity and the second cavity to communicate or isolate with each other, and in condition of the first cavity and the second cavity being in communication, liquid in the first cavity is capable of flowing to the second cavity; where the isolation member is capable of changing to a state under an action of an external force to enable the first cavity and the second cavity to be in communication, and in a case where the external force is removed, the isolation member returns to another state where the first cavity and the second cavity are isolated.

The sample analysis box provided in the embodiments of the present disclosure is arranged with an isolation member in the seat body that can isolate the first cavity and the second cavity, so as to isolate the first cavity and the second cavity when the sample analysis box is not in use. When the sample analysis box is in use, the state of the isolation member is changed by an external force to make the first cavity and the second cavity in communication, thereby allowing the liquid in the first cavity to flow to the second cavity to complete the analysis operation of the sample analysis box. In addition, when the sample analysis box is not in use, the first cavity and the second cavity are isolated from each other, which allows the salt bridge exposed from the second cavity to be stored dry, thereby extending the storage period of the sample analysis box.

A detection assembly, including: a first liquid path, a second liquid path, a liquid inlet, a liquid outlet, and a valve assembly; where an end of the first liquid path is configured to be in communication with the liquid inlet, and another end of the first liquid path is configured to be in communication with the liquid outlet; an end of the second liquid path is configured to be in communication with the liquid inlet, and another end of the second liquid path is configured to be in communication with the liquid outlet; the valve assembly is arranged on the liquid inlet, and/or on the liquid outlet, and/or between the liquid inlet and the liquid outlet, and is capable of being switched between a first turn-on state and a second turn-on state to control the first liquid path and the second liquid path to be selectively connected; in the first turn-on state, a first external liquid of the detection assembly is capable of flowing into the first liquid path from the liquid inlet and flowing out of the first liquid path from the liquid outlet; in the second turn-on state, a second external liquid of the detection assembly is capable of flowing into the second liquid path from the liquid inlet and flowing out of the second liquid path from the liquid outlet.

In the detection assembly provided in the embodiments of the present disclosure, the connection of either the first liquid path or the second liquid path is controlled through the valve assembly. When the second liquid path is connected, external liquid in the detection assembly, such as reference liquid, can enter the second liquid path. When the first liquid path is connected, external liquid in the detection assembly, such as a detection sample, can enter the first liquid path. That is, it is not necessary to prepare auxiliary measurement liquids in advance, such as reference liquid, etc., inside the detection assembly upon completion of assembly. Instead, the auxiliary measurement liquid, such as reference liquid, etc., can be injected into the second liquid path by controlling the connection of the second liquid path by the valve assembly when in use, which may reduce the volume of the detection assembly upon completion of assembly and avoid the problem of the auxiliary measurement liquid such as reference liquid, etc., expiring due to the detection assembly not being used for a long period of time while the auxiliary measurement liquid such as reference liquid, etc., is placed inside the detection assembly.

### BRIEF DESCRIPTION OF THE DRAWINGS

In order to more clearly illustrate the technical solution in the embodiments of the present disclosure, a brief description of the drawings needed for use in the embodiments is provided below. Obviously, the drawings described below are only some embodiments of the present disclosure, and for those skilled in the art, other drawings can be obtained without the expenditure of creative effort.
FIG. 1 is a structural schematic view of a blood gas analysis device according to some embodiments of the present disclosure.
FIG. 2 is a structural schematic view of a detection assembly according to some embodiments of the present disclosure.
FIG. 3 is a structural schematic view of a reagent kit according to some embodiments of the present disclosure.
FIG. 4 is a structural schematic view of a mounting bracket of a blood gas analysis device according to some embodiments of the present disclosure.
FIG. 5 is an exploded structural schematic view of a liquid sample detection apparatus according to some embodiments of the present disclosure.
FIG. 6 is an exploded structural schematic view of a regulating device in FIG. 5.
FIG. 7 is an exploded structural schematic view of a biological tissue detection device according to some embodiments of the present disclosure.
FIG. 8 is an exploded structural schematic view of a biological tissue detection frame in FIG. 7.
FIG. 9 is an exploded structural schematic view of a sample analysis apparatus according to some embodiments of the present disclosure.
FIG. 10 is an exploded structural schematic view of a conveying assembly in FIG. 9.
FIG. 11 is an exploded structural schematic view of a testing assembly in FIG. 9.
FIG. 12 is an exploded structural schematic view of a supporting component in FIG. 11.
FIG. 13 is an exploded structural schematic view of a biological detection device according to some embodiments of the present disclosure.
FIG. 14 is a partial structural schematic view of the biological detection device in FIG. 13.
FIG. 15 is a partial structural schematic view of the biological detection device in FIG. 13.
FIG. 16 is an exploded structural schematic view of a blood sample analysis platform according to some embodiments of the present disclosure.
FIG. 17 is a partial structural schematic view of the blood sample analysis platform in FIG. 16.
FIG. 18 is a partial structural schematic view of the blood sample analysis platform in FIG. 16.
FIG. 19 is an exploded structural schematic view of a fluid detection instrument according to some embodiments of the present disclosure.
FIG. 20 is a partial structural schematic view of the fluid detection instrument in FIG. 19.
FIG. 21 is a partial structural schematic view of the fluid detection instrument in FIG. 19.
FIG. 22 is a structural schematic view of an integrated reagent kit according to some embodiments of the present disclosure.
FIG. 23 is a partial structural schematic view of a reagent storage apparatus according to some embodiments of the present disclosure.
FIG. 24 is a cross-sectional structural schematic view of the reagent storage apparatus in FIG. 23.
FIG. 25 is an exploded structural schematic view of a liquid parameter measurement platform according to some embodiments of the present disclosure.
FIG. 26 is a partial structural schematic view of the liquid parameter measurement platform in FIG. 25.
FIG. 27 is a partially exploded structural schematic view of the liquid parameter measurement platform in FIG. 25.
FIG. 28 is a partially exploded structural schematic view of a biological sample analysis device according to some embodiments of the present disclosure.
FIG. 29 is a partially exploded structural schematic view of the biological sample analysis device in FIG. 28.
FIG. 30 is a partially exploded structural schematic view of the biological sample analysis device in FIG. 28.
FIG. 31 is a partially exploded structural schematic view of a liquid drive apparatus according to some embodiments of the present disclosure.
FIG. 32 is a partial structural schematic view of the liquid drive apparatus in FIG. 31.
FIG. 33 is a partially exploded structural schematic view of a sample analysis apparatus according to some embodiments of the present disclosure.
FIG. 34 is a schematic view of the liquid path structure of a medical detection device according to some embodiments of the present disclosure.
FIG. 35 is a structural schematic view of the medical detection device according to some embodiments of the present disclosure.
FIG. 36 is an exploded structural schematic view of the medical detection device in FIG. 35.
FIG. 37 is a schematic view of a liquid path structure of a medical detection device according to some embodiments of the present disclosure.
FIG. 38 is a structural schematic view of a medical detection device according to some embodiments of the present disclosure.
FIG. 39 is an exploded structural schematic view of the medical detection device in FIG. 38.
FIG. 40 is a structural schematic view of a biological tissue detection apparatus according to some embodiments of the present disclosure.
FIG. 41 is another structural schematic view of the biological tissue detection apparatus in FIG. 40.
FIG. 42 is a schematic view of a liquid path structure of the biological tissue detection apparatus in FIG. 40.
FIG. 43 is a structural schematic view of a sample analysis apparatus according to some embodiments of the present disclosure.
FIG. 44 is a partially exploded structural schematic view of the sample analysis apparatus in FIG. 43.
FIG. 45 is another partially exploded structural schematic view of the sample analysis apparatus in FIG. 43.
FIG. 46 is an exploded structural schematic view of a fluid detection instrument according to some embodiments of the present disclosure.
FIG. 47 is a cross-sectional structural schematic view of a valve assembly of the fluid detection instrument in FIG. 46.
FIG. 48 is a schematic view of a liquid path structure of the fluid detection instrument in FIG. 46.
FIG. 49 is a structural schematic view of a liquid pack according to some embodiments of the present disclosure.
FIG. 50 is a structural schematic view of an integrated reagent kit according to some embodiments of the present disclosure.
FIG. 51 is another structural schematic view of the integrated reagent kit in FIG. 50.
FIG. 52 is another structural schematic view of the integrated reagent kit in FIG. 50.
FIG. 53 is a structural schematic view of a reagent kit according to some embodiments of the present disclosure.
FIG. 54 is a partial cross-sectional structural schematic view of the reagent kit in FIG. 53.
FIG. 55 is a structural schematic view of a sample analysis device according to some embodiments of the present disclosure.
FIG. 56 is an exploded structural schematic view of the sample analysis device in FIG. 55.
FIG. 57 is a partially exploded structural schematic view of the sample analysis device in FIG. 55.
FIG. 58 is a structural schematic view of a biological sample analysis device according to some embodiments of the present disclosure.
FIG. 59 is an exploded structural schematic view of the biological sample analysis device in FIG. 58.
FIG. 60 is a partially exploded structural schematic view of the biological sample analysis device in FIG. 58.
FIG. 61 is a structural schematic view of a biological parameter analysis device according to some embodiments of the present disclosure.
FIG. 62 is an exploded structural schematic view of the biological parameter analysis device in FIG. 61.
FIG. 63 is a partial structural schematic view of the biological parameter analysis device in FIG. 61.
FIG. 64 is a structural schematic view of a sample parameter analysis apparatus according to some embodiments of the present disclosure.
FIG. 65 is an exploded structural schematic view of the sample parameter analysis apparatus in FIG. 64.
FIG. 66 is a partially exploded structural schematic view of the sample parameter analysis apparatus in FIG. 64.
FIG. 67 is a structural schematic view of a sample analysis box according to some embodiments of the present disclosure.
FIG. 68 is an exploded structural schematic view of the sample analysis box in FIG. 67.
FIG. 69 is a cross-sectional structural schematic view of the sample analysis box in FIG. 67.
FIG. 70 is an exploded structural schematic view of a detection assembly according to some embodiments of the present disclosure.
FIG. 71 is a partially exploded structural schematic view of the detection assembly in FIG. 70.
FIG. 72 is a schematic view of a liquid path structure of the detection assembly in FIG. 70.

### DETAILED DESCRIPTION

The following description of the technical solutions in the embodiments of the present disclosure is presented clearly and completely with reference to the accompanying drawings in the embodiments of the present disclosure. It is apparent that the embodiments described are only some embodiments of the present disclosure and not all of the embodiments. All other embodiments obtained by those skilled in the art without creative effort based on the embodiments in the present disclosure fall within the scope of the present disclosure.

References to "embodiments" mean that particular features, structures, or characteristics in combination with the embodiments may be included in at least one embodiment of the present disclosure. The occurrence of the phrase at various locations in the description does not necessarily refer to the same embodiment, nor is it mutually exclusive with other embodiments as independent or alternative embodiments. It is expressly and implicitly understood by those skilled in the art that the embodiments described herein may be combined with other embodiments.

In an aspect, the present disclosure provides a blood gas analysis device, including:
a first detection assembly, including a detection element, a first liquid path, and a first port, where the first liquid path and the first port are in communication, and the detection element is configured to perform a blood gas detection on liquid in the first fluid path; and
a reagent kit, including a cavity, a second fluid path, and a second port, where the cavity is configured to hold a liquid pack; an end of the second fluid path is configured to be in communication with the liquid pack, and another end of the second fluid path is configured to be in communication with the second port;
where the first detection assembly and the reagent kit are capable of being docked and of being separated, and the first port and the second port are caused to be in communication or separated; in condition of the first detection assembly and the reagent kit being docked with each other, the first liquid path is in communication with the second liquid path, and reagent in the liquid pack is configured to reach the first liquid path; in condition of the reagent kit being moved into a mounting bracket of the blood gas analysis device, the first port and the second port are in communication.

In some embodiments, the reagent kit is arranged with a sampling element, a sample inlet of the sampling element being configured to selectively collect the reagent from the liquid pack or liquid external to the reagent kit, and a sample outlet of the sampling element being configured to be in communication with the second port; in condition of that it is required to collect the reagent from the liquid pack, the sampling element forms a part of the second liquid path.

In some embodiments, the reagent kit includes a first connecting pipe, an end of the first connecting pipe being in communication with the liquid pack and another end of the first connecting pipe being in communication with the sample inlet of the sampling element; the first connecting pipe forms another part of the second liquid path.

In some embodiments, the first detection assembly includes a third port spaced apart from the first port, and the third port is in communication with the first liquid path; the reagent kit includes a fourth port and a second connecting pipe, and the fourth port is spaced apart from the second port; an end of the second connecting pipe is in communication with the fourth port, and another end of the second connecting pipe is in communication with the cavity; in condition of the first port and the second port being in communication, the third port and the fourth port are in communication.

In some embodiments, a docking base is arranged on an outer side of a cavity wall of the cavity, the second port and the fourth port are arranged on the docking base, and the second connecting pipe is partially arranged between the cavity wall of the cavity and the docking base.

In some embodiments, the liquid pack includes a reagent pack and a recovery pack that are disposed in the cavity; an end of the first connecting pipe is configured to be in communication with the reagent pack, and an end of the second connecting pipe is configured to be in communication with the recovery pack.

In some embodiments, the reagent kit is arranged with a liquid channel exposed from the cavity, and the liquid channel forms another part of the second liquid path; liquid flowing out of the first liquid path is configured to pass through the liquid channel and reach the cavity;
the blood gas analysis device further includes a second detection assembly arranged on the mounting bracket, the second detection assembly being configured to detect liquid in the liquid channel exposed from the cavity, where the first detection assembly is configured to perform the blood gas detection, and the second detection assembly is configured to perform a blood oxygen detection.

In another aspect, the present disclosure provides a detection assembly, including:
a housing, a detection element and a first liquid path that are disposed in the housing, and a first port and a connection terminal that are disposed on the housing, where the connection terminal is electrically connected to the detection element;
where the first liquid path is in communication with the first port, and the detection element is configured to perform a blood gas detection on liquid in the first liquid path; the first port is capable of being docked with and of being separated from a second port of a reagent kit; in condition of the first port being docked with the second port, reagent in the reagent kit is obtainable through the first port; in condition of the first port being docked with the second port, reagent in the reagent kit is obtainable through the first port, the connection terminal is configured to be electrically connected to a processing circuit of a blood gas analysis device.

In some embodiments, the housing is arranged with a positioning member for positioning a bracket, of the blood gas analysis device, for mounting the detection assembly.

In some embodiments, an end of the first port that is docked with the second port includes a tapered recess or tapered protrusion to guide the second port to dock with the first port.

In another aspect, the present disclosure provides a reagent kit, including:
a cavity, a second liquid path, and a second port;
where the cavity is configured to hold a liquid pack; an end of the second liquid path is in communication with the liquid pack, and another end of the second liquid path is in communication with to the second port; the second port is capable of being docked with and of being separated from a first port of a first detection assembly; in condition of the first port being docked with the second port, reagent in the liquid pack is configured to be delivered to the first detection assembly through the second port; the reagent kit is capable of being moved into and of being moved out of a mounting bracket of a blood gas analysis device, and in condition of the reagent kit being moved into the mounting bracket, the first port and the second port are in communication.

In some embodiments, the reagent kit is arranged with a sampling element, a sample inlet of the sampling element being configured to selectively collect the reagent from the liquid pack or liquid external to the reagent kit, and a sample outlet of the sampling element being configured to be in communication with the second port; in condition of that it is required to collect the reagent from the liquid pack, the sampling element forms a part of the second liquid path.

In some embodiments, the reagent kit is arranged with a liquid channel exposed from the cavity, and the liquid channel forms another part of the second liquid path; liquid flowing out of the first detection assembly is configured to pass through the liquid channel and reach the cavity;
the blood gas analysis device further includes a second detection assembly arranged on the mounting bracket, the second detection assembly being configured to detect liquid in the liquid channel exposed from the cavity, where the first detection assembly is configured to perform a blood gas detection, and the second detection assembly is configured to perform a blood oxygen detection.

In some embodiments, the first detection assembly includes a third port spaced apart from the first port; the reagent kit includes a fourth port spaced apart from the second port; in condition of the first port and the second port being in communication, the third port and the fourth port are in communication.

In some embodiments, a docking base is arranged on an outer side of a cavity wall of the cavity, and the second port and the fourth port are arranged on the docking base.

In another aspect, the present disclosure provides a blood gas analysis device, including:
a mounting bracket, including a first mounting position and a second mounting position, where the first mounting position is configured to detachably mount a first detection assembly, and the second mounting position is configured to hold a reagent kit;
where the first detection assembly and the reagent kit are capable of being docked and of being separated, and the first detection assembly and the reagent kit are caused to be in communication or separated; in condition of the first detection assembly and the reagent kit being docked with each other, reagent in the reagent kit is capable of reaching the first detection assembly.

In some embodiments, the mounting bracket further includes a third mounting position, and the third mounting position is configured to detachably mount a second detection assembly; the reagent kitinclud is arranged with a liquid channel exposed from the cavity from the third mounting position, and liquid flowing out of the first detection assembly is configured to pass through the liquid channel and reach the cavity; the second detection assembly is configured to detect liquid in the liquid channel exposed from the cavity; the first detection assembly is configured to perform a blood gas detection, and the second detection assembly is configured to perform a blood oxygen detection.

In some embodiments, the mounting bracket includes a fixed bracket and a movable bracket; the fixed bracket includes a cavity, the cavity forming the second mounting position; the movable bracket includes a receiving cavity, the receiving cavity forming the first mounting position; in condition of the reagent kit being moved into the cavity, the movable bracket is movable relative to the fixed bracket, and the first detection assembly is configured to be docked with the reagent kit.

In some embodiments, the movable bracket is movable relative to the fixed bracket in a first direction or a second direction;
in condition of the movable bracket moving in the first direction relative to the fixed bracket, the first detection assembly is capable of being moved into and of being moved out of the movable bracket;
in condition of the movable bracket moving in the second direction relative to the fixed bracket, the first detection assembly is capable of being docked with and of being separated from the reagent kit.

In another aspect, the present disclosure provides a regulating device, applied to a liquid sample detection apparatus and including:
a first adjusting component and a second adjusting component;
where the first adjusting component is configured to adjust a rotation angle of a sampling element relative to the liquid sample detection apparatus, so as to switch between a first fluid path and a second fluid path for conveying liquid;
the second adjusting component is configured to adjust an axial position of a sleeve sleeved on the sampling element, so as to switch between a first position and a second position of the sleeve;
where in condition of the sleeve being in the first position, the sampling element is capable of achieving a connection of the first fluid path;
in condition of the sleeve being in the second position, the first adjusting component is capable of adjusting the rotation angle of the sampling element, so as to achieve a connection of the second fluid path.

In some embodiments, the first adjusting component includes an adjusting seat arranged on the liquid sample detection apparatus, a first power member arranged on the adjusting seat, and an adjusting bracket; the first power member is configured to drive the adjusting bracket to rotate; the adjusting bracket is configured to be assembled with the sampling element to adjust the rotation angle of the sampling element relative to the liquid sample detection apparatus.

In some embodiments, the first adjusting component further includes a rotary member arranged between the adjusting seat and the adjusting bracket, the rotary member being connected to the adjusting bracket, and the first power member being capable of driving the rotary member to rotate and thereby drive the adjusting bracket to rotate.

In some embodiments, the adjusting seat defines a rotating groove, and the rotary member is accommodated at least partially in the rotating groove and rotatable relative to the rotating groove;
the rotating groove is arranged with a shaft hole, the adjusting bracket is arranged with a rotating shaft, and the rotating shaft passes through the rotary member and is inserted into the shaft hole.

In some embodiments, the first power member is arranged on a side of the adjusting seat away from the rotary member, and a rotating shaft of the first power member passes through the adjusting seat and is transmission-connected to the rotary member.

In some embodiments, a bottom wall of the rotating groove defines a curved slot extending in a rotation direction of the rotary member; a first positioning member is arranged on the curved slot, and the rotary member is arranged with a slider that is slidable along the curved slot; the slider is configured to cooperate with the first positioning member to obtain a rotation angle of the rotary member relative to the adjusting seat.

In some embodiments, the adjusting bracket is assembled with the second adjusting component; in condition of the first power member driving the adjusting bracket to rotate, the second adjusting component is rotatable synchronously with the adjusting bracket.

In some embodiments, the second adjusting component includes a second power member arranged on the adjusting bracket, and a movable bracket connected to the second power member; the second power member is configured to drive the movable bracket to move relative to the adjusting bracket; in condition of the liquid sample detection apparatus being docked with the reagent kit, the sampling element is nested on a side of the movable bracket, and the movable bracket is connected to the sleeve of the sampling element and capable of driving the sleeve to switch between the first position and the second position.

In some embodiments, the movable bracket includes a first movable bracket and a second movable bracket; the first movable bracket is connected to the second power member, and the second movable bracket is elastically connected to the first movable bracket; the second movable bracket is connected to the sleeve in condition of the liquid sample detection apparatus being docked with the reagent kit.

In another aspect, the present disclosure provides a liquid sample detection apparatus, including:
a body, including a first mounting position; and
a regulating device, arranged on the first mounting position;
where the regulating device includes:
a first adjusting component and a second adjusting component;
where the first adjusting component is configured to adjust a rotation angle of a sampling element relative to the liquid sample detection apparatus, so as to switch between a first fluid path and a second fluid path for conveying liquid; the second adjusting component is configured to adjust an axial position of a sleeve sleeved on the sampling element, so as to switch between a first position and a second position of the sleeve;
where in condition of the sleeve being in the first position, the sampling element is capable of achieving a connection of the first fluid path;
in condition of the sleeve being in the second position, the first adjusting component is capable of adjusting the rotation angle of the sampling element, so as to achieve a connection of the second fluid path.

In some embodiments, the first adjusting component includes an adjusting seat arranged on the liquid sample detection apparatus, a first power member arranged on the adjusting seat, and an adjusting bracket; the first power member is configured to drive the adjusting bracket to rotate; the adjusting bracket is configured to be assembled with the sampling element to adjust the rotation angle of the sampling element relative to the liquid sample detection apparatus.

In some embodiments, the second adjusting component includes a second power member arranged on the adjusting bracket, and a movable bracket connected to the second power member; the second power member is configured to drive the movable bracket to move relative to the adjusting bracket; in condition of the liquid sample detection apparatus being docked with the reagent kit, the sampling element is nested on a side of the movable bracket, and the movable bracket is connected to the sleeve of the sampling element and capable of driving the sleeve to switch between the first position and the second position.

In another aspect, the present disclosure provides a biological tissue detection frame, including:
a plurality of plates, that are interconnected to form a cavity and a first mounting position, where the first mounting position is configured to mount a biological tissue measurement platform, and the cavity is configured to contain an auxiliary consumable;
where an avoidance passage is arranged between the cavity and the first mounting position, the avoidance passage being configured to avoid mechanical docking operations and consumable delivery between the biological tissue measurement platform and the auxiliary consumable; the consumable delivery is a passage connecting a first pipe of the biological tissue measurement platform and a second pipe of the auxiliary consumable.

In some embodiments, the plurality of plates include a top plate and a bottom plate disposed opposite each other, the cavity being defined between the top plate and the bottom plate, the avoidance passage passing through the top plate, and the first mounting position being disposed on a side of the top plate away from the bottom plate.

In some embodiments, the cavity includes a pick-up and placement opening, and the auxiliary consumable is capable of being moved into and of being moved out of the cavity through the pick-up and placement opening; the side of the top plate away from the bottom plate is arranged with a limit member, and the limit member spans the avoidance passage; the avoidance passage is configured to limit a height of the auxiliary consumable protruding from the top plate.

In some embodiments, the plurality of plates further include a side plate disposed between the top plate and the bottom plate; the side plate is arranged opposite the pick-up and placement opening; the side plate includes a first docking port and a second mounting position, where the first docking port is in communication with the cavity, and the second mounting position is configured to mount a regulating device; in condition of the auxiliary consumable being moved into the cavity, a sampling element on the auxiliary consumable is exposed through the first docking port to dock with the regulating device.

In some embodiments, the side plate further includes a second docking port and a third mounting position; the second docking port is in communication with the cavity, and the third mounting position is configured to mount a valve apparatus; in condition of the auxiliary consumable being moved into the cavity, a liquid pack connector in the auxiliary consumable is exposed through the second docking port to dock with the valve apparatus.

In some embodiments, the side plate further includes a third docking port and a fourth mounting position; the third docking port is in communication with the cavity, and the fourth mounting position is configured to mount a drive apparatus; in condition of the auxiliary consumable being moved into the cavity, the drive apparatus extends into the cavity from the third docking port and docks with the auxiliary consumable to drive liquid in the auxiliary consumable to flow.

In some embodiments, the avoidance passage passes through the first mounting position, and a positioning member is arranged on the first mounting position for positioning the biological tissue measurement platform on the top plate.

In another aspect, the present disclosure provides a biological tissue detection device, including:
a biological tissue detection frame, including a cavity and a first mounting position;
a biological tissue measurement platform, detachably arranged on the first mounting position and including a first pipe; and
an auxiliary consumable, capable of being moved into and of being moved out of the cavity, and including a second pipe;
where an avoidance passage is arranged between the cavity and the first mounting position, the avoidance passage being configured to avoid mechanical docking operations and consumable delivery between the biological tissue measurement platform and the auxiliary consumable; the consumable delivery is a passage connecting the first pipe and the second pipe.

In some embodiments, the biological tissue detection frame includes a plurality of plates, and the plurality of plates include a top plate and a bottom plate disposed opposite to each other, as well as a side plate disposed between the top plate and the bottom plate; the top plate, the bottom plate, and the side plate enclose to define the cavity with a pick-up and placement opening, and the auxiliary consumable capable of being moved into and of being moved out of the cavity through the pick-up and placement opening; the avoidance passage runs through the top plate, and the first mounting position is arranged on a side of the top plate away from the bottom plate.

In some embodiments, the auxiliary consumable includes a sampling element, and the biological tissue detection device includes a regulating device; the regulating device is configured to adjust a sampling posture of the sampling element; the side plate includes a first docking port and a second mounting position; the first docking port is in communication with the cavity, and the second mounting position is configured to mount the regulating device; the sampling element is docked with the regulating device in condition of the auxiliary consumable being moved into the cavity.

In some embodiments, the auxiliary consumable includes a receiving cavity for accommodating a liquid pack, and a cavity wall of the receiving cavity is arranged with the liquid pack connector; the biological tissue detection device further includes a valve apparatus, the valve apparatus being configured to be docked with the liquid pack connector to control liquid in the liquid pack to flow out; the side plate includes a second docking port and a third mounting position; the second docking port is in communication with the cavity, and the third mounting position is configured to mount the valve apparatus; the liquid pack connector is docked with the valve apparatus in condition of the auxiliary consumable being moved into the cavity.

In some embodiments, the auxiliary consumable includes a receiving cavity for accommodating a liquid pack, and a cavity wall of the receiving cavity is arranged with a first drive member; the biological tissue detection device further includes a drive apparatus, the drive apparatus being configured to be docked with the first drive member to drive liquid in the liquid pack to flow out; the side plate includes a third docking port and a fourth mounting position; the third docking port is in communication with the cavity, and the fourth mounting position is configured to mount the drive apparatus; the drive apparatus is docked with the first drive member in condition of the auxiliary consumable being moved into the cavity.

In another aspect, the present disclosure provides a sample analysis apparatus, including:
a fixing seat and a mounting seat, where a guide rod is arranged between the fixing seat and the mounting seat;
a conveying assembly, disposed between the fixing seat and the mounting seat, where the conveying assembly is sleeved on the guide rod and is slidable along the guide rod; and
a testing assembly, disposed between fixing seat and mounting seat, where the testing assembly is sleeved on the guide rod and is slidable along the guide rod;
where the testing assembly is arranged on a side of the conveying assembly away from the fixing seat, and a first elastic member is arranged between the conveying assembly and the fixing seat; the conveying assembly and the fixing seat are configured to be separated by the first elastic member; a second elastic member is arranged between the conveying assembly and the testing assembly, and the conveying assembly and the testing assembly are configured to be separated by the second elastic member.

In some embodiments, the conveying assembly includes a carrier seat that is sleeved on and slidable along the guide rod, a telescopic seat slidably connected to the carrier seat, and a first drive member disposed on the carrier seat; the first elastic member is disposed between the carrier seat and the fixing seat, and the first drive member is capable of driving the telescopic seat to move relative to the carrier seat.

In some embodiments, the carrier seat is arranged with a channel extending along a moving direction of the telescopic seat, and the telescopic seat is partially disposed in the channel and is movable along the channel relative to the carrier seat.

In some embodiments, the telescopic seat defines a clamping slot for placing a detection assembly, and the clamping slot is configured to switch between a first position and a second position in condition of the telescopic seat moving relative to the carrier seat;
in condition of the clamping slot being in the first position, the detection assembly is capable of being moved into and of being moved out of the clamping slot;
in condition of the clamping slot being in the second position, the clamping slot is accommodated in the channel.

In some embodiments, the carrier seat includes a top wall and a bottom wall disposed opposite each other, the bottom wall being disposed on a side of the carrier seat close to the fixing seat, the top wall being disposed on a side of the carrier seat close to the testing assembly, and the channel being formed between the top wall and the bottom wall; the top wall defines a first avoidance hole, and the bottom wall defines a second avoidance hole; in condition of the clamping slot being in the second position, a detection element of the detection assembly placed in the clamping slot is exposed from the first avoidance hole, the testing assembly is capable of being docked with the detection element of the detection assembly through the first avoidance hole, and a liquid hole of the detection assembly is exposed from the second avoidance hole.

In some embodiments, the telescopic seat is arranged with a transmission member, and the transmission member is assembled with the first drive member to drive the telescopic seat to move by means of the transmission member under drive of the first drive member.

In some embodiments, the telescopic seat defines an avoidance slot, the transmission member is disposed on a slot wall of the avoidance slot, and an output shaft of the first drive member extends into the avoidance slot and is transmission-connected to the transmission member.

In some embodiments, the testing assembly includes a testing seat that is sleeved on the guide rod and slidable along the guide rod, and a testing plate arranged on the testing seat; the testing plate is arranged with a test head, and the test head contacts the detection element of the detection assembly through the first avoidance hole in condition of the testing seat and the carrier seat being docked.

In some embodiments, the testing assembly further includes a supporting component arranged on the testing seat, and a second elastic member arranged between the supporting component and the testing seat; in condition of the testing seat and the carrier seat being docked such that the testing plate contacts the detection element of the detection assembly, the second elastic member is compressed to generate an elastic force, and the elastic force is configured to cause the supporting component and the testing seat to separate.

In some embodiments, the testing seat includes a first mounting position and a second mounting position on a same side; the testing plate is arranged on the first mounting position, and the supporting component is arranged on the second mounting position; before the second elastic member is compressed, a height of the supporting component protruding from the testing seat is greater than or equal to a height of the test head protruding from the testing seat.

In some embodiments, the supporting component includes a body and a limit member; the limit member is fixedly disposed on the second mounting position, and the second elastic member is disposed between the body and the second mounting position; the body and the limit member are slidably connected, and the limit member is configured to limit a sliding travel of the body under an action of an elastic force.

In some embodiments, the mounting seat is arranged with a second drive member, and the second drive member is configured to drive the testing seat to slide along the guide rod.

In another aspect, the present disclosure provides a biological detection device, including:
a first base and a second base;
a guide member, disposed between the first base and the second base and extending in an arrangement direction of the first base and the second base;
a testing assembly, arranged on the guide member and movable along the guide member; and
a transmission assembly, arranged on the first base and including a drive member;
where the testing assembly is arranged with a supporting member on a side close to the first base, and the drive member is in rolling fit with the supporting member to drive the testing assembly to move along the guide member.

In some embodiments, the transmission assembly includes a power member arranged on the first base and a support shaft passing through the drive member; the power member and the support shaft are transmission-assembled to drive the support shaft to rotate, and a rotation of the support shaft drives the drive member to rotate synchronously.

In some embodiments, the biological detection device further includes a mounting bracket and a drive bracket that are spaced apart and arranged on the first base, the support shaft being arranged between the mounting bracket and the drive bracket, and the power member being arranged on the drive bracket.

In some embodiments, the first base defines an avoidance hole, and the avoidance hole is configured to avoid the drive member; the mounting bracket is arranged on a side of the first base close to the testing assembly, a spacer is arranged on at least one of the first base and the testing assembly, and the spacer is arranged between the first base and the testing assembly to prevent the mounting bracket from interfering with the rolling fit between the drive member and the supporting member.

In some embodiments, the mounting bracket includes a first bracket and a second bracket spaced apart; the second bracket is arranged between the first bracket and the drive bracket, and the support shaft is arranged between the first bracket and the drive bracket; the drive member is arranged between the first bracket and the second bracket and faces the avoidance hole, and the power member and the supporting shaft are transmission-assembled between the second bracket and the drive bracket.

In some embodiments, the first bracket is arranged with a first positioning member, and the drive member or the supporting shaft is arranged with a second positioning member; the second positioning member is configured to rotate synchronously with the drive member or the supporting shaft and cooperate with the first positioning member to obtain a rotation angle of the drive member or the supporting shaft.

In some embodiments, the power member is arranged on a side of the drive bracket away from the second bracket, and an output shaft of the power member passes through the drive bracket and is transmission-assembled with the support shaft.

In some embodiments, the transmission assembly includes a first transmission assembly disposed between the drive bracket and the second bracket; an end of the output shaft of the power member passing through the drive bracket and extending into between the drive bracket and the second bracket is arranged with a first transmission wheel, and a portion of the support shaft disposed between the drive bracket and the second bracket is arranged with a second transmission wheel, the first transmission assembly being transmission-assembled with the first transmission wheel and the second transmission wheel, respectively.

In some embodiments, the first transmission assembly includes a first fixed shaft arranged between the drive bracket and the second bracket, and a first driving wheel and a first driven wheel that are arranged on the first fixed shaft; the first driving wheel is transmission-assembled with the first transmission wheel, and the first driven wheel is transmission-assembled with the second transmission wheel;
where a diameter of the first driving wheel is greater than a diameter of the first driven wheel, an axial thickness of the first driving wheel is less than an axial thickness of the first transmission wheel, the diameter of the first driven wheel is less than a diameter of the second transmission wheel, and an axial thickness of the second transmission wheel is less than an axial thickness of the first driven wheel.

In some embodiments, the transmission assembly further includes a second transmission assembly disposed between the first transmission assembly and the second transmission wheel, and the second transmission assembly is transmission-assembled with the first transmission assembly and the second transmission wheel, respectively.

In some embodiments, the second transmission assembly includes a second fixed shaft arranged between the drive bracket and the second bracket, and a second driving wheel and a second driven wheel that are arranged on the second fixed shaft; the second driving wheel is transmission-assembled with the first driven wheel, and the second driven wheel is transmission-assembled with the second transmission wheel;
where a diameter of the second driving wheel is greater than a diameter of the second driven wheel, an axial thickness of the second driving wheel is less than the axial thickness of the first driven wheel, the diameter of the second driven wheel is less than the diameter of the second transmission wheel, and the axial thickness of the second transmission wheel is less than an axial thickness of the second driven wheel.

In some embodiments, a limit block is arranged on one of the first bracket and the testing assembly, and the other of the first bracket and the testing assembly defines a limit groove; the limit block and the limit groove cooperate to limit a spacing between the first base and the testing assembly.

In some embodiments, the drive member is a turbine, and the supporting member is a roller.

In another aspect, the present disclosure provides a blood sample analysis platform, including:
a blood sample analyzer, including a non-contact detection element and an avoidance slot; and
an auxiliary liquid box, including a cavity and a liquid path, where the cavity is configured to hold an auxiliary liquid and a waste liquid pack; an end of the liquid path is configured to introduce the auxiliary liquid, and another end of the liquid path is configured to communicate with the waste liquid pack;
where the liquid path has a portion that is exposed from the auxiliary liquid box, and the non-contact detection element is configured to detect a portion of the liquid path entering the avoidance slot.

In some embodiments, the blood sample analysis platform includes a mounting bracket, the mounting bracket including a receiving cavity and a first mounting position; the auxiliary liquid box is capable of being moved into and of being moved out of the receiving cavity, and the first mounting position is configured to mount the blood sample analyzer; an avoidance passage is arranged between the receiving cavity and the first mounting position, and in condition of the auxiliary liquid box being moved into the receiving cavity, the portion of the liquid path enters the avoidance slot through the avoidance passage.

In some embodiments, the auxiliary liquid box is arranged with a sampling seat, and the liquid path includes a liquid channel arranged in the sampling seat; in condition of the auxiliary liquid box being moved into the receiving cavity, the sampling seat is moved into the avoidance slot, and the non-contact detection element is configured to detect liquid in the liquid channel.

In some embodiments, the liquid channel includes a liquid inlet section, a liquid outlet section, and a detection section connecting the liquid inlet section and the liquid outlet section; the liquid inlet section is configured to introduce the auxiliary liquid, and the liquid outlet section is configured to be in communication with the waste liquid pack; in condition of the sampling seat being moved into the avoidance slot, the non-contact detection element is capable of detecting liquid in the detection section.

In some embodiments, the sampling seat is arranged with a light-transmitting region corresponding to the detection section, and light emitted from the blood sample analyzer is configured to be directed to the liquid in the detection section through the light-transmitting region for detection.

In some embodiments, the blood sample analyzer further includes a testing seat and a testing plate, the testing seat being arranged on the first mounting position and the testing plate being arranged on the testing seat; the avoidance slot is defined on the testing seat, and the non-contact detection element includes a light source and a light sensor device arranged on the testing plate; the light emitted by the light source is configured to pass through the light-transmitting region to reach the light sensor device to detect the liquid in the detection section.

In some embodiments, the blood sample analyzer further includes an ultrasonic sounder arranged on the testing seat, for ultrasonic processing of the liquid in the liquid channel entering the avoidance slot.

In another aspect, the present disclosure provides a blood sample analyzer, including:
a testing seat and a non-contact detection element, where the testing seat defines an avoidance slot, and the non-contact detection element is connected to the testing seat for detecting liquid entering the avoidance slot;
where the testing seat is capable of being docked with and of being separated from an auxiliary liquid box; a liquid path of the auxiliary liquid box includes a bent part that protrudes from the liquid box; in condition of the testing seat being docked with the liquid box, the bent part enters the avoidance slot to be detected by the non-contact detection element.

In some embodiments, the blood sample analyzer further includes a testing seat and a testing plate, the testing seat being arranged on the first mounting position and the testing plate being arranged on the testing seat; the avoidance slot is defined on the testing seat, and the non-contact detection element includes a light source and a light sensor device arranged on the testing plate; the light emitted by the light source is configured to pass through the light-transmitting region to reach the light sensor device to detect the liquid in the detection section.

In some embodiments, the blood sample analyzer further includes an ultrasonic sounder arranged on the testing seat, for ultrasonic processing of the liquid in the liquid channel entering the avoidance slot.

In another aspect, the present disclosure provides an auxiliary liquid box, including:
a cavity and a liquid path;
where the cavity is configured to hold an auxiliary liquid and a waste liquid pack; an end of the liquid path is configured to introduce the auxiliary liquid, and another end of the liquid path is configured to communicate with the waste liquid pack; the liquid path has a portion that is exposed from the auxiliary liquid box, and in condition of the auxiliary liquid box being docked with a blood sample analyzer, the portion of the liquid path enters an avoidance slot of the blood sample analyzer to be detected by a non-contact detection element of the blood sample analyzer.

In some embodiments, the auxiliary liquid box is arranged with a sampling seat, and the liquid path includes a liquid channel arranged in the sampling seat; in condition of the auxiliary liquid box being docked with the blood sample analyzer, the sampling seat is moved into the avoidance slot, and the non-contact detection element is configured to detect liquid in the liquid channel.

In some embodiments, the liquid channel includes a liquid inlet section, a liquid outlet section, and a detection section connecting the liquid inlet section and the liquid outlet section; the liquid inlet section is configured to introduce the auxiliary liquid, and the liquid outlet section is configured to be in communication with the waste liquid pack; in condition of the sampling seat being moved into the avoidance slot, the non-contact detection element is capable of detecting liquid in the detection section.

In some embodiments, the sampling seat is arranged with a light-transmitting region corresponding to the detection section, and light emitted from the blood sample analyzer is configured to be directed to the liquid in the detection section through the light-transmitting region for detection.

In another aspect, the present disclosure provides a fluid detection instrument, including:
a valve assembly, including a control element, a first pipe, a plurality of inlets, and an outlet, where the control element is configured to control one of the plurality of inlets to be in communication with one end of the first pipe, and the other end of the first pipe is in communication with the outlet; and
a fluid kit, including: a cavity for accommodating a fluid pack, and a plurality of ports, where an end of each port is configured to be in communication with the fluid pack, and the other end of the port is configured to be in communication with a corresponding inlet;
where the valve assembly is capable of being docked with and of being separated from the fluid kit, and the inlet and the port are configured to be docked or separated; in condition of the inlet and the port being docked, the first pipe is capable of being in communication with the port under control of the control element, and fluid in the fluid pack is configured to reach the first pipe.

In some embodiments, the valve assembly includes a vent, and an end of the first pipe is selectively in communication with the inlet or the vent under the control of the control element.

In some embodiments, the fluid kit defines a docking groove configured to dock with a sampling element, and the docking groove is arranged with a through hole and a connecting pipe passing through the through hole; an end of the connecting pipe is in communication with the outlet, and the other end of the connecting pipe is configured to be in communication with a sample inlet of the sampling element; the connecting pipe is in sealed contact with a hole wall of the through hole.

**In** some embodiments, the sampling element includes a sampling needle, a sleeve sleeved on the sampling needle, and a docking member arranged on an end of the sleeve; an outer diameter of the sleeve is less than or equal to an inner diameter of the docking groove; the docking member defines another through hole; the docking member is movable along an axial direction of the sampling needle following the sleeve, and the sampling needle is configured to be inserted into an end of the through hole on the docking member, or pass through the through hole on the docking member and be exposed on a side of the docking member;
where in condition of the sleeve moving along the axial direction of the sampling needle and towards the connecting pipe such that the docking member is inserted into the docking groove, the sampling needle and the connecting pipe are inserted into both ends of the through hole on the docking member to achieve connection.

**In** another aspect, the present disclosure provides a valve assembly, including:
a control element, a first pipe, a plurality of inlets, and an outlet;
where the control element is configured to control one of the plurality of inlets to be in communication with one end of the first pipe, and the other end of the first pipe is in communication with the outlet; each inlet is capable of being docked with and of being separated from a port of a fluid kit, to obtain fluid of the fluid kit by controlling the inlet through the control element in condition of the inlet being docked with the port.

**In** some embodiments, the valve assembly includes a vent, and an end of the first pipe is selectively in communication with the inlet or the vent under control of the control element.

**In** another aspect, the present disclosure provides a fluid kit, including:
a cavity for accommodating a fluid pack, and a plurality of ports;
where an end of each port is configured to be in communication with the fluid pack, and the other end of the port is configured to be in communication with a corresponding inlet of a valve assembly; the valve assembly is capable of being docked with and of being separated from the fluid kit, and in condition of the valve assembly and the fluid kit being docked, fluid in the fluid kit is configured to be delivered to the valve assembly through the port.

**In** some embodiments, the fluid kit defines a docking groove configured to dock with a sampling element, and the docking groove is arranged with a through hole and a connecting pipe passing through the through hole; an end of the connecting pipe is in communication with the outlet, and the other end of the connecting pipe is configured to be in communication with a sample inlet of the sampling element; the connecting pipe is in sealed contact with a hole wall of the through hole.

In some embodiments, the sampling element includes a sampling needle, a sleeve sleeved on the sampling needle, and a docking member arranged on an end of the sleeve; an outer diameter of the sleeve is less than or equal to an inner diameter of the docking groove; the docking member defines another through hole; the docking member is movable along an axial direction of the sampling needle following the sleeve, and the sampling needle is configured to be inserted into an end of the through hole on the docking member, or pass through the through hole on the docking member and be exposed on a side of the docking member;
where in condition of the sleeve moving along the axial direction of the sampling needle and towards the connecting pipe such that the docking member is inserted into the docking groove, the sampling needle and the connecting pipe are inserted into both ends of the through hole on the docking member to achieve connection.

In another aspect, the present disclosure provides an integrated reagent kit, including:
a case, defining a storage space, where the storage space is configured to store a reagent pack and a recovery pack;
a sampling apparatus, arranged on the case and disposed outside the storage space; and
a first pipe and a second pipe, that are arranged on the case, where an input end of the sampling apparatus is capable of being in communication with the reagent pack through the first pipe, and an output end of the sampling apparatus is in communication with the recovery pack through the second pipe; the sampling apparatus is rotatable relative to the case, and the input end of the sampling apparatus is configured to collect liquid from the reagent pack or an external container.

In some embodiments, the case includes a first port and a second port arranged at intervals; the first port is in communication with the output end of the sampling apparatus, and the second port is in communication with the recovery pack through the second pipe; the first port and the second port are configured to dock with a testing assembly, and liquid collected by the sampling apparatus is configured to reach the testing assembly.

In some embodiments, the case is arranged with a sample introduction seat disposed outside the storage space, the first port and the second port being arranged on the sample introduction seat, and a first connecting pipe and the second connecting pipe are arranged in the sample introduction seat; an end of the first connecting pipe is in communication with the first port, and another end of the first connecting pipe is in communication with the output end of the sampling apparatus; an end of the second connecting pipe is in communication with the second port, and another end of the second connecting pipe is configured to be in communication with the recovery pack.

In some embodiments, the output end of the sampling apparatus is inserted into the sample introduction seat and in communication with the first connecting pipe.

In some embodiments, the sample introduction seat includes a seat body and a pipe body arranged on an end of the seat body; the first port and the second port are arranged on the seat body; an end of the first connecting pipe is inserted into the pipe body, and another end of the first connecting pipe is in communication with the first port; the output end of the sampling apparatus is inserted into the pipe body to be in communication with the first connecting pipe.

In some embodiments, the sampling apparatus includes a sampling element, a sleeve sleeved on an input end of the sampling element, and a swivel sleeved on an output end of the sampling element; the swivel is rotatably assembled with the pipe body, and the swivel is configured to be rotated relative to the pipe body; a rotation of the swivel is configured to drive the sleeve to rotate, and the input end of the sampling element is configured to collect the liquid from the reagent pack or the external container.

In some embodiments, the pipe body is arranged with a first hole segment and a second hole segment that are in communication, the first hole segment being configured to be rotatably connected to the swivel, and the output end of the sampling element and the first connecting pipe are in communication through the second hole segment; a hole diameter of the first hole segment is greater than a hole diameter of the second hole segment.

In some embodiments, the swivel includes a first swivel and a second swivel clamped to the sleeve, the first swivel being rotatably assembled with the pipe body, and the second swivel being rotatable relative to the pipe body under an action of an external force, for driving the sleeve to rotate.

In some embodiments, the sleeve is movable axially along the sample inlet of the sampling element under an external force to expose or cover the sample inlet of the sampling element; the sleeve defines an avoidance hole to avoid the sampling element when the sleeve is moved axially.

In some embodiments, the case is arranged with a docking base, the docking base including a port; the port is configured to be docked with the input end of the sampling apparatus; an end of the first pipe is in communication with the port, and another end of the first pipe is configured to be in communication with the reagent pack.

In another aspect, the present disclosure provides a reagent storage apparatus, including:
a storage box, and a sampling assembly and a docking assembly that are disposed on the storage box and outside the storage box;
where an outlet end of the sampling assembly is in communication with a first position in the storage box, the docking assembly defines a sampling groove, and the sampling groove is in communication with a second position in the storage box; the sampling assembly is capable of being connected with and of being separated from the docking assembly; in condition of the sampling assembly and the docking assembly being connected, an inlet end of the sampling assembly is inserted into the sampling groove to sample from the second position in storage box; in condition of the sampling assembly and the docking assembly being separated, the inlet end of sampling assembly is capable of sampling from outside the storage box.

In some embodiments, the docking assembly includes a docking base arranged on the storage box, a sampling groove defined on the docking base, and a connector pipe disposed in the sampling groove; an end of the connector pipe is configured to be in communication with the inlet end of the sampling assembly, and another end of the connector pipe is configured to be in communication with the second position in the storage box; the inlet end of the sampling assembly is arranged with a connector, and in condition of the sampling assembly being connected to the docking assembly, the connector pipe is inserted into the connector.

In some embodiments, the sampling assembly includes a sampling element capable of being inserted into or passing through the connector; in condition of the sampling assembly and the docking assembly being connected, a sampling end of the sampling element is inserted into the connector, and the connector pipe is arranged on the connector to be in communication with the sampling end of the sampling element.

In some embodiments, the docking assembly further includes a sealing member embedded in the sampling groove, and the connector pipe passes through the sealing member; in condition of the connector pipe being inserted into the connector, the sealing member abuts against the connector.

In some embodiments, an end of the connector close to the sealing member is tapered and concave to guide the connector pipe to be inserted into the connector.

In some embodiments, the docking base defines a matching groove opened facing the storage box; an end of the connector pipe extends into the matching groove to be in communication with the second position in the storage box through a connecting pipe, and another end of the connector pipe extends into sampling groove to be inserted in the connector in condition of the sampling assembly being connected to the docking assembly.

In some embodiments, the sampling assembly includes a sleeve sleeved on the sampling element, and a connector embedded in an end portion of the sleeve; a clamping member is sleeved on the connector at the end portion of the sleeve, and the clamping member is connected to the sleeve; a radial width of the clamping member is greater than a radial width of the sealing member, and the radial width of the clamping member is less than or equal to a radial width of the sampling groove.

In some embodiments, the docking assembly further includes a sliding member that is slidably connected to the docking base, and the sliding member is capable of abutting against the sampling assembly to stabilize a sampling state of the sampling assembly.

In some embodiments, the docking base defines a mating hole, the sliding member passes through the mating hole and is elastically connected to the storage box, and the sliding member is configured to slide relative to the docking base.

In some embodiments, the mating hole includes a first mating section and a second mating section that are in communication; the first mating section has a cross-sectional area that is smaller than a cross-sectional area of the second mating section, and the first mating section is arranged between the second mating section and the storage box; the sliding member includes a sliding portion and a limit portion, the sliding portion being slidable relative to the first mating portion, and the limit portion being slidable relative to the second mating portion and stopped by the second mating portion.

In some embodiments, the docking assembly further includes an elastic member arranged between the storage box and the sliding member; an end of the elastic member is connected to the sliding member, and another end of the elastic member is connected to the storage box.

In some embodiments, the docking base defines an avoidance slot that passes through a side wall of the sampling groove; the inlet end of the sampling assembly is capable of being unscrewed out of the sampling groove through the avoidance slot, and the inlet end of the sampling assembly is capable of sampling from outside the storage box.

In another aspect, the present disclosure provides a liquid parameter measurement platform, including:
a liquid loading box, including a liquid taking assembly and an adjusting assembly, where the adjusting assembly is configured to be driven under an external force to drive the liquid taking assembly to rotate relative to the liquid loading box; and
a rotatable assembly and a movable assembly, where the rotatable assembly is rotatable to drive the movable assembly to rotate synchronously, and the movable assembly is movable relative to the rotatable assembly;
where the adjusting assembly is capable of being docked with and of being separated from the rotatable assembly; in condition of the adjusting assembly and the rotatable assembly being docked, the rotatable assembly is capable of driving the adjusting assembly to rotate relative to the liquid loading box, thereby driving the liquid taking assembly to rotate relative to the liquid loading box;
the liquid taking assembly and the movable assembly are configured to follow docking or separation of the adjusting assembly and the rotatable assembly to achieve corresponding docking or separation;
in condition of the liquid taking assembly and the movable assembly being docked, the movable assembly is configured to adjust an axial position of at least part of the liquid taking assembly, and the rotatable assembly is configured to drive the liquid taking assembly to switch between a first attitude and a second attitude;
where the first attitude corresponds to that the liquid taking assembly is limited to collect liquid from inside the liquid loading box, and the second attitude corresponds to that the liquid taking assembly is limited to collect liquid from outside the liquid loading box.

In some embodiments, the liquid taking assembly includes a liquid taking element and a sleeve; a liquid taking end of the liquid taking element is configured to collect the liquid inside the liquid loading box or the liquid outside the liquid loading box; liquid collected by the liquid taking element is configured to flow to an interior of liquid loading box or a liquid recovery container through a liquid outlet end of the liquid taking element;
the sleeve is sleeved on the liquid taking end of the liquid taking element and is capable of being docked with the movable assembly, and an axial position of the sleeve relative to the liquid taking element is adjustable under drive of the movable assembly.

In some embodiments, the adjusting assembly includes a swivel clamped to the sleeve; an end of the swivel is sleeved on a liquid outlet end of the liquid taking element, and another end of the swivel is capable of being docked with and of being separated from the rotatable assembly; in condition of the rotatable assembly being docked with the swivel, the rotatable assembly is capable of driving the swivel to rotate, thereby driving the sleeve and the liquid taking element to rotate.

In some embodiments, the rotatable assembly includes an adjusting seat to be arranged on the liquid parameter measurement platform, a first power member arranged on the adjusting seat, and an adjusting bracket; the first power member is configured to drive the adjusting bracket to rotate; the adjusting bracket is configured to be assembled with the swivel to adjust a rotation angle of the swivel relative to the liquid loading box.

In some embodiments, the rotatable assembly further includes a rotary member disposed between the adjusting seat and the adjusting bracket, the rotary member being connected to the adjusting bracket for synchronous rotation, and the first power member being capable of driving the rotary member to rotate.

In some embodiments, the first power member is arranged on a side of the adjusting seat away from the rotary member, and an output shaft of the first power member passes through the adjusting seat and is transmission-connected to the rotary member.

In some embodiments, the adjusting seat defines a rotating groove, the rotary member is at least partially embedded in the rotating groove, and an output shaft of the first power member is transmission-connected to the rotatory member to drive the rotary member to rotate relative to the rotating groove;
a shaft hole is defined on the rotating groove, a rotating shaft is arranged on the adjusting bracket, and the rotating shaft passes through the rotary member and is inserted into the shaft hole.

In some embodiments, a bottom wall of the rotating groove defines a curved slot extending in a rotation direction of the rotary member, the curved slot is arranged with a first positioning member, and the rotary member is arranged with a slider that is slidable along the curved slot; the slider is configured to cooperate with the first positioning member to obtain a rotation angle of the rotary member relative to the adjusting seat.

In some embodiments, the adjusting bracket is assembled with the movable assembly; in condition of the first power member driving the adjusting bracket to rotate, the movable assembly is rotatable synchronously with the adjusting bracket; the adjusting bracket defines an adjusting slot, and the movable assembly is embedded in the adjusting slot to rotate synchronously with the adjusting bracket.

In some embodiments, the movable assembly includes a second power member arranged on the adjusting bracket, and a movable bracket connected to the second power member; the second power member is configured to drive the movable bracket to move relative to the adjusting bracket;
in condition of the adjusting assembly being docked with the rotatable assembly, the sleeve of the liquid taking assembly is nested on a side of the movable bracket, and the movable bracket is connected to the sleeve and capable of driving the sleeve to switch between a first position and a second position.

In some embodiments, the movable assembly further includes a fixed bracket embedded in the adjusting slot, and an end of the output shaft of the second power member passes through the fixed bracket to connect with the movable bracket.

In some embodiments, an elastic member is arranged between the fixed bracket and the movable bracket, and the movable bracket is configured to move relative to the fixed bracket under drive of the second power member, causing the elastic member to generate elasticity; in condition of the drive being cancelled, the elasticity causes the movable bracket to reset.

In some embodiments, the movable bracket includes a first engagement portion, and the sleeve includes a second engagement portion; in condition of the adjusting assembly being docked with the rotatable assembly, the first engagement portion and the second engagement portion are engaged and connected.

In another aspect, the present disclosure provides a liquid loading box, applied to a liquid parameter measurement platform and including:
a liquid taking assembly and an adjusting assembly, where the adjusting assembly is configured to, under an external force, drive the liquid taking assembly to rotate relative to the liquid loading box;
where the adjusting assembly is capable of being docked with and of being separated from a rotatable assembly, and in condition of the adjusting assembly being docked with the rotatable assembly, the rotatable assembly is capable of driving the adjusting assembly to rotate relative to the liquid loading box, thereby driving the liquid taking assembly to rotate relative to the liquid loading box;
the liquid taking assembly is capable of being docked with and of being separated from a movable assembly, and the liquid taking assembly and the movable assembly are configured to follow docking or separation of the adjusting assembly and the rotatable assembly to achieve corresponding docking or separation; in condition of the liquid taking assembly and the movable assembly being docked, the movable assembly is configured to adjust an axial position of at least part of the liquid taking assembly, and the rotatable assembly is configured to drive the liquid taking assembly to switch between a first attitude and a second attitude;
where the first attitude corresponds to that the liquid taking assembly is limited to collect liquid from inside the liquid loading box, and the second attitude corresponds to that the liquid taking assembly is limited to collect liquid from outside the liquid loading box.

In some embodiments, the liquid taking assembly includes a liquid taking element and a sleeve; a liquid taking end of the liquid taking element is configured to collect the liquid inside the liquid loading box or the liquid outside the liquid loading box; liquid collected by the liquid taking element is configured to flow to an interior of liquid loading box or a liquid recovery container through a liquid outlet end of the liquid taking element;
the sleeve is sleeved on the liquid taking end of the liquid taking element and is capable of being docked with the movable assembly, and an axial position of the sleeve relative to the liquid taking element is adjustable under drive of the movable assembly.

In some embodiments, the adjusting assembly includes a swivel clamped to the sleeve; an end of the swivel is sleeved on a liquid outlet end of the liquid taking element, and another end of the swivel is capable of being docked with and of being separated from the rotatable assembly; in condition of the rotatable assembly being docked with the swivel, the rotatable assembly is capable of driving the swivel to rotate, thereby driving the sleeve and the liquid taking element to rotate.

In another aspect, the present disclosure provides a motion apparatus, applied to a liquid parameter measurement platform and including:
a rotatable assembly and a movable assembly, where the rotatable assembly is rotatable to drive the movable assembly to rotate synchronously, and the movable assembly is movable relative to the rotatable assembly;
where the rotatable assembly is capable of being docked with and of being separated from an adjusting assembly of a liquid loading box; in condition of the adjusting assembly and the rotatable assembly being docked, the rotatable assembly is capable of driving the adjusting assembly to rotate relative to the liquid loading box, thereby driving a liquid taking assembly of the liquid loading box to rotate relative to the liquid loading box;
where the movable assembly is capable of being docked with and of being separated from the liquid taking assembly; the liquid taking assembly and the movable assembly are configured to follow docking or separation of the adjusting assembly and the rotatable assembly to achieve corresponding docking or separation;
in condition of the liquid taking assembly and the movable assembly being docked, the movable assembly is configured to adjust an axial position of at least part of the liquid taking assembly, and the rotatable assembly is configured to drive the liquid taking assembly to switch between a first attitude and a second attitude;
where the first attitude corresponds to that the liquid taking assembly is limited to collect liquid from inside the liquid loading box, and the second attitude corresponds to that the liquid taking assembly is limited to collect liquid from outside the liquid loading box.

In some embodiments, the rotatable assembly includes an adjusting seat to be arranged on the liquid parameter measurement platform, a first power member arranged on the adjusting seat, and an adjusting bracket; the first power member is configured to drive the adjusting bracket to rotate; the adjusting bracket is configured to be assembled with the swivel to adjust a rotation angle of the swivel relative to the liquid loading box.

In some embodiments, the adjusting bracket is assembled with the movable assembly; in condition of the first power member driving the adjusting bracket to rotate, the movable assembly is rotatable synchronously with the adjusting bracket; the adjusting bracket defines an adjusting slot, and the movable assembly is embedded in the adjusting slot to rotate synchronously with the adjusting bracket.

In some embodiments, the movable assembly includes a second power member arranged on the adjusting bracket, and a movable bracket connected to the second power member; the second power member is configured to drive the movable bracket to move relative to the adjusting bracket;
in condition of the adjusting assembly being docked with the rotatable assembly, the sleeve of the liquid taking assembly is nested on a side of the movable bracket, and the movable bracket is connected to the sleeve and capable of driving the sleeve to switch between a first position and a second position.

In another aspect, the present disclosure provides a biological sample analysis device, including:
a drive assembly, including an output shaft; and
a reagent storage apparatus, including a receiving cavity, a connecting pipe, and a squeezing assembly, where the receiving cavity is configured to hold a reagent pack and a recovery pack, and the connecting pipe is partially embedded in the squeezing assembly; an end of the connecting pipe is configured to be in communication with the reagent pack, and another end of the connecting pipe is configured to be in communication with the recovery pack;
where the drive assembly is capable of being docked with and of being separated from the reagent storage apparatus, causing the output shaft to be docked with or separated from the squeezing assembly; in condition of the drive assembly and the reagent storage apparatus being docked, the output shaft is capable of driving the squeezing assembly to squeeze the connecting pipe to cause liquid in the connecting pipe to flow.

In some embodiments, the biological sample analysis device further includes a mounting bracket; the mounting bracket includes a cavity and a first mounting position; the reagent storage apparatus is capable of being moved into and of being moved out of the cavity, and the drive assembly is arranged on the first mounting position; the first mounting position is arranged on an end of a travel of the reagent storage apparatus moving into the cavity.

In some embodiments, the drive assembly is arranged outside the cavity, and an avoidance hole is defined between the cavity and the first mounting position, with the output shaft of the drive assembly extending into the cavity through the avoidance hole; in condition of the reagent storage apparatus being moved into the cavity, the output shaft of the drive assembly is transmission-assembled with the squeezing assembly.

In some embodiments, the squeezing assembly includes a first squeezing member and a second squeezing member, the second squeezing member being arranged coaxially with the first squeezing member and being embedded in the first squeezing member, and a part of the connecting pipe being arranged between the first squeezing member and the second squeezing member; the second squeezing member is configured to be transmission-assembled with the output shaft, and the second squeezing member is configured to be driven by the output shaft to rotate relative to the first squeezing member and thereby squeeze the connecting pipe.

In some embodiments, the first squeezing member is annular in shape, and the second squeezing member includes a rotating portion and a squeezing portion; the rotating portion is configured to be docked with the output shaft, and the squeezing portion is disposed on a peripheral side of the rotating portion and rotatable synchronously with the rotating portion;
the squeezing portion is arranged spaced apart from the first squeezing portion, and the connecting pipe is partially arranged between the squeezing portion and the first squeezing portion.

In some embodiments, the rotating portion defines a shaft hole, and an end of the output shaft is assembled with the shaft hole; the squeezing portion is sleeved with a shaft sleeve, the shaft sleeve being configured to abut against the connecting pipe.

In some embodiments, a cavity wall of the receiving cavity defines a storage groove, the squeezing assembly is embedded in the storage groove and arranged coaxially with the storage groove, and the connecting pipe is partially arranged between the storage groove and the squeezing assembly; the squeezing assembly is configured to be transmission-assembled with the output shaft, and the squeezing assembly is configured to be driven by the output shaft to rotate relative to the storage groove and thereby squeeze the connecting pipe.

In another aspect, the present disclosure provides a reagent storage apparatus, including:
a receiving cavity, a connecting pipe, and a squeezing assembly, where the receiving cavity is configured to hold a reagent pack and a recovery pack, and the connecting pipe is partially embedded in the squeezing assembly; an end of the connecting pipe is configured to be in communication with the reagent pack, and another end of the connecting pipe is configured to be in communication with the recovery pack;
where the squeezing assembly is capable of being docked with and of being separated from an output shaft of a drive assembly; in condition of the squeezing assembly and the output shaft being docked, the output shaft of the drive assembly is configured to drive the squeezing assembly to squeeze the connecting pipe to cause liquid in the connecting pipe to flow.

In some embodiments, the squeezing assembly includes a first squeezing member and a second squeezing member, the second squeezing member being arranged coaxially with the first squeezing member and being embedded in the first squeezing member, and a part of the connecting pipe being arranged between the first squeezing member and the second squeezing member; the second squeezing member is configured to be transmission-assembled with the output shaft, and the second squeezing member is configured to be driven by the output shaft to rotate relative to the first squeezing member and thereby squeeze the connecting pipe.

In some embodiments, the first squeezing member is annular in shape, and the second squeezing member includes a rotating portion and a squeezing portion; the rotating portion is configured to be docked with the output shaft, and the squeezing portion is disposed on a peripheral side of the rotating portion and rotatable synchronously with the rotating portion;
the squeezing portion is arranged spaced apart from the first squeezing portion, and the connecting pipe is partially arranged between the squeezing portion and the first squeezing portion.

In some embodiments, the rotating portion defines a shaft hole, and an end of the output shaft is assembled with the shaft hole; the squeezing portion is sleeved with a shaft sleeve, the shaft sleeve being configured to abut against the connecting pipe.

In some embodiments, a cavity wall of the receiving cavity defines a storage groove, the squeezing assembly is embedded in the storage groove and arranged coaxially with the storage groove, and the connecting pipe is partially arranged between the storage groove and the squeezing assembly; the squeezing assembly is configured to be transmission-assembled with the output shaft, and the squeezing assembly is configured to be driven by the output shaft to rotate relative to the storage groove and thereby squeeze the connecting pipe.

In another aspect, the present disclosure provides a liquid drive apparatus, including:
a power assembly, a rotatable assembly, and a liquid pipe;
where one of the power assembly and the rotatable assembly includes a rotating shaft, and the other of the power assembly and the rotatable assembly includes a mating portion into which the rotating shaft is configured to be inserted;
one of the rotating shaft and the mating portion is arranged with a snap fastener, and the other of the rotating shaft and the mating portion defines a snap groove;
the liquid pipe is arranged in the rotatable assembly, and the rotatable assembly is rotatable to squeeze the liquid pipe;
where the power assembly is capable of being docked with and of being separated from the rotatable assembly, and the rotating shaft is configured to be inserted into the mating portion or pulled out of the mating portion; in condition of the rotating shaft being inserted into the mating portion, the rotating shaft is rotatable to engage the snap fastener and the snap groove to achieve a locking limit.

In some embodiments, the snap fastener is arranged on the rotating shaft, the snap groove is defined on the mating portion, and the snap fastener protrudes from a peripheral side of the rotating shaft in condition of no external force being applied; in condition of the rotating shaft being inserted into the mating portion, the snap fastener is restrained by the mating portion and is retracted in the rotating shaft; in condition of the rotating shaft rotating relative to the mating portion until the snap fastener is opposite the snap groove, the snap fastener protrudes from the peripheral side of the rotating shaft and snaps into the snap groove.

In some embodiments, the snap fastener is arranged on the mating portion, the snap groove is defined on the rotating shaft, and the snap fastener protrudes from the mating portion in condition of no external force being applied; in condition of the rotating shaft being inserted into the mating portion, the snap fastener is restrained by the rotating shaft and is retracted in the mating portion; in condition of the rotating shaft rotating relative to the mating portion until the snap fastener is opposite the snap groove, the snap fastener protrudes from the mating portion and snaps into the snap groove.

In some embodiments, an end of the snap fastener that engages with the snap groove includes a guide ramp, and the guide ramp is configured to provide resistance in condition of the rotating shaft being inserted into the mating portion, causing the snap fastener to be retracted.

In some embodiments, an end of the rotating shaft includes a chamfer, and the chamfer is configured to guide the rotating shaft to be inserted into the mating portion.

In some embodiments, a mounting hole is defined on the rotating shaft or the mating portion, the snap fastener is assembled in the mounting hole, and an elastic member that abuts against the snap fastener is arranged in the mounting hole; the elastic member is configured to generate an elastic force in condition of the snap fastener being retracted; in condition of the snap fastener being opposite the snap groove, the elastic force causes the snap fastener to extend to snap with the snap groove.

In some embodiments, a first limit portion is arranged in the mounting hole, and the snap fastener is arranged with a second limit portion; the first limit portion and the second limit portion are configured to cooperate to limit an extension travel of the snap fastener.

In some embodiments, a first gear is sleeved on the rotating shaft, and the power assembly includes a drive member and a second gear, the second gear being transmission-assembled with an output shaft of the drive member; the first gear is connected to the second gear by meshing, and a thickness of the first gear along an axial direction of the rotating shaft is less than a thickness of the second gear along the axial direction of the rotating shaft.

In some embodiments, the first gear is spaced from the drive member along the axial direction of the rotating shaft.

In some embodiments, the rotatable assembly includes a holding space for holding the liquid pipe and a window communicating with the holding space, with two ends of the liquid pipe extending out of the holding space through the window.

In another aspect, the present disclosure provides a reagent kit, including:
a cavity, a liquid pipe, and a rotatable assembly;
where the cavity is configured to hold a reagent pack and a recovery pack, the liquid pipe is arranged in the rotatable assembly, and the rotatable assembly is rotatable to squeeze the liquid pipe, causing liquid in the reagent pack to pass through the liquid pipe and reach the recovery pack;
the rotatable assembly is configured to capable of being docked with and of being separated from a power assembly; one of the power assembly and the rotatable assembly includes a rotating shaft, and the other of the power assembly and the rotatable assembly includes a mating portion into which the rotating shaft is configured to be inserted; one of the rotating shaft and the mating portion is arranged with a snap fastener, and the other of the rotating shaft and the mating portion defines a snap groove;
in condition of the rotatable assembly being docked with the power assembly, the rotating shaft is inserted into the mating portion, and the rotating shaft is rotatable to engage the snap fastener and the snap groove to achieve a locking limit.

In another aspect, the present disclosure provides a sample analysis apparatus, including:
a mounting bracket, including a first mounting position and a second mounting position, where the first mounting position is configured to mount a reagent kit, and the second mounting position is configured to mount a power assembly;
where the power assembly is capable of being docked with and of being separated from a rotatable assembly of the reagent kit; one of the power assembly and the rotatable assembly includes a rotating shaft, and the other of the power assembly and the rotatable assembly includes a mating portion into which the rotating shaft is configured to be inserted; one of the rotating shaft and the mating portion is arranged with a snap fastener, and the other of the rotating shaft and the mating portion defines a snap groove;
in condition of the rotatable assembly being docked with the power assembly, the rotating shaft is inserted into the mating portion, and the rotating shaft is rotatable to engage the snap fastener and the snap groove to achieve a locking limit.

In another aspect, the present disclosure provides a medical detection device, including:
a detection assembly, including a detection element, a first liquid path, and a first port, where the first liquid path is in communication with the first port, and the detection element is configured to detect liquid in the first liquid path to obtain a detection signal;
a processing circuit, detachably connected to the detection element of the detection assembly and configured to receive the detection signal in condition of being connected to the detection element; and
a reagent kit, including a cavity, a second liquid path, and a second port, where the cavity is configured to hold a liquid pack; an end of the second liquid path is configured to be in communication with the liquid pack, and another end of the second liquid path is in communication with the second port;
where the detection assembly and the reagent kit are capable of being docked and of being separated, causing the first port and the second port to be connected or separated; in condition of the first port and the second port being connected, the first liquid path is in communication with the second liquid path, causing reagent in the liquid pack to reach the first liquid path; in condition of the detection assembly and the reagent kit being separated to replace the reagent kit, the processing circuit keeps electrically connected to the detection element of the detection assembly.

In some embodiments, the medical detection device includes a mounting bracket, the mounting bracket including a first mounting position, a second mounting position, and a third mounting position; the first mounting position is configured to mount the detection assembly, the second mounting position is configured to place the reagent kit, and the processing circuit is arranged in the third mounting position;
the detection assembly and the processing circuit are movable synchronously relative to the reagent kit while maintaining an electrical connection, causing the detection assembly to be docked with or separated from the reagent kit; the reagent kit is replaceable in condition of the detection assembly and the reagent kit being separated.

In some embodiments, the mounting bracket includes a fixed bracket, a first drive mechanism, a first movable bracket, and a second movable bracket; the first movable bracket is arranged on the fixed bracket, and the first movable bracket is connected to the second movable bracket; the first movable bracket is configured to hold the detection assembly, and the second movable bracket is configured to assemble the processing circuit; the detection assembly and the processing circuit are configured to undergo a relative movement driven by the first drive mechanism, causing the detection element of the detection assembly to maintain electrically connected to or be disconnected from the processing circuit; the first drive mechanism is configured to drive the first movable bracket to move, causing the detection assembly to be docked with or separated from the reagent kit.

In some embodiments, the first movable bracket defines a storage groove for accommodating the detection assembly, the detection assembly is capable of being moved into and of being moved out of the storage groove, and the second movable bracket is elastically connected to the first movable bracket; in condition of the detection assembly being moved into the storage groove, the processing circuit is capable of moving towards the detection assembly and being electrically connected under drive of the first drive mechanism.

In some embodiments, the first movable bracket is elastically connected to the mounting bracket; under drive of the first drive mechanism, the detection assembly is movable towards the reagent kit to generate an elastic force and achieve communication between the first port and the second port; the detection assembly is movable away from the reagent kit by means of an elastic force to achieve separation between the first port and the second port.

In some embodiments, the mounting bracket includes a second drive mechanism and a liquid path selector; the second drive mechanism is arranged on the second movable bracket and configured to drive the liquid path selector to move; in condition of the detection assembly being docked with the reagent kit, the liquid path selector is driven by the second drive mechanism to enable the detection assembly to realize different liquid path circulations.

In some embodiments, the fixed bracket includes a cavity for accommodating the reagent kit, and a cavity wall of the cavity defines an avoidance opening; the detection assembly is arranged outside the cavity; in condition of the reagent kit being moved into the cavity, the second port of the reagent kit is exposed from the avoidance opening to achieve connection or separation with the first port of the detection assembly.

In some embodiments, a region in which the second port of the reagent kit is located forms a concave portion, the shape of the concave portion being adapted to the shape of the detection assembly.

In some embodiments, the reagent kit is arranged with a sampling element, a sample inlet of the sampling element being configured to selectively collect reagent from the liquid pack or liquid outside the reagent kit, and a sample outlet of the sampling element being configured to be in communication with the second port; in condition of the reagent from the liquid pack being is required to be collected, the sampling element forms a part of the second liquid path.

In some embodiments, the reagent kit further includes a first connecting pipe, an end of the first connecting pipe being in communication with the liquid pack and another end of the first connecting pipe being in communication with the sample inlet of the sampling element; the first connecting pipe forms another part of the second liquid path.

In some embodiments, the detection assembly further includes a third port spaced apart from the first port, and the third port is in communication with the first liquid path; the reagent kit further includes a fourth port and a second connecting pipe, and the fourth port is spaced apart from the second port; an end of the second connecting pipe is in communication with the fourth port, and another end of the second connecting pipe is in communication with the cavity; in condition of the first port and the second port being in communication, the third port and the fourth port are in communication.

In another aspect, the present disclosure provides a detection assembly, including:
a housing, a detection element and a first liquid path that are disposed in the housing, and a first port and a connection terminal that are disposed on the housing, where the connection terminal is electrically connected to the detection element;
where the first liquid path is in communication with the first port, and the detection element is configured to perform a blood gas detection on liquid in the first liquid path to obtain a detection signal; the first port is capable of being docked with and of being separated from a second port of a reagent kit for obtaining reagent of the reagent kit through the first port in condition of the first port being docked with the second port; in condition of the detection assembly and the reagent kit being separated to replace the reagent kit, the connection terminal remains electrically connected to a processing circuit of a medical detection device.

In some embodiments, the housing is arranged with a first positioning member, so as to position a bracket for mounting the detection assembly on the medical detection device.

In some embodiments, an end of the first port that is docked with the second port includes a tapered recess or tapered protrusion for guiding the second port to dock with the first port.

In another aspect, the present disclosure provides a reagent kit, including:
a cavity, a second liquid path, and a second port;
where the cavity is configured to hold a liquid pack; an end of the second liquid path is configured to be in communication with the liquid pack, and another end of the second liquid path is in communication with the second port; the second port is configured to be docked with a first port of a detection assembly in condition of a detection being performed using the reagent kit, and reagent in the liquid pack is configured to be delivered to the detection assembly through the second port; the second port is separated from the detection assembly before the detection is performed using the reagent kit; the reagent kit is capable of being moved into and of being moved out of a mounting bracket of a medical detection device, and in condition of the reagent kit being moved in, the first port and the second port are in communication.

In some embodiments, a region in which the second port of the reagent kit is located forms a concave portion, the shape of the concave portion being adapted to the shape of the detection assembly.

In some embodiments, the concave portion is arranged with a second positioning member for positioning the detection assembly.

In some embodiments, the reagent kit is arranged with a sampling element, a sample inlet of the sampling element being configured to selectively collect reagent from the liquid pack or liquid outside the reagent kit, and a sample outlet of the sampling element being configured to be in communication with the second port; in condition of the reagent from the liquid pack being required to be collected, the sampling element forms a part of the second liquid path.

In some embodiments, the detection assembly includes a third port spaced apart from the first port, and the reagent kit further includes a fourth port spaced apart from the second port; in condition of the first port and the second port being in communication, the third port and the fourth port are in communication.

In another aspect, the present disclosure provides a medical detection device, including:
a mounting bracket, including a first mounting position, a second mounting position, and a third mounting position, where the first mounting position is configured to mount a detection assembly, and the second mounting position is configured to mount a reagent kit; and
a processing circuit, arranged on the third mounting position and detachably connected to the detection assembly;
where the detection assembly and the reagent kit are capable of being docked and of being separated, and the detection assembly and the reagent kit are caused to be in communication or separated; in condition of the detection assembly and the reagent kit being in communication, reagent in the reagent kit is capable of reaching the detection assembly; in condition of the detection assembly and the reagent kit being separated to replace the reagent kit, the processing circuit remains electrically connected to the detection assembly.

In some embodiments, a processing circuit is arranged on the mounting bracket, the mounting bracket including a first mounting position, a second mounting position, and a third mounting position, where the first mounting position is configured to mount the detection assembly, the second mounting position is configured to hold the reagent kit, and the processing circuit is arranged on the third mounting position;
the detection assembly and the processing circuit is movable synchronously relative to the reagent kit while maintaining an electrical connection, causing the detection assembly to be capable of being docked with and of being separated from the reagent kit; the reagent kit is replaceable in condition of the detection assembly being separated from the reagent kit.

In some embodiments, the mounting bracket includes a fixed bracket, a first drive mechanism, a first movable bracket, and a second movable bracket; the first movable bracket is arranged on the fixed bracket, and the first movable bracket is connected to the second movable bracket; the first movable bracket is configured to hold the detection assembly, and the second movable bracket is configured to assemble the processing circuit; the detection assembly and the processing circuit are configured to undergo a relative movement driven by the first drive mechanism, causing the detection element of the detection assembly to maintain electrically connected to or be disconnected from the processing circuit; the detection assembly is configured to drive the first movable bracket to move under drive of the first drive mechanism, causing the detection assembly to be capable of being docked with and of being separated from the reagent kit.

In some embodiments, the first movable bracket defines a storage groove for accommodating the detection assembly, the detection assembly is capable of being moved into and of being moved out of the storage groove, and the second movable bracket is elastically connected to the first movable bracket; in condition of the detection assembly being moved into the storage groove, the processing circuit is capable of moving towards the detection assembly and being electrically connected under the drive of the first drive mechanism.

In some embodiments, the first movable bracket is elastically connected to the mounting bracket; under the drive of the first drive mechanism, the detection assembly is movable towards the reagent kit to generate an elastic force and achieve communication between a first port of the detection assembly and a second port of the reagent kit; the detection assembly is movable away from the reagent kit by means of an elastic force to achieve separation between the first port and the second port.

In some embodiments, the mounting bracket includes a second drive mechanism and a liquid path selector; the second drive mechanism is arranged on the second movable bracket and configured to drive the liquid path selector to move; in condition of the detection assembly being docked with the reagent kit, the liquid path selector is driven by the second drive mechanism to enable the detection assembly to realize different liquid path circulations.

In another aspect, the present disclosure provides a medical detection device, including:
a detection assembly, including a detection element, a first liquid path, and a first port, where the first liquid path is in communication with the first port, and the detection element is configured to detect liquid in the first liquid path to obtain a detection signal;
a reagent kit, including a cavity, a second liquid path, and a second port, where the cavity is configured to hold a liquid pack; an end of the second liquid path is configured to be in communication with the liquid pack, and another end of the second liquid path is in communication with the second port; and
a processing circuit, detachably connected to the detection element of the detection assembly for receiving the detection signal in condition of being connected to the detection element, where in condition of the processing circuit and the detection element being disassembled, the detection assembly is capable of being disassembled synchronously with the reagent kit;
where the detection assembly is detachably arranged on the reagent kit, and the first port and the second port are caused to be in communication or separated; in condition of the first port and the second port being in communication, the first liquid path is in communication with the second liquid path, and reagent in the liquid pack is capable of reaching the first liquid path.

In some embodiments, the medical detection device further includes a mounting bracket, and the mounting bracket includes a first mounting position for positioning the reagent kit; the reagent kit includes a second mounting position for mounting the detection assembly;
where the mounting bracket is arranged with an avoidance channel that avoids the second mounting position, and the detection element of the detection assembly is capable of protruding from the avoidance channel and being connected to the processing circuit.

In some embodiments, the mounting bracket further includes a fixed bracket, and a movable bracket and a drive mechanism that are arranged on the fixed bracket; the movable bracket is configured to place the processing circuit, and the detection assembly and the processing circuit are configured to undergo a relative movement under drive of the drive mechanism, causing the detection element of the detection assembly to come into contact or break contact with the processing circuit; in condition of the detection element breaking contact with the processing circuit, the detection assembly is capable of being disassembled synchronously with the reagent kit.

In some embodiments, a region in which the second port of the reagent kit located forms a concave portion, the shape of the concave portion being adapted to the shape of the detection assembly.

In some embodiments, the depth of the concave portion is greater than or equal to the thickness of the detection assembly.

In some embodiments, a side of the concave portion defines an avoidance notch, and the avoidance notch is configured to remove the detection assembly disposed on the concave portion.

In some embodiments, a peripheral portion of the concave portion defines a positioning groove in communication with the concave portion, and the positioning groove is configured to guide the processing circuit into contact with the detection element of the detection assembly.

In some embodiments, a region in which the second port of the reagent kit is located in formed with a first positioning portion, and the first positioning portion is configured to guide the detection assembly to be mounted on the second mounting position, thereby guiding the first port and the second port to dock with each other.

In some embodiments, the reagent kit is arranged with a sampling element, a sample inlet of the sampling element being configured to selectively collect reagent from the liquid pack or liquid outside the reagent kit, and a sample outlet of the sampling element being configured to be in communication with the second port; in condition of the reagent from the liquid pack being required to be collected, the sampling element forms a part of the second liquid path.

In some embodiments, the reagent kit further includes a first connecting pipe, an end of the first connecting pipe being in communication with the liquid pack and another end of the first connecting pipe being in communication with the sample inlet of the sampling element; the first connecting pipe forms another part of the second liquid path.

In some embodiments, the detection assembly includes a third port spaced apart from the first port, and the third port is in communication with the first liquid path; the reagent kit further includes a fourth port and a second connecting pipe, and the fourth port is spaced apart from the second port; an end of the second connecting pipe is in communication with the fourth port, and another end of the second connecting pipe is in communication with the; in condition of the first port and the second port being in communication, the third port and the fourth port are in communication.

In another aspect, the present disclosure provides a detection assembly, including:
a housing, a detection element and a first liquid path that are disposed in the housing, and a first port and a connection terminal that are disposed on the housing, where the connection terminal is electrically connected to the detection element;
where the first liquid path is in communication with the first port, and the detection element is configured to perform a detection on liquid in the first liquid path to obtain a detection signal; the first port is capable of being docked with and of being separated from a second port of a reagent kit for obtaining reagent of the reagent kit through the first port in condition of the first port being docked with the second port; the detection assembly is configured to be arranged on the reagent kit, and the connection terminal is configured to be electrically connected to a processing circuit of a medical detection device.

In some embodiments, a region in which the second port of the reagent kit is located in formed with a first positioning portion, and the housing is arranged with a second positioning portion; the first positioning portion and the second positioning portion are configured to cooperate to guide the detection assembly to be mounted on the reagent kit.

In some embodiments, a region in which the second port of the reagent kit is located defines a positioning groove, and the positioning groove is configured to guide the processing circuit into contact with the detection element of the detection assembly.

In some embodiments, an end of the first port that is docked with the second port includes a tapered recess or tapered protrusion to guide the second port to dock with the first port.

In another aspect, the present disclosure provides a reagent kit, including:
a cavity, a second liquid path, and a second port;
where the cavity is configured to hold a liquid pack; an end of the second liquid path is configured to be in communication with the liquid pack, and another end of the second liquid path is in communication with the second port; the second port is configured to be docked with a first port of a detection assembly, and reagent in the liquid pack is capable of being delivered to the detection assembly through the second port in condition of the first port and the second port being docked; the detection assembly is detachably arranged on the reagent kit, and in condition of the reagent kit being disassembled, the detection assembly is capable of being disassembled simultaneously with the reagent kit.

In some embodiments, a region in which the second port of the reagent kit is located forms a concave portion, the shape of the concave portion being adapted to the shape of the detection assembly.

In some embodiments, the concave portion is arranged with a first positioning portion for positioning the detection assembly.

In some embodiments, the reagent kit is arranged with a sampling element, a sample inlet of the sampling element being configured to selectively collect reagent from the liquid pack or liquid outside the reagent kit, and a sample outlet of the sampling element being configured to be in communication with the second port; in condition of the reagent from the liquid pack being required to be collected, the sampling element forms a part of the second liquid path.

In some embodiments, the detection assembly includes a third port spaced apart from the first port, and the reagent kit further includes a fourth port spaced apart from the second port; in condition of the first port and the second port being in communication, the third port and the fourth port are in communication.

In another aspect, the present disclosure provides a medical detection device, including:
a mounting bracket, including a first mounting position for detachably mounting a reagent kit, where the reagent kit is arranged with a second mounting position for detachably mounting a detection assembly;
where the detection assembly and the reagent kit are capable of being docked and of being separated, and the detection assembly and the reagent kit are caused to be in communication or separated; in condition of the detection assembly and the reagent kit being in communication, reagent in the reagent kit is capable of reaching the detection assembly; in condition of the reagent kit being disassembled, the detection assembly is capable of being disassembled synchronously with the reagent kit.

In some embodiments, the medical detection device further includes a processing circuit; the mounting bracket is arranged with an avoidance passage that avoids the second mounting position, and a detection element of the detection assembly is capable of protruding from the avoidance passage and thereby achieving an electrical connection to the processing circuit.

In some embodiments, the mounting bracket further includes a fixed bracket, and a movable bracket and a drive mechanism that are arranged on the fixed bracket; the movable bracket is configured to place the processing circuit, and the detection assembly and the processing circuit are configured to undergo a relative movement under drive of the drive mechanism, causing the detection element of the detection assembly to maintain electrically connected to or be electrically disconnected from the processing circuit; in condition of the detection element being electrically disconnected from the processing circuit, the detection assembly is capable of being disassembled synchronously with the reagent kit.

In another aspect, the present disclosure provides a biological tissue detection apparatus, including:
a bracket, including a storage space and a first mounting position, where the first mounting position is configured to mount a sample measuring device, and a reagent kit is capable of being moved into and of being moved out of the storage space; the reagent kit is configured to convey liquid to the sample measuring device to assist the sample measuring device in sample measurement, and the sample measuring device is configured to measure the liquid flowing into the sample measuring device;
where the dimension of the receiving space in a moving direction of the reagent kit is larger than an intended placement dimension of the reagent kit, and the bracket is arranged with a limit portion and an elastic portion; the limit portion is configured to position the reagent kit, and the elastic member is configured to provide an elastic force in condition of the reagent kit being moved into the storage space; and the reagent kit is capable of retracting part of a moving travel and being restrained by the limit portion after being moved into the storage space.

In some embodiments, the bracket includes a plurality of plates that are interconnected to form the storage space and the first mounting position;
an avoidance window is arranged between the storage space and the first mounting position, and the avoidance window is configured to avoid mechanical docking operations and a liquid delivery path between the sample measuring device and the reagent kit, the liquid delivery path being a path connecting a first pipe of the sample measuring device and a second pipe of the reagent kit; the first pipe and the second pipe are delivery pipes for the liquid.

In some embodiments, the plurality of plates include a frame plate connected end to end and a mounting plate disposed on a side of the frame plate, the frame plate and the mounting plate are configured to cooperate to enclose for defining the storage space, and the first mounting position and the avoidance window are arranged on the frame plate; a side of the frame plate away from the mounting plate defines a pick-up and placement opening for the reagent kit to move in or out.

In some embodiments, the frame plate is arranged with at least one guide member, the at least one guide member being configured to guide the reagent kit to move into or out of the storage space.

In some embodiments, the elastic member is arranged on the mounting plate or the frame plate, and in condition of the reagent kit being moved into the storage space, the elastic member is compressed or stretched to generate an elastic force in a direction of the reagent kit moving out of the storage space.

In some embodiments, the limit portion is arranged on the frame plate and is arranged adjacent to the pick-up and placement opening in the direction of the reagent kit moving out of the storage space; the reagent kit is arranged with an engagement portion corresponding to the limit portion, and the engagement portion is configured to cooperate with the limit portion to position the reagent kit in condition of the reagent kit retracting part of the moving travel after being moved into the storage space.

In some embodiments, the mounting plate includes a first docking port and a second mounting position; the first docking port is in communication with the storage space, and the second mounting position is configured to mount a regulating device; in condition of the reagent kit being moved into the storage space, a sampling element on the reagent kit is exposed via the first docking port to dock with the regulating device, and the sampling element is capable of obtaining liquid inside or outside the reagent kit and causing the liquid to enter the second pipe, under regulation of the regulating device.

In some embodiments, the mounting plate further includes a second docking port and a third mounting position; the second docking port is in communication with the storage space, and the third mounting position is configured to mount a valve apparatus; in condition of the reagent kit being moved into the storage space, a liquid pack connector within the reagent kit is exposed via the second docking port to dock with the valve apparatus, and the valve apparatus is capable of controlling the liquid in the reagent kit to flow into the second pipe; in condition of the sampling element being docked with the reagent kit, the sampling element is in communication with a liquid outlet of the valve apparatus, and the sampling element is capable of obtaining the liquid in the reagent kit and causing the liquid to enter the second pipe.

In some embodiments, the mounting plate further includes a third docking port and a fourth mounting position; the third docking port is in communication with the storage space, and the fourth mounting position is configured to mount a drive apparatus; in condition of the reagent kit being moved into the storage space, the drive apparatus is docked with the reagent kit via the third docking port to drive the liquid in the second pipe to flow.

In some embodiments, the avoidance window extends through the first mounting position, and the first mounting position includes a positioning member for positioning the sample measuring device on the frame plate.

In another aspect, the present disclosure provides a biological tissue detection apparatus, including:
a bracket, including a storage space and a first mounting position;
a sample measuring device, detachably arranged on the first mounting position and including a first pipe; and
a reagent kit, capable of being moved into and of being moved out the storage space, where the reagent kit includes a second pipe;
where a dimension of the storage space in a moving direction of the reagent kit is larger than an intended placement dimension of the reagent kit; the bracket is arranged with a limit portion and an elastic member, the limit portion being configured to position the reagent kit, and the elastic member being configured to provide an elastic force for the reagent kit to move into the storage space, causing the reagent kit to retract a part of a movement travel and be restrained by the limit portion after being moved into the storage space.

In some embodiments, an avoidance window is arranged between the storage space and the first mounting position, and the avoidance window is configured to avoid mechanical docking operations and a liquid delivery path between the sample measuring device and the reagent kit, the liquid delivery path being a path connecting a first pipe of the sample measuring device and a second pipe of the reagent kit.

In some embodiments, the plurality of plates include a frame plate connected end to end and a mounting plate disposed on a side of the frame plate, the frame plate and the mounting plate are configured to cooperate to enclose for defining the storage space, and the first mounting position and the avoidance window are arranged on the frame plate; a side of the frame plate away from the mounting plate defines a pick-up and placement opening for the reagent kit to move in or out.

In some embodiments, the reagent kit is arranged with a sampling element, the biological tissue detection apparatus includes an regulating device, and the regulating device is configured to adjust a sampling posture of the sampling element; the mounting plate includes a first docking port and a second mounting position, the first docking port is in communication with the storage space, and the second mounting position is configured to mount the regulating device; in condition of the reagent kit being moved into the storage space, the sampling element is docked with the regulating device, and the sampling element is capable of obtaining liquid inside or outside the reagent kit and causing the liquid to enter the second pipe, under regulation of the regulating device.

In some embodiments, the reagent kit includes a receiving cavity for accommodating a liquid pack, and a cavity wall of the receiving cavity is arranged with a liquid pack connector; the biological tissue detection apparatus includes a valve apparatus for docking with the liquid pack connector to control an outflow of liquid from the liquid pack; the mounting plate includes a second docking port and a third mounting position; the second docking port is in communication with the storage space, and the third mounting position is configured to mount the valve apparatus; in condition of the reagent kit being moved into the storage space, the liquid pack connector is docked with the valve apparatus, and the valve apparatus is capable of controlling the liquid in the reagent kit to flow into the second pipe; in condition of the sampling element being docked with the reagent kit, the sampling element is in communication with a liquid outlet of the valve apparatus, and the sampling element is capable of obtaining the liquid in the reagent kit and causing the liquid to enter the second pipe.

In some embodiments, the reagent kit includes a receiving cavity for accommodating a liquid pack, and a cavity wall of the receiving cavity is arranged with a first drive member; the biological tissue detection apparatus includes a drive apparatus configured to be docked with the first drive member to drive an outflow of liquid from the liquid pack; the mounting plate includes a third docking port and a fourth mounting position; the third docking port is in communication with the storage space, and the fourth mounting position is configured to mount the drive apparatus; in condition of the reagent kit being moved into the storage space, the drive apparatus is docked with the first drive member.

In another aspect, the present disclosure provides a sample analysis apparatus, including:
a seat body, defining a storage groove and a pick-up and placement opening in communication with the storage groove, where a detection card is capable of being moved into and of being moved out of the storage groove from the pick-up and placement opening; at least one of the storage groove and detection card is arranged with a locking member, the locking member being configured to lock the detection card in the seat body in condition of the detection card being moved into the storage groove; at least one of the seat body and the detection card is arranged with an elastic member;
where in condition of the detection card being moved into the storage groove, the detection card squeezes or stretches the elastic member, causing the elastic member to deform and generate an elastic force; in condition of the locking member releasing locking and fixing of the detection card, the detection card pops up from the pick-up and placement opening under an action of the elastic force.

In some embodiments, an inner wall of the pick-up and placement opening is arranged with a damping portion, and the damping portion is configured to limit a pop-up travel of the detection card.

In some embodiments, the seat body is arranged with a drive member, and the drive member is configured to drive the locking member to release the locking and fixing of the detection card.

In some embodiments, the seat body defines a receiving groove, the receiving groove and the storage groove are arranged at intervals in a moving direction of the detection card, and the drive member is arranged in the receiving groove; an end of the locking member is assembled and connected to the drive member, and another end of the locking member is disposed in the storage groove to lock and fix the detection card in the storage groove.

In some embodiments, the seat body defines an avoidance space in communication with the storage groove and the receiving groove, and the locking member is at least partially disposed in the avoidance space; the locking member is rotatably connected to the seat body to rotate under drive of the drive member and thereby release the locking and fixing of the detection card.

In some embodiments, the elastic member is arranged in the receiving groove, and the seat body is arranged with a supporting member; an end of the supporting member is assembled and connected to the elastic member, and another end of the supporting member is configured to support the detection card; in condition of the detection card being moved into the storage groove, the detection card abuts against the supporting member, causing the elastic member to deform and generate the elastic force.

In some embodiments, the receiving groove is arranged with a limit portion, and the limit portion is configured to limit a movement travel of the supporting member.

In some embodiments, the elastic member is sleeved on the supporting member, and in condition of the detection card being moved into the storage groove, the detection card abuts against the supporting member, causing the elastic member to abut against the limit portion and generate the elastic force.

In some embodiments, a side of the detection card is arranged with a first port, a second port, and a snap-fit portion, and the storage groove includes an avoidance opening; in condition of the detection card being moved into the storage groove, the first port and the second port are exposed from the avoidance opening for communication to an external liquid path, and the snap-fit portion is locked and fixed with the locking member.

In some embodiments, the storage groove is arranged with at least one elastic tab in a region adjacent to the pick-up and placement opening, and the at least one elastic tab is configured to position the detection card in condition of the detection card being moved into the storage groove.

In another aspect, the present disclosure provides a fluid detection instrument, including:
a valve assembly, including a control element, a first pipe, a first inlet, and a first outlet, where the control element is configured to control the first inlet to be in communication with an end of the first pipe, and another end of the first pipe is in communication with the first outlet; and
a reagent kit, including a cavity, a second pipe, and a first port, where the cavity is configured to hold a reagent pack and a recovery pack, the first port is configured to connect the reagent pack to the first inlet, and the first outlet is in communication with a docking groove defined on the reagent kit; the docking groove is configured to be docked with a sampling element, an end of the second pipe being in communication with the docking groove, and another end of the second pipe being in communication with the recovery pack;
where the valve assembly and the reagent kit are capable of being docked and of being separated, and the first inlet and the first port are caused to be in communication or separated; in condition of the first inlet and the first port being in communication, the first pipe is configured to be in communication with the docking groove under control of the control element, and liquid in the reagent pack is capable of reaching the docking groove, and liquid in the docking groove is capable of reaching the recovery pack through the second pipe.

In some embodiments, the docking groove is arranged with a liquid inlet hole and a liquid outlet hole; the liquid inlet hole is configured to be in communication with the first outlet, and the liquid outlet hole is configured to be in communication with the second pipe.

In some embodiments, the docking groove is arranged with an isolation portion disposed between the liquid inlet hole and the liquid outlet hole, and a flow channel in communication with the liquid inlet hole and the liquid outlet hole and bypassing the isolation portion.

In some embodiments, the sampling element includes a sampling needle, a sleeve sleeved on the sampling needle, and a docking member arranged on an end of the sleeve; an outer diameter of the sleeve is less than or equal to an inner diameter of the docking groove, and the docking member defines a through hole; the docking member is movable along an axial direction of the sampling needle following the sleeve, causing the sampling needle to be inserted into an end of the through hole, or pass through the through hole and be exposed on a side of the docking member.

In some embodiments, the valve assembly further includes a third pipe, a third inlet, and a third outlet; the control element is configured to control the third inlet to be in communication with an end of the third pipe, and another end of the third pipe is in communication with the third outlet;
the reagent kit further includes a fourth pipe and a second port, the second port being configured to connect the third inlet to the reagent pack, and the third outlet being configured to be in communication with a sample inlet of the sampling element; an end of the fourth pipe is configured to be in communication with a sample outlet of the sampling element, and another end of the fourth pipe is configured to be in communication with a detection assembly of the fluid detection instrument.

In some embodiments, the docking groove is arranged with a through hole and a connecting pipe passing through the through hole; an end of the connecting pipe is in communication with the third outlet, and another end of the connecting pipe is configured to be in communication with the sample inlet of the sampling element; the connecting pipe is in sealed contact with a hole wall of the through hole; in condition of the sleeve moving along the axial direction of the sampling needle and towards the connecting pipe such that the docking member is inserted into the docking groove, the sampling needle and the connecting pipe are respectively inserted into both ends of the through hole to achieve communication.

In some embodiments, the valve assembly defines a first air hole and a second air hole; an end of the first pipe is selectively connected to the first inlet or the first air hole under control of the control element; an end of the third pipe is selectively connected to the third inlet or the second air hole under the control of the control element.

In another aspect, the present disclosure provides a valve assembly, including:
a control element, a first pipe, a first inlet, and a first outlet;
where the control element is configured to control the first inlet to be in communication with an end of the first pipe, and another end of the first pipe is in communication with the first outlet; the first inlet is capable of being docked with and of being separated from a first port of a reagent kit; in condition of the first port being docked with the first inlet, fluid in the reagent kit is capable of being obtained by controlling the first inlet through the control element; the first outlet is configured to be in communication with a docking groove of the reagent kit; in condition of the first outlet being docked with the docking groove, the liquid in the reagent kit is capable of passing through the first outlet to reach the docking groove, and liquid in the docking groove is capable of passing through a second pipe of the reagent kit to reach a recovery pack of the reagent kit.

In some embodiments, the valve assembly further includes a third pipe, a third inlet, and a third outlet; the control element is configured to control the third inlet to be in communication with an end of the third pipe, and another end of the third pipe is in communication with the third outlet;
the third inlet is capable of being docked with and of being separated from a second port of the reagent kit, and the third outlet is configured to be in communication with a sample inlet of a sampling element; in condition of the third outlet being docked with the sample inlet, the liquid in the reagent kit is capable of passing through the third outlet to reach the sampling element.

In some embodiments, the valve assembly defines a first air hole and a second air hole; an end of the first pipe is selectively connected to the first inlet or the first air hole under control of the control element; an end of the third pipe is selectively connected to the third inlet or the second air hole under the control of the control element.

In another aspect, the present disclosure provides a reagent kit, including:
a cavity, a second pipe, a first port, and a docking groove;
where the cavity is configured to hold a reagent pack and a recovery pack; an end of the second pipe is in communication with the docking groove, and another end of the second pipe is in communication with the recovery pack; the first port is capable of being docked with and of being separated from a first inlet of a valve assembly, and the docking groove is configured to be in communication with a first outlet of the valve assembly; in condition of the first port being docked with the first inlet, liquid in the reagent pack is capable of being delivered to the valve assembly through the first port, and liquid in the docking groove is capable of reaching the recovery pack through the second pipe.

In some embodiments, the docking groove is arranged with a liquid inlet hole and a liquid outlet hole; the liquid inlet hole is configured to be in communication with the first outlet, and the liquid outlet hole is configured to be in communication with the second pipe.

In some embodiments, the docking groove is arranged with an isolation portion disposed between the liquid inlet hole and the liquid outlet hole, and a flow channel in communication with the liquid inlet hole and the liquid outlet hole and bypassing the isolation portion.

In some embodiments, the reagent kit further includes a fourth pipe and a second port, the second port being configured to connect a third inlet of the valve assembly to the reagent kit, and the third outlet being configured to be in communication with a sample inlet of a sampling element; an end of the fourth pipe is configured to be in communication with a sample outlet of the sampling element, and another end of the fourth pipe is configured to be in communication with a detection assembly of a fluid detection instrument.

In some embodiments, the docking groove is arranged with a through hole and a connecting pipe passing through the through hole; an end of the connecting pipe is in communication with the third outlet, and another end of the connecting pipe is configured to be in communication with the sample inlet of the sampling element; the connecting pipe is in sealed contact with a hole wall of the through hole.

In some embodiments, the sampling element includes a sampling needle, a sleeve sleeved on the sampling needle, and a docking member arranged on an end of the sleeve; an outer diameter of the sleeve is less than or equal to an inner diameter of the docking groove, and the docking member defines another through hole; the docking member is movable along an axial direction of the sampling needle following the sleeve, causing the sampling needle to be inserted into an end of the through hole on the docking member, or pass through the through hole on the docking member and be exposed on a side of the docking member;
in condition of the sleeve moving along the axial direction of the sampling needle and towards the connecting pipe such that the docking member is inserted into the docking groove, the sampling needle and the connecting pipe are respectively inserted into both ends of the through hole to achieve communication.

In another aspect, the present disclosure provides a liquid pack, including a body and a connector, where the body is configured to contain liquid, and the connector is configured to control the liquid to flow from the liquid pack through the connector or to seal the body;
where the connector includes a pipe in communication with an internal space of the body, and a seal disposed in the pipe; the seal is movable in the pipe under an external force to open the pipe, and return to an initial position to seal the pipe in a case where the external force is removed.

In some embodiments, a first abutting portion is arranged in the pipe, and the seal is arranged with a second abutting portion; in condition of the seal being in the initial position, the first abutting portion abuts against the second abutting portion to seal the pipe; the seal is configured to move within the pipe under the external force to separate the first abutting portion and the second abutting portion, so as to open the pipe.

In some embodiments, the pipe includes a first pipe and a second pipe that are in communication; an end of the first pipe is in communication with the body, and another end of the first pipe is in communication with the second pipe, an inner diameter of the first pipe being greater than an inner diameter of the second pipe to form the first abutting portion, that is stepped in shape, at a connection between the first pipe and the second pipe; a gap exists between the seal and the first pipe, and the second abutting portion is configured to seal an end of the second pipe connected to the first pipe.

In some embodiments, an outer diameter of the seal is less than the inner diameter of the first pipe, and an outer diameter of the seal is greater than the inner diameter of the second pipe; the seal in the initial position is disposed in the first pipe, and the second abutting portion is formed by an end of the seal near the second pipe to abut against the first abutting portion.

In some embodiments, the seal includes a sealing portion and a guiding portion; an outer diameter of the sealing portion is greater than an outer diameter of the guiding portion, the outer diameter of the sealing portion is less than the inner diameter of first pipe, and the outer diameter of the sealing portion is greater than the inner diameter of the second pipe; in condition of the seal being in the initial position, the sealing portion is disposed in the first pipe, and an end of the seal close to the second pipe forms the second abutting portion to abut against the first abutting portion, and the guiding portion is disposed in the second pipe and has a gap with the second pipe; the guiding portion is configured to receive the external force.

In some embodiments, an end of the first pipe away from the second pipe includes a limit portion, and the limit portion is configured to limit entry of the sealing portion into the body.

In some embodiments, the connector includes a docking port, the docking port being arranged on an end of the second pipe away from the first pipe and communicating with the second pipe; the docking port is configured to guide an external connector into the pipe to exert a force on the seal.

In some embodiments, a sealing ring, which is annular in shape, is arranged in the docking port; an end of the guiding portion is at least partially exposed from the liquid pack from an annular hollow portion of the sealing ring; the external connector is capable of entering the pipe from the annular hollow portion of the sealing ring to exert the force on the seal; in condition of the external connector abutting against the guiding portion and pushing the guiding portion to move towards the first pipe, the sealing ring seals a gap between the external connector and the docking port.

In some embodiments, the sealing ring includes a first sealing ring and a second sealing ring, the first sealing ring being arranged between the guiding portion and the second sealing ring; the external connector is configured to exert a force on the guiding portion through the first sealing ring and the second sealing ring.

In some embodiments, the first sealing ring and the second sealing ring are arranged coaxially; an outer diameter of the first sealing ring is less than an outer diameter of the second sealing ring.

In some embodiments, the first sealing ring and the second sealing ring are both elastomers, and the external connector is configured to enter annular hollow portions of the first sealing ring and the second sealing ring to exert the force on the seal;
the external connector is sealed and assembled with at least one of the first sealing ring and the second sealing ring when exerting the force on the seal.

In some embodiments, an end of the second sealing ring away from the first sealing ring includes a guide ramp, and the guide ramp is configured to guide the external connector into the seal.

In another aspect, the present disclosure provides an integrated reagent kit, including:
a case, and a sampling assembly disposed on the case;
where the sampling assembly includes a rotary member rotatably connected to the case and a sampling member connected to the rotary member;
the rotary member is capable of changing to a state under an action of a first external force to drive the sampling member to rotate synchronously relative to the case, and of returning to an original state in a case where the first external force is removed;
where the sampling member includes a sampling needle and a sleeve sleeved on the sampling needle; the sleeve is capable of changing to a state under an action of a second external force to move relative to the rotary member to expose the sampling needle, and of returning to an original state to cover the sampling needle in a case where the second external force is removed.

In some embodiments, the rotary member includes a first rotary member and a second rotary member connected to each other, the first rotary member being movably connected to the sleeve in an axial direction of the sleeve, the second rotary member being rotatably connected to the case, and the first rotary member being configured to take the first external force.

In some embodiments, the integrated reagent kit further includes a first elastic member connecting the first rotary member and the sleeve; the first elastic member changes a state to generate an elastic force in condition of the sleeve being moved under force, and in a case where the force is removed from the sleeve, the first elastic member restores the state under an action of an elastic force to restore the sleeve to the original state.

In some embodiments, the sampling needle includes a sampling portion and a sample discharge portion connected by a bend; the sleeve is arranged on the sampling portion and movable along an axial direction of the sampling portion; the sample discharge portion extends from an end of the sampling portion to the second rotary member or extends and passes through the second rotary member; the sleeve defines an avoidance slot to avoid the sample discharge portion in condition of the sleeve being moved axially.

In some embodiments, an end of the sleeve adjacent to a sample inlet of the sampling portion includes a bulge; the bulge is pushed to move the sleeve under the action of the second external force exerted by an external container, in condition of the sampling portion obtaining liquid from the external container.

In some embodiments, the sleeve passes through the first rotary portion, and an end of the sleeve is connected to the first rotary portion through the first elastic member.

In some embodiments, the integrated reagent kit further includes a second elastic member arranged between the second rotary member and the case, and the rotary member changes a state of the second elastic member to generate an elastic force in condition of the rotary member rotating relative to the case under the action of the first external force; in a case where the first external force is removed, the second elastic member restores the state of the rotary member under an action of the elastic force.

In some embodiments, the case defines a rotating groove, the second rotary member is inserted in the rotating groove, the second elastic member is sleeved on the second rotary member, and an end of the second elastic member abuts against the rotating groove; the sampling needle is capable of being in communication with a pipe on the case through the rotating groove.

In some embodiments, the case is arranged with an assembly portion, the assembly portion being arranged in a corner region of the case; the rotating groove is defined on the assembly portion, and the second rotary member is inserted into the rotating groove; in condition of the sampling assembly being rotated, a sample inlet of the sampling assembly is capable of being unscrewed out of the corner region of the case.

In another aspect, the present disclosure provides a reagent kit that is easy to clean, including:
a case, a first pipe, a second pipe, and a docking groove, where a reagent pack and a recovery pack are arranged in the case; an end of the first pipe is in communication with the docking groove, and another end of the first pipe is in communication with the reagent pack; an end of the second pipe is in communication with the docking groove, and another end of the second pipe is in communication with the recovery pack;
where the docking groove is capable of being docked with and of being separated from a sampling element; in condition of the docking groove being docked with the sampling element, liquid in the reagent pack is capable of reaching the docking groove through the first pipe for cleaning a sample inlet of the sampling element, and liquid after the cleaning is capable of reaching the recovery pack through the second pipe.

In some embodiments, the docking groove includes a liquid inlet in communication with the first pipe and a liquid outlet in communication with the second pipe; in condition of the docking groove being docked with the sampling element, the sample inlet of the sampling element seals the docking groove and cooperates with the docking groove to define a cavity, and the liquid inlet and the liquid outlet are respectively in communication with the cavity.

In some embodiments, the docking groove includes a first groove section and a second groove section that are in communication; the first groove section is away from a bottom of the docking groove relative to the second groove section; the first groove section is capable of being docked with and of being separated from the sampling element, and the second groove section includes the liquid inlet and the liquid outlet; an inner diameter of the first groove section is greater than an inner diameter of the second groove section, the inner diameter of the first groove section is greater than an outer diameter of the sample inlet of the sampling element, and the inner diameter of the second groove section is less than the outer diameter of the sample inlet of the sampling element.

In some embodiments, the sampling element includes a sleeve, and a sampling needle and a docking head that are arranged in the sleeve, the docking head defining a through hole; the docking head is embedded in an end of the sleeve, and a sample inlet of the sampling needle is inserted in the through hole; in condition of the docking groove being docked with the sampling element, the sleeve abuts against the first groove section, and the docking head abuts against the second groove section, the cavity being defined between the docking head and the second groove section.

In some embodiments, the second groove section includes an open end and a narrow end that are oppose to each other, an inner diameter of the open end being greater than an inner diameter of the narrow end; the inner diameter of the open end is greater than an outer diameter of the docking head and less than an outer diameter of the sleeve.

In some embodiments, the inner diameter of the narrow end is less than or equal to an inner diameter of the sleeve.

In some embodiments, the reagent kit further includes a third pipe partially disposed in the docking groove; in condition of the docking groove being docked with the sampling element, the docking head is inserted into the second groove section, and the third pipe is inserted into the docking head to communicate with the sampling needle.

In some embodiments, an end of the docking head abutting against the second groove section includes a groove, and the through hole is in communication with a bottom wall of the groove; the docking head is in sealed contact with a sidewall of the second groove section, and the groove and the second groove section enclose to define the cavity where the sample inlet is configured to be cleaned.

In some embodiments, the second groove section is arranged with an isolation portion, and the liquid inlet and liquid outlet are disposed on opposite sides of the isolation portion and are respectively in communication with the cavity; the liquid in the reagent pack is capable of reaching the cavity from the liquid inlet, so as to clean the sample inlet of the sampling element; the liquid after the cleaning is configured to flow out of the cavity from the liquid outlet and reach the recovery pack through the second pipe.

In some embodiments, the sampling element includes a sleeve 5305a, and a sampling needle and a docking connector that are arranged in the sleeve, the docking head defining a through hole; the docking head is embedded in an end of the sleeve, and a sample inlet of the sampling needle is inserted in the through hole; an inner wall of the sleeve includes a step portion, and the docking head includes an engagement portion; the engagement portion is disposed on a side of the step portion away from a bottom wall of the docking groove and abuts against the step portion, and the docking head is movable axially along with the sleeve.

In another aspect, the present disclosure provides a sample analysis device, including:
a reagent kit, including a sampling assembly, a regulating assembly, and a movable assembly, where the regulating assembly is configured to drive the sampling assembly to rotate relative to the reagent kit; the movable assembly is arranged facing the sampling assembly; the movable assembly is capable of being docked with and of being separated from the sampling assembly;
where the movable assembly is movable between a first position and a second position relative to the sampling assembly; the movable assembly is docked with the sampling assembly in the first position, and the movable assembly is separated from the sampling assembly in the second position;
the first position corresponds to that the sampling assembly is subjected to positional restriction such that the sampling assembly is capable of collecting liquid inside the reagent kit; the second position corresponds to that positional restriction on the sampling assembly is cancelled such that the regulating assembly is capable of driving the sampling assembly to rotate, and the sampling assembly is capable of collecting liquid outside the reagent kit.

In some embodiments, the reagent kit further includes a case for holding a liquid pack, and the case is arranged with a first port and a second port; the first port is configured to be docked with a detection assembly, and the second port is configured to be docked with the liquid pack; the movable assembly includes a third port; an end of the sampling assembly is in communication with the first port through a first liquid path, and another end of the sampling assembly is capable of being docked with and of being separated from the third port, the second port being in communication with the third port through a second liquid path.

In some embodiments, the regulating assembly includes a seat body and a shaft portion disposed on the seat body; the sampling assembly includes a sampling element; an end of the sampling element is at least partially disposed in the seat body and is in communication with the first port through the first liquid path, and another end of the sampling element is disposed outside the seat body for selectively docking with or separating from the third port; the shaft portion is docked with the case and is rotatable relative to the case.

In some embodiments, the sampling assembly further includes a sleeve sleeved on the sampling element; the sleeve is movable along an axial direction of the sampling element; the sleeve defines an avoidance slot to avoid the first liquid path or the sampling element in condition of the sleeve being moved.

In some embodiments, the sleeve includes a bulge on an end adjacent to the movable assembly, and the bulge is pushed to move the sleeve under an action of a force exerted by an external container in condition of the sampling element obtaining liquid from the external container.

In some embodiments, the sampling assembly further includes a first elastic member assembled between the sleeve and the seat body; the first elastic member changes a state to generate an elastic force in condition of the sleeve being moved under the action of the force; in a case where the force on the sleeve is removed, the first elastic member restores the state under an action of the elastic force, causing the sleeve to reset and cover a sample inlet of the sampling element.

In some embodiments, the sampling assembly further includes a second elastic member assembled between the regulating assembly and the case, and the regulating assembly changes a state of the second elastic member to generate an elastic force in condition of rotating relative to the case under an action of a force; in a case where the force on the regulating assembly is removed, the second elastic member returns to an original state under an action of the elastic force to reset the regulating assembly.

In some embodiments, the seat body is arranged with a docking portion configured to be docked with a first drive component of the sample analysis device for transmission fit; the movable assembly is capable of being docked with and of being separated from the sampling element under drive of a second drive component of the sample analysis device, in condition of the movable assembly being docked with the second drive component.

In some embodiments, the sample analysis device further includes a mounting bracket; the mounting bracket includes a cavity, a first mounting position, and a second mounting position; the reagent kit is capable of being moved into and of being moved out of the cavity, the first mounting position is configured to mount the first drive component, and the second mounting position is configured to mount the second drive component;
where in condition of the reagent kit being moved into the cavity, the docking portion is docked with the first drive component and the movable assembly is docked with the second drive component.

In some embodiments, the first drive component includes a first power member arranged on the first mounting position, and the docking portion of the seat body defines a shaft hole; in condition of the docking portion and the first drive component being docked, an output shaft of the first drive component cooperates with the shaft hole to achieve a transmission fit between the docking portion and the first drive component.

In some embodiments, the movable assembly includes a docking seat that is movable relative to the case; an end of the docking seat forms the third port, and another end of the docking seat is configured to be docked with the second drive component; in condition of the reagent kit being moved into the cavity, the docking base is docked with the second drive component to move under drive of the second drive component.

In some embodiments, the second drive component includes a second power member arranged on the second mounting position, and a clamping member cooperating with an output shaft of the second power member, and the second power member is configured to drive the clamping member to move;
where in condition of the reagent kit being moved into the cavity, the clamping member is clamped and connected with the docking base, causing the second power member to synchronously drive the docking base to move while driving the clamping member to move.

In another aspect, the present disclosure provides a biological sample analysis device, including:
a drive assembly, including an output shaft; and
a reagent storage apparatus, including a receiving cavity, a first connecting pipe, a second connecting pipe, and a squeezing assembly, where the receiving cavity is configured to hold a reagent pack, a cleaning pack, and a recovery pack; the first connecting pipe and the second connecting pipe are partially embedded in the squeezing assembly; an end of the first connecting pipe is configured to be in communication with the reagent pack, and another end of the first connecting pipe is configured to be in communication with the recovery pack; an end of the second connecting pipe is configured to be in communication with the cleaning pack, and another end of the second connecting pipe is configured to be in communication with the recovery pack;
where the drive assembly and the reagent storage apparatus are capable of being docked and of being separated, and the output shaft is caused to be docked with or separated from the squeezing assembly; in condition of the output shaft being docked with the squeezing assembly, the output shaft is capable of driving the squeezing assembly to synchronously squeeze the first connecting pipe and the second connecting pipe, and liquid in the first connecting pipe or the second connecting pipe is capable of flowing.

In some embodiments, the biological sample analysis device further includes a mounting bracket, the mounting bracket including a cavity and a first mounting position; the reagent storage apparatus is capable of being moved into and of being moved out of the cavity, and the drive assembly is arranged on the first mounting position; the first mounting position is arranged on an end of a travel of the reagent storage apparatus moving into the cavity.

In some embodiments, the drive assembly is arranged outside the cavity; an avoidance hole is defined between the cavity and the first mounting position, and the output shaft of the drive assembly extends from the avoidance hole into the cavity; in condition of the reagent storage apparatus being moved into the cavity, the output shaft of the drive assembly is transmission-assembled with the squeezing assembly.

In some embodiments, the squeezing assembly includes a first squeezing member and a second squeezing member, the second squeezing member being embedded in the first squeezing member and arranged coaxially with the first squeezing member; the first connecting pipe and the second connecting pipe are partially arranged between the first squeezing member and the second squeezing member; the second squeezing member is configured to be transmission-assembled with the output shaft, so as to rotate relative to the first squeezing member under drive of the output shaft and thereby squeeze the first connecting pipe and the second connecting pipe.

In some embodiments, the first squeezing member is annular in shape, and the second squeezing member includes a rotary portion and a squeezing portion; the rotary portion is configured to be docked with the output shaft, and the squeezing portion is arranged on a peripheral side of the rotary portion and rotatable synchronously with the rotary portion;
where the squeezing portion is arranged spaced apart from the first squeezing member, and the first connecting pipe and the second connecting pipe are partially arranged between the squeezing portion and the first squeezing member.

In some embodiments, the rotary portion defines a shaft hole; an end of the output shaft is assembled with the shaft hole, and the first connecting pipe and the second connecting pipe are arranged along an axial direction of the shaft hole on a position between the squeezing portion and the first squeezing member.

In some embodiments, the squeezing portion is arranged with a bushing, the bushing is arranged with a spacer, and the spacer is disposed between the first connecting pipe and the second connecting pipe.

In some embodiments, a cavity wall of the receiving cavity defines a receiving groove, the squeezing assembly is embedded in the receiving groove and arranged coaxially with the receiving groove, and the first connecting pipe and the second connecting pipe are partially arranged between the receiving groove and the squeezing assembly; the squeezing assembly is configured to be transmission-assembled with the output shaft, so as to rotate relative to the storage groove under drive of the output shaft and thereby squeeze the first connecting pipe and the second connecting pipe.

In another aspect, the present disclosure provides a reagent storage apparatus, including:
a receiving cavity, a first connecting pipe, a second connecting pipe, and a squeezing assembly;
where the receiving cavity is configured to hold a reagent pack, a cleaning pack, and a recovery pack; the first connecting pipe and the second connecting pipe are partially embedded in the squeezing assembly; an end of the first connecting pipe is configured to be in communication with the reagent pack, and another end of the first connecting pipe is configured to be in communication with the recovery pack; an end of the second connecting pipe is configured to be in communication with the cleaning pack, and another end of the second connecting pipe is configured to be in communication with the recovery pack;
the squeezing assembly is capable of being docked with and of being separated from the output shaft of the drive assembly; in condition of the squeezing assembly being docked with the output shaft, the squeezing assembly is driven by the output shaft to synchronously squeeze the first connecting pipe and the second connecting pipe, and liquid in the first connecting pipe or the second connecting pipe is capable of flowing.

In some embodiments, the squeezing assembly includes a first squeezing member and a second squeezing member, the second squeezing member being embedded in the first squeezing member and arranged coaxially with the first squeezing member; the first connecting pipe and the second connecting pipe are partially arranged between the first squeezing member and the second squeezing member; the second squeezing member is configured to be transmission-assembled with the output shaft, so as to rotate relative to the first squeezing member under drive of the output shaft and thereby squeeze the first connecting pipe and the second connecting pipe.

In some embodiments, the first squeezing member is annular in shape, and the second squeezing member includes a rotary portion and a squeezing portion; the rotary portion is configured to be docked with the output shaft, and the squeezing portion is arranged on a peripheral side of the rotary portion and rotatable synchronously with the rotary portion;
where the squeezing portion is arranged spaced apart from the first squeezing member, and the first connecting pipe and the second connecting pipe are partially arranged between the squeezing portion and the first squeezing member.

In some embodiments, the rotary portion defines a shaft hole; an end of the output shaft is assembled with the shaft hole, and the first connecting pipe and the second connecting pipe are arranged along an axial direction of the shaft hole on a position between the squeezing portion and the first squeezing member.

In some embodiments, the squeezing portion is arranged with a bushing, the bushing is arranged with a spacer, and the spacer is disposed between the first connecting pipe and the second connecting pipe.

In some embodiments, a cavity wall of the receiving cavity defines a receiving groove, the squeezing assembly is embedded in the receiving groove and arranged coaxially with the receiving groove, and the first connecting pipe and the second connecting pipe are partially arranged between the receiving groove and the squeezing assembly; the squeezing assembly is configured to be transmission-assembled with the output shaft, so as to rotate relative to the storage groove under drive of the output shaft and thereby squeeze the first connecting pipe and the second connecting pipe.

In another aspect, the present disclosure provides a biological parameter analysis device, including:
a housing, arranged with a window and a door capable of covering the window;
a reagent kit, capable of being moved into and of being moved out of the housing, where the reagent kit includes a sampling assembly and a docking assembly; in condition of the reagent kit being arranged within the housing, the sampling assembly is capable of being unscrewed from the window to collect liquid outside the housing; the docking assembly is capable of being docked with the sampling assembly to enable the sampling assembly to collect liquid inside the reagent kit; and
a linkage assembly, coupled to the docking assembly and the door, where the door covers the window in condition of the sampling assembly being docked with the docking assembly, and the door opens the window in condition of the sampling assembly being separated from the docking assembly.

In some embodiments, the housing includes a shell and a bracket arranged in the shell; the window is arranged on the shell, and the door is arranged on an inner side of the housing; the bracket includes a cavity and a first mounting position, and the reagent kit is capable of being moved into and of being moved out of the cavity, the first mounting position being arranged on an end of a travel of the reagent kit moving into the cavity; the docking assembly is arranged facing the first mounting position in condition of the reagent kit being moved into the cavity; a part of the linkage assembly is assembled on the first mounting position, and another part of the linkage assembly is assembled on the shell.

In some embodiments, the bracket includes a frame plate and a mounting plate arranged on a side of the frame plate, and the frame plate and the mounting plate cooperate to define the cavity; an avoidance opening is defined between the frame plate and the mounting plate to avoid the linkage assembly and the sampling assembly; the avoidance opening is arranged opposite the door, and the linkage assembly is configured to drive the sampling assembly to be separated from the docking assembly while driving the door to open the window; a sample inlet of the sampling assembly is capable of being unscrewed from the window.

In some embodiments, the linkage assembly includes a drive member and a first linkage member; the drive member is arranged on the first mounting position, and the first linkage member is disposed on an output end of the drive member; the first linkage member is docked with the docking assembly in condition of the reagent kit being moved into the cavity; the drive member is configured to drive the first linkage member to move the docking assembly and to drive the first linkage member to move the door.

In some embodiments, the drive member and the first linkage member are arranged on opposite sides of the frame plate, and the drive member is arranged outside the cavity.

In some embodiments, the first linkage member includes a first docking portion for docking with the docking assembly and a first linkage portion for linkage with the door.

In some embodiments, the linkage assembly includes a second linkage assembly arranged on the shell; an end of the second linkage assembly is docked with the first linkage member, and another end of the second linkage assembly is docked with the door.

In some embodiments, the second linkage assembly includes a second linkage member and a transmission component that is transmission-connected to the second linkage member; the second linkage member is docked with the first linkage member to move relative to the shell under an action of the first linkage member; a moving direction of the second linkage member is perpendicular to a direction of opening or closing of the door.

In some embodiments, the transmission component includes a first transmission member which is transmission-connected to the second linkage member, a second transmission member which is transmission-connected to the first transmission member, a third transmission member which is transmission-connected to the second transmission member, and a fourth transmission member which is transmission-connected to the third transmission member; the fourth transmission member is assembled with the door;

where the second linkage member is configured to move to drive the first transmission member to rotate, and the second transmission member rotates synchronously following a rotation of the first transmission member; a rotation of the second transmission member drives the third transmission member to move, and the third transmission member moves to drive the fourth transmission member to rotate, causing the door to rotate synchronously with the fourth transmission member; a moving direction of the third transmission member is perpendicular to a moving direction of the second linkage member.

In some embodiments, the linkage assembly includes a seat body arranged on the shell, and the second linkage member is slidably connected to the seat body; one of the second linkage member and the seat body defines a travel groove, and the other of the second linkage member and the seat body is arranged with a limit block; the limit block is slidable in the travel groove to limit a sliding travel of the second linkage member relative to the seat body.

In some embodiments, a first elastic member is arranged between the second linkage member and the seat body; in condition of the second linkage member moving relative to the housing under an action of the first linkage member, the first elastic member changes a state to generate an elastic force; in a case where the action of the first linkage member is cancelled, the first elastic member returns to an original state, causing the second linkage member to return to an original position.

In some embodiments, the fourth transmission member is rotatably connected to the seat body, and a second elastic member is arranged between the fourth transmission member and the seat body; in condition of the third transmission member driving the fourth transmission member to rotate and thereby driving the door to open the window, the second elastic member changes a state to generate an elastic force; in condition of a force exerted by the third transmission member on the fourth transmission member being cancelled, the fourth transmission member drives the door to cover the window under an action of the elastic force.

In another aspect, the present disclosure provides a sample parameter analysis apparatus, including:
a housing, arranged with a window and a door that is capable of covering the window, where the housing includes a first side and a second side arranged opposite each other, and the door is arranged on the first side and is rotatably connected to the housing; and
a connecting assembly, arranged on the second side of the housing to connect the housing and the door in condition of the door covering the window, where the connecting assembly includes a clamping member, a drive member, and a first elastic member; an end of the first elastic 6402c is connected to a middle portion of the clamping member, and another end of the first elastic member is connected to the housing; an end of the clamping member is connected to the drive member, and another end of the clamping member is configured to limit a position of the door in condition of the door covering the window;
where the drive member is configured to drive the clamping member to release a restriction on the door, and the door is rotatable relative to the housing to open the window; the first elastic member deforms to generate an elastic force in condition of the clamping member moving, and the elastic force is configured to act on the clamping member to cause the clamping member to return to an original position in condition of the drive member cancelling a drive on the clamping member.

In some embodiments, the second side of the housing defines an engagement groove, and a side of the door close to the housing is arranged with an engagement portion; the engagement portion is capable of being moved into and of being moved out of the engagement groove; in condition of the engagement portion being moved into the engagement groove, the clamping member abuts against the engagement portion to limit the position of the door.

In some embodiments, an end of the clamping member is arranged with a protrusion, and the engagement portion defines a groove; in condition of the engagement portion being moved into the engagement groove, the protrusion is inserted into the groove to limit the position of the door.

In some embodiments, the engagement groove includes a first notch and a second notch that are disposed on adjacent sides of the engagement groove; the engagement portion is capable of being moved into and of being moved out of the first notch, and the protrusion is capable of being moved into and of being moved out of the second notch;
where in condition of the engagement portion moving from the first notch into the engagement groove, the engagement portion pushes the protrusion out of the second notch; the protrusion is capable of being moved into the engagement groove or the groove under an action of the elastic force of the first elastic member.

In some embodiments, at least one of the engagement portion and the protrusion is arranged with a guide ramp; the guide ramp is configured to guide the engagement portion to push the protrusion out of the second notch in condition of the engagement portion being moved into the engagement groove.

In some embodiments, the engagement groove includes a first side wall and a second side wall arranged at intervals, the first side wall and the second side wall being respectively connected to the housing; the clamping member is rotatable around the first side wall, and the protrusion is caused to be capable of being moved into and of being moved out of a space between the first side wall and the second side wall;
the clamping member is rotatable around the first side wall; an end of the clamping member that is arranged with the protrusion and another end of the clamping member that is connected to the drive member are arranged on both sides of the first side wall, and an end of the first elastic member is connected between the ends of the clamping member.

In some embodiments, the connecting assembly further includes a second elastic member arranged on the engagement groove; in condition of the engagement portion being moved into the engagement groove, the engagement portion squeezes or stretches the second elastic member, causing the second elastic member to deform and generate an elastic force; in condition of the drive member driving the clamping member to release the restriction on the door, the elastic force acts on the door to cause the door to rotate relative to the housing, so as to open the window.

In some embodiments, the engagement groove includes a third side wall spaced apart from the housing, the connecting assembly further includes a supporting member passing through the third side wall, and the second elastic member acts between the supporting member and the third side wall; the engagement portion pushes the supporting member to move in condition of the engagement portion being moved into the engagement groove to change a state of the second elastic member to generate the elastic force.

In some embodiments, the third side wall defines a storage groove, and the supporting member is arranged with a limit portion; an end of the supporting member is inserted into the storage groove to be connected to the second elastic member, and the limit portion is disposed in the storage groove to limit a movement travel of the limit portion in condition of the engagement portion pushing the supporting member to move.

In some embodiments, the connecting assembly further includes a limit member disposed on the second side of the housing, and the clamping member is disposed between the limit member and the housing.

In another aspect, the present disclosure provides a sample analysis box, including:
a seat body, including a first cavity, a second cavity, and an isolation member, where the isolation member is configured to cause the first cavity and the second cavity to communicate or isolate with each other, and in condition of the first cavity and the second cavity being in communication, liquid in the first cavity is capable of flowing to the second cavity;
where the isolation member is capable of changing to a state under an action of an external force to enable the first cavity and the second cavity to be in communication, and in a case where the external force is removed, the isolation member returns to another state where the first cavity and the second cavity are isolated.

In some embodiments, the seat body includes a first liquid port for communicating the first cavity with the second cavity; the isolation member is arranged on a cavity wall of the first cavity corresponding to the first liquid port and is configured to open or cover the first liquid port; the isolation member covers the first liquid port to isolate the first cavity from the second cavity in condition of no external force being applied to the isolation member; in condition of the external force being applied to the isolation member, the first liquid port is opened to allow the first cavity and the second cavity to be in communication through the first liquid port.

In some embodiments, the cavity wall of the first cavity is arranged with a squeezing member; the squeezing member abuts against the isolation member and is configured to apply a force to the isolation member, causing the isolation member to open the first liquid port.

In some embodiments, the first cavity includes a squeezing opening, and the squeezing member is sealed and assembled with the squeezing opening; the squeezing member extends into the first cavity from the squeezing opening to abut against the isolation member and apply the force to the isolation member.

In some embodiments, the isolation member includes a connecting portion and an isolation portion; the connecting portion is connected to the cavity wall of the first cavity; an end of the isolation portion is connected to the connecting portion, and another end of the isolation portion is configured to abut against the squeezing member; the isolation portion is movable relative to the connecting portion under the action of the external force to open the first liquid port, and in condition of the external force being removed, the isolation portion returns to a state that covers the first liquid port.

In some embodiments, at least one of the isolation portion and the connecting portion is arranged with an elastic member, the elastic member being disposed between the connecting portion and the isolation portion; the isolation portion is deformed under the action of the external force to deform the elastic member to generate an elastic force; in condition of the external force being removed, the elastic force causes the isolation portion to return to an original state.

In some embodiments, the isolation portion is arranged with a seal on a side close to the first liquid port; in condition of the isolation portion returning to an original state to cover the first liquid port, the seal is sealed and assembled with the first liquid port.

In some embodiments, the connecting portion is fixedly arranged on the cavity wall of the first cavity, and the isolation portion is rotatably connected to the connecting portion through a rotating shaft.

In some embodiments, the squeezing member includes an assembly portion and a squeezing portion, the assembly portion being configured to mount the squeezing member to the squeezing opening; the assembly portion is annular in shape, and the squeezing portion is arranged in an annular hollow region of the assembly portion and is elastically connected to the assembly portion; the assembly portion is sealed and assembled with the squeezing opening.

In some embodiments, the seat body includes a third cavity and a salt bridge; an end of the salt bridge is exposed from the second cavity, and another end of the salt bridge is exposed from the third cavity and connected to an electrode terminal in the third cavity.

In another aspect, the present disclosure provides a detection assembly, including:
a first liquid path, a second liquid path, a liquid inlet, a liquid outlet, and a valve assembly;
where an end of the first liquid path is configured to be in communication with the liquid inlet, and another end of the first liquid path is configured to be in communication with the liquid outlet; an end of the second liquid path is configured to be in communication with the liquid inlet, and another end of the second liquid path is configured to be in communication with the liquid outlet;
the valve assembly is arranged on the liquid inlet, and/or on the liquid outlet, and/or between the liquid inlet and the liquid outlet, and is capable of being switched between a first turn-on state and a second turn-on state to control the first liquid path and the second liquid path to be selectively connected;
in the first turn-on state, a first external liquid of the detection assembly is capable of flowing into the first liquid path from the liquid inlet and flowing out of the first liquid path from the liquid outlet;
in the second turn-on state, a second external liquid of the detection assembly is capable of flowing into the second liquid path from the liquid inlet and flowing out of the second liquid path from the liquid outlet.
In some embodiments, the first liquid path includes a first inlet and a first outlet, the second liquid path includes a second inlet and a second outlet, and the valve assembly includes a first valve and a second valve; the first inlet and the second inlet are configured to be in communication with the liquid inlet, and the first outlet and the second outlet are configured to be in communication with the liquid outlet; the first valve is arranged between the first inlet and the first outlet to control connection and disconnection of the first liquid path, and the second valve is arranged between the second inlet and the second outlet to control connection and disconnection of the second liquid path;
either the first valve or the second valve is selectively turned on.

In some embodiments, the valve assembly further includes a third valve, which is arranged between the liquid inlet and the second inlet and is configured to control opening and closing of the liquid inlet and the second inlet; either the third valve or the first valve is selectively turned on.

In some embodiments, the first liquid path includes a first liquid inlet section and a first liquid outlet section, the first liquid inlet section being in communication with the liquid inlet and the first liquid outlet section being in communication with the liquid outlet; the first valve is arranged between the first liquid inlet section and the first liquid outlet section to control connection and disconnection of the first liquid inlet section and the first liquid outlet section, and the third valve is arranged between the second inlet and the first liquid inlet section to control opening and closing of the second inlet and connection and disconnection of the first liquid inlet section.

In some embodiments, the detection assembly includes a housing and a plate; the plate defines a first through opening and a second through opening, and the housing includes a cavity; a cavity wall of the cavity defines a first fluid slot and a second fluid slot; the plate is disposed in the cavity; the first through opening is in communication with a part of the first fluid slot, and the second through opening is in communication with a part of the second fluid slot; the plate encloses and seals another part of the first fluid slot and another part of the second fluid slot to form the first liquid path and the second liquid path; the liquid inlet and the liquid outlet are respectively in communication with the cavity; the valve assembly is arranged outside the cavity, and the first valve is configured to control the connection and disconnection of the first liquid path, and the second valve is configured to control connection and disconnection of the second liquid path.

In some embodiments, the housing further includes a first housing and a second housing surrounding the first housing to define the cavity, and the first housing and the second housing cooperate to clamp the plate to form the first liquid path and the second liquid path;
the first housing defines a first through hole and a second through hole;
where the liquid inlet and the first inlet are arranged on both ends of the first through hole of the first housing, or the liquid inlet and the second inlet are arranged on both ends of the first through hole of the first housing; and/or,
where the first outlet and the liquid outlet are arranged on both ends of the second through hole of the first housing, or the second outlet and the liquid outlet are arranged on both ends of the second through hole of the first housing.

In some embodiments, the second housing includes a receiving cavity, the first housing is at least partially embedded in the receiving cavity and cooperates with the second housing to define the cavity, and the plate is disposed between the first housing and the second housing.

In some embodiments, a first groove is defined on the housing, and the housing is arranged with a first liquid inlet channel and a first liquid outlet channel that are in communication with the first groove; a second groove is defined on the housing, and the housing is arranged with a second liquid inlet channel and a second liquid outlet channel that are in communication with the second groove;
the first liquid path between the first inlet and the first outlet includes two first sub-liquid paths that are discontinuous, an end of the first liquid inlet channel being in communication with the first inlet, and an end of the first liquid outlet channel being in communication with the first outlet; the two first sub-liquid paths are in communication through the first groove, the first liquid inlet channel, and the first liquid outlet channel;
the second liquid path between the second inlet and the second outlet includes two second sub-liquid paths that are discontinuous, an end of the second liquid inlet channel being in communication with the second inlet, and an end of the second liquid outlet channel being in communication with the second outlet; the two second sub-liquid paths are in communication through the second groove, the second liquid inlet channel, and the second liquid outlet channel;
the first valve is an elastomer and disposed on the housing, and capable of abutting against the first groove under an action of an external force to block the first liquid inlet channel and the first liquid outlet channel, for disconnecting the first liquid path; the second valve is an elastomer and disposed on the housing, and capable of abutting against the second groove under an action of an external force to block the second liquid inlet channel and the second liquid outlet channel, for disconnecting the second liquid path.

In some embodiments, the housing is arranged with a third groove, a third valve, and a third liquid inlet channel and a third liquid outlet channel that are respectively in communication with the third groove;
the first liquid path and the second liquid path are discontinuous; an end of the third liquid inlet channel is in communication with the first inlet, and an end of the third liquid outlet channel is in communication with the second inlet; the first liquid path and the second liquid path are in communication through the third groove, the third liquid inlet channel, and the third liquid channel;
the third valve is an elastomer and capable of abutting against the third groove under an action of an external force to block the third liquid inlet channel and the third liquid outlet channel, for disconnecting the first liquid path from the second liquid path.

In some embodiments, the first valve, the second valve, and the third valve form an integrated structure.

The following describes a blood gas analysis device that is adopted with blood gas analysis technology. The blood gas analysis technology refers to a technical means applied in blood gas analysis devices to understand human body's respiratory function and acid-base balance by measuring the H⁺ concentration in the blood sample and the gases dissolved in the blood (mainly CO₂, O₂, etc.). It can directly reflect the lung's gas exchange function and its acid-base balance. The sample used is usually a blood sample.

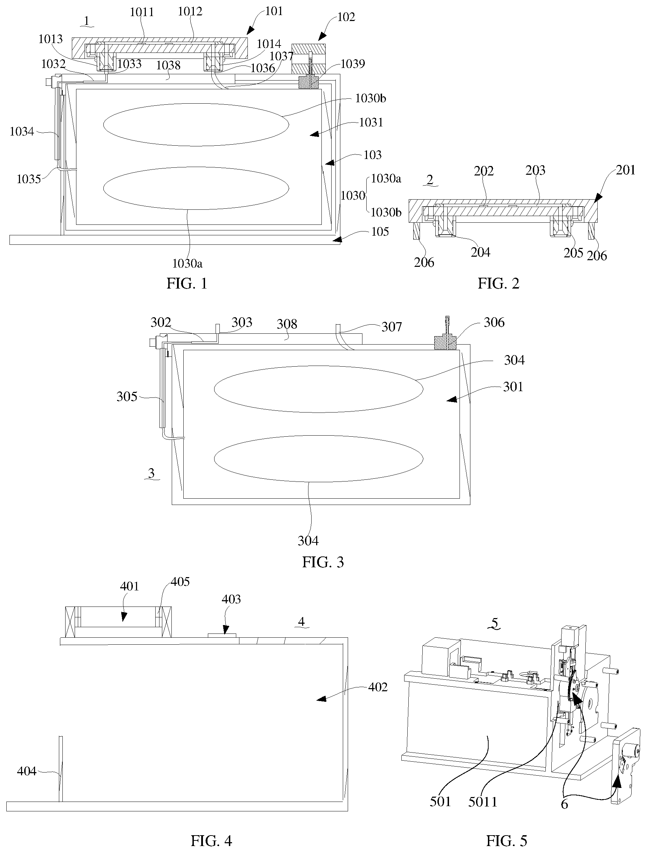

Referring to FIG. 1, FIG. 1 is a structural schematic view of a blood gas analysis device 1 according to some embodiments of the present disclosure. The blood gas analysis device 1 generally includes: a first detection assembly 101, which includes a detection element 1011, a first liquid path 1012, and a first port 1013, where the first liquid path 1012 and the first port 1013 are in communication, and the detection element 1011 is configured to perform a blood gas detection on liquid in the first fluid path 1012; and a reagent kit 103, including a cavity 1031, a second fluid path 1032, and a second port 1033, where the cavity 1031 is configured to hold a liquid pack 1030; an end of the second fluid path 1032 is configured to be in communication with the liquid pack 1030, and the other end of the second fluid path 1032 is configured to be in communication with the second port 1033.

The first detection assembly 101 and the reagent kit 103 can be docked as well as separated, such that the first port 1013 and the second port 1033 are in communication or separated. When the first detection assembly 101 and the reagent kit 103 are docked, the first liquid path 1012 is in communication with the second liquid path 1032, such that reagent in the liquid pack 1030 can reach the first liquid path 1012; when the reagent kit 103 is moved into a mounting bracket 105 of the blood gas analysis device 1, the first port 1013 and the second port 1033 are in communication.

The reagent kit 103 is arranged with a sampling element 1034, a sample inlet of the sampling element 1034 being configured to selectively collect the reagent from the liquid pack 1030 or a liquid external to the reagent kit 103, and a sample outlet of the sampling element 1034 being configured to be in communication with the second port 1033. When it is required to collect the reagent from the liquid pack 1030, the sampling element 1034 forms a part of the second liquid path 1032.

The reagent kit 103 includes a first connecting pipe 1035, an end of the first connecting pipe 1035 being in communication with the liquid pack 1030 and the other end of the first connecting pipe 1035 being in communication with the sample inlet of the sampling element 1034; the first connecting pipe 1035 forms another part of the second liquid path 1032.

The first detection assembly 101 includes a third port 1014 spaced apart from the first port 1013, and the third port 1014 is in communication with the first liquid path 1012; the reagent kit 103 includes a fourth port 1036 and a second connecting pipe 1037, and the fourth port 1036 is spaced apart from the second port 1033; an end of the second connecting pipe 1037 is in communication with the fourth port 1036, and the other end of the second connecting pipe 1037 is in communication with the cavity 1031; when the first port 1013 and the second port 1033 are in communication, the third port 1014 and the fourth port 1036 are in communication.

A docking base 1038 is arranged on an outer side of a cavity wall of the cavity 1031, the second port 1033 and the fourth port 1036 are arranged on the docking base 1038, and the second connecting pipe 1037 is partially arranged between the cavity wall of the cavity 1031 and the docking base 1038.

The liquid pack 1030 includes a reagent pack 1030a and a recovery pack 1030b disposed in the cavity 1031; an end of the first connecting pipe 1035 is configured to be in communication with the reagent pack 1030a, and an end of the second connecting pipe 1037 is configured to be in communication with the recovery pack 1030b.

The reagent kit 103 is arranged with a liquid channel 1039 that is exposed from the cavity 1031, and the liquid channel 1039 forms another part of the second liquid path 1032. The liquid flowing out of the first liquid path 1012 passes through the liquid channel 1039 and reaches the cavity 1031.

The blood gas analysis device 1 includes a second detection assembly 102 arranged on the mounting bracket 105, the second detection assembly 102 being configured to detect the liquid in the liquid channel 1039 exposed from the cavity 1031. The first detection assembly 101 is configured to perform the blood gas detection, and the second detection assembly 102 is configured to perform a blood oxygen detection.

It should be noted that the terms "first," "second," "third," etc. in this document are for descriptive purposes only and are not to be construed as indicating or implying relative importance or as implying that the indicated technical features are in any particular number. Accordingly, features that are defined with "first," "second," "third," etc. can expressly or implicitly include one or more of the features.

Referring to FIG. 2, FIG. 2 is a structural schematic view of a detection assembly 2 according to some embodiments of the present disclosure. The detection assembly 2 generally includes a housing 201, a detection element 202 and a first liquid path 203 that are disposed in the housing 201, and a first port 204 and a connection terminal that are disposed on the housing 201, the connection terminal being electrically connected to the detection element 202. The first liquid path 203 is in communication with the first port 204, and the detection element 202 is configured to perform a blood gas detection on the liquid in the first liquid path 203. The first port 204 is configured to dock with as well as separate from a second port of a reagent kit, such that when they are docked, reagent in the reagent kit can be obtained through the first port 204. Further, when they are docked, the connection terminal is configured to be electrically connected to a processing circuit of the blood gas analysis device 1.

An end of the first port 204 that docks with the second port includes a tapered recess or tapered protrusion to guide the second port 204 to dock with the first port. A third port 205 spaced apart from the first port 204 is arranged on the housing 201, and the third port 205 is in communication with the first liquid path 203.

The housing 201 is arranged with a positioning member 206 for positioning a bracket on the blood gas analysis device 1 for mounting the detection assembly 2.

Referring to FIG. 3, FIG. 3 is a structural schematic view of a reagent kit according to some embodiments of the present disclosure. The reagent kit 3 generally includes a cavity 301, a second liquid path 302, and a second port 303.

The cavity 301 is configured to hold a liquid pack 304. An end of the second liquid path 302 is in communication with the liquid pack 302, and the other end of the second liquid path 302 is in communication with to the second port 303. The second port 303 is configured to dock with as well as separate from a first port of a first detection assembly, such that when they are docked, the reagent in the liquid pack 304 is delivered to the first detection assembly through the second port 303. The reagent kit 3 can be moved into or out of a mounting bracket of the blood gas analysis device, and when the reagent kit 3 is moved in, the first port and the second port 303 are in communication.

A sampling element 305 is arranged on the reagent kit 3, and a sample inlet of the sampling element 305 is configured to selectively collect the reagent from the liquid pack 304 or liquid external to the reagent kit 3, and a sample outlet of the sampling element 305 is configured to be in communication with the second port 303. When it is required to collect the reagent from the liquid pack 304, the sampling element 305 forms a part of the second liquid path 302.

The reagent kit 3 is arranged with a liquid channel 306 that is exposed from the cavity 301, and the liquid channel 306 forms another part of the second liquid path 302. The liquid flowing out of the first detection assembly flows through the liquid channel 306 and reaches the cavity 301; the blood gas analysis device includes a second detection assembly arranged on a mounting bracket, the second detection assembly being configured to detect the liquid in the liquid channel 306 exposed from the cavity 301. The first detection assembly is configured to perform a blood gas detection, and the second detection assembly is configured to perform a blood oxygen detection.

The first detection assembly includes a third port spaced apart from the first port; the reagent kit 3 includes a fourth port 307 spaced apart from the second port 303; and the third port and the fourth port 307 are in communication, when the first port and the second port 303 are in communication.

A docking base 308 is arranged on an outer side of a cavity wall of the cavity 301, and the second port 303 and the fourth port 307 are arranged on the docking base 308.

Referring to FIG. 4, FIG. 4 is a structural schematic view of a mounting bracket 4 of a blood gas analysis device according to some embodiments of the present disclosure. The mounting bracket 4 generally includes a first mounting position 401 and a second mounting position 402. The first mounting position 401 is configured to detachably mount the first detection assembly, and the second mounting position 402 is configured to place the reagent kit.

The first detection assembly and the reagent kit can be docked as well as separated such that the first detection assembly and the reagent kit are in communication or separated. When the first detection assembly and the reagent kit are connected, the reagent in the reagent kit can reach the first detection assembly.

In particular, the mounting bracket 4 further includes a third mounting position 403, and the third mounting position 403 is configured to detachably mount the second detection assembly. The reagent kit is arranged with a liquid channel exposed from the cavity from the third mounting position, and the liquid flowing out of the first detection assembly passes through the liquid channel to reach the cavity. The second detection assembly is configured to detect the liquid in the liquid channel exposed from the cavity, the first detection assembly is configured to perform a blood gas detection, and the second detection assembly is configured to perform a blood oxygen detection.

The mounting bracket 4 includes a fixed bracket 404 and a movable bracket 405. The fixed bracket 404 has a cavity, the cavity forming the second mounting position 402. The movable bracket 405 has a receiving cavity, the receiving cavity forming the first mounting position 401. When the reagent kit is moved into the cavity, the movable bracket 405 can be moved relative to the fixed bracket 404 such that the first detection assembly can be docked with the reagent kit.

The movable bracket 405 can be moved relative to the fixed bracket in a first direction or a second direction; when the movable bracket 405 is moved in the first direction relative to the fixed bracket 404, the first detection assembly can move into or out of the movable bracket 405; when the movable bracket 405 is moved in the second direction relative to the fixed bracket 404, the first detection assembly can dock with or separate from the reagent kit.

The blood gas analysis device provided in the embodiments of the present disclosure can achieve a detachable assembly of the first detection assembly and the reagent kit by enabling the first detection assembly and the reagent kit to be docked as well as separated. In particular, when the first detection assembly and the reagent kit are docked, the first liquid path of the first detection assembly can be in communication with the second liquid path of the reagent kit, such that the reagent of the reagent kit can reach the first liquid path for blood gas detection. In addition, the mounting bracket is provided for assembling the reagent kit and the first detection assembly, and when the assembly is complete, the first detection assembly and the reagent kit can be docked as well as separated. When the first detection assembly and the reagent kit are separated, the reagent kit can be removed separately for replacement. Further, the mounting bracket can further be assembled with the second detection assembly, and the reagent kit is arranged with an external liquid conduit, and the second detection assembly can perform a blood oxygen detection on the liquid in the liquid conduit, thereby completing the blood gas analysis device detection.

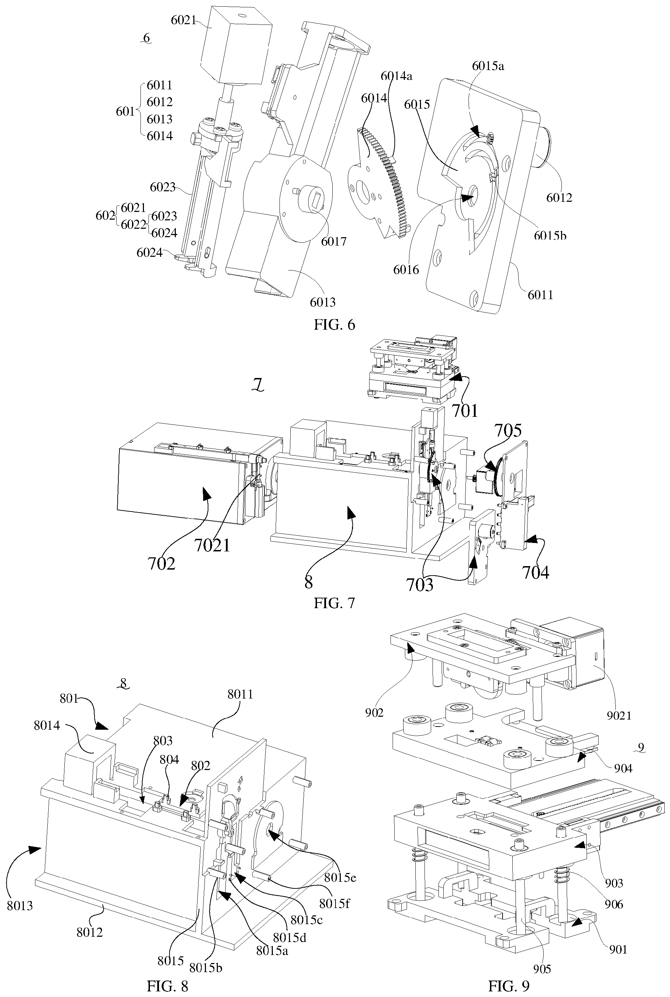

Referring to FIGS. 5 and 6, FIG. 5 is an exploded structural schematic view of a liquid sample detection apparatus 5 according to some embodiments of the present disclosure, and FIG. 6 is an exploded structural schematic view of a regulating device 6 in FIG. 5.

The liquid sample detection apparatus 5 generally includes a body 501 and a regulating device 6, the body 501 having a first mounting position 5011; the regulating device 6 is arranged on the first mounting position 5011.

The regulating device 6 includes, in particular, a first adjusting component 601 and a second adjusting component 602. The first adjusting component 601 is configured to adjust the rotation angle of the sampling element relative to the body 501 of the liquid sample detection apparatus 5 so as to switch between a first fluid path and a second fluid path for conveying the liquid.

The second adjusting component 602 is configured to adjust the axial position of a sleeve sleeved on the sampling element, so as to switch between a first position and a second position of the sleeve. When the sleeve is in the first position, the sampling element can achieve the connection of the first fluid path; when the sleeve is in the second position, the first adjusting component can adjust the rotation angle of the sampling element, so as to achieve the connection of the second fluid path.

The first adjusting component 601 includes an adjusting seat 6011 arranged on the body 501 of the liquid sample detection apparatus 5, a first power member 6012 arranged on the adjusting seat 6011, and an adjusting bracket 6013, where the first power member 6012 can drive the adjusting bracket 6013 to rotate; the adjusting bracket 6013 is configured to be assembled with the sampling element to adjust the rotation angle of the sampling element relative to the body 501 of the liquid sample detection apparatus 5.

The second adjusting component 602 includes a second power member 6021 arranged on the adjustment bracket 6013, and a movable bracket 6022 connected to the second power member 6021, where the second power member 6021 can drive the movable bracket 6022 to move relative to the adjusting bracket 6013; when the body 501 is docked with the reagent kit, the sampling element is nested on a side of the movable bracket 6022, the movable bracket 6022 is connected to the sleeve and can drive the sleeve to switch between the first position and the second position.

The first adjusting component 601 further includes a rotary member 6014 arranged between the adjusting seat 6011 and the adjusting bracket 6013, the rotary member 6014 being connected to the adjusting bracket 6013, and the first power member 6012 being capable of driving the rotary member 6014 to rotate and thereby drive the adjusting bracket 6013 to rotate.

In particular, the adjusting seat 6011 defines a rotating groove 6015, and the rotary member 6014 is accommodated at least partially in the rotating groove 6015 and can be rotated relative to the rotating groove 6015; the rotating groove 6015 is arranged with a shaft hole 6016, the adjusting bracket is arranged with a rotating shaft 6017, and the rotating shaft 6017 passes through the rotary member 6014 and is inserted into the shaft hole 6016.

The first power member 6012 is arranged on a side of the adjusting seat 6011 away from the rotary member 6014, and a rotating shaft of the first power member 6012 passes through the adjusting seat 6011 and is transmission-connected to the rotary member 6014.

A bottom wall of the rotating groove 6015 defines a curved slot 6015a extending in a rotation direction of the rotary member 6014. A first positioning member 6015b is arranged on the curved slot 6015a, and the rotary member 6014 is arranged with a slider 6014a that can slide along the curved slot 6015a; where the slider 6014a cooperates with the first positioning member 6015b to obtain the rotation angle of the rotary member 6014 relative to the adjusting seat 6011.

In particular, the adjusting bracket 6013 is assembled with the second adjusting component 602 such that when the first power member 6012 drives the adjusting bracket 6013 to rotate, the second adjusting component 602 can rotate synchronously with the adjusting bracket 6013.

The second adjusting component 602 includes a second power member 6021 arranged on the adjusting bracket 6013, and a movable bracket 6022 connected to the second power member 6021, where the second power member 6021 can drive the movable bracket 6022 to move relative to the adjusting bracket 6013; when the liquid sample detection apparatus is docked with the reagent kit, the sampling element is nested on a side of the movable bracket 6022, the movable bracket 6022 is connected to the sleeve of the sampling element and can drive the sleeve to switch between the first position and the second position.

The movable bracket 6022 includes a first movable bracket 6023 and a second movable bracket 6024, where the first movable bracket 6023 is connected to the second power member 6021, and the second movable bracket 6024 is elastically connected to the first movable bracket 6023. The second movable bracket 6024 is connected to the sleeve when the liquid sample detection apparatus is docked with the reagent kit.

The regulating device provided in the embodiments of the present disclosure can adjust the rotation angle of the sampling element by means of the first adjusting component and the axial position of the sleeve sleeved on the sampling element by means of the second adjusting component, such that the sampling element can be switched between the first fluid path and the second fluid path, thereby enriching the sampling attitude of the sampling element.

Referring to FIGS. 7 and 8, FIG. 7 is an exploded structural schematic view of a biological tissue detection device 7 according to some embodiments of the present disclosure, and FIG. 8 is an exploded structural schematic view of a biological tissue detection frame 8 in FIG. 7.

The biological tissue detection device 7 generally includes a biological tissue detection frame 8, a biological tissue measurement platform 701, and an auxiliary consumable 702. The biological tissue detection frame 8 includes a cavity 801 and a first mounting position 802; the biological tissue measurement platform 701 is detachably arranged on the first mounting position 802, and the biological tissue measurement platform 701 includes a first pipe; the auxiliary consumable 702 can be moved into or out of the cavity 801, and the auxiliary consumable 702 includes a second pipe; where an avoidance passage 803 is arranged between the cavity 801 and the first mounting position 802, the avoidance passage 803 being configured to avoid mechanical docking operations and consumable delivery between the biological tissue measurement platform 701 and the auxiliary consumable 702. The consumable delivery is a passage connecting the first pipe and the second pipe.

The biological tissue detection frame 8 includes multiple plates, which are interconnected to form the cavity 801 and the first mounting position 802, where the first mounting position 802 is configured to mount the biological tissue measurement platform 701, and the cavity 801 is configured to contain the auxiliary consumable 702.

In particular, the multiple plates include a top plate 8011 and a bottom plate 8012 disposed opposite each other, the cavity 801 being defined between the top plate 8011 and the bottom plate 8012, the avoidance passage 803 passing through the top plate 8011, and the first mounting position 802 being disposed on a side of the top plate 8011 away from the bottom plate 8012.

The cavity 801 includes a pick-up and placement opening 8013, and the auxiliary consumable 702 can be moved into or out of the cavity 801 through the pick-up and placement opening 8013. The side of the top plate 8011 away from the bottom plate 8012 is arranged with a limit member 8014, and the limit member 8014 spans the avoidance passage 803; where the avoidance passage 803 is configured to limit the height of the auxiliary consumable 702 protruding from the top plate 8011.

In particular, the multiple plates include a top plate 8011 and a bottom plate 8012 disposed opposite to each other, as well as a side plate 8015 disposed between the top plate 8011 and the bottom plate 8012. The top plate 8011, the bottom plate 8012, and the side plate 8015 enclose to form the cavity 801 with a pick-up and placement opening 8013, and the auxiliary consumable 702 can be moved into or out of the cavity 801 through the pick-up and placement opening 8013. The avoidance passage 803 runs through the top plate 8011, and the first mounting position 802 is arranged on a side of the top plate 8011 away from the bottom plate 8012.

The side plate 8015 is opposite the pick-up and placement opening 8013; the side plate 8015 includes a first docking port 8015a and a second mounting position 8015b, where the first docking port 8015a is in communication with the cavity 801, and the second mounting position 8015b is configured to mount a regulating device 703. When the auxiliary consumable 702 is moved into the cavity 801, the sampling element on the auxiliary consumable 702 is exposed via the first docking port 8015a to dock with the regulating device 703.

The auxiliary consumable 702 includes a sampling element 7021, and the biological tissue detection device 7 includes a regulating device 703; the regulating device 703 is configured to adjust the sampling posture of the sampling element 7021; the sampling element 7021 docks with the regulating device 703 when the auxiliary consumable 702 is moved into the cavity 801.

The side plate 8015 includes a second docking port 8015c and a third mounting position 8015d. The second docking port 8015c is in communication with the cavity 801, and the third mounting position 8015d is configured to mount a valve apparatus 704. When the auxiliary consumable 702 is moved into the cavity 801, a liquid pack connector in the auxiliary consumable 702 is exposed via the second docking port 8015c to dock with the valve apparatus 704.

The auxiliary consumable 702 includes a receiving cavity for accommodating a liquid pack, and a cavity wall of the receiving cavity is arranged with the liquid pack connector; the biological tissue detection device 7 includes the valve apparatus 704, the valve apparatus 704 being configured to dock with the liquid pack connector to control the outflow of liquid from the liquid pack, the liquid pack connector docking with the valve apparatus 704 when the auxiliary consumable 702 is moved into the cavity 801.

The side plate 8015 includes a third docking port 8015e and a fourth mounting position 8015f. The third docking port 8015e is in communication with the cavity 801, and the fourth mounting position 8015f is configured to mount a drive apparatus 705; when the auxiliary consumable 702 is moved into the cavity 801, the drive apparatus 705 extends into the cavity 801 from the third docking port 8015e and docks with the auxiliary consumable 702 to drive the flow of liquid in the auxiliary consumable 702.

The auxiliary consumable 702 includes a receiving cavity for accommodating a liquid pack, and the cavity wall of the receiving cavity is arranged with a first drive member; the biological tissue detection device 7 includes the drive apparatus 705, the drive apparatus 705 being configured to engage with the first drive member to drive the liquid in the liquid pack to flow out. When the auxiliary consumable 702 is moved into the cavity 801, the drive apparatus 705 docks with the first drive member.

The avoidance passage 803 passes through the first mounting position 802, and a positioning member 804 is arranged on the first mounting position 802 for positioning the biological tissue measurement platform 701 on the top plate 8011.

The biological tissue detection frame provided in the embodiments of the present disclosure has a simple structure, as an avoidance passage is provided between the cavity and the first mounting position of the biological tissue detection frame, such that mechanical docking operations and consumable delivery between the auxiliary consumable in the cavity and the biological tissue measurement platform on the first mounting position can be achieved.

Referring to FIG. 9, FIG. 9 is an exploded structural schematic view of a sample analysis apparatus 9 according to some embodiments of the present disclosure. The sample analysis apparatus 9 generally includes a fixing seat 901, a mounting seat 902, a conveying assembly 903, and a testing assembly 904, and a guide rod 905 is arranged between the fixing seat 901 and the mounting seat 902. The conveying assembly 903 is disposed between the fixing seat 901 and the mounting seat 902, and the conveying assembly 903 is sleeved on the guide rod 905 and is slidable along the guide rod 905; the testing assembly 904 is arranged between fixing seat 901 and mounting seat 902, and the testing assembly 904 is sleeved on the guide rod 905 and is slidable along the guide rod 905.

In particular, the testing assembly 904 is arranged on a side of the conveying assembly 903 away from the fixing seat 901, and a first elastic member 906 is arranged between the conveying assembly 903 and the fixing seat 901, such that the conveying assembly 903 and the fixing seat 901 can be separated by the first elastic member 906.

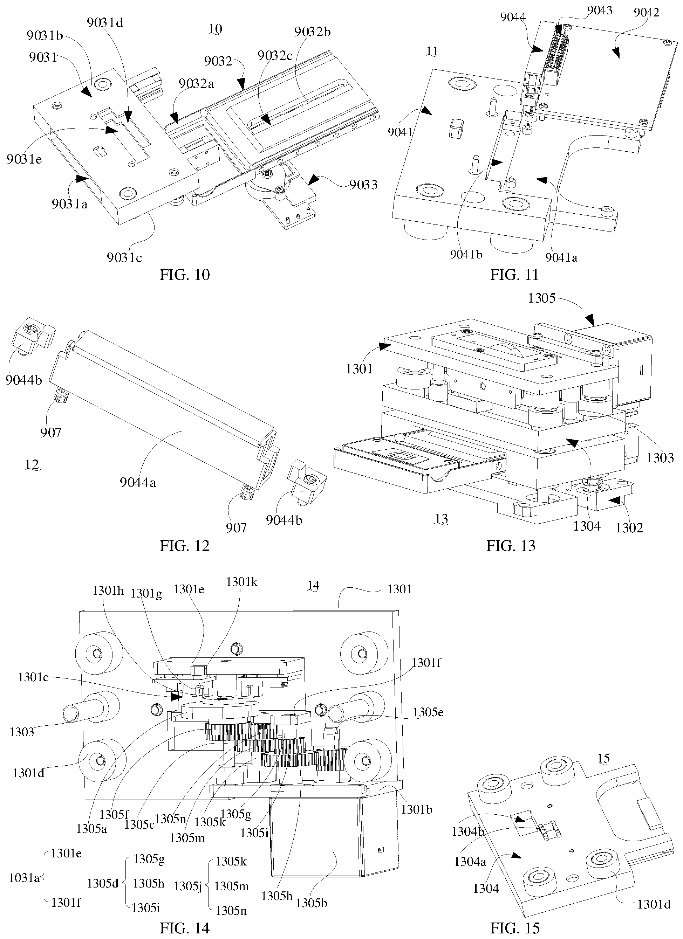

Referring to FIG. 10, FIG. 10 is an exploded structural schematic view of the conveying assembly 903 in FIG. 9. The conveying assembly 903 generally includes a carrier seat 9031 that is sleeved on and slidable along the guide rod 905, a telescopic seat 9032 slidably connected to the carrier seat 9031, and a first drive member 9033 disposed on the carrier seat 9031; where the first elastic member 906 is disposed between the carrier seat 9031 and the fixing seat 901, and the first drive member 9033 is capable of driving the telescopic seat 9032 to move relative to the carrier seat 9031.

The carrier seat 9031 is arranged with a channel 9031a extending along a moving direction of the telescopic seat 9032, and the telescopic seat 9032 is partially disposed in the channel 9031a and can move along the channel 9031a relative to the carrier seat 9031.

In particular, the telescopic seat 9032 defines a clamping slot 9032a for placing a detection assembly, and the clamping slot 9032a can switch between a first position and a second position when the telescopic seat 9032 moves relative to the carrier seat 9031; in particular, when the clamping slot 9032a is in the first position, the detection assembly can be moved into or out of the clamping slot 9032a; when the clamping slot 9032a is in the second position, the clamping slot 9032a is accommodated in the channel 9031a.

The carrier seat 9031 includes a top wall 9031b and a bottom wall 9031c disposed opposite each other, the bottom wall 9031c being disposed on a side of the carrier seat 9031 close to the fixing seat 901, the top wall 9031b being disposed on a side of the carrier seat 9031 close to the testing assembly 904, and the channel 9031a being formed between the top wall 9031b and the bottom wall 9031c; the top wall 9031b defines a first avoidance hole 9031d, and the bottom wall 9031c defines a second avoidance hole 9031e; when the clamping slot 9032a is in the second position, a detection element of the detection assembly placed in the clamping slot 9032a is exposed from the first avoidance hole 9031d, the testing assembly 904 can dock with the detection element of the detection assembly through the first avoidance hole 9031d, and a liquid hole of the detection assembly is exposed from the second avoidance hole 9031e.

In particular, the telescopic seat 9032 is arranged with a transmission member 9032b, and the transmission member 9032b is assembled with the first drive member 9033 to drive the telescopic seat 9032 to move by means of the transmission member 9032b under the drive of the first drive member 9033.

In particular, the telescopic seat 9032 defines an avoidance slot 9032c, the transmission member 9032b is disposed on a slot wall of the avoidance slot 9032c, and an output shaft of the first drive member 9033 extends into the avoidance slot 9032c and is transmission-connected to the transmission member 9032b.

Referring further to Figure 11, FIG. 11 is an exploded structural schematic view of the testing assembly 904 in FIG. 9. A second elastic member 907 is arranged between the conveying assembly 903 and the testing assembly 904, such that the conveying assembly 903 and the testing assembly 904 can be separated by the second elastic member 907.

The testing assembly 904 includes a testing seat 9041 that is sleeved on the guide rod 905 and slidable along the guide rod 905, and a testing plate 9042 arranged on the testing seat 9041. The testing plate 9042 is arranged with a test head 9043, and the test head 9043 contacts the detection element of the detection assembly via the first avoidance hole 9031d when the testing seat 9041 and the carrier seat 9031 are docked.

In particular, the testing assembly 904 further includes a supporting component 9044 arranged on the testing seat 9041, and a second elastic member 907 arranged between the supporting component 9044 and the testing seat 9041. When the testing seat 9041 and the carrier seat 9031 are docked such that the testing plate 9042 contacts the detection element of the detection assembly, the second elastic member 907 is compressed to generate an elastic force, and the elastic force can cause the supporting component 9044 and the testing seat 9041 to separate.

The testing seat 9041 includes a first mounting position 9041a and a second mounting position 9041b on the same side. The testing plate 9042 is arranged on the first mounting position 9041a, and the supporting component 9044 is arranged on the second mounting position 9041b. Before the second elastic member 907 is compressed, the height of the supporting component 9044 protruding from the testing seat 9041 is greater than or equal to the height of the test head 9043 protruding from the testing seat 9041.

The mounting seat 902 is arranged with a second drive member 9021, and the second drive member 9021 is configured to drive the testing seat 9041 to slide along the guide rod 905.

In particular, the testing seat 9041 is arranged with a first positioning member on a side close to the carrier seat 9031, and the carrier seat 9031 is arranged with a second positioning member on a side close to the testing seat 9041. During the docking process of the testing seat 9041 and the carrier seat 9031, the first positioning member and the second positioning member cooperate to guide the testing seat 9041 and the carrier seat 9031 to dock.

Referring further to FIG. 12, FIG. 12 is an exploded structural schematic view of the supporting component 9044 in FIG. 11. The supporting component 9044 includes a body 9044a and a limit member 9044b, where the limit member 9044b is fixedly disposed on the second mounting position 9041b, and the second elastic member 907 is disposed between the body 9044a and the second mounting position 9041b; the body 9044a and the limit member 9044b are slidably connected, and the limit member 9044b is configured to limit the sliding travel of the body 9044a under the action of the elastic force.

The sample analysis apparatus provided in the embodiments of the present disclosure is arranged with a first elastic member such that the conveying assembly and the fixing seat can be separated by the first elastic member, and a second elastic member such that the conveying assembly and the testing assembly can be separated by the second elastic member. That is, when the sample analysis apparatus completes an analysis operation, no additional drive mechanism is required, and the separation of the conveying assembly and the fixing seat and the separation of the conveying assembly and the testing assembly can be achieved by the first elastic member and the second elastic member, such that the overall structure is simple.

Referring to FIGS. 13 to 15, FIG. 13 is an exploded structural schematic view of a biological detection device 13 according to some embodiments of the present disclosure, FIG. 14 is a partial structural schematic view of the biological detection device 13 in FIG. 13, and FIG. 15 is a partial structural schematic view of the biological detection device 13 in FIG. 13.

The biological detection device 13 generally includes a first base 1301, a second base 1302, a guide member 1303, a testing assembly 1304, and a transmission assembly 1305. The guide member 1303 is disposed between the first base 1301 and the second base 1302 and extends in an arrangement direction of the first base 1301 and the second base 1302; the testing assembly 1304 is arranged on the guide member 1303 and can move along the guide member 1303; the transmission assembly 1305 is arranged on the first base 1301, and the transmission assembly 1305 includes a drive member 1305a.

In particular, the testing assembly 1304 is arranged with a supporting member 1304a on a side close to the first base 1301, and the drive member 1305a is in rolling fit with the supporting member 1304a to drive the testing assembly 1304 to move along the guide member 1303.

The transmission assembly 1305 includes a power member 1305b arranged on the first base 1301 and a support shaft 1305c passing through the drive member 1305a. The power member 1305b and the support shaft 1305c are transmission-assembled to drive the support shaft 1305c to rotate, and the rotation of the support shaft 1305c drives the drive member 1305a to rotate synchronously.

The biological detection device 13 further includes a mounting bracket 1301a and a drive bracket 1301b spaced apart and arranged on the first base 1301, the support shaft 1305c being arranged between the mounting bracket 1301a and the drive bracket 1301b, and the power member 1305b being arranged on the drive bracket 1301b.

The first base 1301 defines an avoidance hole 1301c, and the avoidance hole 1301c is configured to avoid the drive member 1305a. The mounting bracket 1301a is arranged on a side of the first base 1301 close to the testing assembly 1304, a spacer 1301d is arranged on at least one of the first base 1301 and the testing assembly 1301d, and the spacer 1301d is arranged between the first base 1301 and the testing assembly 1304 to prevent the mounting bracket 1301a from interfering with the rolling fit between the drive member 1305a and the supporting member 1304a.

The mounting bracket 1301a includes a first bracket 1301e and a second bracket 1301f spaced apart, where the second bracket 1301f is arranged between the first bracket 1301e and the drive bracket 1301b, and the support shaft 1305c is arranged between the first bracket 1301e and the drive bracket 1301b; the drive member 1305a is arranged between the first bracket 1301e and the second bracket 1301f and faces the avoidance hole 1301c, and the power member 1305b and the supporting shaft 1305c are transmission-assembled between the second bracket 1301f and the drive bracket 1301b.

The first bracket 1301e is arranged with a first positioning member 1301g, and the drive member 1305a or the supporting shaft 1305c is arranged with a second positioning member 1301h. The second positioning member 1301h rotates synchronously with the drive member 1305a or the supporting shaft 1305c and cooperates with the first positioning member 1301g to obtain the rotation angle of the drive member 1305a or the supporting shaft 1305c.

The power member 1305b is arranged on a side of the drive bracket 1301b away from the second bracket 1301f, and an output shaft of the power member 1305b passes through the drive bracket 1301b and is transmission-assembled with the support shaft 1305c.

The transmission assembly 1305 includes a first transmission assembly 1305d disposed between the drive bracket 1301b and the second bracket 1301f, an end of the output shaft of the power member 1305b passing through the drive bracket 1301b and extending into between the drive bracket 1301b and the second bracket 1301f is arranged with a first transmission wheel 1305e, and a portion of the support shaft 1305c disposed between the drive bracket 1301b and the second bracket 1301f is arranged with a second transmission wheel 1305f, the first transmission assembly 1305d being transmission-assembled with the first transmission wheel 1305e and the second transmission wheel 1305f, respectively.

The first transmission assembly 1305d includes a first fixed shaft 1305g arranged between the drive bracket 1301b and the second bracket 1301f, and a first driving wheel 1305h and a first driven wheel 1305i that are arranged on the first fixed shaft 1305g; where the first driving wheel 1305h is transmission-assembled with the first transmission wheel 1305e, and the first driven wheel 1305i is transmission-assembled with the second transmission wheel 1305f.

The diameter of the first driving wheel 1305h is greater than the diameter of the first driven wheel 1305i, the axial thickness of the first driving wheel 1305h is less than the axial thickness of the first transmission wheel 1305e, the diameter of the first driven wheel 1305i is less than the diameter of the second transmission wheel 1305f, and the axial thickness of the second transmission wheel 1305f is less than the axial thickness of the first driven wheel 1305i.

The transmission assembly 1305 includes a second transmission assembly 1305j disposed between the first transmission assembly 1305d and the second transmission wheel 1305f, and the second transmission assembly 1305j is transmission-assembled with the first transmission assembly 1305d and the second transmission wheel 1305f, respectively.

The second transmission assembly 1305j includes a second fixed shaft 1305k arranged between the drive bracket 1301b and the second bracket 1301f, and a second driving wheel 1305m and a second driven wheel 1305n arranged on the second fixed shaft 1305k; where the second driving wheel 1305m is transmission-assembled with the first driven wheel 1305i, and the second driven wheel 1305n is transmission-assembled with the second transmission wheel 1305f.

The diameter of the second driving wheel 1305m is greater than the diameter of the second driven wheel 1305n, the axial thickness of the second driving wheel 1305m is less than the axial thickness of the first driven wheel 1305i, the diameter of the second driven wheel 1305n is less than the diameter of the second transmission wheel 1305f, and the axial thickness of the second transmission wheel 1305f is less than the axial thickness of the second driven wheel 1305n.

A limit block 1301k is arranged on one of the first bracket 1301e and the testing assembly 1304, and the other defines a limit groove 1304b. The limit block 1301k and the limit groove 1304b cooperate to limit a spacing between the first base 1301 and the testing assembly 1304.

The drive member 1305a may be a turbine, and the supporting member 1304a may be a roller.

The biological detection device provided in the embodiments of the present disclosure can ensure the stability of the movement of the testing assembly and move the testing assembly to a predetermined position for corresponding testing, by providing the supporting member and the drive member that are in rolling fit on the testing assembly to drive the testing assembly to move along the guide rod.

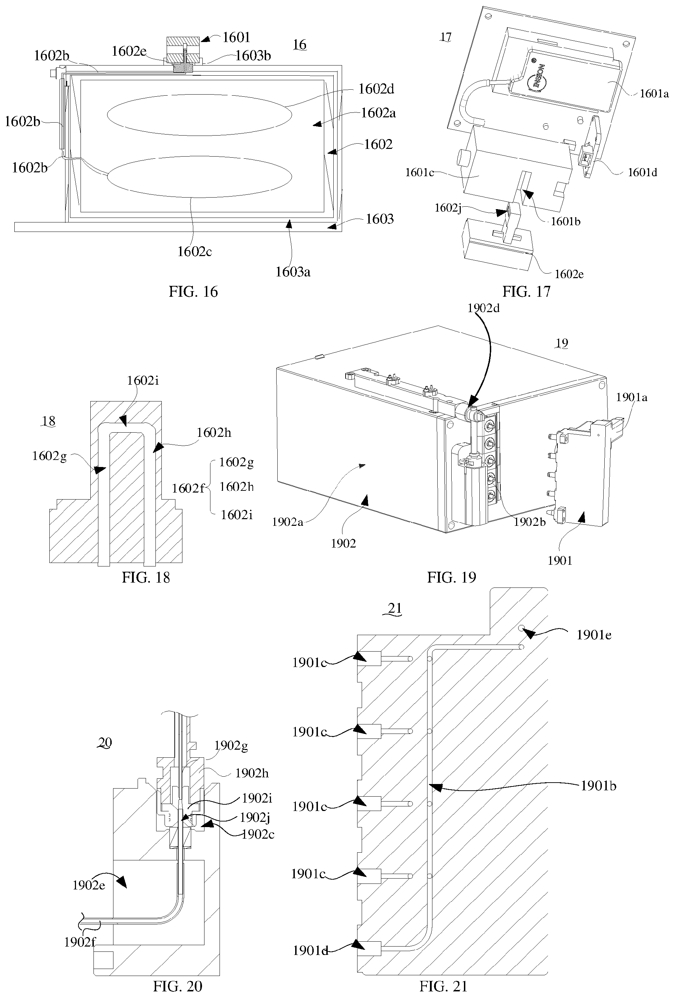

Referring to FIGS. 16 to 18, FIG. 16 is an exploded structural schematic view of a blood sample analysis platform 16 according to some embodiments of the present disclosure, FIG. 17 is a partial structural schematic view of the blood sample analysis platform 16 in FIG. 16, and FIG. 18 is a partial structural schematic view of the blood sample analysis platform 16 in FIG. 16.

The blood sample analysis platform 16 generally includes a blood sample analyzer 1601 and an auxiliary liquid box 1602. The blood sample analyzer 1601 includes a non-contact detection element 1601a and an avoidance slot 1601b. The auxiliary liquid box 1602 includes a cavity 1602a and a liquid path 1602b, where the cavity 1602a is configured to hold an auxiliary liquid 1602c and a waste liquid pack 1602d; an end of the liquid path 1602b is configured to introduce the auxiliary liquid 1602c, and the other end is configured to communicate with the waste liquid pack 1602d.

The liquid path 1602b has a portion that is exposed from the auxiliary liquid box 1602, and the non-contact detection element 1601a is configured to detect a portion of the liquid path 1602b entering the avoidance slot 1601b.

The blood sample analysis platform 16 includes a mounting bracket 1603, the mounting bracket 1603 including a receiving cavity 1603a and a first mounting position 1603b; where the auxiliary liquid box 1602 can be moved into or out of the receiving cavity 1603a, and the first mounting position 1603b is configured to mount the blood sample analyzer 1601; an avoidance passage is arranged between the receiving cavity 1603a and the first mounting position 1603b, and when the auxiliary liquid box 1602 is moved into the receiving cavity, the portion of the liquid path 1602b exposed from the auxiliary liquid box 1602 enters the avoidance slot 1601b through the avoidance passage.

In particular, the auxiliary liquid box 1602 is arranged with a sampling seat 1602e, and the liquid path 1602b includes a liquid channel 1602f arranged in the sampling seat 1602e. When the auxiliary liquid box 1602 is moved into the receiving cavity 1603a, the sampling seat 1602e is moved into the avoidance slot 1601b, such that the non-contact detection element 1601a can detect the liquid in the liquid channel 1602f.

The liquid channel 1602f includes a liquid inlet section 1602g, a liquid outlet section 1602h, and a detection section 1602i connecting the liquid inlet section 1602g and the liquid outlet section 1602h; the liquid inlet section 1602g is configured to input the auxiliary liquid 1602c, and the liquid outlet section 1602h is configured to be in communication with the waste liquid pack 1602d. When the sampling seat 1602e is moved into the avoidance slot 1601b, the non-contact detection element 1601a can detect the liquid in the detection section 1602i.

In particular, the sampling seat 1602e is arranged with a light-transmitting region 1602j corresponding to the detection section 1602i, such that light emitted from the blood sample analyzer 1601 can be directed to the liquid in the detection section 1602i through the light-transmitting region 1602j for detection.

The blood sample analyzer 1601 includes a testing seat 1601c and a testing plate 1601d, the testing seat 1601c being arranged on the first mounting position 1603b and the testing plate 1601d being arranged on the testing seat 1601c; the avoidance slot 1601b is defined on the testing seat 1601c, and the non-contact detection element 1601a is connected to the testing seat 1601c for detecting the liquid entering the avoidance slot 1601b. The non-contact detection element 1601a includes a light source and a light sensor device arranged on the testing plate 1601d. The light emitted by the light source can pass through the light-transmitting region 1602j to reach the light sensor device to detect the liquid in the detection section 1602i.

The blood sample analyzer 1601 includes an ultrasonic sounder, which is arranged on the testing seat 1601c, for ultrasonic processing of the liquid in the liquid channel 1602f entering the avoidance slot 1601b.

The testing seat 1601c is configured to dock with as well as separate from the auxiliary liquid box 1602, and the liquid path of the auxiliary liquid box 1602 has a bent part that protrudes from the auxiliary liquid box 1602. When the testing seat 1601c docks with the auxiliary liquid box 1602, the part enters the avoidance slot 1601b to be detected by the non-contact detection element 1601a.

The liquid path of the auxiliary liquid box 1602 has a part that is exposed from the auxiliary liquid box 1602, and the part enters the avoidance slot of the blood sample analyzer 1601 to be detected by the non-contact detection element 1601a of the blood sample analyzer 1601 when the auxiliary liquid box 1602 is docked with the blood sample analyzer 1601.

In the blood sample analysis platform provided in the embodiments of the present disclosure, a part of the liquid path of the auxiliary liquid box is exposes to the auxiliary liquid box, and the non-contact detection element of the blood sample analyzer can detect the part when the part enters the avoidance slot of the blood sample analyzer, thereby realizing non-contact detection of the liquid in the liquid path.

Referring to FIGS. 19 to 21, FIG. 19 is an exploded structural schematic view of a fluid detection instrument 19 according to some embodiments of the present disclosure, FIG. 20 is a partial structural schematic view of the fluid detection instrument 19 in FIG. 19, and FIG. 21 is a partial structural schematic view of the fluid detection instrument 19 in FIG. 19.

The fluid detection instrument 19 generally includes a valve assembly 1901 and a fluid kit 1902. The valve assembly 1901 includes a control element 1901a, a first pipe 1901b, an inlet 1901c, and an outlet 1901d. The number of the inlets 1901c is plural, and the control element 1901a is configured to control one of the multiple inlets 1901c to be in communication with one end of the first pipe 1901b, and the other end of the first pipe 1901b is in communication with the outlet 1901d.

The fluid kit 1902 includes a cavity 1902a for accommodating the fluid pack and a port 1902b. An end of the port 1902b is in communication with the fluid pack, and the other end of the port 1902b is in communication with the inlet 1901c.

The valve assembly 1901 and the fluid kit 1902 can be docked or separated, such that the inlet 1901c and the port 1902c can be docked or separated. When they are docked, the first pipe 1901b can be in communication with the port 1902c under the control of the control element 1901a, such that the fluid in the fluid pack can reach the first pipe 1901b.

In particular, the valve assembly 1901 includes a vent 1901e, and an end of the first pipe 1901b is selectively in communication with the inlet 1901c or the vent 1901e under the control of the control element 1901a.

The fluid kit 1902 defines a docking groove 1902c, which is configured to dock with the sampling element 1902d, and the docking groove 1902c is arranged with a through hole 1902e and a connecting pipe 1902f passing through the through hole 1902e. An end of the connecting pipe 1902f is in communication with the outlet 1901d, and the other end is configured to be in communication with a sample inlet of the sampling element 1902d; the connecting pipe 1902f is in sealed contact with a hole wall of the through hole 1902e.

The sampling element 1902d includes a sampling needle 1902g, a sleeve 1902h sleeved on the sampling needle 1902g, and a docking member 1902i arranged on an end of the sleeve 1902h. The outer diameter of the sleeve 1902h does not exceed the inner diameter of the docking groove 1902c. The docking member 1902i defines a through hole 1902j; the docking member 1902i can move along the axial direction of the sampling needle 1902g following the sleeve 1902h, such that the sampling needle 1902g is inserted into an end of the through hole 1902j or passes through the through hole 1902j and is exposed on a side of the docking member 1902i.

When the sleeve 1902h moves along the axial direction of the sampling needle 1902g and towards the connecting pipe 1902f such that the docking member 1902i is inserted into the docking groove 1902c, the sampling needle 1902g and the connecting pipe 1902f are respectively inserted into both ends of the through hole 1902j to achieve connection.

The valve assembly 1901 includes a control element 1901a, a first pipe 1901b, an inlet 1901c, and an outlet 1901d. The number of the inlets 1901c is plural, and the control element 1901a is configured to control one of the multiple inlets 1901c to be in communication with one end of the first pipe 1901b, and the other end of the first pipe 1901b is in communication with the outlet 1901d; the inlet 1901c is configured to dock with as well as separate from a port of the fluid kit 1902 to obtain the fluid of the fluid kit 1902 by controlling the inlet 1901c through the control element 1901a when docked.

The fluid kit 1902 includes a cavity 1902a for accommodating the fluid pack and a port 1902b. An end of the port 1902b is in communication with the fluid pack, and the other end of the port 1902b is in communication with the inlet 1901c of the valve assembly 1901. The valve assembly 1901 and the fluid kit 1902 can be docked or separated, such that when they are docked, the fluid in the fluid kit 1902 can be delivered to the valve assembly 1901 through the port 1902b.

The fluid detection apparatus provided in the embodiments of the present disclosure can realize the detachable assembly of the fluid kit and valve assembly, by providing the valve assembly and fluid kit that can be docked or separated. In particular, when they are docked, the control element of the valve assembly can control the first pipe of the valve assembly to be in communication with the port of the fluid kit, such that the liquid in the fluid kit can reach the first pipe to complete the corresponding detection. In addition, the control element can control one of multiple inlets to be in communication with the first pipe, such that the liquid flowing into the first pipe can be conveniently selected.

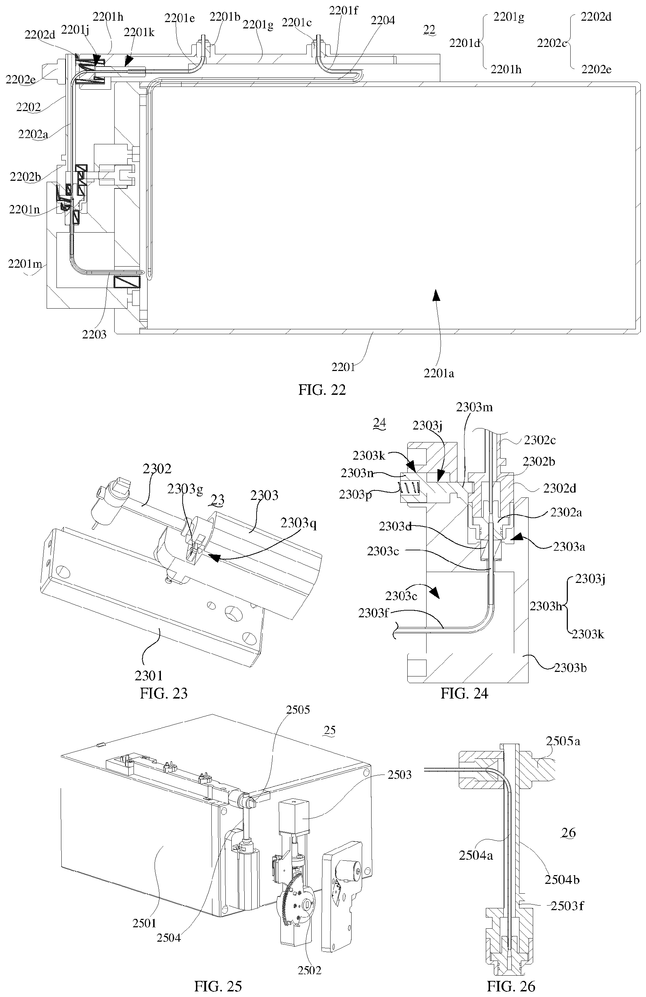

Referring to FIG. 22, FIG. 22 is a structural schematic view of an integrated reagent kit 22 according to some embodiments of the present disclosure. The integrated reagent kit 22 generally includes a case 2201, a sampling apparatus 2202, a first pipe 2203, and a second pipe 2204. The case 2201 has a storage space 2201a, and the storage space 2201a is configured to store a reagent pack and a recovery pack. The sampling apparatus 2202 is arranged on the case 2201 and is disposed outside the storage space 2201a. The first pipe 2203 and the second pipe 2204 are arranged on the case 2201. An input end of the sampling apparatus 2202 can be in communication with the reagent pack through the first pipe 2203, and an output end of the sampling apparatus 2202 is in communication with the recovery pack through the second pipe 2204. The sampling apparatus 2202 is rotatable relative to the case 2201 such that the input end of the sampling apparatus 2202 can collect liquid from the reagent pack or an external container.

In particular, the case 2201 includes a first port 2201b and a second port 2201c arranged at intervals, where the first port 2201b is in communication with the output end of the sampling apparatus 2202 and the second port 2201c is in communication with the recovery pack through the second pipe 2204. The first port 2201b and the second port 2201c are configured to dock with a testing assembly such that the liquid collected by the sampling apparatus 2202 can reach the testing assembly.

In particular, the case 2201 is arranged with a sample introduction seat 2201d disposed outside the storage space 2201a, the first port 2201b and the second port 2201c being arranged on the sample introduction seat 2201d, and a first connecting pipe 2201e and the second connecting pipe 2201f are arranged in the sample introduction seat 2201d. An end of the first connecting pipe 2201e is in communication with the first port 2201b, and the other end of the first connecting pipe 2201e is in communication with the output end of the sampling apparatus 2202. An end of the second connecting pipe 2201f is in communication with the second port 2201c, and the other end of the second connecting pipe 2201f is configured to be in communication with the recovery pack.

The output end of the sampling apparatus 2202 is inserted into the sample introduction seat 2201d and in communication with the first connecting pipe 2201e.

The sample introduction seat 2201d includes a seat body 2201g and a pipe body 2201h arranged on an end of the seat body 2201g. The first port 2201b and the second port 2201c are arranged on the seat body 2201g. An end of the first connecting pipe 2201e is inserted into the pipe body 2201h and the other end of the first connecting pipe 2201e is in communication with the first port 2201b, and the output end of the sampling apparatus 2202 is inserted into the pipe body 2201h to be in communication with the first connecting pipe 2201e.

The sampling apparatus 2202 includes a sampling element 2202a, a sleeve 2202b sleeved on an input end of the sampling element 2202a, and a swivel 2202c sleeved on an output end of the sampling element 2202a. The swivel 2202c is rotatably assembled with the pipe body 2201h such that the swivel 2202c can be rotated relative to the pipe body 2201h. The rotation of the swivel 2202c can drive the sleeve 2202b to rotate such that the input end of the sampling element 2202a can collect liquid from the reagent pack or external container.

In particular, the pipe body 2201h is arranged with a first hole segment 2201j and a second hole segment 2201k that are in communication, the first hole segment 2201j being configured to be rotatably connected to the swivel 2202c, and the output end of the sampling element 2202a and the first connecting pipe 2201e are in communication through the second hole segment 2201k; where the hole diameter of the first hole segment 2201j is greater than the hole diameter of the second hole segment 2201k.

The swivel 2202c includes a first swivel 2202d and a second swivel 2202e clamped to the sleeve 2202b, the first swivel 2202d being rotatably assembled with the pipe body 2201h, and the second swivel 2202e being rotatable relative to the pipe body 2201h under the action of an external force, thereby driving the sleeve 2202b to rotate.

In particular, the sleeve 2202b can be moved axially along the sample inlet of the sampling element 2202a under an external force to expose or cover the sample inlet of the sampling element 2202a. The sleeve 2202b defines an avoidance hole to avoid the sampling element 2202a when the sleeve 2202b is moved axially.

The case 2201 is arranged with a docking base 2201m, the docking base 2201m including a port 2201n; where the port 2201n is configured to dock with the input end of the sampling apparatus 2202; an end of the first pipe 2203 is in communication with the port 2201n, and the other end of the first pipe 2203 is configured to be in communication with the reagent pack.

The embodiments of the present disclosure provide an integrated reagent kit, which is arranged with a sampling apparatus, a first pipe and a second pipe arranged on the case, such that the sampling apparatus can collect the liquid in the reagent pack through the first pipe, and the liquid in the sampling apparatus can reach the recovery pack through the second pipe. In addition, the sampling apparatus can be rotated relative to the case, such that the sampling apparatus can collect liquid from an external container or reagent pack, thereby enhancing the versatility of the sampling apparatus for collecting liquid and the flexibility of the reagent kit.

Referring to FIGS. 23 and 24, FIG. 23 is a partial structural schematic view of a reagent storage apparatus 23 according to some embodiments of the present disclosure, and FIG. 24 is a cross-sectional structural schematic view of the reagent storage apparatus 23 in FIG. 23.

The reagent storage apparatus 23 generally includes a storage box 2301, and a sampling assembly 2302 and a docking assembly 2303 that are disposed on the storage box 2301 and outside the storage box 2301. An outlet end of the sampling assembly 2302 is in communication with a first position in the storage box 2301, the docking assembly 2303 defines a sampling groove 2303a, and the sampling groove 2303a is in communication with a second position in the storage box 2301; the sampling assembly 2302 and the docking assembly 2303 can be connected or separated. When the sampling assembly 2302 and the docking assembly 2303 are connected, an inlet end of the sampling assembly 2302 is inserted into the sampling groove 2303a to sample from the second position in storage box 2301. When the sampling assembly 2302 and the docking assembly 2303 are separated, the inlet end of sampling assembly 2302 can sample from outside the storage box 2301.

The docking assembly 2303 includes a docking base 2303b arranged on the storage box 2301, a sampling groove 2303a defined on the docking base 2303b, and a connector pipe 2303c disposed in the sampling groove 2303a. An end of the connector pipe 2303c is configured to be in communication with the inlet end of the sampling assembly 2302, and the other end is configured to be in communication with the second position in the storage box 2301. The inlet end of the sampling assembly 2302 is arranged with a connector 2302a, and when the sampling assembly 2302 is connected to the docking assembly 2303, the connector pipe 2303c is inserted into the connector 2302a.

The sampling assembly 2302 includes a sampling element 2302b, which may be inserted into or pass through the connector 2302a. When the sampling assembly 2302 and the docking assembly 2303 are connected, a sampling end of the sampling element 2302b is inserted into the connector 2302a, and the connector pipe 2303c is arranged on the connector 2302a to be in communication with the sampling end of the sampling element 2302b.

The docking assembly 2303 includes a sealing member 2303d embedded in the sampling groove 2303a, and the connector pipe 2303c passes through the sealing member 2303d. When the connector pipe 2303c is inserted into the connector 2302a, the sealing member 2303d abuts against the connector 2302a.

An end of the connector 2302a close to the sealing member 2303d is tapered and concave to guide the connector pipe 2303c to be inserted into the connector 2302a.

In particular, the docking base 2303b defines a matching groove 2303e opened facing the storage box 2301. An end of the connector pipe 2303c extends into the matching groove 2303e to be in communication with the second position in the storage box 2301 through a connecting pipe 2303f, and the other end of the connector pipe 2303c extends into sampling groove 2303a to be inserted in the connector 2302a when the sampling assembly 2302 is connected to the docking assembly 2303.

The sampling assembly 2302 includes a sleeve 2302c sleeved on the sampling element 2302b, and a connector 2302a embedded in an end portion of the sleeve 2302c; where a clamping member 2302d is sleeved on the connector 2302a at the end portion of the sleeve 2302c, and the clamping member 2302d is connected to the sleeve 2302c. The radial width of the clamping member 2302d is greater than the radial width of the sealing member 2303d, and the radial width of the clamping member 2302d does not exceed the radial width of the sampling groove 2303a.

The docking assembly 2303 includes a sliding member 2303g that is slidably connected to the docking base 2303b, and the sliding member 2303g can abut against the sampling assembly 2302 to stabilize the sampling state of the sampling assembly 2302.

In particular, the docking base 2303b defines a mating hole 2303h, and the sliding member 2303g passes through the mating hole 2303h and is elastically connected to the storage box 2301, such that the sliding member 2303g can slide relative to the docking base 2303b.

The mating hole 2303h includes a first mating section 2303j and a second mating section 2303k that are in communication, where the first mating section 2303j has a cross-sectional area that is smaller than the cross-sectional area of the second mating section 2303k, and the first mating section 2303j is arranged between the second mating section 2303k and the storage box 2301; the sliding member 2303g includes a sliding portion 2303m and a limit portion 2303n, the sliding portion 2303m being slidable relative to the first mating portion 2303j, and the limit portion 2303n being slidable relative to the second mating portion 2303k and stopped by the second mating portion 2303k.

The docking assembly 2303 includes an elastic member 2303p, which is arranged between the storage box 2301 and the sliding member 2303g, and is connected to the sliding member 2303g at one end and to the storage box 2301 at the other end.

In particular, the docking base 2303b defines an avoidance slot 2303q that passes through a side wall of the sampling groove 2303a, and the inlet end of the sampling assembly 2302 can be unscrewed out of the sampling groove 2303a through the avoidance slot 2303q, such that the inlet end of the sampling assembly 2302 can sample from outside the storage box 2301.

In the reagent storage apparatus provided in the embodiments of the present disclosure, the docking assembly can cooperate with the sampling assembly arranged on the storage box, such that the sampling assembly can sample from the inside or outside of the storage box. Specifically, when the sampling assembly and the docking assembly are docked, the inlet end of the sampling assembly is inserted into the sampling groove of the docking assembly to sample from the inside of the storage box; when the sampling assembly and the docking assembly are separated, the inlet end of the sampling assembly can sample from the outside of the storage box. That is, the sampling position of the sampling assembly can be flexibly rotated by the above structural arrangement of the embodiments of the present disclosure, and the amount and type of liquid in the storage box can be reasonably allocated.

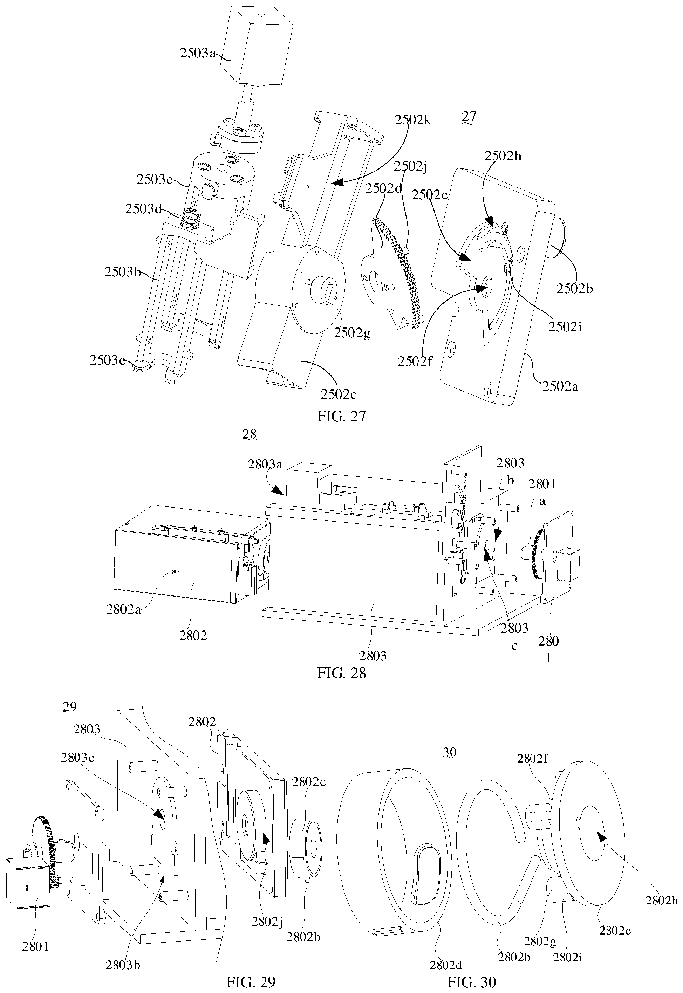

Referring to FIGS. 25 to 27, FIG. 25 is an exploded structural schematic view of a liquid parameter measurement platform 25 according to some embodiments of the present disclosure, FIG. 26 is a partial structural schematic view of the liquid parameter measurement platform 25 in FIG. 25, and FIG. 27 is a partially exploded structural schematic view of the liquid parameter measurement platform 25 in FIG. 25.

The liquid parameter measurement platform 25 generally includes a liquid loading box 2501, a rotatable assembly 2502, and a movable assembly 2503. The liquid loading box 2501 includes a liquid taking assembly 2504 and an adjusting assembly 2505; where the adjusting assembly 2505 is configured to be driven under an external force to drive the liquid taking assembly 2504 to rotate relative to the liquid loading box 2501; the rotatable assembly 2502 rotates to drive the movable assembly 2503 to rotate synchronously, and the movable assembly 2503 can move relative to the rotatable assembly 2502; the adjusting assembly 2505 and the rotatable assembly 2502 can be docked or separated, and when the adjusting assembly 2505 and the rotatable assembly 2502 are docked, the rotatable assembly 2502 can drive the adjusting assembly 2505 to rotate relative to the liquid loading box 2501, thereby driving the liquid taking assembly 2504 to rotate relative to the liquid loading box 2501; the liquid taking assembly 2504 and the movable assembly 2503 can follow the docking or separation of the adjusting assembly 2505 and the rotatable assembly 2502 to achieve the corresponding docking or separation. When the liquid taking assembly 2504 and the movable assembly 2503 are docked, the movable assembly 2503 is configured to adjust the axial position of at least part of the liquid taking assembly 2504 such that the rotatable assembly 2502 can drive the liquid taking assembly 2504 to switch between a first attitude and a second attitude; the first attitude corresponds to that the liquid taking assembly 2504 is limited to collect liquid from inside the liquid loading box 2501, and the second attitude corresponds to that the liquid taking assembly 2504 is limited to collect liquid from outside the liquid loading box 2501.

The liquid taking assembly 2504 includes a liquid taking element 2504a and a sleeve 2504b. A liquid taking end of the liquid taking element 2504a is configured to collect internal liquid from the liquid loading box 2501 or external liquid. The liquid collected by the liquid taking element 2504a flows to the interior of liquid loading box 2501 or a liquid recovery container. The sleeve 2504b is sleeved on the liquid taking end of the liquid taking element 2504a and is able to dock with the movable assembly 2503, such that the axial position of the sleeve 2504b relative to the liquid taking element 2504a can be adjusted under the drive of the movable assembly 2503.

The adjusting assembly 2505 includes a swivel 2505a clamped to the sleeve 2504b. An end of the swivel 2505a is sleeved on a liquid outlet end of the liquid taking element 2504a, and the other end of the swivel 2505a is able to dock with or separate from the rotatable assembly 2502. When the rotatable assembly 2502 docks with the swivel 2505a, the rotatable assembly 2502 can drive the swivel 2505a to rotate, thereby driving the sleeve 2504b and the liquid taking element 2504a to rotate.

The rotatable assembly 2502 includes an adjusting seat 2502a to be arranged on the liquid parameter measurement platform 25, a first power member 2502b arranged on the adjusting seat 2502a, and an adjusting bracket 2502c. The first power member 2502b can drive the adjusting bracket 2502c to rotate; the adjusting bracket 2502c is configured to be assembled with the swivel 2505a to adjust the rotation angle of the swivel 2505a relative to the liquid loading box 2501.

The rotatable assembly 2502 further includes a rotary member 2502d disposed between the adjusting seat 2502a and the adjusting bracket 2502c, the rotary member 2502d being connected to the adjusting bracket 2502c for synchronous rotation, and the first power member 2502b being capable of driving the rotary member 2502d to rotate.

In particular, the first power member 2502b is arranged on a side of the adjusting seat 2502a away from the rotary member 2502d, and an output shaft of the first power member 2502b passes through the adjusting seat 2502a and is transmission-connected to the rotary member 2502d.

The adjusting seat 2502a defines a rotating groove 2502e, the rotary member 2502d is at least partially embedded in the rotating groove 2502e, and the output shaft of the first power member 2502b is transmission-connected to the rotatory member 2502d to drive the rotary member 2502d to rotate relative to the rotating groove 2502e.

A shaft hole 2502f is defined on the rotating groove 2502e, a rotating shaft 2502g is arranged on the adjusting bracket 2502c, and the rotating shaft 2502g passes through the rotary member 2502d and is inserted into the shaft hole 2502f.

In particular, a bottom wall of the rotating groove 2502e defines a curved slot 2502h extending in a rotation direction of the rotary member 2502d, the curved slot 2502h is arranged with a first positioning member 2502i, and the rotary member 2502d is arranged with a slider 2502j that can slide along the curved slot 2502h; where the slider 2502j cooperates with the first positioning member 2502i to obtain the rotation angle of the rotary member 2502d relative to the adjusting seat 2502a.

The adjusting bracket 2502c is assembled with the movable assembly 2503, such that when the first power member 2502b drives the adjusting bracket 2502c to rotate, the movable assembly 2503 can rotate synchronously with the adjusting bracket 2502c; the adjusting bracket 2502c defines an adjusting slot 2502k, and the movable assembly 2503 is embedded in the adjusting slot 2502k to rotate synchronously with the adjusting bracket 2502c.

The movable assembly 2503 includes a second power member 2503a arranged on the adjusting bracket 2502c, and a movable bracket 2503b connected to the second power member 2503a; where the second power member 2503a can drive the movable bracket 2503b to move relative to the adjusting bracket 2502c. When the adjusting assembly 2505 is docked with the rotatable assembly 2502, the sleeve 2504b of the liquid taking assembly 2504 is nested on a side of the movable bracket 2503b, and the movable bracket 2503b is connected to the sleeve 2504b and can drive the sleeve 2504b to switch between a first position and a second position.

The movable assembly 2503 includes a fixed bracket 2503c embedded in the adjusting slot 2502k, and an end of the output shaft of the second power member 2503a passes through the fixed bracket 2503c to connect with the movable bracket 2503b.

In particular, an elastic member 2503d is arranged between the fixed bracket 2503c and the movable bracket 2503b, and the movable bracket 2503b moves relative to the fixed bracket 2503c under the drive of the second power member 2503a, causing the elastic member 2503d to generate elasticity. When the drive is cancelled, the elasticity causes the movable bracket 2503b to reset.

The movable bracket 2503b includes a first engagement portion 2503e, and the sleeve 2504b includes a second engagement portion 2503f. When the adjusting assembly 2505 is docked with the rotatable assembly 2502, the first engagement portion 2503e and the second engagement portion 2503f are engaged and connected.

The liquid loading box 2501 that can be applied to the liquid parameter measurement platform 25 generally includes a liquid taking assembly 2504 and an adjusting assembly 2505, the adjusting assembly 2505 being configured to be driven by an external force to drive the liquid taking assembly 2504 to rotate relative to the liquid loading box 2501.

The adjusting assembly 2505 is configured to dock with as well as separate from the rotatable assembly 2502, and when the adjusting assembly 2505 docks with the rotatable assembly 2502, the rotatable assembly 2502 can drive the adjusting assembly 2505 to rotate relative to the liquid loading box 2501, thereby driving the liquid taking assembly 2504 to rotate relative to the liquid loading box 2501.

The liquid taking assembly 2504 is configured to dock with as well as separate from the movable assembly 2503. The liquid taking assembly 2504 and the movable assembly 2503 can dock with or separate accordingly by following the docking or separation of the adjusting assembly 2505 and the rotatable assembly 2502. When the liquid taking assembly 2504 and the movable assembly 2503 are docked, the movable assembly 2503 can adjust the axial position of at least part of the liquid taking assembly 2504 such that the rotatable assembly 2502 can drive the liquid taking assembly 2504 to switch between a first attitude and a second attitude.

The first attitude corresponds to that the liquid taking assembly 2504 is limited to collect liquid from inside the liquid loading box 2501, and the second attitude corresponds to that the liquid taking assembly 2504 is limited to collect liquid from outside the liquid loading box 2501.

The liquid taking assembly 2504 includes a liquid taking element 2504a and a sleeve 2504b. A liquid taking end of the liquid taking element 2504a is configured to collect internal liquid from the liquid loading box 2501 or external liquid. The liquid collected by the liquid taking element 2504a flows through the liquid taking end of the liquid taking element 2504a to the interior of liquid loading box 2501 or a liquid recovery container. The sleeve 2504b is sleeved on the liquid taking end of the liquid taking element 2504a and is able to dock with the movable assembly 2503, such that the axial position of the sleeve 2504b relative to the liquid taking element 2504a can be adjusted under the drive of the movable assembly 2503.

The adjusting assembly 2505 includes a swivel 2505a clamped to the sleeve 2504b. An end of the swivel 2505a is sleeved on a liquid outlet end of the liquid taking element 2504a, and the other end of the swivel 2505a is able to dock with or separate from the rotatable assembly 2502. When the rotatable assembly 2502 docks with the swivel 2505a, the rotatable assembly 2502 can drive the swivel 2505a to rotate, thereby driving the sleeve 2504b and the liquid taking element 2504a to rotate.

A motion apparatus that can be applied to the liquid parameter measurement platform 25 generally includes a rotatable assembly 2502 and a movable assembly 2503. The rotatable assembly 2502 rotates to drive the movable assembly 2503 to rotate synchronously, and the movable assembly 2503 can move relative to the rotatable assembly 2502.

The rotatable assembly 2502 is configured to dock with as well as separate from the adjusting assembly 2505 of the liquid loading box 2501, and when the rotatable assembly 2502 docks with the adjusting assembly 2505, the rotatable assembly 2502 can drive the adjusting assembly 2505 to rotate relative to the liquid loading box 2501, thereby driving the liquid taking assembly 2504 to rotate relative to the liquid loading box 2501.

The movable assembly 2503 is configured to dock with as well as separate from the liquid taking assembly 2504; the movable assembly 2503 and the liquid taking assembly 2504 can be docked or separated accordingly by following the docking or separation of the adjusting assembly 2505 and the rotatable assembly 2502; when the liquid taking assembly 2504 and the movable assembly 2503 are docked, the movable assembly 2503 is configured to adjust the axial position of at least part of the liquid taking assembly 2504 such that the rotatable assembly 2502 can drive the liquid taking assembly 2504 to switch between a first attitude and a second attitude.

The first attitude corresponds to that the liquid taking assembly 2504 is limited to collect liquid from inside the liquid loading box 2501, and the second attitude corresponds to that the liquid taking assembly 2504 is limited to collect liquid from outside the liquid loading box 2501.

The rotatable assembly 2502 includes an adjusting seat 2502a to be arranged on the liquid parameter measurement platform 25, a first power member 2502b arranged on the adjusting seat 2502a, and an adjusting bracket 2502c. The first power member 2502b can drive the adjusting bracket 2502c to rotate; the adjusting bracket 2502c is configured to be assembled with the adjusting assembly 2505 to adjust the rotating angle of the adjusting assembly 2505 relative to the liquid loading box 2501.

In the liquid parameter measurement platform provided in the embodiments of the present disclosure, the adjusting assembly and the rotatable assembly that can be docked or separated are provided, and when they are docked, the rotatable assembly can drive the adjusting assembly to rotate relative to the liquid loading box, thereby driving the liquid taking assembly to rotate relative to the liquid loading box. The liquid taking assembly and the movable assembly are set to be docked or separated accordingly following the docking and separating of the adjusting assembly and the rotatable assembly, and when they are docked, the movable assembly can adjust the axial position of at least part of the liquid taking assembly such that the rotatable assembly can drive the liquid taking assembly to switch between a first attitude and a second attitude. The first attitude corresponds to that the liquid taking assembly is limited to collect liquid from inside the liquid loading box, and the second attitude corresponds to that the liquid taking assembly is limited to collect liquid from outside the liquid loading box. That is, through the above structural settings, the liquid collection attitude of the liquid taking assembly can be flexibly adjusted, and thus the diversity of liquid parameter measurement platform detection may be enriched.

Referring to FIGS. 28 to 30, FIG. 28 is a partially exploded structural schematic view of a biological sample analysis device 28 according to some embodiments of the present disclosure, FIG. 29 is a partially exploded structural schematic view of the biological sample analysis device 28 in FIG. 28, and FIG. 30 is a partially exploded structural schematic view of the biological sample analysis device 28 in FIG. 28.

The biological sample analysis device 28 generally includes a drive assembly 2801 and a reagent storage apparatus 2802. The drive assembly 2801 includes an output shaft 2801a. The reagent storage apparatus 2802 includes a receiving cavity 2802a, a connecting pipe 2802b, and a squeezing assembly 2802c, the receiving cavity 2802a being configured to hold a reagent pack and a recovery pack, and the connecting pipe 2802b being partially embedded in the squeezing assembly 2802c; an end of the connecting pipe 2802b is configured to be in communication with the reagent pack, and the other end of the connecting pipe 2802b is configured to be in communication with the recovery pack. The drive assembly 2801 and the reagent storage apparatus 2802 can be docked or separated, such that the output shaft 2801a can be docked with or separated from the squeezing assembly 2802c. When the drive assembly 2801 and the reagent storage apparatus 2802 are docked, the output shaft 2801a can drive the squeezing assembly 2802c to squeeze the connecting pipe 2802b to allow the liquid in the connecting pipe 2802b to flow.

The biological sample analysis device 28 further includes a mounting bracket 2803, the mounting bracket 2803 including a cavity 2803a and a first mounting position 2803b; where the reagent storage apparatus 2802 is able to move into or out of the cavity 2803a, and the drive assembly 2801 is arranged on the first mounting position 2803b; the first mounting position 2803b is arranged on an end of a travel of the reagent storage apparatus 2802 into the cavity 2803a.

The drive assembly 2801 is arranged outside the cavity 2803a, and an avoidance hole 2803c is defined between the cavity 2803a and the first mounting position 2803b, with the output shaft 2801a of the drive assembly 2801 extending into the cavity 2803a through the avoidance hole 2803c. When the reagent storage apparatus 2802 is moved into the cavity 2803a, the output shaft 2801a of the drive assembly 2801 is transmission-assembled with the squeezing assembly 2802c.

The squeezing assembly 2802c includes a first squeezing member 2802d and a second squeezing member 2802e, the second squeezing member 2802e being arranged coaxially with the first squeezing member 2802d and being embedded in the first squeezing member 2802d, and a part of the connecting pipe 2802b being arranged between the first squeezing member 2802d and the second squeezing member 2802e. The second squeezing member 2802e is configured to be transmission-assembled with the output shaft 2801a, such that the second squeezing member 2802e is driven by the output shaft 2801a to rotate relative to the first squeezing member 2802d and thereby squeeze the connecting pipe 2802b.

The first squeezing member 2802d is annular in shape, and the second squeezing member 2802e includes a rotating portion 2802f and a squeezing portion 2802g. The rotating portion 2802f is configured to dock with the output shaft 2801a, and the squeezing portion 2802g is disposed on a peripheral side of the rotating portion 2802f and can rotate synchronously with the rotating portion 2802f.

The squeezing portion 2802g is arranged spaced apart from the first squeezing portion 2802d, and the connecting pipe 2802b is partially arranged between the squeezing portion 2802g and the first squeezing portion 2802d.

The rotating portion 2802f defines a shaft hole 2802h, and an end of the output shaft 2801a is assembled with the shaft hole 2802h. The squeezing portion 2802g is sleeved with a shaft sleeve 2802i, which is configured to abut against the connecting pipe 2802b.

In particular, a cavity wall of the receiving cavity 2802a defines a storage groove 2802j, the squeezing assembly 2802c is embedded in the storage groove 2802j and arranged coaxially with the storage groove 2802j, and the connecting pipe 2802b is partially arranged between the storage groove 2802j and the squeezing assembly 2802c. The squeezing assembly 2802c is configured to be transmission-assembled with the output shaft 2801a, such that the squeezing assembly 2802c is driven by the output shaft 2801a to rotate relative to the storage groove 2802j and thereby squeeze the connecting pipe 2802b.

The reagent storage apparatus 2802 generally includes a receiving cavity 2802a, a connecting pipe 2802b, and a squeezing assembly 2802c. The receiving cavity 2802a is configured to hold a reagent pack and a recovery pack, and the connecting pipe 2802b is partially embedded in the squeezing assembly 2802c; an end of the connecting pipe 2802b is configured to be in communication with the reagent pack, and the other end of the connecting pipe 2802b is configured to be in communication with the recovery pack. The squeezing assembly 2802c is configured to dock with as well as separate from the output shaft of the drive assembly, such that when they are docked, the output shaft of the drive assembly drives the squeezing assembly 2802c to squeeze the connecting pipe 2802b to allow the liquid in the connecting pipe 2802b to flow.

The biological sample analysis device provided in the embodiments of the present disclosure can realize a detachable assembling of the drive assembly and reagent storage apparatus, by providing the drive assembly and reagent storage apparatus that can be docked or separated. When they are docked, the output shaft of the drive assembly can drive the squeezing assembly on the reagent storage apparatus to squeeze the connecting pipe to allow the liquid in the connecting pipe to flow. Therefore, there is no need to additionally provide a drive apparatus in the reagent storage apparatus to drive the flow of liquid, and the drive assembly and reagent storage apparatus may thus be detachably assembled for ease of replacement.

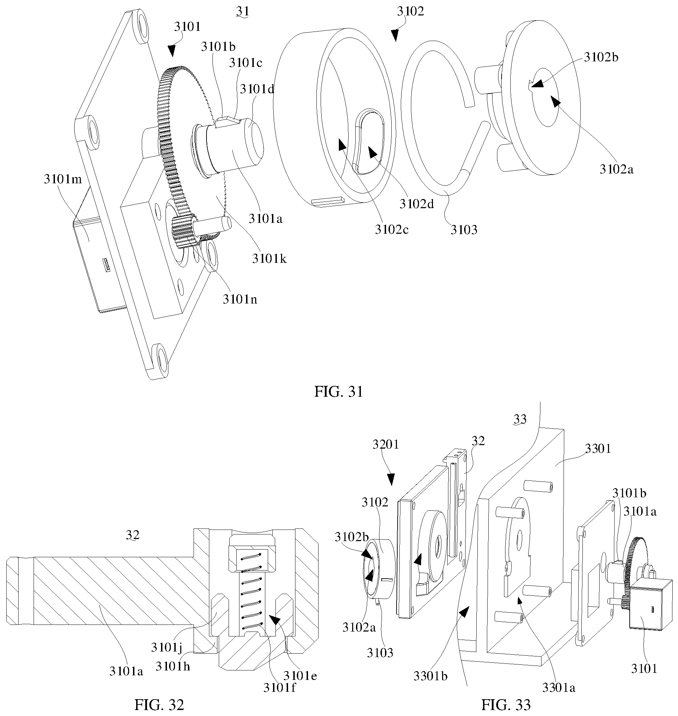

Referring to FIG. 31, FIG. 31 is a partially exploded structural schematic view of a liquid drive apparatus 31 according to some embodiments of the present disclosure. The liquid drive apparatus 31 generally includes a power assembly 3101, a rotatable assembly 3102, and a liquid pipe 3103. One of the power assembly 3101 and the rotatable assembly 3102 includes a rotating shaft 3101a, and the other includes a mating portion 3102a into which the rotating shaft 3101a can be inserted. One of the rotating shaft 3101a and the mating portion 3102a is arranged with a snap fastener 3101b, and the other of the rotating shaft 3101a and the mating portion 3102a defines a snap groove 3102b. The liquid pipe 3103 is arranged in the rotatable assembly 3102, and the rotatable assembly 3102 is rotatable to squeeze the liquid pipe 3103. The power assembly 3101 and the rotatable assembly 3102 can be docked or separated, such that the rotating shaft 3101a is inserted into the mating portion 3102a or pulled out of the mating portion 3102a; when the rotating shaft 3101a is inserted into the mating portion 3102a, the rotating shaft 3101a can rotate to engage the snap fastener 3101b and the snap groove 3102b to achieve a locking limit.

In particular, the snap fastener 3101b is arranged on the rotating shaft 3101a, the snap groove 3102b is arranged on the mating portion 3102a, and the snap fastener 3101b protrudes from a peripheral side of the rotating shaft 3101a when no external force is applied. When the rotating shaft 3101a is inserted into the mating portion 3102a, the snap fastener 3101b is restrained by the mating portion 3102a and is retracted in the rotating shaft 3101a. When the rotating shaft 3101a rotates relative to the mating portion 3102a until the snap fastener 3101b is opposite the snap groove 3102b, the snap fastener 3101b protrudes from the peripheral side of the rotating shaft 3101a and snaps into the snap groove 3102b.

The snap fastener 3101b is arranged on the mating portion 3102a, the snap groove 3102b is arranged on the rotating shaft 3101a, and the snap fastener 3101b protrudes from the mating portion 3102a when no external force is applied. When the rotating shaft 3101a is inserted into the mating portion 3102a, the snap fastener 3101b is restrained by the rotating shaft 3101a and is retracted in the mating portion 3102a. When the rotating shaft 3101a rotates relative to the mating portion 3102a until the snap fastener 3101b is opposite the snap groove 3102b, the snap fastener 3101b protrudes from the mating portion 3102a and snaps into the snap groove 3102b.

An end of the snap fastener 3101b that engages with the snap groove 3102b includes a guide ramp 3101c, and the guide ramp 3101c provides resistance when the rotating shaft 3101a is inserted into the mating portion 3102a such that the snap fastener 3101b is retracted.

In particular, an end of the rotating shaft 3101a includes a chamfer 3101d, and the chamfer 3101d is configured to guide the insertion of the rotating shaft 3101a in the mating portion 3102a.

Referring further to FIG. 32, FIG. 32 is a partial structural schematic view of the liquid drive apparatus 31 in FIG. 31. A mounting hole 3101e is defined on the rotating shaft 3101a or the mating portion 3102a, the snap fastener 3101b is assembled in the mounting hole 3101e, and an elastic member 3101f that abuts against the snap fastener 3101b is arranged in the mounting hole 3101e. The elastic member 3101f generates an elastic force when the snap fastener 3101b is retracted. When the snap fastener 3101b is opposite the snap groove 3102b, the elastic force causes the snap fastener 3101b to extend to snap with the snap groove 3102b.

A first limit portion 3101h is arranged in the mounting hole 3101e, and the snap fastener 3101b is arranged with a second limit portion 3101j. The first limit portion 3101h and the second limit portion 3101j cooperate to limit the extension travel of the snap fastener 3101b.

A first gear 3101k is sleeved on the rotating shaft 3101a, and the power assembly 3101 includes a drive member 3101m and a second gear 3101n, where the second gear 3101n is transmission-assembled with an output shaft of the drive member 3101m; the first gear 3101k is connected to the second gear 3101n by meshing, and the thickness of the first gear 3101k along the axial direction of the rotating shaft 3101a is less than the thickness of the second gear 3101n along the axial direction of the rotating shaft 3101a.

The first gear 3101k is spaced from the drive member 3101m along the axial direction of the rotating shaft 3101a.

The rotatable assembly 3102 has a holding space 3102c for holding the liquid pipe 3103 and a window 3102d communicating with the holding space 3102c, with two ends of the liquid pipe 3103 extending out of the holding space 3102c through the window 3102d.

Referring to FIG. 33, FIG. 33 is a partially exploded structural schematic view of a sample analysis apparatus 33 according to some embodiments of the present disclosure. The sample analysis apparatus 33 includes a mounting bracket 3301, the mounting bracket 3301 including a first mounting position 3301a and a second mounting position 3301b; where the first mounting position 3301a is configured to mount a reagent kit 32, and the second mounting position 3301b is configured to mount the power assembly 3101. The reagent kit 32 generally includes a receiving cavity 3201, a liquid pipe 3103, and a rotatable assembly 3102. The receiving cavity 3201 is configured to hold a reagent pack and a recovery pack, and the liquid pipe 3103 is arranged in the rotatable assembly 3102. The rotatable assembly 3102 is rotatable to squeeze the liquid pipe 3103, such that the liquid in the reagent pack can pass through the liquid pipe 3103 to reach the recovery pack. The rotatable assembly 3102 is configured to dock with as well as separate from the power assembly 3101. One of the power assembly 3101 rotatable assemblyand the power assembly 3101 includes a rotating shaft 3101a, and the other of the power assembly 3101 rotatable assemblyand the power assembly 3101 includes a mating portion 3102a into which the rotating shaft 3101a can be inserted. One of the rotating shaft 3101a and the mating portion 3102a is arranged with a snap fastener 3101b, and the other of the rotating shaft 3101a and the mating portion 3102a defines a snap groove 3102b. When the rotatable assembly 3102 is docked with the power assembly 3101, the rotating shaft 3101a is inserted into the mating portion 3102a, and the rotating shaft 3101a can rotate to engage the snap fastener 3101b and the snap groove 3102b to achieve a locking limit.

The power assembly 3101 is configured to dock with as well as separate from the rotatable assembly 3102 of the reagent kit 32. One of the power assembly 3101 and the rotatable assembly 3102 includes a rotating shaft 3101a, and the other includes a mating portion 3102a into which the rotating shaft 3101a can be inserted. One of the rotating shaft 3101a and the mating portion 3102a is arranged with a snap fastener 3101b, and the other of the rotating shaft 3101a and the mating portion 3102a defines a snap groove 3102b. When the rotatable assembly 3102 is docked with the power assembly 3101, the rotating shaft 3101a is inserted into the mating portion 3102a, and the rotating shaft 3101a can be rotated to cause the snap fastener 3101b and the snap groove 3102b to engage to achieve a locking limit.

The liquid drive apparatus provided in the embodiments of the present disclosure is arranged with a snap fastener on one of the rotating shaft and the mating portion, and a snap groove on the other. The snap fastener and the snap groove can be engaged to achieve a locking limit by rotating the rotating shaft. In this way, there is no need to align the snap fastener and the snap groove when assembling the rotating shaft and the mating portion, that is, after the rotating shaft and the mating portion are assembled, the rotating shaft can be rotated to achieve the locking limit of the snap fastener and the snap groove, thereby improving the assembly efficiency.

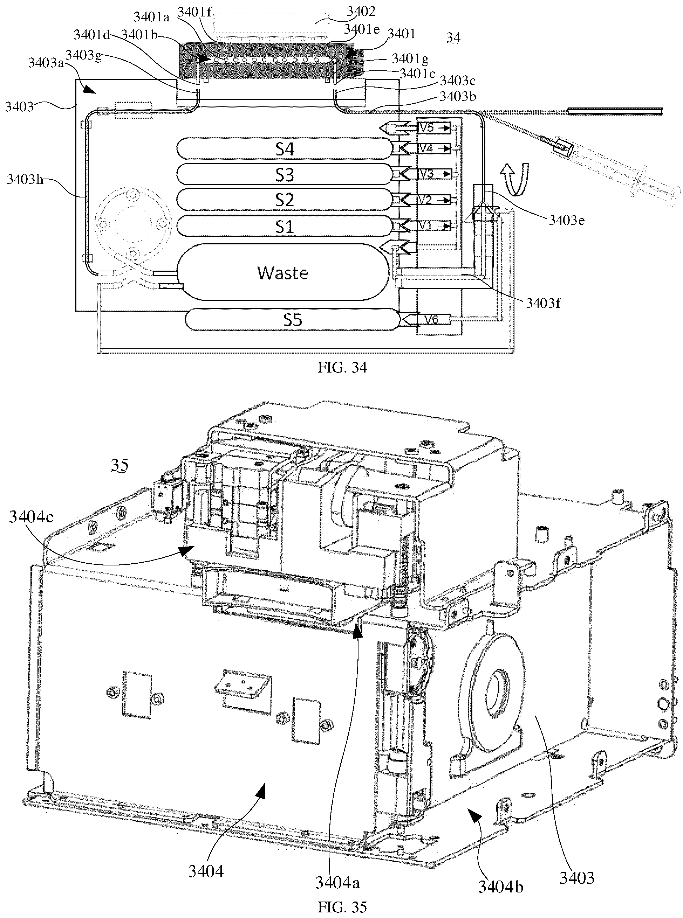

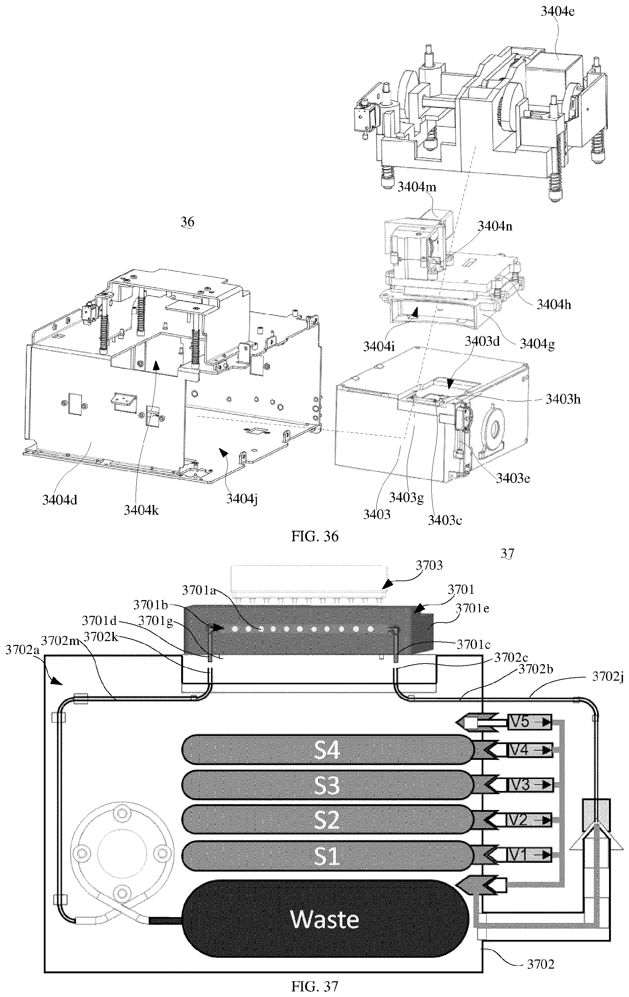

Referring to FIGS. 34 to 36, FIG. 34 is a schematic view of the liquid path structure of a medical detection device 34 according to some embodiments of the present disclosure, FIG. 35 is a structural schematic view of the medical detection device 34 according to some embodiments of the present disclosure, and FIG. 36 is an exploded structural schematic view of the medical detection device 34 in FIG. 35.

The medical detection device 34 generally includes a detection assembly 3401, a processing circuit 3402, and a reagent kit 3403. The detection assembly 3401 includes a detection element 3401a, a first liquid path 3401b, and a first port 3401c, the first liquid path 3401b being in communication with the first port 3401c, the detection element 3401a being configured to detect the liquid in the first liquid path 3401b to obtain a detection signal.

The processing circuit 3402 is detachably connected to the detection element 3401a of the detection assembly 3401 and is configured to receive the detection signal when the detection element 3401a is connected.

The reagent kit 3403 includes a cavity 3403a, a second liquid path 3403b, and a second port 3403c. The cavity 3403a is configured to hold a liquid pack. An end of the second liquid path 3403b is configured to be in communication with the liquid pack, and the other end is in communication with the second port 3403c.

The detection assembly 3401 and the reagent kit 3403 can be docked or separated to allow the first port 3401c and the second port 3403c to be connected or separated. When they are connected, the first liquid path 3401b is in communication with the second liquid path 3403b to allow the reagent in the liquid pack to reach the first liquid path 3401b. When the detection assembly 3401 and the reagent kit 3403 are separated to replace the reagent kit 3403, the processing circuit 3402 keeps electrically connected to the detection element 3401a of the detection assembly 3401.

The medical detection device 34 includes a mounting bracket 3404, the mounting bracket 3404 including a first mounting position 3404a, a second mounting position 3404b, and a third mounting position 3404c. The first mounting position 3404a is configured to mount the detection assembly 3401, the second mounting position 3404b is configured to place the reagent kit 3403, and the processing circuit 3402 is arranged in the third mounting position 3404c.

The detection assembly 3401 and the processing circuit 3402 are movable synchronously relative to the reagent kit 3403 while maintaining an electrical connection, such that the detection assembly 3401 can dock with or separate from the reagent kit 3403; and the reagent kit 3403 can be replaced while the detection assembly 3401 and the reagent kit 3403 are separated.

The mounting bracket 3404 includes a fixed bracket 3404d, a first drive mechanism 3404e, a first movable bracket 3404g, and a second movable bracket 3404h. The first movable bracket 3404g is arranged on the fixed bracket 3404d, and the first movable bracket 3404g is connected to the second movable bracket 3404h. The first movable bracket 3404g is configured to hold the detection assembly 3401, and the second movable bracket 3404h is configured to assemble the processing circuit 3402.

The detection assembly 3401 and the processing circuit 3402 undergo a relative movement driven by the first drive mechanism 3404e, such that the detection element 3401a of the detection assembly 3401 maintains electrically connected to or is disconnected from the processing circuit 3402; the first drive mechanism 3404e drives the first movable bracket 3404g to move such that the detection assembly 3401 can dock with or separate from the reagent kit 3403.

In particular, the first movable bracket 3404g defines a storage groove 3404i for accommodating the detection assembly 3401, the detection assembly 3401 can be moved in or out of the storage groove 3404i, and the second movable bracket 3404h is elastically connected to the first movable bracket 3404g. When the detection assembly 3401 is moved into the storage groove 3404i, the processing circuit 3402 can move towards the detection assembly 3401 and be electrically connected under the drive of the first drive mechanism 3404e.

The first movable bracket 3404h is elastically connected to the mounting bracket 3404. Driven by the first drive mechanism 3404f, the detection assembly 3401 can move towards the reagent kit 3403 to generate an elastic force and achieve the communication between the first port 3401c and the second port 3403c. The detection assembly 3401 can move away from the reagent kit 3403 by means of the elastic force to achieve the separation between the first port 3401c and the second port 3403c.

The mounting bracket 3404 includes a second drive mechanism 3404m and a liquid path selector 3404n. The second drive mechanism 3404m is arranged on the second movable bracket 3404h and is configured to drive the liquid path selector 3404n to move. When the detection assembly 3401 docks with the reagent kit 3403, the liquid path selector 3404n is driven by the second drive mechanism 3404m to enable the detection assembly 3401 to realize different liquid path circulations.

The fixed bracket 3404d has a cavity 3404j for accommodating the reagent kit 3403, and a cavity wall of the cavity 3404j defines an avoidance opening 3404k. The detection assembly 3401 is arranged outside the cavity 3404j. When the reagent kit 3403 is moved into the cavity 3404j, the second port 3403c of the reagent kit 3403 is exposed from the avoidance opening 3404k to achieve connection or separation with the first port 3401c of the detection assembly 3401.

A region in which the second port 3403c of the reagent kit 3403 is located forms a concave portion 3403d, the shape of which is adapted to the shape of the detection assembly 3401.

The reagent kit 3403 is arranged with a sampling element 3403e, a sample inlet of the sampling element 3403e being configured to selectively collect reagent from the liquid pack or liquid outside the reagent kit 3403, and a sample outlet of the sampling element 3403e being configured to be in communication with the second port 3403c. When it is required to collect the reagent from the liquid pack, the sampling element 3403e forms a part of the second liquid path 3403b.

The reagent kit 3403 includes a first connecting pipe 3403f, an end of the first connecting pipe 3403f being in communication with the liquid pack and the other end of the first connecting pipe 3403f being in communication with the sample inlet of the sampling element 3403e; the first connecting pipe 3403f forms another part of the second liquid path 3403b.

The detection assembly 3401 includes a third port 3401d spaced apart from the first port 3401c, and the third port 3401d is in communication with the first liquid path 3401b. The reagent kit 3403 includes a fourth port 3403g and a second connecting pipe 3403h, and the fourth port 3403g is spaced apart from the second port 3403c; an end of the second connecting pipe 3403h is in communication with the fourth port 3403g, and the other end of the second connecting pipe 3403h is in communication with the cavity; when the first port 3401c and the second port 3403c are in communication, the third port 3401d and the fourth port 3403g are in communication.

The detection assembly 3401 includes a housing 3401e, a detection element 3401a and a first liquid path 3401b that are disposed in the housing 3401e, and a first port 3401c and a connection terminal 3401f that are disposed on the housing 3401e, the connection terminal 3401f being electrically connected to the detection element 3401a.

The first liquid path 3401b is in communication with the first port 3401c, and the detection element 3401a is configured to perform a blood gas detection on the liquid in the first liquid path 3401b to obtain a detection signal. The first port 3401c is configured to dock with as well as separate from the second port 3403c of the reagent kit 3403 to obtain the reagent of the reagent kit 3403 through the first port 3401c when docking; when the detection assembly 3401 and the reagent kit 3403 are separated to replace the reagent kit 3403, the connection terminal 3401f remains electrically connected to the processing circuit 3402 of the medical detection device 34.

The housing 3401e is arranged with a first positioning member 3401g for positioning a bracket for mounting the detection assembly 3401 on the medical detection device 34.

An end of the first port 3401c that docks with the second port 3403c includes a tapered recess or tapered protrusion for guiding the second port 3403c to dock with the first port 3401c.

The reagent kit 3403 generally includes a cavity 3403a, a second liquid path 3403b, and a second port 3403c. The cavity 3403a is configured to hold the liquid pack. An end of the second liquid path 3403b is in communication with the liquid pack, and the other end of the second liquid path 3403b is in communication with to the second port 3403c. The second port 3403c is configured to dock with the first port 3401c of the detection assembly 3401 when a detection is performed using the reagent kit 3403, such that the reagent in the liquid pack is delivered to the detection assembly 3401 through the second port 3403c. The second port 3403c is separated from the detection assembly 3401 before the detection is performed using the reagent kit 3403. The reagent kit 3403 can be moved into or out of the mounting bracket of the medical detection device 34, and when the reagent kit 3403 is moved in, the first port 3401c and the second port 3403c are in communication.

A region in which the second port 3403c of the reagent kit 3403 is located forms a concave portion 3403d, the shape of which is adapted to the shape of the detection assembly 3401.

In particular, the concave portion 3403d is arranged with a second positioning member 3403h for positioning the detection assembly 3401.

The reagent kit 3403 is arranged with a sampling element 3403e, a sample inlet of the sampling element 3403e being configured to selectively collect reagent from the liquid pack or liquid outside the reagent kit 3403, and a sample outlet of the sampling element 3403e being configured to be in communication with the second port 3403c. When it is required to collect the reagent from the liquid pack, the sampling element 3403e forms a part of the second liquid path 3403b.

The detection assembly 3401 includes a third port 3401d spaced apart from the first port 3401c, and the reagent kit 3403 includes a fourth port 3403g spaced apart from the second port 3403c. When the first port 3401c and the second port 3403c are in communication, the third port 3401d and the fourth port 3403g are in communication.

The medical detection device 34 generally includes a mounting bracket 3404 and a processing circuit 3402. The mounting bracket 3404 includes a first mounting position 3404a, a second mounting position 3404b, and a third mounting position 3404c. The first mounting position 3404a is configured to mount the detection assembly 3401, and the second mounting position 3404b is configured to place the reagent kit 3403; the processing circuit 3402 is arranged on the third mounting position 3404c and is detachably connected to the detection assembly 3401.

The detection assembly 3401 and the reagent kit 3403 can be docked or separated to allow the detection assembly 3401 and the reagent kit 3403 to be connected or separated. When they are connected, the reagent in the reagent kit 3403 can reach the detection assembly 3401. When the detection assembly 3401 and the reagent kit 3403 are separated to replace the reagent kit 3403, the processing circuit 3402 remains electrically connected to the detection assembly 3401.

The processing circuit 3402 is arranged on the mounting bracket 3404, which includes a first mounting position 3404a, a second mounting position 3404b, and a third mounting position 3404c. The first mounting position 3404a is configured to mount the detection assembly 3401, the second mounting position 3404b is configured to place the reagent kit 3403, and the processing circuit 3402 is arranged on the third mounting position 3404c.

The detection assembly 3401 and the processing circuit 3402 are movable synchronously relative to the reagent kit 3403 while maintaining an electrical connection, such that the detection assembly 3401 can dock with or separate from the reagent kit 3403. The reagent kit 3403 can be replaced while the detection assembly 3401 is separated from the reagent kit 3403.

The mounting bracket 3404 includes a fixed bracket 3404d, a first drive mechanism 3404e, a first movable bracket 3404g, and a second movable bracket 3404h. The first movable bracket 3404g is arranged on the fixed bracket 3404d, and the first movable bracket 3404g is connected to the second movable bracket 3404h. The first movable bracket 3404g is configured to hold the detection assembly 3401, and the second movable bracket 3404h is configured to assemble the processing circuit 3402. The detection assembly 3401 and the processing circuit 3402 undergo a relative movement driven by the first drive mechanism 3404e, such that the detection element 3401a of the detection assembly 3401 maintains electrically connected to or is disconnected from the processing circuit 3402; the detection assembly 3401 drives the first movable bracket 3404g to move under the drive of the first drive mechanism 3404e, such that the detection assembly 3401 can dock with or separate from the reagent kit 3403.

In particular, the first movable bracket 3404g defines a storage groove 3404i for accommodating the detection assembly 3401, the detection assembly 3401 can be moved in or out of the storage groove 3404i, and the second movable bracket 3404h is elastically connected to the first movable bracket 3404g. When the detection assembly 3401 is moved into the storage groove 3404i, the processing circuit 3402 can move towards the detection assembly 3401 and be electrically connected under the drive of the first drive mechanism 3404e.

The first movable bracket 3404h is elastically connected to the mounting bracket 3404. Driven by the first drive mechanism 3404f, the detection assembly 3401 can move towards the reagent kit 3403 to generate an elastic force and achieve the communication between the first port 3401c and the second port 3403c. The detection assembly 3401 can move away from the reagent kit 3403 by means of the elastic force to achieve the separation between the first port 3401c and the second port 3403c.

The mounting bracket 3404 includes a second drive mechanism 3404m and a liquid path selector 3404n. The second drive mechanism 3404m is arranged on the second movable bracket 3404h and is configured to drive the liquid path selector 3404n to move. When the detection assembly 3401 docks with the reagent kit 3403, the liquid path selector 3404n is driven by the second drive mechanism 3404m to enable the detection assembly 3401 to realize different liquid path circulations.

The medical testing device provided in the embodiments of the present disclosure allows the testing assembly and the reagent kit to be docked or separated, such that when the first port and the second port are in communication, the first liquid path is in communication with the second liquid path, allowing the reagent in the liquid pack to reach the first liquid path for detection. Furthermore, when the detection assembly and the reagent kit are separated to replace the reagent kit, the processing circuit remains electrically connected to the detection element of the detection assembly, thereby preventing the power failure between the processing circuit and the detection element from affecting the continuity of subsequent detection and the accuracy of the detection data.

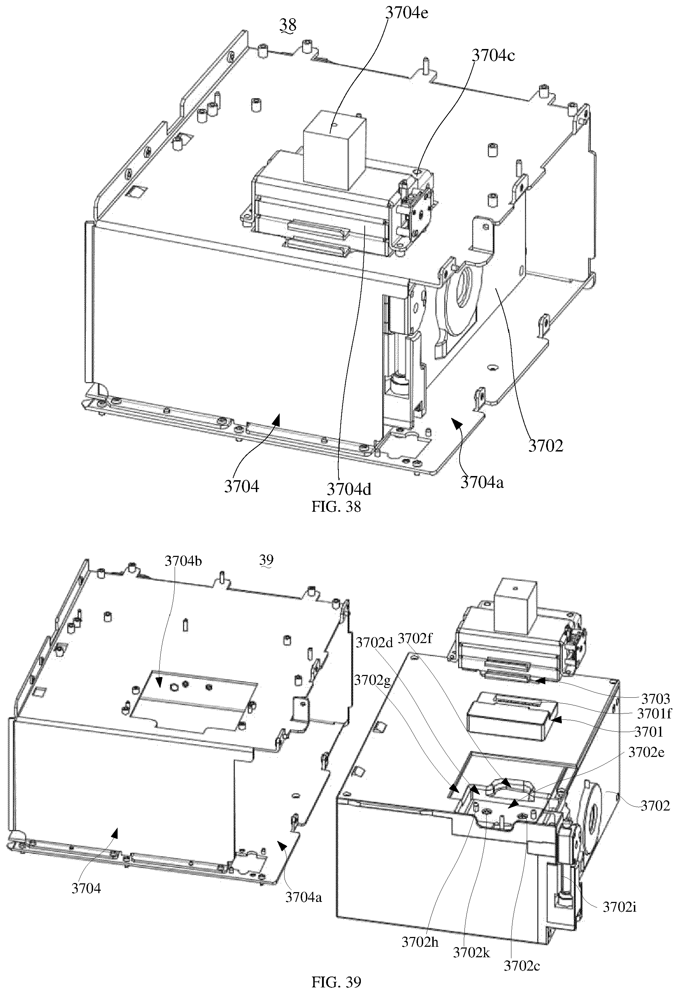

Referring to FIGS. 37 to 39, FIG. 37 is a schematic view of a liquid path structure of a medical detection device 37 according to some embodiments of the present disclosure, FIG. 38 is a structural schematic view of a medical detection device 37 according to some embodiments of the present disclosure, and FIG. 39 is an exploded structural schematic view of the medical detection device 37 in FIG. 38.

The medical detection device 37 generally includes a detection assembly 3701, a reagent kit 3702, and a processing circuit 3703. The detection assembly 3701 includes a detection element 3701a, a first liquid path 3701b, and a first port 3701c, the first liquid path 3701b being in communication with the first port 3701c, the detection element 3701a being configured to detect the liquid in the first liquid path 3701b to obtain a detection signal;
The reagent kit 3702 includes a cavity 3702a, a second liquid path 3702b, and a second port 3702c. The cavity 3702a is configured to hold a liquid pack. An end of the second liquid path 3702b is configured to be in communication with the liquid pack, and the other end is in communication with the second port 3702c.

The processing circuit 3703 is detachably connected to the detection element 3701a of the detection assembly 3701 for receiving the detection signal when the detection element 3701a is connected; when the processing circuit 3703 and the detection element 3701a are disassembled, the detection assembly 3701 can be disassembled synchronously with the reagent kit 3702.

The detection assembly 3701 is detachably arranged on the reagent kit 3702 such that the first port 3701c and the second port 3702c can be connected or separated. When they are connected, the first liquid path 3701b is in communication with the second liquid path 3702b such that the reagent in the liquid pack can reach the first liquid path 3701b.

The medical detection device 37 includes a mounting bracket 3704 including a first mounting position 3704a for positioning the reagent kit 3702, where the reagent kit 3702 includes a second mounting position 3702d for mounting the detection assembly 3701.

The mounting bracket 3704 is arranged with an avoidance channel 3704b that avoids the second mounting position 3702d, such that the detection element 3701a of the detection assembly 3701 can protrude from the avoidance channel 3704b and be connected to the processing circuit 3703.

The mounting bracket 3704 includes a fixed bracket 3704c, and a movable bracket 3704d and a drive mechanism 3704e that are arranged on the fixed bracket 3704c. The movable bracket 3704d is configured to place the processing circuit 3703, and the detection assembly 3701 and the processing circuit 3703 undergo a relative movement under the drive of the drive mechanism 3704e, such that the detection element 3701a of the detection assembly 3701 comes into contact or breaks contact with the processing circuit 3703; when the detection element 3701a breaks contact with the processing circuit 3703, the detection assembly 3701 can be disassembled synchronously with the reagent kit 3702.

A region in which the second port 3702c of the reagent kit 3702is located forms a concave portion 3702e, the shape of which is adapted to the shape of the detection assembly 3701.

In particular, the depth of the concave portion 3702e is greater than or equal to the thickness of the detection assembly 3701.

A side of the concave portion 3702e defines an avoidance notch 3702f, which is configured to remove the detection assembly 3701 disposed on the concave portion 3702e.

A peripheral portion of the concave portion 3702e defines a positioning groove 3702g in communication with the concave portion 3702e, and the positioning groove 3702g is configured to guide the processing circuit 3703 into contact with the detection element 3701a of the detection assembly 3701.

A region in which the second port 3702c of the reagent kit 3702 is located in formed with a first positioning portion 3702h, and the first positioning portion 3702h is configured to guide the detection assembly 3701 to be mounted on the second mounting position 3702d, thereby guiding the first port 3701c and the second port 3702c to dock with each other.

The reagent kit 3702 is arranged with a sampling element 3702i, a sample inlet of the sampling element 3702i being configured to selectively collect reagent from the liquid pack or liquid outside the reagent kit 3702, and a sample outlet of the sampling element 3702i being configured to be in communication with the second port 3702c. When it is required to collect the reagent from the liquid pack, the sampling element 3702i forms a part of the second liquid path 3702b.

The reagent kit 3702 includes a first connecting pipe 3702j, an end of the first connecting pipe 3702j being in communication with the liquid pack and the other end of the first connecting pipe 3702j being in communication with the sample inlet of the sampling element 3702i; the first connecting pipe 3702j forms another part of the second liquid path 3702b.

The detection assembly 3701 includes a third port 3701d spaced apart from the first port 3701c, and the third port 3701d is in communication with the first liquid path 3701b; the reagent kit 3702 includes a fourth port 3702k and a second connecting pipe 3702m, and the fourth port 3702k is spaced apart from the second port 3702c; an end of the second connecting pipe 3702m is in communication with the fourth port 3702k, and the other end of the second connecting pipe 3702m is in communication with the cavity3702a; when the first port 3701c and the second port 3702c are in communication, the third port 3701d and the fourth port 3702k are in communication.

The detection assembly 3701 generally includes a housing 3701e, a detection element 3701a and a first liquid path 3701b that are disposed in the housing 3701e, and a first port 3701c and a connection terminal 3701f that are disposed on the housing 3701e, the connection terminal 3701f being electrically connected to the detection element 3701a.

The first liquid path 3701b is in communication with the first port 3701c, and the detection element 3701a is configured to perform a blood gas detection on the liquid in the first liquid path 3701b to obtain a detection signal. The first port 3701c is configured to dock with as well as separate from the second port 3702c of the reagent kit 3702 to obtain the reagent of the reagent kit 3702 through the first port 3701c when docking; the detection assembly 3701 is configured to be arranged on the reagent kit 3702, and the connection terminal 3701f is configured to be electrically connected to the processing circuit 3703 of the medical detection device 37.

A region in which the second port 3702c of the reagent kit 3702 is located in formed with a first positioning portion 3702h, and the housing 3701e is arranged with a second positioning portion 3701g. The first positioning portion 3702h and the second positioning portion 3701g cooperate to guide the mounting of the detection assembly 3701 on the reagent kit 3702.

A region in which the second port 3702c of the reagent kit 3702 is located defines a positioning groove 3702g, and the positioning groove 3702g is configured to guide the processing circuit 3703 into contact with the detection element 3701a of the detection assembly 3701.

An end of the first port 3701c that docks with the second port 3702c includes a tapered recess or tapered protrusion to guide the second port 3702c to dock with the first port 3701c.

The reagent kit 3702 includes a cavity 3702a, a second liquid path 3702b, and a second port 3702c. The cavity 3702a is configured to hold a liquid pack. An end of the second liquid path 3702b is configured to be in communication with the liquid pack, and the other end is in communication with the second port 3702c. The second port 3702c is configured to dock with the first port 3701c of the detection assembly 3701, such that when they are docked, the reagent in the liquid pack is delivered to the detection assembly 3701 through the second port 3702c. The detection assembly 3701 is detachably arranged on the reagent kit 3702, and when the reagent kit 3702 is disassembled, the detection assembly 3701 can be simultaneously disassembled with the reagent kit 3702.

The medical detection device 37 includes a mounting bracket 3704, the mounting bracket 3704 having a first mounting position 3704a for detachably mounting the reagent kit 3702; the reagent kit 3702 is arranged with a second mounting position 3702d for detachably mounting the detection assembly 3701.

The detection assembly 3701 and the reagent kit 3702 can be docked or separated to allow the detection assembly 3701 and the reagent kit 3702 to be connected or separated. When they are connected, the reagent in the reagent kit 3702 can reach the detection assembly 3701. When the reagent kit 3702 is disassembled, the detection assembly 3701 can be disassembled synchronously with the reagent kit 3702.

The medical detection device 37 further includes a processing circuit 3703, and the mounting bracket 3704 is arranged with an avoidance passage 3704b that avoids the second mounting position 3702d, such that the detection element 3701a of the detection assembly 3701 can protrude from the avoidance passage 3704b and thereby enable an electrical connection to the processing circuit 3703.

The mounting bracket 3704 includes a fixed bracket 3704c, and a movable bracket 3704d and a drive mechanism 3704e that are arranged on the fixed bracket 3704c. The movable bracket 3704d is configured to place the processing circuit 3703, and the detection assembly 3701 and the processing circuit 3703 undergo a relative movement under the drive of the drive mechanism 3704e, such that the detection element 3701a of the detection assembly 3701 maintains electrically connected to or is disconnected from the processing circuit 3703; when the detection element 3701a is electrically disconnected from the processing circuit 3703, the detection assembly 3701 can be disassembled synchronously with the reagent kit 3702.

The medical detection device provided in the embodiments of the present disclosure is arranged with a detection assembly detachably arranged on the reagent kit, such that when the first port and the second port are connected, the reagent in the liquid pack of the reagent kit can reach the first liquid path of the detection assembly for detection to obtain a detection signal. The processing circuit is further detachably connected to the detection element of the detection assembly for receiving the detection signal when the detection element is connected. In addition, when the processing circuit and the detection element are disassembled, the detection assembly can be disassembled simultaneously with the reagent kit to avoid disassembling the detection assembly and the reagent kit separately, thereby improving the ease of disassembly.

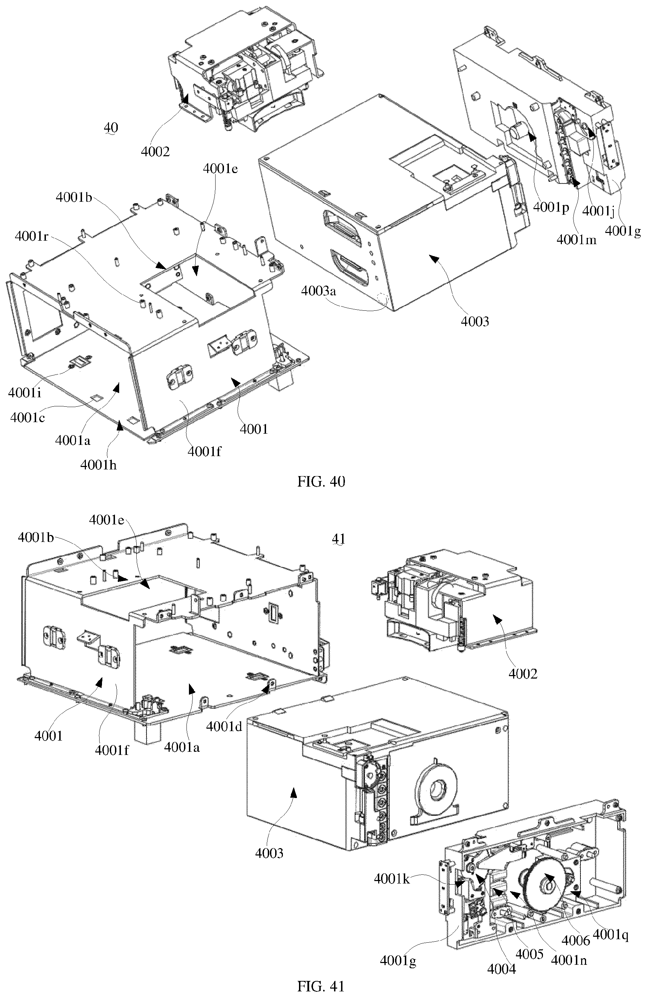

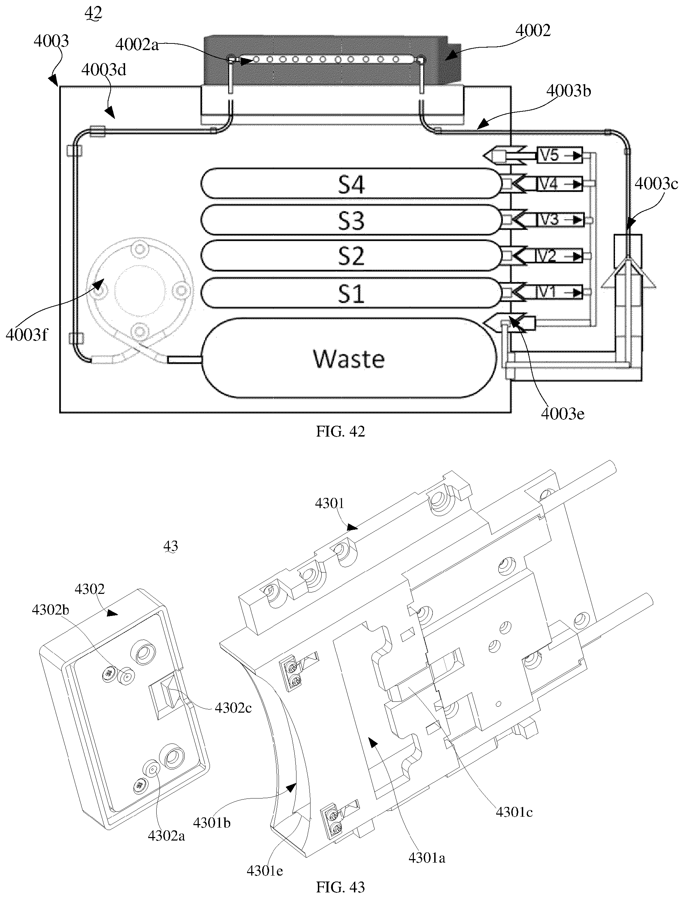

Referring to FIGS. 40 to 42, FIG. 40 is a structural schematic view of a biological tissue detection apparatus 40 according to some embodiments of the present disclosure, FIG. 41 is another structural schematic view of the biological tissue detection apparatus 40 in FIG. 40, and FIG. 42 is a schematic view of a liquid path structure of the biological tissue detection apparatus 40 in FIG. 40.

The biological tissue detection apparatus 40 generally includes a bracket 4001, the bracket 4001 including a storage space 4001a and a first mounting position 4001b; where the first mounting position 4001b is configured to mount a sample measuring device 4002, and the storage space 4001 a is configured to move in or out a reagent kit 4003; the reagent kit 4003 is configured to convey liquid to the sample measuring device 4002 to assist the sample measuring device 4002 in measuring the sample, and the sample measuring device 4002 is configured to measure the liquid flowing into the sample measuring device.

The dimension of the receiving space 4001a in the moving direction of the reagent kit 4003 is larger than an intended placement dimension of the reagent kit 4003, and the bracket 4001 is arranged with a limit portion 4001c and an elastic portion 4001d; where the limit portion 4001c is configured to position the reagent kit 4003, and the elastic member 4001d is configured to provide elastic force when the reagent kit 4003 is moved into the storage space 4001a, such that the reagent kit 4003 retracts part of the travel and is restrained by the limit portion 4001c after being moved into the storage space 4001a.

The bracket 4001 includes multiple plates, where the multiple plates are connected to each other to form the storage space 4001a and the first mounting position 4001b.

An avoidance window 4001e is arranged between the storage space 4001a and the first mounting position 4001b, and the avoidance window 4001e is configured to avoid mechanical docking operations and the liquid delivery path between the sample measuring device 4002 and the reagent kit 4003, the liquid delivery path being a path connecting a first pipe of the sample measuring device 4002 and a second pipe of the reagent kit 4003; the first pipe and the second pipe are the delivery pipes for the liquid.

The multiple plates include a frame plate 4001f connected end to end and a mounting plate 4001g disposed on a side of the frame plate 4001f, the frame plate 4001f and the mounting plate 4001g cooperate to enclose for defining the storage space 4001a, and the first mounting position 4001b and the avoidance window 4001e are arranged on the frame plate 4001f. A side of the frame plate 4001f away from the mounting plate 4001g defines a pick-up and placement opening 4001h for the reagent kit 4003 to move in or out.

The frame plate 4001f is arranged with at least one guide member 4001i, the guide member 4001i being configured to guide the reagent kit 4003 to move into or out of the storage space 4001a.

The elastic member 4001d is arranged on the mounting plate 4001g or the frame plate 4001f, and when the reagent kit 4003 is moved into the storage space 4001a, the elastic member 4001d is compressed or stretched to generate an elastic force in the direction of the reagent kit 4003 moving out of the storage space 4001a.

The limit portion 4001c is arranged on the frame plate 4001f and is arranged adjacent to the pick-up and placement opening 4001h in the moving direction of the reagent kit 4003 out of the storage space 4001a. The reagent kit 4003 is arranged with an engagement portion 4003a corresponding to the limit portion 4001c, and the engagement portion 4003a cooperates with the limit portion 4001c to position the reagent kit 4003 when the reagent kit 4003 retracts part of the travel after being moved into the storage space 4001a.

The mounting plate 4001g includes a first docking port 4001j and a second mounting position 4001k. The first docking port 4001j is in communication with the storage space 4001a, and the second mounting position 4001k is configured to mount a regulating device 4004. When the reagent kit 4003 is moved into the storage space 4001a, a sampling element on the reagent kit 4003 is exposed via the first docking port 4001j to dock with the regulating device 4004, such that the sampling element can obtain liquid inside or outside the reagent kit 4003 and cause the liquid to enter the second pipe, under the regulation of the regulating device 4004.

The mounting plate 4001g includes a second docking port 4001m and a third mounting position 4001n. The second docking port 4001m is in communication with the storage space 4001a, and the third mounting position 4001n is configured to mount a valve apparatus 4005. When the reagent kit 4003 is moved into the storage space 4001a, a liquid pack connector within the reagent kit 4003 is exposed via the second docking port 4001m to dock with the valve apparatus 4005, such that the valve apparatus 4005 can control the liquid in the reagent kit 4003 to flow into the second pipe. When the sampling element docks with the reagent kit 4003, the sampling element is in communication with a liquid outlet of the valve apparatus 4005 such that the sampling element can obtain the liquid in the reagent kit 4003 and cause the liquid to enter the second pipe.

The mounting plate 4001g includes a third docking port 4001p and a fourth mounting position 4001q. The third docking port 4001p is in communication with the storage space 4001a, and the fourth mounting position 4001q is configured to mount a drive apparatus 4006. When the reagent kit 4003 is moved into the storage space 4001a, the drive apparatus 4006 docks with the reagent kit 4003 via the third docking port 4001p to drive the liquid in the second pipe to flow.

The avoidance window 4001e extends through the first mounting position 4001b, where the first mounting position 4001b includes a positioning member 4001r for positioning the sample measuring device 4002 on the frame plate 4001f.

The biological tissue detection apparatus 40 generally includes a bracket 4001, a sample measuring device 4002 and a reagent kit 4003, where the bracket 4001 includes a storage space 4001a and a first mounting position 4001b; the sample measuring device 4002 is detachably arranged on the first mounting position 4001b and includes a first pipe 4002a; the reagent kit 4003 can be moved into and out of the storage space 4001a, and the reagent kit 4003 includes a second pipe 4003b; the dimension of the storage space 4001a in the moving direction of the reagent kit 4003 is larger than an intended placement dimension of the reagent kit 4003; the bracket 4001 is arranged with a limit portion 4001c and an elastic member 4001d, the limit portion 4001c being configured to position the reagent kit 4003, and the elastic member 4001d being configured to provide elastic force for the reagent kit 4003 to move into the storage space 4001a, such that the reagent kit 4003 retracts part of the travel and is restrained by the limit portion 4001c after being moved into the storage space 4001a.

The reagent kit 4003 is arranged with a sampling element 4003c, the biological tissue detection apparatus 40 includes an regulating device 4004, and the regulating device 4004 is configured to adjust the sampling posture of the sampling element 4003c; the mounting plate 4001g includes a first docking port 4001j and a second mounting position 4001k, the first docking port 4001j is in communication with the storage space 4001a, and the second mounting position 4001k is configured to mount the regulating device 4004; when the reagent kit 4003 is moved into the storage space 4001a, the sampling element 4003c is docked with the regulating device 4004, such that the sampling element can obtain liquid inside or outside the reagent kit 4003 and cause the liquid to enter the second pipe, under the regulation of the regulating device 4004.

The reagent kit 4003 includes a receiving cavity 4003d for accommodating a liquid pack, and a cavity wall of the receiving cavity 4003d is arranged with a liquid pack connector 4003e. The biological tissue detection apparatus 40 includes a valve apparatus 4005 for docking with the liquid pack connector 4003e to control the outflow of liquid from the liquid pack. The mounting plate 4001g includes a second docking port 4001m and a third mounting position 4001n. The second docking port 4001m is in communication with the storage space 4001a, and the third mounting position 4001n is configured to mount the valve apparatus 4005. When the reagent kit 4003 is moved into the storage space 4001a, the liquid pack connector 4003e is docked with the valve apparatus 4005, such that the valve apparatus 4005 can control the liquid in the reagent kit 4003 to flow into the second pipe. When the sampling element docks with the reagent kit 4003, the sampling element is in communication with a liquid outlet of the valve apparatus 4005 such that the sampling element can obtain the liquid in the reagent kit 4003 and cause the liquid to enter the second pipe.

The reagent kit 4003 includes a receiving cavity 4003d for accommodating the liquid pack, and a cavity wall of the receiving cavity 4003d is arranged with a first drive member 4003f. The biological tissue detection apparatus 40 includes a drive apparatus 4006 configured to dock with the first drive member 4003f to drive the liquid in the liquid pack to flow out. The mounting plate 4001g includes a third docking port 4001p and a fourth mounting position 4001q. The third docking port 4001p is in communication with the storage space 4001a, and the fourth mounting position 4001q is configured to mount the drive apparatus 4006. When the reagent kit 4003 is moved into the storage space 4001a, the drive apparatus 4006 is docked with the first drive member 4003f.

**In** the biological tissue detection apparatus provided in the embodiments of the present disclosure, a limit portion is arranged on the bracket to position the reagent kit when it is moved into the storage space of the bracket, and an elastic member is arranged on the bracket to provide an elastic force when the reagent kit is moved into the storage space. The elastic force allows the reagent kit to retract part of the travel after it is moved into the solute space to resist against the limit member, so as to facilitate the positioning of the reagent kit in the storage space.

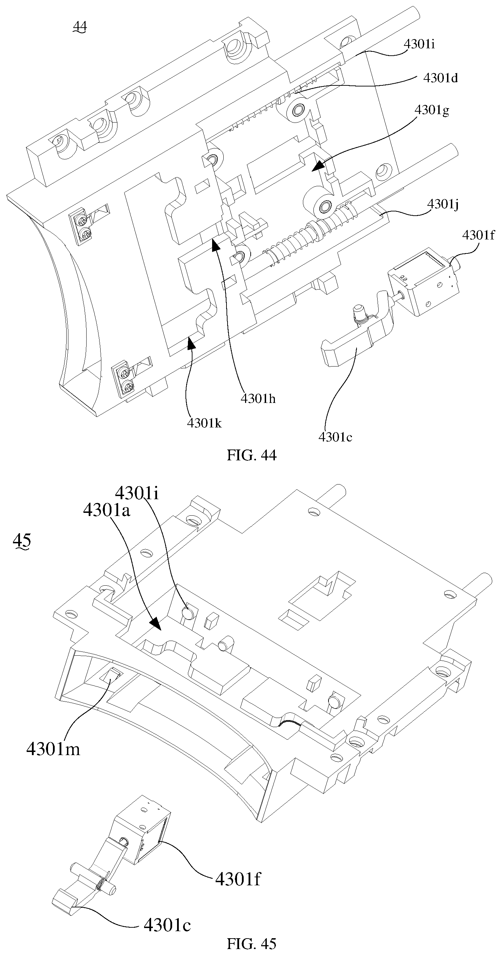

Referring to FIGS. 43 to 45, FIG. 43 is a structural schematic view of a sample analysis apparatus 43 according to some embodiments of the present disclosure, FIG. 44 is a partially exploded structural schematic view of the sample analysis apparatus 43 in FIG. 43, and FIG. 45 is another partially exploded structural schematic view of the sample analysis apparatus 43 in FIG. 43.

The sample analysis apparatus 43 generally includes a seat body 4301. The seat body 4301 defines a storage groove 4301a and a pick-up and placement opening 4301b in communication with the storage groove 4301a, the storage groove 4301a being configured to move in or out a detection card 4302, such that the detection card 4302 can be moved in or out of the storage groove 4301a from the pick-up and placement opening 4301b; at least one of the storage groove 4301a and detection card 4302 is arranged with a locking member 4301c, the locking member 4301c being configured to lock the detection card 4302 in the seat body 4301 when the detection card 4302 is moved into the storage groove 4301a; at least one of the seat body 4301 and the detection card 4302 is arranged with an elastic member 4301d; when the detection card 4302 is moved into the storage groove 4301a, the detection card 4302 squeezes or stretches the elastic member 4301d such that the elastic member 4301d deforms and generates an elastic force; and when the locking member 4301c releases the locking and fixing of the detection card 4302, the detection card 4302 pops up from the pick-up and placement opening 4301b under the action of the elastic force.

In particular, an inner wall of the pick-up and placement opening 4301b is arranged with a damping portion 4301e, and the damping portion 4301e is configured to limit the pop-up travel of the detection card 4302.

The seat body 4301 is arranged with a drive member 4301f, and the drive member 4301f is configured to drive the locking member 4301c to release the locking of the detection card 4302.

The seat body 4301 defines a receiving groove 4301g, the receiving groove 4301g and the storage groove 4301a are arranged at intervals in the moving direction of the detection card 4302, and the drive member 4301f is arranged in the receiving groove 4301g; an end of the locking member 4301c is assembled and connected to the drive member 4301f, and the other end of the locking member 4301c is disposed in the storage groove 4301a to lock and fix the detection card 4302 in the storage groove 4301a.

The seat body 4301 defines an avoidance space 4301h in communication with the storage groove 4301a and the receiving groove 4301g, and the locking member 4301c is at least partially disposed in the avoidance space 4301h; where the locking member 4301c is rotatably connected to the seat body 4301 to rotate under the drive of the drive member 4301f and thereby release the locking of the detection card 4302.

The elastic member 4301d is arranged in the receiving groove 4301g, the seat body 4301 is arranged with a supporting member 4301i; an end of the supporting member 4301i is assembled and connected to the elastic member 4301d, and the other end of the supporting member 4301i is configured to support the detection card 4302; when the detection card 4302 is moved into the storage groove 4301a, the detection card 4302 abuts against the supporting member 4301i such that the elastic member 4301d deforms and generates an elastic force.

The receiving groove 4301g is arranged with a limit portion 4301j, and the limit portion 4301j is configured to limit the movement travel of the supporting member 4301i.

The elastic member 4301d is sleeved on the supporting member 4301i, and when the detection card 4302 is moved into the storage groove 4301a, the detection card 4302 abuts against the supporting member 4301i such that the elastic member 4301d abuts against the limit portion 4301j and generates an elastic force.

A side of the detection card 4302 is arranged with a first port 4302a, a second port 4302b, and a snap-fit portion 4302c, and the storage groove 4301a includes an avoidance opening 4301k. When the detection card 4302 is moved into the storage groove 4301a, the first port 4302a and the second port 4302b are exposed from the avoidance opening 4301k for communication to an external liquid path, and the snap-fit portion 4302c is locked and fixed with the locking member 4301c.

In particular, the storage groove 4301a is arranged with at least one elastic tab 4301m in a region adjacent to the pick-up and placement opening 4301b, and the elastic tab 4301m is configured to position the detection card 4302 when the detection card 4302 is moved into the storage groove 4301a.

The sample analysis apparatus provided in the embodiments of the present disclosure facilitates the placement of the detection card on the seat body by providing a storage groove on the seat body for moving the detection card in or out. Further, the detection card can be locked to the seat body by a locking member to facilitate the corresponding detection. In addition, an elastic member is provided to eject the detection card when the locking member releases the lock on the detection card, so as to facilitate replacement.

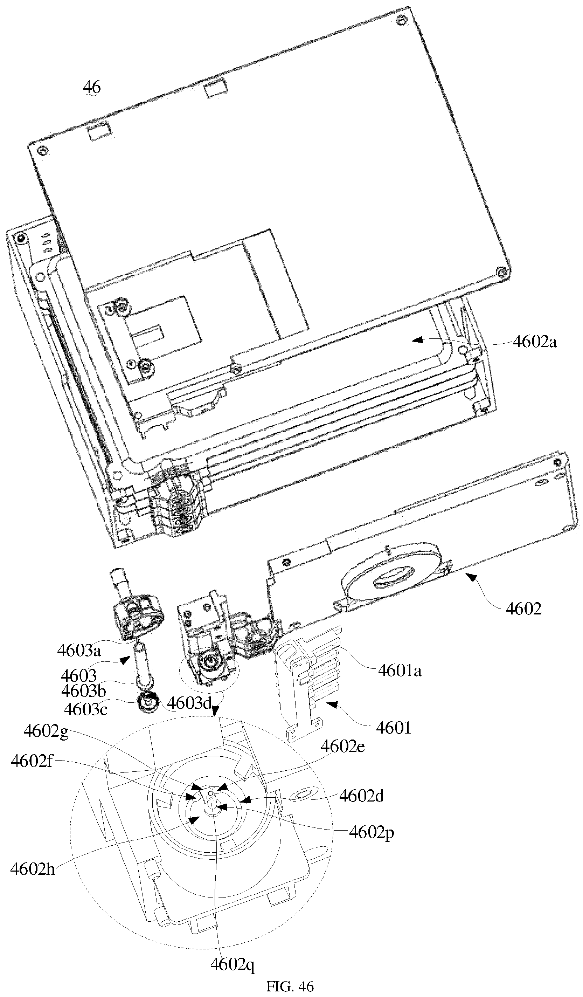

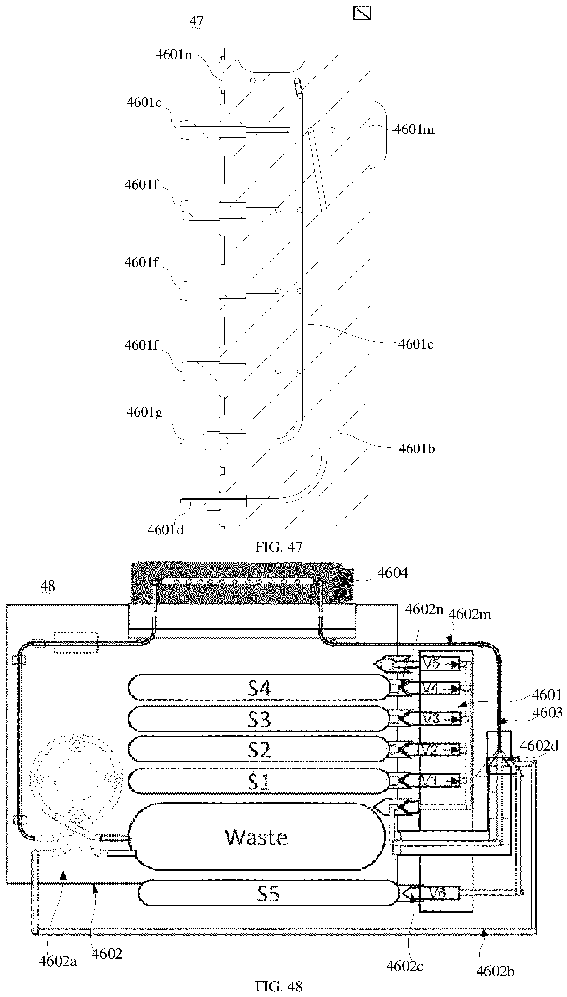

Referring to FIGS. 46 to 48, FIG. 46 is an exploded structural schematic view of a fluid detection instrument 46 according to some embodiments of the present disclosure, FIG. 47 is a cross-sectional structural schematic view of a valve assembly of the fluid detection instrument 46 in FIG. 46, and FIG. 48 is a schematic view of a liquid path structure of the fluid detection instrument 46 in FIG. 46.

The fluid detection instrument 46 generally includes a valve assembly 4601 and a reagent kit 4602.

The valve assembly 4601 includes a control element 4601a, a first pipe 4601b, a first inlet 4601c, and a first outlet 4601d, the control element 4601a being configured to control the first inlet 4601c to be in communication with an end of the first pipe 4601b, and the other end of the first pipe 4601b is in communication with the first outlet 4601d.

The reagent kit 4602 includes a cavity 4602a, a second pipe 4602b, and a first port 4602c, the cavity 4602a being configured to place a reagent pack and a recovery pack, the first port 4602c being configured to connect the reagent pack to the first inlet 4601c, and the first outlet 4601d being in communication with a docking groove 4602d defined on the reagent kit 4602. The docking groove 4602d is configured to dock with a sampling element 4603, where an end of the second pipe 4602b is in communication with the docking groove 4602d, and the other end of the second pipe 4602b is in communication with the recovery pack.

The valve assembly 4601 and the reagent kit 4602 can be docked or separated to allow the first inlet 4601c and the first port 4602c to be connected or separated. When they are connected, the first pipe 4601b can be in communication with the docking groove 4602d under the control of the control element 4601a, such that the liquid in the reagent pack can reach the docking groove 4602d, and the liquid in the docking groove 4602d can reach the recovery pack through the second pipe 4602b.

The docking groove 4602d is arranged with a liquid inlet hole 4602e and a liquid outlet hole 4602f. The liquid inlet hole 4602e is configured to be in communication with the first outlet 4601d, and the liquid outlet hole 4602f is configured to be in communication with the second pipe 4602b.

The docking groove 4602d is arranged with an isolation portion 4602g disposed between the liquid inlet hole 4602e and the liquid outlet hole 4602f, and a flow channel 4602h in communication with the liquid inlet hole 4602e and the liquid outlet hole 4602f and bypassing the isolation portion 4602g.

The sampling element 4603 includes a sampling needle 4603a, a sleeve 4603b sleeved on the sampling needle 4603a, and a docking member 4603c arranged on an end of the sleeve 4603b. The outer diameter of the sleeve 4603b does not exceed the inner diameter of the docking groove 4602d, and the docking member 4603c defines a through hole 4603d; the docking member 4603c can move along the axial direction of the sampling needle 4603a following the sleeve 4603b such that the sampling needle 4603a is inserted into an end of the through hole 4603d or passes through the through hole 4603d and is exposed on a side of the docking member 4603c.

The valve assembly 4601 further includes a third pipe 4601e, a third inlet 4601f, and a third outlet 4601g; where the control element 4601a is configured to control the third inlet 4601f to be in communication with an end of the third pipe 4601e, and the other end of the third pipe 4601e is in communication with the third outlet 4601g.

The reagent kit 4602 further includes a fourth pipe 4602m and a second port 4602n, the second port 4602n being configured to connect the third inlet 4601f to the reagent kit, and the third outlet 4601g being configured to be in communication with a sample inlet of the sampling element 4603; an end of the fourth pipe 4602m is configured to be in communication with a sample outlet of the sampling element 4603, and the other end of the fourth pipe 4602m is configured to be in communication with a detection assembly 4604 of the fluid detection instrument 46.

The docking groove 4602d is arranged with a through hole 4602p and a connecting pipe 4602q passing through the through hole 4602p. An end of the connecting pipe 4602q is in communication with the third outlet 4601g, and the other end of the connecting pipe 4602q is configured to be in communication with the sample inlet of the sampling element 4603. The connecting pipe 4602q is in sealed contact with a hole wall of the through hole 4602p. When the sleeve 4603b moves along the axial direction of the sampling needle 4603a and towards the connecting pipe 4602q such that the docking member 4603c is inserted into the docking groove 4602d, the sampling needle 4603a and the connecting pipe 4602q are respectively inserted into both ends of the through hole 4603d to achieve communication.

The valve assembly 4601 defines a first air hole 4601m and a second air hole 4601n. An end of the first pipe 4601b is selectively connected to the first inlet 4601c or the first air hole 4601m under the control of the control element 4601a; an end of the third pipe 4601e is selectively connected to the third inlet 4601f or the second air hole 4601n under the control of the control element 4601a.

The valve assembly 4601 generally includes a control element 4601a, a first pipe 4601b, a first inlet 4601c, and a first outlet 4601d; where the control element 4601a is configured to control the first inlet 4601c to be in communication with an end of the first pipe 4601b, and the other end of the first pipe 4601b is in communication with the first outlet 4601d; the first inlet 4601c is configured to dock with as well as separate from the first port 4602c of the reagent kit 4602, such that when they are docked, the fluid in the reagent kit 4602 can be obtained by controlling the first inlet 4601c through the control element 4601a; and the first outlet 4601d is configured to be in communication with the docking groove 4602d of the reagent kit 4602d, such that when they are docked, the liquid in the reagent kit 4602 can pass through the first outlet 4601d to reach the docking groove 4602d, and the liquid in the docking groove 4602d can pass through the second pipe 4602b of the reagent kit 4602 to reach the recovery pack 4602 of the reagent kit 4602.

The valve assembly 4601 further includes a third pipe 4601e, a third inlet 4601f, and a third outlet 4601g. The control element 4601a is configured to control the third inlet 4601f to be in communication with an end of the third pipe 4601e, and the other end of the third pipe 4601e is in communication with the third outlet 4601g.

The third inlet 4601f is configured to dock with as well as separate from the second port 4602n of the reagent kit 4602, and the third outlet 4601g is configured to be in communication with the sample inlet of the sampling element 4603, such that when they are docked, the liquid in the reagent kit 4602 can pass through the third outlet 4601g to reach the sampling element 4603.

The valve assembly 4601 defines a first air hole 4601m and a second air hole 4601n. An end of the first pipe 4601b is selectively connected to the first inlet 4601c or the first air hole 4601m under the control of the control element 4601a; an end of the third pipe 4601e is selectively connected to the third inlet 4601f or the second air hole 4601n under the control of the control element 4601a.

The reagent kit 4602 includes a cavity 4602a, a second pipe 4602b, a first port 4602c, and a docking groove 4602d; where the cavity 4602a is configured to place a reagent pack and a recovery pack; an end of the second pipe 4602b is in communication with the docking groove 4602d, and the other end of the second pipe 4602b is in communication with the recovery pack; the first port 4602c is configured to dock with as well as separate from the first inlet 4601c of the valve assembly 4601, and the docking groove 4602d is configured to be in communication with the first outlet 4601d of the valve assembly 4601, such that when they are docked, the liquid in the reagent pack can be delivered to the valve assembly 4601 through the first port 4602c, and the liquid in the docking groove 4602d can reach the recovery pack through the second pipe 4602b.

The docking groove 4602d is arranged with a liquid inlet hole 4602e and a liquid outlet hole 4602f. The liquid inlet hole 4602e is configured to be in communication with the first outlet 4601d, and the liquid outlet hole 4602f is configured to be in communication with the second pipe 4602b.

The docking groove 4602d is arranged with an isolation portion 4602g disposed between the liquid inlet hole 4602e and the liquid outlet hole 4602f, and a flow channel 4602h in communication with the liquid inlet hole 4602e and the liquid outlet hole 4602f and bypassing the isolation portion 4602g.

The reagent kit 4602 further includes a fourth pipe 4602m and a second port 4602n, the second port 4602n being configured to connect the third inlet 4601f of the valve assembly 4601 to the reagent kit, and the third outlet 4601g being configured to connect to a sample inlet of the sampling element 4603; an end of the fourth pipe 4602m is configured to be in communication with a sample outlet of the sampling element 4603, and the other end of the fourth pipe 4602m is configured to be in communication with a detection assembly of the fluid detection instrument 46.

The docking groove 4602d is arranged with a through hole 4602p and a connecting pipe 4602q passing through the through hole 4602p. An end of the connecting pipe 4602q is in communication with the third outlet 4601g, and the other end of the connecting pipe 4602q is configured to be in communication with the sample inlet of the sampling element 4603. The connecting pipe 4602q is in sealed contact with the hole wall of the through hole 4602p.

The sampling element 4603 includes a sampling needle 4603a, a sleeve 4603b sleeved on the sampling needle 4603a, and a docking member 4603c arranged on an end of the sleeve 4603b. The outer diameter of the sleeve 4603b does not exceed the inner diameter of the docking groove 4602d. The docking member 4603c defines a through hole 4603d; the docking member 4603c can move along the axial direction of the sampling needle 4603a following the sleeve 4603b such that the sampling needle 4603a is inserted into an end of the through hole 4603d or passes through the through hole 4603d and is exposed on a side of the docking member 4603c.

When the sleeve 4603b moves along the axial direction of the sampling needle 4603a and towards the connecting pipe 4602q such that the docking member 4603c is inserted into the docking groove 4602d, the sampling needle 4603a and the connecting pipe 4602q are respectively inserted into both ends of the through hole 4603d to achieve communication.

In the fluid detection instrument provided in the embodiments of the present disclosure, the valve assembly and the reagent kit that can be docked or separated are provided, such that the reagent kit can be replaced easily. In addition, when the valve assembly is docked with the reagent kit, the first pipe can be in communication with the docking groove through the control element, such that the liquid in the reagent kit can reach the docking groove. In addition, the second pipe of the reagent kit is in communication with the recovery pack in the reagent kit, such that the liquid in the docking groove can reach the recovery pack, thereby forming a liquid conduit, such that the liquid in the reagent pack can reach the recovery pack after reaching the docking groove, and the liquid reaching the docking groove can be configured to clean the sample inlet of the sampling element on the reagent kit, thereby maintaining the cleanliness of the sample inlet and thus extending the service life of the reagent kit.

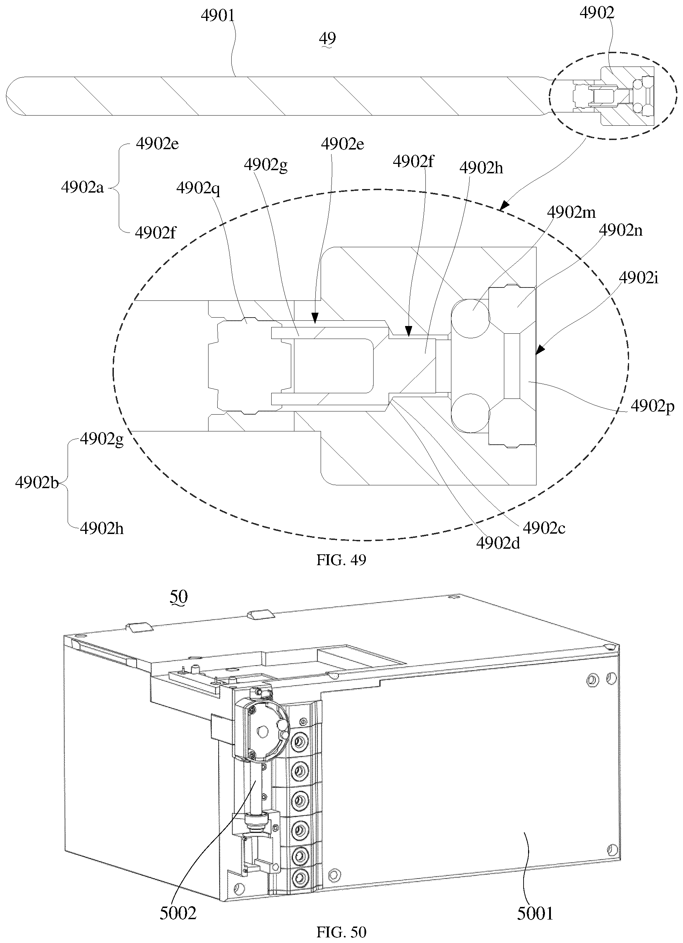

Referring to FIG. 49, FIG. 49 is a structural schematic view of a liquid pack 49 according to some embodiments of the present disclosure.

The liquid pack 49 includes a body 4901 and a connector 4902. The body 4901 is configured to contain liquid, and the connector 4902 is configured to control the liquid to flow from the liquid pack 49 through the connector 4902 or to seal the body 4901. The connector 4902 includes a pipe 4902a connected to an internal space of the body 4901, and a seal 4902b disposed in the pipe 4902a, where the seal 4902b can move in the pipe 4902a under an external force to open the pipe 4902a, and return to an initial position when the external force is removed to seal the pipe 4902a.

In particular, a first abutting portion 4902c is arranged in the pipe 4902a, and the seal 4902b is arranged with a second abutting portion 4902d. When the seal 4902b is in the initial position, the first abutting portion 4902c abuts against the second abutting portion 4902d to seal the pipe 4902a; the seal 4902b moves within the pipe 4902a under the action of an external force to separate the first abutting portion 4902c and the second abutting portion 4902d to open the pipe 4902a.

The pipe 4902a includes a first pipe 4902e and a second pipe 4902f that are in communication. An end of the first pipe 4902e is in communication with the body 4901, and the other end of the first pipe 4902e is in communication with the second pipe 4902f, the inner diameter of the first pipe 4902e being greater than the inner diameter of the second pipe 4902f to form the first abutting portion 4902c, that is stepped in shape, at the connection between the first pipe 4902e and the second pipe 4902f; a gap exists between the seal 4902b and the first pipe 4902e, and the second abutting portion 4902d can seal the end of the second pipe 4902f connected to the first pipe 4902e.

The outer diameter of the seal 4902b is less than the inner diameter of the first pipe 4902e, and the outer diameter of the seal 4902b is greater than the inner diameter of the second pipe 4902f. The seal 4902b in the initial position is disposed in the first pipe 4902e, and the second abutting portion 4902d is formed by an end of the seal 4902b near the second pipe 4902f to abut against the first abutting portion 4902c.

In particular, the seal 4902b includes a sealing portion 4902g and a guiding portion 4902h, where the outer diameter of the sealing portion 4902g is greater than the outer diameter of the guiding portion 4902h, the outer diameter of the sealing portion 4902g is less than the inner diameter of first pipe 4902e, and the outer diameter of the sealing portion 4902g is greater than the inner diameter of the second pipe 4902f; when the seal 4902b is in the initial position, the sealing portion 4902g is disposed in the first pipe 4902e, and an end of the seal 4902b close to the second pipe 4902f forms the second abutting portion 4902d to abut against the first abutting portion 4902c, and the guiding portion 4902h is disposed in the second pipe 4902f and has a gap with the second pipe 4902f. The guiding portion 4902h is configured to receive an external force.

The outer diameter of the guiding portion 4902h is less than the inner diameter of the second pipe 4902f. Under the action of the external force, the seal 4902b moves within the pipe 4902a to separate the first abutting portion 4902c and the second abutting portion 4902d, so as to open the pipe 4902a.

When the seal 4902b is in the initial position, the guiding portion 4902h and the second pipe 4902f are sealed and assembled; the seal 4902b moves in the pipe 4902a under the action of the external force to cause the guiding portion 4902h to move from the second pipe 4902f to the first pipe 4902e.

In particular, the connector 4902 includes a docking port 4902i, the docking port 4902i being arranged on an end of the second pipe 4902f away from the first pipe 4902e and communicating with the second pipe 4902f; the docking port 4902i is configured to guide an external connector into the pipe 4902a to exert a force on the seal 4902b.

The docking port 4902i is arranged with a sealing ring 4902m which is annular in shape, an end of the guiding portion 4902h being at least partially exposed from the liquid pack 49 from an annular hollow portion of the sealing ring 4902m. The external connector can enter the pipe 4902a from the annular hollow portion of the sealing ring 4902m to exert a force on the seal 4902b. When the external connector abuts against the guiding portion 4902h and pushes the guiding portion 4902h to move towards the first pipe 4902e, the sealing ring 4902m seals a gap between the external connector and the docking port 4902i.

The sealing ring 4902m includes a first sealing ring 4902m and a second sealing ring 4902n, where the first sealing ring 4902m is arranged between the guiding portion 4902h and the second sealing ring 4902n, and the external connector 4902 can exert a force on the guiding portion 4902h through the first sealing ring 4902m and the second sealing ring 4902n.

The first sealing ring 4902m and the second sealing ring 4902n are both annular in shape, and the first sealing ring 4902m and the second sealing ring 4902n are arranged coaxially; the outer diameter of the first sealing ring 4902m is less than the outer diameter of the second sealing ring 4902n.

The first sealing ring 4902m and the second sealing ring 4902n are both elastomers, and the external connector can enter the annular hollow portions of the first sealing ring 4902m and the second sealing ring 4902n to exert a force on the seal 4902b.

The external connector is sealed and assembled with at least one of the first sealing ring 4902m and the second sealing ring 4902n when exerting a force on the seal 4902b.

An end of the second sealing ring 4902n away from the first sealing ring 4902m includes a guide ramp 4902p, and the guide ramp 4902p is configured to guide the external connector into the seal.

In particular, an end of the first pipe 4902e away from the second pipe 4902f includes a limit portion 4902q, and the limit portion 4902q is configured to limit the entry of the sealing portion 4902g into the body 4901.

The liquid pack provided in the embodiments of the present disclosure includes a pipe connected to the internal space of the liquid pack body and a seal arranged in the pipe, such that the seal can be moved in the pipe to open or seal the pipe, without the need to provide an extra structure on the liquid pack to open or seal the pipe, thereby improving the convenience of using the liquid pack.

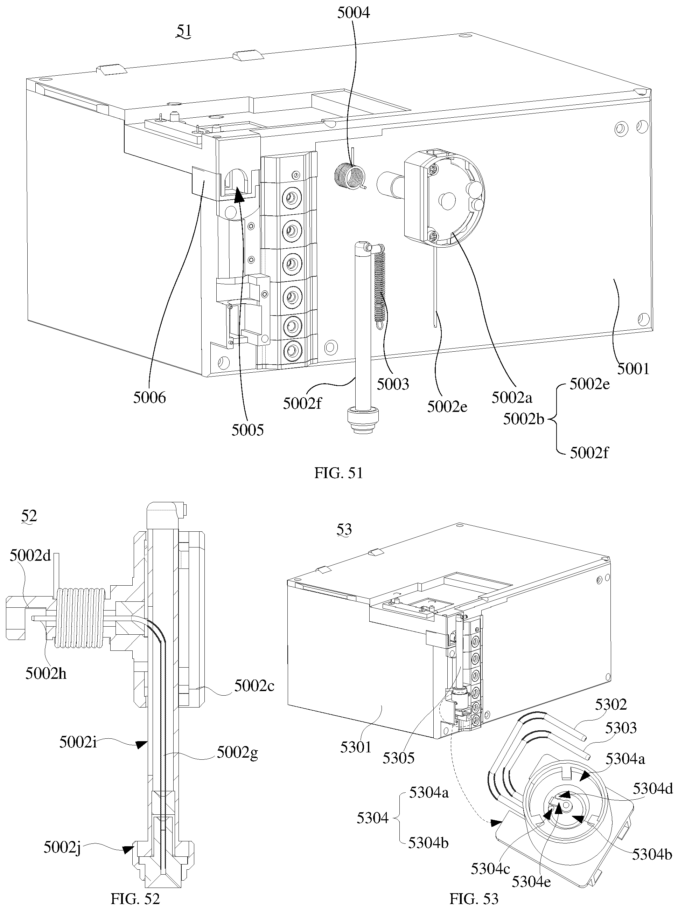

Referring to FIGS. 50 to 52, FIG. 50 is a structural schematic view of an integrated reagent kit 50 according to some embodiments of the present disclosure, FIG. 51 is another structural schematic view of the integrated reagent kit 50 in FIG. 50, and FIG. 52 is another structural schematic view of the integrated reagent kit 50 in FIG. 50.

The integrated reagent kit 50 generally includes a case 5001 and a sampling assembly 5002 disposed on the case 5001; where the sampling assembly 5002 includes a rotary member 5002a rotatably connected to the case 5001 and a sampling member 5002b connected to the rotary member 5002a.

The rotary member 5002a is capable of changing to a state under the action of a first external force to drive the sampling member 5002b to rotate synchronously relative to the case 5001, and of returning to an original state when the first external force is removed.

The sampling member 5002b includes a sampling needle 5002e and a sleeve 5002f sleeved on the sampling needle 5002e. The sleeve 5002f can change to a state under the action of a second external force to move relative to the rotary member 5002a to expose the sampling needle 5002e, and returning to an original state to cover the sampling needle 5002e when the second external force is removed.

In particular, the rotary member 5002a includes a first rotary member 5002c and a second rotary member 5002d connected to each other, the first rotary member 5002c being movably connected to the sleeve 5002f in the axial direction of the sleeve 5002f, the second rotary member 5002d being rotatably connected to the case 5001, and the first rotary member 5002c being configured to take the first external force.

The integrated reagent kit 50 includes a first elastic member 5003 connecting the first rotary member 5002c and the sleeve 5002f. The first elastic member 5003 changes state to generate an elastic force when the sleeve 5002f is moved under force, and when the force is removed from the sleeve 5002f, the first elastic member 5003 restores its state under the action of the elastic force to restore the sleeve 5002f to its original state.

The sampling member 5002b includes a sampling needle 5002e and a sleeve 5002f sleeved on the sampling needle 5002e, the sleeve 5002f being connected to the first rotary member 5002c and movable along the axial direction of the sampling needle 5002e, and the first elastic member 5003 being connected between the first rotary member 5002c and the sleeve 5002f.

The sampling needle 5002e includes a sampling portion 5002g and a sample discharge portion 5002h connected by a bend. The sleeve 5002f is arranged on the sampling portion 5002g and can move along the axial direction of the sampling portion 5002g. The sample discharge portion 5002h extends from an end of the sampling portion 5002g to the second rotary member 5002d or extends and passes through the second rotary member 5002d; the sleeve 5002f defines an avoidance slot 5002i to avoid the sample discharge portion 5002h when the sleeve 5002f is moved axially.

The sleeve 5002f passes through the first rotary member 5002c, and an end of the sleeve 5002f is connected to the first rotary member 5002c through the first elastic member 5003. The first elastic member 5003 changes state to generate an elastic force when the sleeve 5002f is moved under force, and when the force on the sleeve 5002f is removed, the first elastic member 5003 returns to its original state under the action of the elastic force, such that the sleeve 5002f is reset and covers a sample inlet of the sampling portion 5002g.

An end of the sleeve 5002f adjacent to the sample inlet of the sampling portion 5002g includes a bulge 5002j, and the bulge 5002j is pushed to move the sleeve 5002f under the action of the second external force exerted by an external container when the sampling portion 5002g obtains liquid from the external container.

In particular, the integrated reagent kit 50 includes a second elastic member 5004 arranged between the second rotary member 5002d and the case 5001, and the rotary member 5002a changes the state of the second elastic member 5004 to generate an elastic force when the rotary member 5002a rotates relative to the case 5001 under the action of the first external force, and when the first external force is removed, the second elastic member 5004 restores the state of the rotary member 5002a under the action of the elastic force.

The case 5001 defines a rotating groove 5005, the second rotary member 5002d is inserted in the rotating groove 5005, the second elastic member 5004 is sleeved on the second rotary member 5002d, and an end of the second elastic member 5004 abuts against the rotating groove 5005; the sampling needle 5002e is connected to a pipe on the case 5001 through the rotating groove 5005.

The case 5001 is arranged with an assembly portion 5006, the assembly portion 5006 being arranged in a corner region of the case 5001; the rotating groove 5005 is defined on the assembly portion 5006, and the second rotary member 5002d is inserted into the rotating groove 5005 such that when the sampling assembly 5002 is rotated, the sample inlet of the sampling assembly 5002 can be unscrewed out of the corner region of the case 5001.

The embodiments of the present disclosure provide an integrated reagent kit, in which the sampling member and the rotary member are arranged on the case, and the rotary member can be driven to rotate the sampling member relative to the case under the action of a first external force to adjust the sampling angle of the sampling member, and the sampling member can restore its state when the first external force is removed. In addition, the sleeve sleeved on the sampling needle can move relative to the rotary member under the action of a second external force to expose the sampling needle, such that the sampling needle can sample from a liquid container, and the sleeve can cover the sampling needle when the second external force is removed. Therefore, by providing the sampling member and the rotary member with the above structure on the case, the sampling state of the sampling needle may be flexibly adjusted.

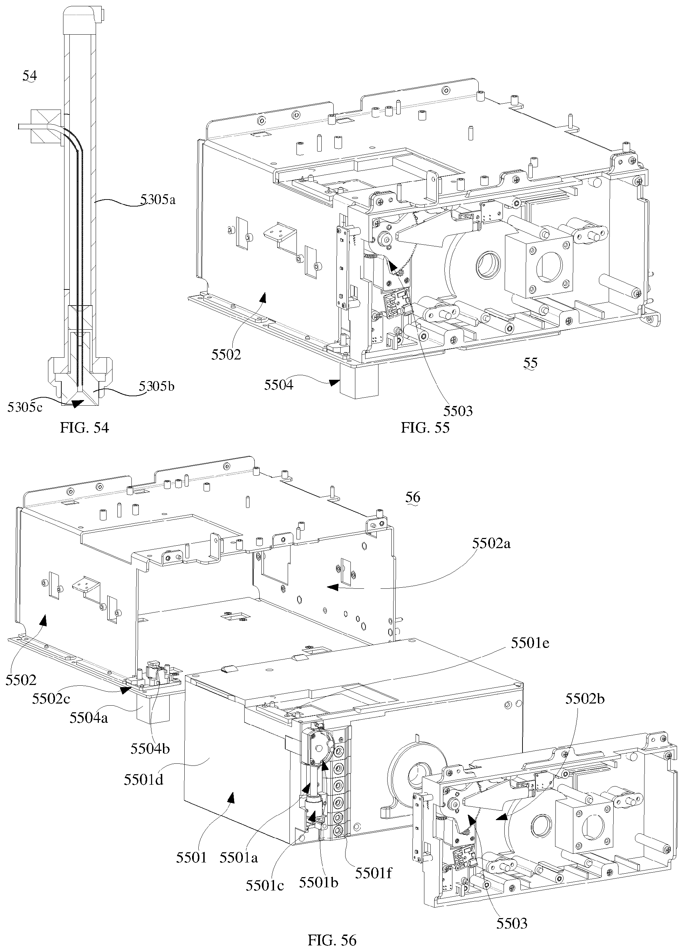

Referring to FIGS. 53 and 54, FIG. 53 is a structural schematic view of a reagent kit 53 according to some embodiments of the present disclosure, and FIG. 54 is a partial cross-sectional structural schematic view of the reagent kit 53 in FIG. 53.

The reagent kit 53 generally includes a case 5301, a first pipe 5302, a second pipe 5303, and a docking groove 5304. The case 5301 is arranged with a reagent pack and a recovery pack therein. An end of the first pipe 5302 is in communication with the docking groove 5304, and the other end of the first pipe 5302 is in communication with the reagent pack. An end of the second pipe 5303 is in communication with the docking groove 5304, and the other end of the second pipe 5303 is in communication with the recovery pack.

The docking groove 5304 is configured to dock with as well as separate from a sampling element 5305. When they are docked, the liquid in the reagent pack can reach the docking groove 5304 through the first pipe 5302 and thus clean a sample inlet of the sampling element 5305, such that the liquid after the cleaning can reach the recovery pack through the second pipe 5303.

The docking groove 5304 includes a liquid inlet 5304c in communication with the first pipe 5302 and a liquid outlet 5304d in communication with the second pipe 5303; when the docking groove 5304 is docked with the sampling element 5305, the sample inlet of the sampling element 5305 seals the docking groove 5304 and cooperates with the docking groove 5304 to form a cavity, and the liquid inlet 5304c and liquid outlet 5304d are respectively in communication with the cavity.

The docking groove 5304 includes a first groove section 5304a and a second groove section 5304b that are in communication; where the first groove section 5304a is away from a bottom of the docking groove 5304 relative to the second groove section 5304b. The first groove section 5304a is configured to dock with as well as separate from the sampling element 5305, and the second groove section 5304b includes the liquid inlet 5304c in communication with the first pipe 5302 and the liquid outlet 5304d in communication with the second pipe 5303. When the first groove section 5304a is docked with the sampling element 5305, the sample inlet of the sampling element 5305 abuts against the first groove section 5304a and is exposed from the second groove section 5304b.

The inner diameter of the first groove section 5304a is greater than the inner diameter of the second groove section 5304b, the inner diameter of the first groove section 5304a is greater than the outer diameter of the sample inlet of the sampling element 5305, and the inner diameter of the second groove section 5304b is less than the outer diameter of the sample inlet of the sampling element 5305.

The sampling element 5305 includes a sleeve 5305a, and a sampling needle and a docking head 5305b that are arranged in the sleeve 5305a, the docking head 5305b defining a through hole. The docking head 5305b is embedded in an end of the sleeve 5305a, and the sample inlet of the sampling needle is inserted in the through hole. When the docking groove 5304 is docked with the sampling element 5305, the sleeve 5305a abuts against the first groove section 5304a, and the docking head 5305b abuts against the second groove section 5304b, the cavity being defined between the docking head 5305b and the second groove section 5304b.

An end of the sampling element 5305 is sleeved with a sleeve 5305a, which abuts against the first groove section 5304a when the docking groove 5304 is docked with the sampling element 5305.

The sleeve 5305a is embedded with a docking head 5305b, and the sample inlet of the sampling element 5305 is inserted into the docking head 5305b. When the docking groove 5304 is docked with the sampling element 5305, the docking head 5305b abuts against the second groove section 5304b.

The second groove section 5304b includes an open end and a narrow end that are oppose to each other, the inner diameter of the open end being greater than the inner diameter of the narrow end; the inner diameter of the open end is greater than the outer diameter of the docking head and less than the outer diameter of the sleeve.

The inner diameter of the narrow end does not exceed the inner diameter of the sleeve 5305a.

The reagent kit 53 includes a third pipe partially disposed in the docking groove 5304. The second groove section 5304b is arranged with an isolation portion 5304e, and the third pipe passes through the isolation portion 5304e. When the docking groove 5304 is docked with the sampling element 5305, the docking head 5305b is inserted into the second groove section 5304b, and the third pipe is inserted into the docking head 5305b to communicate with the sampling needle.

In particular, an end of the docking head 5305b abutting against the second groove section 5304b includes a groove 5305c, and the through hole is in communication with a bottom wall of the groove 5305c. The docking head 5305b is in sealed contact with a sidewall of the second groove section 5304b, and the groove 5305c and the second groove section 5304b enclose to define the cavity where the sample inlet can be cleaned.

The second groove section 5304b is arranged with an isolation portion 5304e, and the liquid inlet 5304c and liquid outlet 5304d are disposed on opposite sides of the isolation portion 5304e and are respectively in communication with the cavity, such that the liquid in the reagent pack can reach the cavity 5305d from the liquid inlet 5304c, so as to clean the sample inlet of the sampling element 5305. After cleaning, the liquid flows out of the cavity from the liquid outlet 5304d and reaches the recovery pack through the second pipe 5303.

The sampling element 5305 includes a sleeve 5305a, and a sampling needle and a docking connector 5305b that are arranged in the sleeve 5305a, the docking head 5305b defining a through hole. The docking head 5305b is embedded in an end of the sleeve 5305a, and the sample inlet of the sampling needle is inserted in the through hole. An inner wall of the sleeve 5305a includes a step portion, and the docking head 5305b includes an engagement portion. The engagement portion is disposed on a side of the step portion away from the bottom wall of the docking groove 5304 and abuts against the step portion, such that the docking head 5305b can move axially along with the sleeve 5305a.

The reagent kit provided in the embodiments of the present disclosure is arranged with a first pipe and a second pipe that are in communication with the docking groove respectively, such that the liquid in the reagent pack can reach the docking groove and clean the sample inlet of the sampling element, and the cleaned liquid can reach the recovery pack. In this way, the cleanliness of the sample inlet of the sampling element of the reagent kit can be maintained during use.

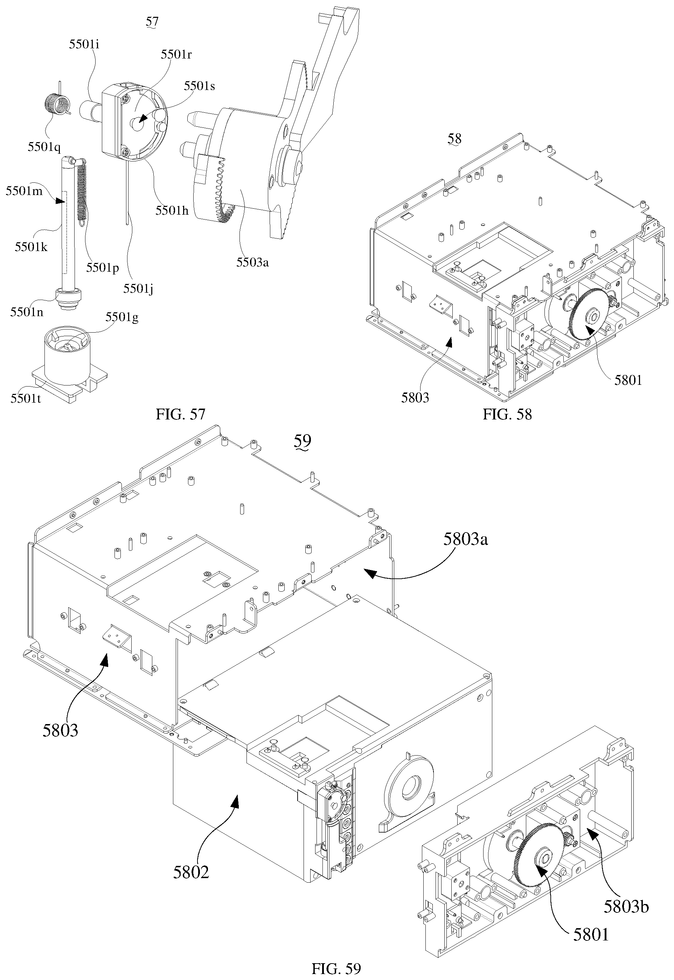

Referring to FIGS. 55 to 57, FIG. 55 is a structural schematic view of a sample analysis device 55 according to some embodiments of the present disclosure, FIG. 56 is an exploded structural schematic view of the sample analysis device 55 in FIG. 55, and FIG. 57 is a partially exploded structural schematic view of the sample analysis device 55 in FIG. 55.

The sample analysis device 55 generally includes:
a reagent kit 5501, including a sampling assembly 5501a, a regulating assembly 5501b, and a movable assembly 5501c; where the regulating assembly 5501b is configured to drive the sampling assembly 5501a to rotate relative to the reagent kit 5501; the movable assembly 5501c is arranged facing the sampling assembly 5501a; the movable assembly 5501c can dock with or separate from the sampling assembly 5501a.

The movable assembly 5501c can move between a first position and a second position relative to the sampling assembly 5501a, where the movable assembly 5501c is docked with the sampling assembly 5501a in the first position and the movable assembly 5501c is separated from the sampling assembly 5501a in the second position.

The first position corresponds to that the sampling assembly 5501a is positionally limited such that the sampling assembly 5501a can collect liquid inside the reagent kit 5501, and the second position corresponds to that the restriction on the sampling assembly 5501a is cancelled such that the regulating assembly 5501b can drive the sampling assembly 5501a to rotate, thereby enabling the sampling assembly 5501a to collect liquid outside the reagent kit 5501.

The reagent kit 5501 includes a case 5501d for placing a liquid pack, and the case 5501d is arranged with a first port 5501e and a second port 5501f. The first port 5501e is configured to dock with a detection assembly, and the second port 5501f is configured to dock with the liquid pack. The movable assembly 5501c includes a third port 5501g. An end of the sampling assembly 5501a is in communication with the first port 5501e through a first liquid path, and the other end of the sampling assembly 5501a is selectively docked with or separated from the third port 5501g, the second port 5501f being in communication with the third port 5501g through a second liquid path.

The regulating assembly 5501b includes a seat body 5501h and a shaft portion 5501i disposed on the seat body 5501h. The sampling assembly 5501a includes a sampling element 5501j, where an end of the sampling element 5501j is at least partially disposed in the seat body 5501h and is in communication with the first port 5501e through the first liquid path, and the other end of the sampling element 5501j is disposed outside the seat body 5501h for selectively docking with or separating from the third port 5501g. The shaft portion 5501i docks with the case 5501d and is rotatable relative to the case 5501d.

The sampling assembly 5501a further includes a sleeve 5501k sleeved on the sampling element 5501j. The sleeve 5501k can be moved along the axial direction of the sampling element 5501j; the sleeve 5501k defines an avoidance slot 5501m to avoid the first liquid path or the sampling element 5501j when the sleeve 5501k is moved.

The sleeve 5501k includes a bulge 5501n on an end adjacent to the movable assembly 5501c, and the bulge 5501n is pushed to move the sleeve 5501k under the action of a force exerted by an external container when the sampling element 5501j obtains liquid from the external container.

**In** particular, the sampling assembly 5501a includes a first elastic member 5501p assembled between the sleeve 5501k and the seat body 5501h. The first elastic member 5501p changes state to generate an elastic force when the sleeve 5501k is moved under the action of a force. When the force on the sleeve 5501k is removed, the first elastic member 5501p restores its state under the action of the elastic force to cause the sleeve 5501k to reset and cover the sample inlet of the sampling element 5501j.

**In** particular, the sampling assembly 5501a includes a second elastic member 5501q assembled between the regulating assembly 5501b and the case 5501d, and the regulating assembly 5501b changes the state of the second elastic member 5501q to generate an elastic force when subjected to a force to rotate relative to the case 5501d. When the force on the regulating assembly 5501b is removed, the second elastic member 5501q returns to its original state under the action of the elastic force to reset the regulating assembly 5501b.

The seat body 5501h is arranged with a docking portion 5501r configured to dock with a first drive component 5503 of the sample analysis device 55 for transmission fit; the movable assembly 5501c is configured to dock with as well as separate from the sampling element 5501j under the drive of a second drive component 5504 of the sample analysis device 55 when docking with the second drive component 5504.

The sample analysis device 55 further includes a mounting bracket 5502. The mounting bracket 5502 includes a cavity 5502a, a first mounting position 5502b, and a second mounting position 5502c. The reagent kit 5501 can be moved into or out of the cavity 5502a, the first mounting position 5502b is configured to mount the first drive component 5503, and the second mounting position 5502c is configured to mount the second drive component 5504.

When the reagent kit 5501 is moved into the cavity 5502a, the docking portion 5501r docks with the first drive component 5503 and the movable assembly 5501c docks with the second drive component 5504.

The first drive component 5503 includes a first power member 5503a arranged on the first mounting position 5502b, and the docking portion 5501r of the seat body 5501h defines a shaft hole 5501s. When the docking portion 5501r and the first drive component 5503 are docked, the output shaft of the first drive component 5503a cooperates with the shaft hole 5501s to achieve a transmission fit between the docking portion 5501r and the first drive component 5503a.

The movable assembly 5501c includes a docking seat 5501t that is movable relative to the case 5501d. An end of the docking seat 5501t forms the third port 5501g, and the other end of the docking seat 5501t is configured to dock with the second drive component 5504. When the reagent kit 5501 is moved into the cavity 5502a, the docking base 5501t docks with the second drive component 5504 to move under the drive of the second drive component 5504.

In particular, the second drive component 5504 includes a second power member 5504a arranged on the second mounting position 5502c, and a clamping member 5504b cooperating with the output shaft of the second power member 5504a, and the second power member 5504a can drive the clamping member 5504b to move.

When the reagent kit 5501 is moved into the cavity 5502a, the clamping member 5504b is clamped and connected with the docking base 5501t, such that the second power member 5504a synchronously drives the docking base 5501t to move when driving the clamping member 5504b to move.

The sample analysis device provided in the embodiments of the present disclosure can achieve different sampling states of the sampling assembly by arranging the movable assembly to be able to move between a first position and a second position relative to the sampling assembly. In particular, the sampling assembly can be limited to collect liquid inside the reagent kit when the movable assembly is in the first position, and the limitation of the sampling assembly can be released when the movable assembly is in the second position to enable the regulating assembly to drive the sampling assembly to rotate, such that the sampling assembly can collect liquid outside the reagent kit. That is, the sampling assembly can be switched between different sampling states through the cooperation of the movable assembly and the when the movable assembly is, which may meet the needs of the sample analysis device for changing sampling positions.

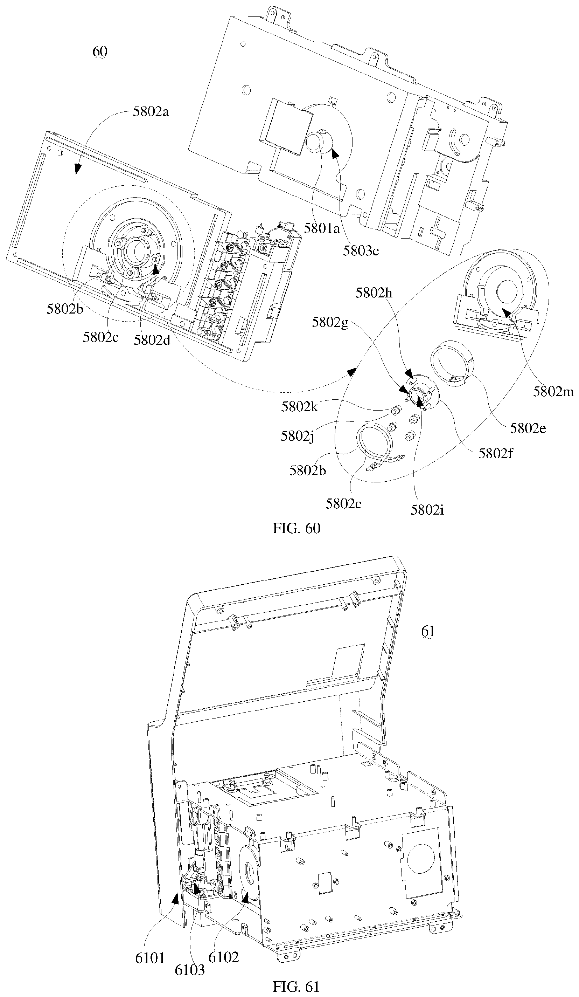

Referring to FIGS. 58 to 60, FIG. 58 is a structural schematic view of a biological sample analysis device 58 according to some embodiments of the present disclosure, FIG. 59 is an exploded structural schematic view of the biological sample analysis device 58 in FIG. 58, and FIG. 60 is a partially exploded structural schematic view of the biological sample analysis device 58 in FIG. 58.

The biological sample analysis device 58 includes a drive assembly 5801 and a reagent storage apparatus 5802. The drive assembly 5801 includes an output shaft 5801a.

The reagent storage apparatus 5802 includes a receiving cavity 5802a, a first connecting pipe 5802b, a second connecting pipe 5802c, and a squeezing assembly 5802d; where the receiving cavity 5802a is configured to hold a reagent pack, a cleaning pack, and a recovery pack; the first connecting pipe 5802b and the second connecting pipe 5802c are partially embedded in the squeezing assembly 5802d; an end of the first connecting pipe 5802b is configured to be in communication with the reagent pack, and the other end of the first connecting pipe 5802b is configured to be in communication with the recovery pack; an end of the second connecting pipe 5802c is configured to be in communication with the cleaning pack, and the other end of the second connecting pipe 5802c is configured to be in communication with the recovery pack.

The drive assembly 5801 and the reagent storage apparatus 5802 can be docked or separated, such that the output shaft 5801a can be docked with or separated from the squeezing assembly 5802d; when they are docked, the output shaft 5801a can drive the squeezing assembly 5802d to synchronously squeeze the first connecting pipe 5802b and the second connecting pipe 5802c, such that the liquid in the first connecting pipe 5802b or the second connecting pipe 5802c can flow.

The biological sample analysis device 58 further includes a mounting bracket 5803, the mounting bracket 5803 including a cavity 5803a and a first mounting position 5803b; where the reagent storage apparatus 5802 is able to move into or out of the cavity 5803a, and the drive assembly 5801 is arranged on the first mounting position 5803b; the first mounting position 5803b is arranged on the end of the travel of the reagent storage apparatus 5802 into the cavity 5803a.

In particular, the drive assembly 5801 is arranged outside the cavity 5803a; an avoidance hole 5803c is defined between the cavity 5803a and the first mounting position 5803b, and the output shaft 5801a of the drive assembly 5801 extends from the avoidance hole 5803c into the cavity 5803a; when the reagent storage apparatus 5802 is moved into the cavity 5803a, the output shaft 5801a of the drive assembly 5801 is transmission-assembled with the squeezing assembly 5802d.

The squeezing assembly 5802d includes a first squeezing member 5802e and a second squeezing member 5802f, the second squeezing member 5802f being embedded in the first squeezing member 5802e and arranged coaxially with the first squeezing member 5802e; the first connecting pipe 5802b and the second connecting pipe 5802c are partially arranged between the first squeezing member 5802e and the second squeezing member 5802f; the second squeezing member 5802f is configured to be transmission-assembled with the output shaft 5801a, so as to rotate relative to the first squeezing member 5802e under the drive of the output shaft 5801a, thereby squeezing the first connecting pipe 5802b and the second connecting pipe 5802c.

The first squeezing member 5802e is annular in shape, and the second squeezing member 5802f includes a rotary portion 5802g and a squeezing portion 5802h; the rotary portion 5802g is configured to dock with the output shaft 5801a, and the squeezing portion 5802h is arranged on a peripheral side of the rotary portion 5802g and can rotate synchronously with the rotary portion 5802g.

The squeezing portion 5802h is arranged spaced apart from the first squeezing member 5802e, and the first connecting pipe 5802b and the second connecting pipe 5802c are partially arranged between the squeezing portion 5802h and the first squeezing member 5802e.

The rotary portion 5802g defines a shaft hole 5802i. An end of the output shaft 5801a is assembled with the shaft hole 5802i, and the first connecting pipe 5802b and the second connecting pipe 5802c are arranged along the axial direction of the shaft hole 5802i on a position between the squeezing portion 5802h and the first squeezing member 5802e.

In particular, the squeezing portion 5802h is arranged with a bushing 5802j, the bushing 5802j is arranged with a spacer 5802k, and the spacer 5802k is disposed between the first connecting pipe 5802b and the second connecting pipe 5802c.

A cavity wall of the receiving cavity 5802a defines a receiving groove 5802m, the squeezing assembly 5802d is embedded in the receiving groove 5802m and arranged coaxially with the receiving groove 5802m, and the first connecting pipe 5802b and the second connecting pipe 5802c are partially arranged between the receiving groove 5802m and the squeezing assembly 5802d; the squeezing assembly 5802d is configured to be transmission-assembled with the output shaft 5801a, so as to rotate relative to the storage groove 5802m under the drive of the output shaft 5801a, thereby squeezing the first connecting pipe 5802b and the second connecting pipe 5802c.

The reagent storage apparatus 5802 includes: a receiving cavity 5802a, a first connecting pipe 5802b, a second connecting pipe 5802c, and a squeezing assembly 5802d.

The receiving cavity 5802a is configured to hold a reagent pack, a cleaning pack, and a recovery pack; the first connecting pipe 5802b and the second connecting pipe 5802c are partially embedded in the squeezing assembly 5802d; an end of the first connecting pipe 5802b is configured to be in communication with the reagent pack, and the other end of the first connecting pipe 5802b is configured to be in communication with the recovery pack; an end of the second connecting pipe 5802c is configured to be in communication with the cleaning pack, and the other end of the second connecting pipe 5802c is configured to be in communication with the recovery pack.

The squeezing assembly 5802d is configured to dock with as well as separate from the output shaft 5801a of the drive assembly 5801, such that when they are docked, the squeezing assembly 5802d is driven by the output shaft 5801a to synchronously squeeze the first connecting pipe 5802b and the second connecting pipe 5802c to allow the liquid in the first connecting pipe 5802b or the second connecting pipe 5802c to flow.

The squeezing assembly 5802d includes a first squeezing member 5802e and a second squeezing member 5802f, the second squeezing member 5802f being embedded in the first squeezing member 5802e and arranged coaxially with the first squeezing member 5802e; the first connecting pipe 5802b and the second connecting pipe 5802c are partially arranged between the first squeezing member 5802e and the second squeezing member 5802f; the second squeezing member 5802f is configured to be transmission-assembled with the output shaft 5801a, so as to rotate relative to the first squeezing member 5802e under the drive of the output shaft 5801a, thereby squeezing the first connecting pipe 5802b and the second connecting pipe 5802c.

The first squeezing member 5802e is annular in shape, and the second squeezing member 5802f includes a rotary portion 5802g and a squeezing portion 5802h; the rotary portion 5802g is arranged to dock with the output shaft 5801a, and the squeezing portion 5802h is arranged on a peripheral side of the rotary portion 5802g and can rotate synchronously with the rotary portion 5802g.

The squeezing portion 5802h is arranged spaced apart from the first squeezing member 5802e, and the first connecting pipe 5802b and the second connecting pipe 5802c are partially arranged between the squeezing portion 5802h and the first squeezing member 5802e.

The rotary portion 5802g defines a shaft hole 5802i. An end of the output shaft 5801a is assembled with the shaft hole 5802i, and the first connecting pipe 5802b and the second connecting pipe 5802c are arranged along the axial direction of the shaft hole 5802i on a position between the squeezing portion 5802h and the first squeezing member 5802e.

In particular, the squeezing portion 5802h is arranged with a bushing 5802j, the bushing 5802j is arranged with a spacer 5802k, and the spacer 5802k is disposed between the first connecting pipe 5802b and the second connecting pipe 5802c.

A cavity wall of the receiving cavity 5802a defines a receiving groove 5802m, the squeezing assembly 5802d is embedded in the receiving groove 5802m and arranged coaxially with the receiving groove 5802m, and the first connecting pipe 5802b and the second connecting pipe 5802c are partially arranged between the receiving groove 5802m and the squeezing assembly 5802d; the squeezing assembly 5802d is configured to be transmission-assembled with the output shaft 5801a, so as to rotate relative to the storage groove 5802m under the drive of the output shaft 5801a, thereby squeezing the first connecting pipe 5802b and the second connecting pipe 5802c.

In the biological sample analysis device provided in the embodiments of the present disclosure, the first connecting pipe and the second connecting pipe are partially embedded in the squeezing assembly, such that the squeezing assembly can synchronously squeeze the first connecting pipe and the second connecting pipe to allow the liquid in the first connecting pipe or the second connecting pipe to flow, thereby saving the space occupied by the squeezing assembly of the reagent storage apparatus for each connecting pipe. Further, the drive assembly can be docked with or separated from the reagent storage apparatus, that is, the output shaft of the drive assembly can be docked with or separated from the squeezing assembly, such that when they are docked, the squeezing assembly can be driven to perform the above operations. In this way, the reagent storage apparatus can be separated from the drive assembly to facilitate replacement of the reagent storage apparatus.

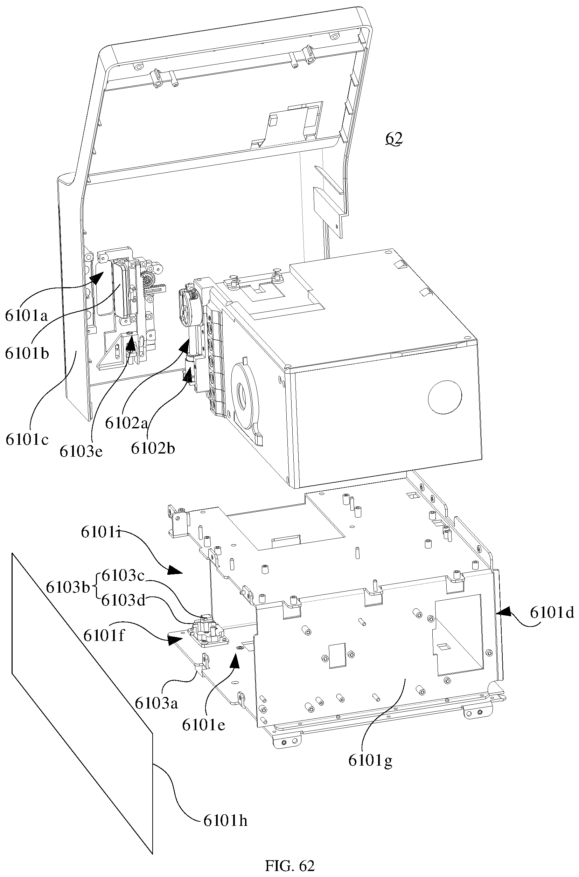

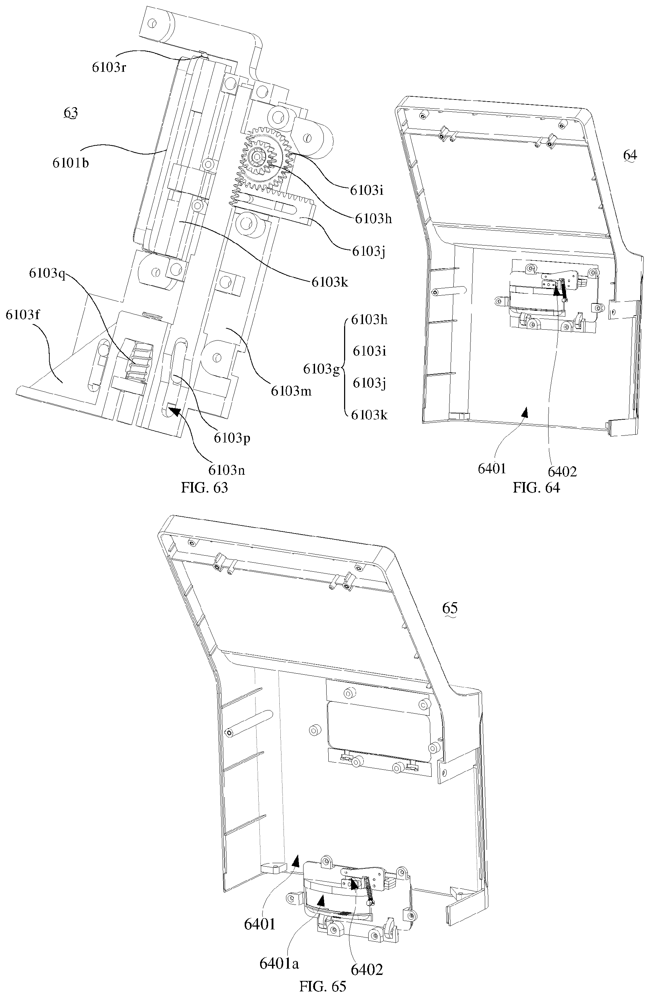

Referring to FIGS. 61 to 63, FIG. 61 is a structural schematic view of a biological parameter analysis device 61 according to some embodiments of the present disclosure, FIG. 62 is an exploded structural schematic view of the biological parameter analysis device 61 in FIG. 61, and FIG. 63 is a partial structural schematic view of the biological parameter analysis device 61 in FIG. 61.

The biological parameter analysis device 61 generally includes a housing 6101, a reagent kit 6102, and a linkage assembly 6103, the housing 6101 being arranged with a window 6101a and a door 6101b capable of covering the window 6101a.

The reagent kit 6102 can be moved into and out of the housing 6101, and the reagent kit 6102 includes a sampling assembly 6102a and a docking assembly 6102b. When the reagent kit 6102 is arranged within the housing 6101, the sampling assembly 6102a can be unscrewed from the window 6101a to collect liquid outside the housing 6101; the docking assembly 6102b can be docked with the sampling assembly 6102a to enable the sampling assembly 6102a to collect liquid inside the reagent kit 6102.

The linkage assembly 6103 is coupled to the docking assembly 6102b and the door 6101b, such that the door 6101b covers the window 6101a when the sampling assembly 6102a is docked with the docking assembly 6102b, and that the door 6101b opens the window 6101a when the sampling assembly 6102a is separated from the docking assembly 6102b.

The housing 6101 includes a shell 6101c and a bracket 6101d arranged in the shell 6101c; the window 6101a is arranged on the shell 6101c, and the door 6101b is arranged on an inner side of the housing 6101; the bracket 6101d includes a cavity 6101e and a first mounting position 6101f, and the reagent kit 6102 can be moved into or out of the cavity 6101e, the first mounting position 6101f being arranged on the end of the travel of the reagent kit 6102 into the cavity 6101e; the docking assembly 6102b is arranged facing the first mounting position 6101f when the reagent kit 6102 is moved into the cavity 6101e; a part of the linkage assembly 6103 is assembled on the first mounting position 6101f, and the other part of the linkage assembly 6103 is assembled on the shell 6101c.

The bracket 6101d includes a frame plate 6101g and a mounting plate 6101h arranged on a side of the frame plate 6101g, and the frame plate 6101g and the mounting plate 6101h cooperate to define the cavity 6101e; an avoidance opening 6101i is defined between the frame plate 6101g and the mounting plate 6101h to avoid the linkage assembly 6103 and the sampling assembly 6102a. The avoidance opening 6101i is arranged opposite the door 6101b, and the linkage assembly 6103 drives the separation of the sampling assembly 6102a from the docking assembly 6102b while driving the door 6101b to open the window 6101a, and a sample inlet of the sampling assembly 6102a can be unscrewed from the window 6101a.

The linkage assembly 6103 includes a drive member 6103a and a first linkage member 6103b. The drive member 6103a is arranged on the first mounting position 6101f, and the first linkage member 6103b is disposed on an output end of the drive member 6103a. The first linkage member 6103b is docked with the docking assembly 6102b when the reagent kit 6102 is moved into the cavity 6101e; the drive member 6103a is configured to drive the first linkage member 6103b to move the docking assembly 6102b and to drive the first linkage member 6103b to move the door 6101b.

In particular, the drive member 6103a and the first linkage member 6103b are arranged on opposite sides of the frame plate 6101g, and the drive member 6103a is arranged outside the cavity 6101e.

The first linkage member 6103b includes a first docking portion 6103c for docking with the docking assembly 6102b and a first linkage portion 6103d for linkage with the door 6101b.

The linkage assembly 6103 includes a second linkage assembly 6103e arranged on the shell 6101c, where an end of the second linkage assembly 6103e is docked with the first linkage member 6103d, and the other end of the second linkage assembly 6103e is docked with the door 6101b.

The second linkage assembly 6103e includes a second linkage member 6103f and a transmission component 6103g that is transmission-connected to the second linkage member 6103f. The second linkage member 6103f is docked with the first linkage member 6103d to move relative to the shell 6101c under the action of the first linkage member 6103d. The moving direction of the second linkage member 6103f is perpendicular to the direction of opening or closing of the door 6101b.

The transmission component 6103g includes a first transmission member 6103h which is transmission-connected to the second linkage member 6103f, a second transmission member 6103i which is transmission-connected to the first transmission member 6103h, a third transmission member 6103j which is transmission-connected to the second transmission member 6103i, and a fourth transmission member 6103k which is transmission-connected to the third transmission member 6103j, and the fourth transmission member 6103k is assembled with the door 6101b.

The second linkage member 6103f moves to drive the first transmission member 6103h to rotate, and the second transmission member 6103i rotates synchronously with the first transmission member 6103h; the second transmission member 6103i rotates to drive the third transmission member 6103j to move, the third transmission member 6103j moves to drive the fourth transmission member 6103k to rotate, and the door 6101b rotates synchronously with the fourth transmission member 6103k. The moving direction of the third transmission member 6103j is perpendicular to the moving direction of the second linkage member 6103f.

The linkage assembly 6103 includes a seat body 6103m arranged on the shell 6101c, and the second linkage member 6103f is slidably connected to the seat body 6103m; one of the second linkage member 6103f and the seat body 6103m defines a travel groove 6103n, and the other of the second linkage member 6103f and the seat body 6103m is arranged with a limit block 6103p. The limit block 6103p is slidable in the travel groove 6103n to limit the sliding travel of the second linkage member 6103f relative to the seat body 6103m.

A first elastic member 6103q is arranged between the second linkage member 6103f and the seat body 6103m. When the second linkage member 6103f moves relative to the housing 6101c under the action of the first linkage member 6103d, the first elastic member 6103q changes its state to generate an elastic force. When the action of the first linkage member 6103d is cancelled, the first elastic member 6103q returns to its original state, causing the second linkage member 6103f to return to its original position.

The fourth transmission member 6103k is rotatably connected to the seat body 6103m, and a second elastic member 6103r is arranged between the fourth transmission member 6103k and the seat body 6103m. When the third transmission member 6103j drives the fourth transmission member 6103k to rotate and thereby drives the door 6101b to open the window 6101a, the second elastic member 6103r changes its state to generate an elastic force. When the force exerted by the third transmission member 6103j on the fourth transmission member 6103k is cancelled, the fourth transmission member 6103k drives the door 6101b to cover the window 6101a under the action of the elastic force.

In the biological parameter analysis device provided in the embodiments of the present disclosure, by providing a linkage assembly that can couple with the door on the shell and the docking assembly on the reagent kit, the door can be driven to close the window when the sampling assembly on the reagent kit docks with the docking assembly when collecting the liquid inside the reagent kit, and the door can be driven to close the window when the sampling assembly separates with the docking assembly, in which case the sampling assembly can be unscrewed out of the window to collect liquid outside the reagent kit. By providing the above linkage assembly to link the movement relationship between the docking assembly and the door, an extra drive mechanism that drives the door to open and close may be cancelled, which is beneficial to simplifying the overall structure of the device.

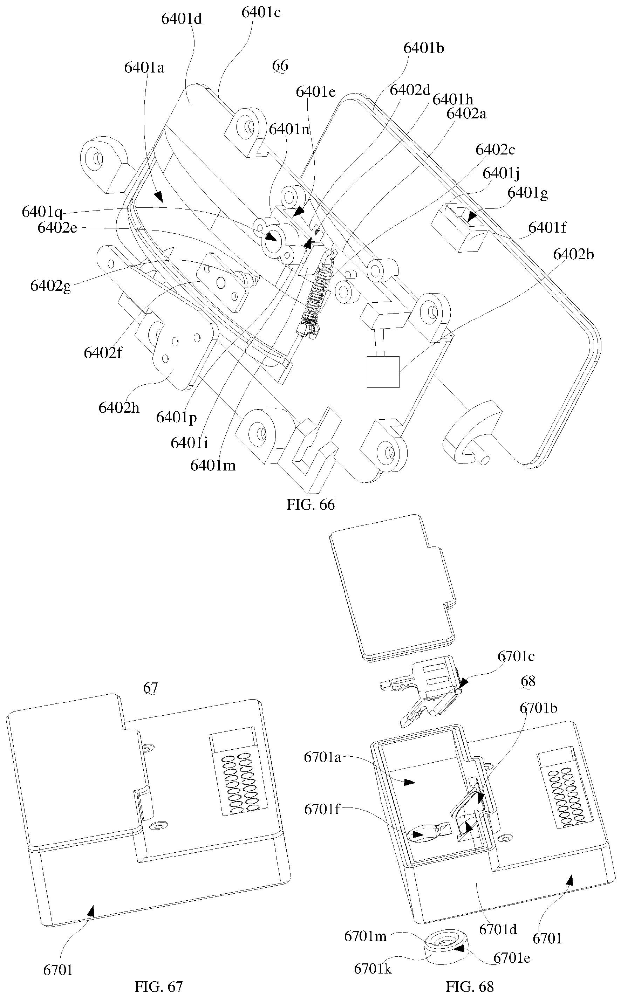

Referring to FIGS. 64 to 66, FIG. 64 is a structural schematic view of a sample parameter analysis apparatus 64 according to some embodiments of the present disclosure, FIG. 65 is an exploded structural schematic view of the sample parameter analysis apparatus 64 in FIG. 64, and FIG. 66 is a partially exploded structural schematic view of the sample parameter analysis apparatus 64 in FIG. 64.

The sample parameter analysis apparatus 64 generally includes a housing 6401 and a connecting assembly 6402, the housing 6401 being arranged with a window 6401a and a door 6401b that can cover the window 6401a; the housing 6401 includes a first side 6401c and a second side 6401d arranged opposite each other, and the door 6401b is arranged on the first side 6401c and is rotatably connected to the housing 6401.

The connecting assembly 6402 is arranged on the second side 6401d of the housing 6401 to connect the housing 6401 and the door 6401b when the door 6401b covers the window 6401a; the connecting assembly 6402 includes a clamping member 6402a, a drive member 6402b, and a first elastic member 6402c; an end of the first elastic member 6402c is connected to the middle of the clamping member 6402a, and the other end of the first elastic member 6402c is connected to the housing 6401; an end of the clamping member 6402a is connected to the drive member 6402b, and the other end of the clamping member 6402a limits the position of the door 6401b when the door 6401b covers the window 6401a.

The drive member 6402b is configured to drive the clamping member 6402a to release the restriction on the door 6401b, such that the door 6401b can rotate relative to the housing 6401 to open the window 6401a. The first elastic member 6402c deforms to generate an elastic force when the clamping member 6402a moves, and the elastic force is configured to act on the clamping member 6402a to cause the clamping member 6402a to return to its original position when the drive member 6402b cancels the drive to the clamping member 6402a.

In particular, the second side 6401d of the housing 6401 defines an engagement groove 6401e, and a side of the door 6401b close to the housing 6401 is arranged with an engagement portion 6401f, where the engagement portion 6401f can be moved into or out of the engagement groove 6401e; when the engagement portion 6401f is moved into the engagement groove 6401e, the clamping member 6402a abuts against the engagement portion 6401f to restrict the position of the door 6401b.

An end of the clamping member 6402a is arranged with a protrusion 6402d, and the engagement portion 6401f defines a groove 6401g. When the engagement portion 6401f is moved into the engagement groove 6401e, the protrusion 6402d is inserted into the groove 6401g to restrict the position of the door 6401b.

The engagement groove 6401e includes a first notch 6401h and a second notch 6401i disposed on the adjacent sides of the engagement groove 6401e, the first notch 6401h being configured for the engagement portion 6401f to move in or out, and the second notch 6401i being configured for the protrusion 6402d to move in or out.

When the engagement portion 6401f moves from the first notch 6401h into the engagement groove 6401e, the engagement portion 6401f pushes the protrusion 6402d out of the second notch 6401i; the protrusion 6402d can be moved into the engagement groove 6401e or the groove 6401g under the elastic force of the first elastic member 6402c.

At least one of the engagement portion 6401f and the protrusion 6402d is arranged with a guide ramp 6401j. The guide ramp 6401j is configured to guide the engagement portion 6401f to push the protrusion 6402d out of the second notch 6401i when the engagement portion 6401f moves into the engagement groove 6401e.

The engagement groove 6401e includes a first side wall 6401m and a second side wall 6401n arranged at intervals, the first side wall 6401m and the second side wall 6401n being respectively connected to the housing 6401, and the clamping member 6402a is rotatable around the first side wall 6401m such that the protrusion 6402d can be moved into or out of a space between the first side wall 6401m and the second side wall 6401n.

The clamping member 6402a is rotatable around the first side wall 6401m; an end of the clamping member 6402a that is arranged with the protrusion 6402d and an end of the clamping member 6402a that is connected to the drive member 6402b are arranged on both sides of the first side wall 6401m, and an end of the first elastic member 6402c is connected between the two ends of the clamping member 6402a.

The connecting assembly 6402 further includes a second elastic member 6402e, which is arranged on the engagement groove 6401e. When the engagement portion 6401f moves into the engagement groove 6401e, the engagement portion 6401f squeezes or stretches the second elastic member 6402e, causing the second elastic member 6402e to deform and generate an elastic force. When the drive member 6402b drives the clamping member 6402a to release the restriction on the door 6401b, the elastic force acts on the door 6401b to cause the door 6401b to rotate relative to the housing 6401, so as to open the window 6401a.

The engagement groove 6401e includes a third side wall 6401p spaced apart from the housing 6401, the connecting assembly 6402 includes a supporting member 6402f passing through the third side wall 6401p, and the second elastic member 6402e acts between the supporting member 6402f and the third side wall 6401p. The engagement portion 6401f pushes the supporting member 6402f to move when the engagement portion 6401f is moved into the engagement groove 6401e to change the state of the second elastic member 6402e to produce an elastic force.

The third side wall 6401p defines a storage groove 6401q, and the supporting member 6402f is arranged with a limit portion 6402g; an end of the supporting member 6402f is inserted into the storage groove 6401q to be connected to the second elastic member 6402e, and the limit portion 6402g is disposed in the storage groove 6401q to limit the movement travel of the limit portion 6402f when the engagement portion 6401f pushes the supporting member 6402f to move.

The connecting assembly 6402 includes a limit member 6402h disposed on the second side 6401d of the housing 6401, and the clamping member 6402a is disposed between the limit member 6402h and the housing 6401.

The sample parameter analysis apparatus provided in the embodiments of the present disclosure is arranged with a connecting assembly to fix the door when the door covers the window, and the connecting assembly is capable of releasing the restriction on the door such that the door can move to open the window. In particular, a clamping member is arranged to restrict and fix the door when the door is closed, and a drive member is arranged to drive the clamping member to move so as to release the restriction on the door. Further, a first elastic member is arranged to connect the clamping member and the housing, such that when the clamping member releases the restriction on the door, the first elastic member deforms to generate an elastic force. The elastic force can cause the clamping member to return to its original position without the need to drive the clamping member to return to its original position again through the drive member, which is beneficial to simplifying the overall structure of the device.

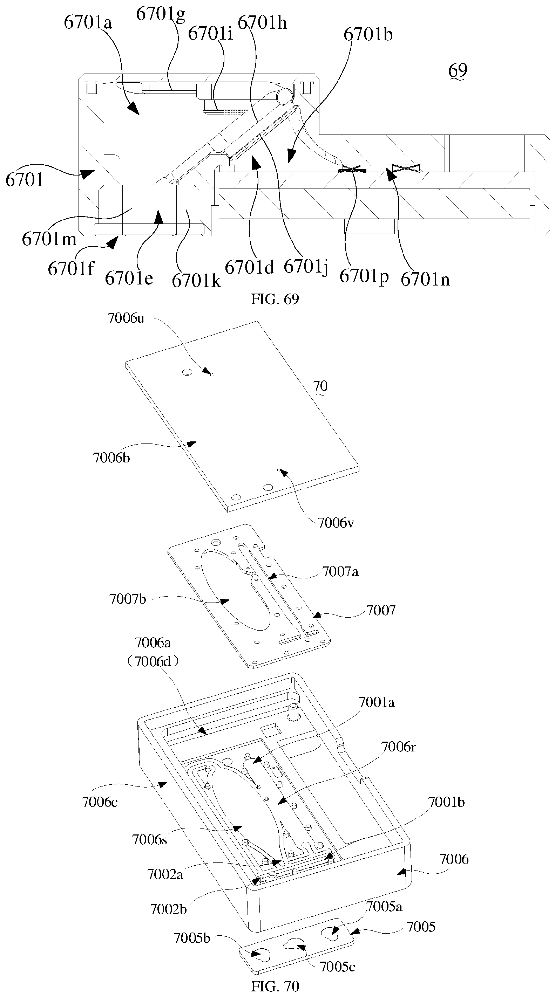

Referring to FIGS. 67 to 69, FIG. 67 is a structural schematic view of a sample analysis box 67 according to some embodiments of the present disclosure, FIG. 68 is an exploded structural schematic view of the sample analysis box 67 in FIG. 67, and FIG. 69 is a cross-sectional structural schematic view of the sample analysis box 67 in FIG. 67.

The sample analysis box 67 generally includes a seat body 6701, the seat body 6701 including a first cavity 6701a, a second cavity 6701b, and an isolation member 6701c; where the isolation member 6701c is configured to cause the first cavity 6701a and the second cavity 6701b to communicate or isolate with each other, and when the first cavity 6701a and the second cavity 6701b are in communication, the liquid in the first cavity 6701a can flow to the second cavity 6701b.

The isolation member 6701c can change states under the action of an external force to enable the first cavity 6701a and the second cavity 6701b to be in communication, and when the external force is removed, the isolation member 6701c returns to a state that isolates the first cavity 6701a and the second cavity 6701b.

The seat body 6701 includes a first liquid port 6701d for communicating the first cavity 6701a with the second cavity 6701b. The isolation member 6701c is arranged on a cavity wall of the first cavity 6701a corresponding to the first liquid port 6701d and is configured to open or cover the first liquid port 6701d. The isolation member 6701c covers the first liquid port 6701d to isolate the first cavity 6701a from the second cavity 6701b when no external force is applied to the isolation member 6701c. When an external force is applied to the isolation member 6701c, the first liquid port 6701d opens to allow the first cavity 6701a and the second cavity 6701b to be in communication through the first liquid port 6701d.

The cavity wall of the first cavity 6701a is arranged with a squeezing member 6701e. The squeezing member 6701e abuts against the isolation member 6701c and is configured to apply a force to the isolation member 6701c to cause the isolation member 6701c to open the first liquid port 6701d.

The first cavity 6701a includes a squeezing opening 6701f, and the squeezing member 6701e is sealedly assembled with the squeezing opening 6701f; the squeezing member 6701e extends into the first cavity 6701a from the squeezing opening 6701f to abut against the isolation member 6701c and can exert a force on the isolation member 6701c.

The isolation member 6701c includes a connecting portion 6701g and an isolation portion 6701h. The connecting portion 6701g is connected to the cavity wall of the first cavity 6701a, an end of the isolation portion 6701h is connected to the connecting portion 6701g, and the other end of the isolation portion 6701h is configured to abut against the squeezing member 6701e; the isolation portion 6701h can move relative to the connecting portion 6701g under the action of an external force to open the first liquid port 6701d, and when the external force is removed, the isolation portion 6701h returns to a state that covers the first liquid port 6701d.

At least one of the isolation portion 6701h and the connecting portion 6701g is arranged with an elastic member 6701i, the elastic member 6701i being disposed between the connecting portion 6701g and the isolation portion 6701h, and the isolation portion 6701h is deformed under an external force to deform the elastic member 6701i to generate an elastic force. When the external force is removed, the elastic force causes the isolation portion 6701h to return to its original state.

In particular, the isolation portion 6701h is arranged with a seal 6701j on a side close to the first liquid port 6701d. When the isolation portion 6701h returns to its original state to cover the first liquid port 6701d, the seal 6701j is sealedly assembled with the first liquid port 6701d.

The connecting portion 6701g is fixedly arranged on the cavity wall of the first cavity 6701a, and the isolation portion 6701h is rotatably connected to the connecting portion 6701g through a rotating shaft.

The squeezing member 6701e includes an assembly portion 6701k and a squeezing portion 6701m, the assembly portion 6701k being configured to mount the squeezing member 6701e to the squeezing opening 6701f; the assembly portion 6701k is annular in shape, and the squeezing portion 6701m is arranged in an annular hollow region of the assembly portion 6701k and is elastically connected to the assembly portion 6701k. The assembly portion 6701k is sealed and assembled with the squeezing opening 6701f.

The seat body 6701 includes a third cavity 6701n and a salt bridge 6701p. An end of the salt bridge 6701p is exposed from the second cavity 6701b, and the other end of the salt bridge 6701p is exposed from the third cavity 6701n and connected to an electrode terminal in the third cavity 6701n.

The sample analysis box provided in the embodiments of the present disclosure is arranged with an isolation member in the seat body that can isolate the first cavity and the second cavity, so as to isolate the first cavity and the second cavity when the sample analysis box is not in use. When the sample analysis box is in use, the state of the isolation member is changed by an external force to make the first cavity and the second cavity in communication, thereby allowing the liquid in the first cavity to flow to the second cavity to complete the analysis operation of the sample analysis box. In addition, when the sample analysis box is not in use, the first cavity and the second cavity are isolated from each other, which allows the salt bridge exposed from the second cavity to be stored dry, thereby extending the storage period of the sample analysis box.

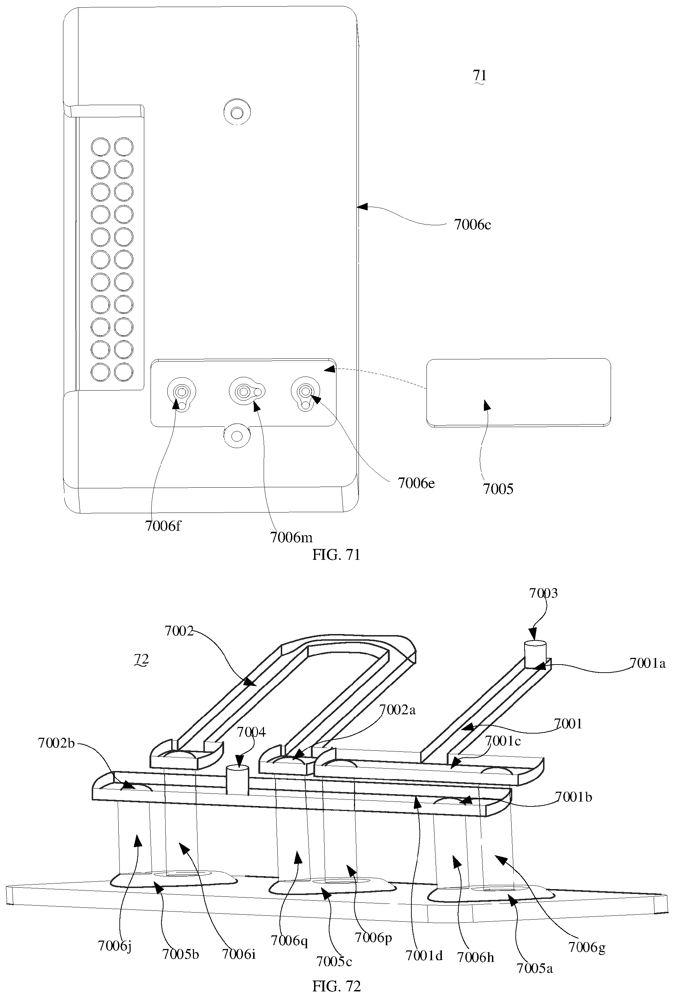

Referring to FIGS. 70 to 72, FIG. 70 is an exploded structural schematic view of a detection assembly 70 according to some embodiments of the present disclosure, FIG. 71 is a partially exploded structural schematic view of the detection assembly 70 in FIG. 70, and FIG. 72 is a schematic view of a liquid path structure of the detection assembly 70 in FIG. 70.

The detection assembly 70 generally includes a first liquid path 7001, a second liquid path 7002, a liquid inlet 7003, a liquid outlet 7004, and a valve assembly 7005. An end of the first liquid path 7001 is configured to be in communication with the liquid inlet 7003, and the other end of the first liquid path 7001 is configured to be in communication with the liquid outlet 7004; an end of the second liquid path 7002 is configured to be in communication with the liquid inlet 7003, and the other end of the second liquid path 7002 is configured to be in communication with the liquid outlet 7004.

The valve assembly 7005 may be arranged on the liquid inlet 7003, and/or on the liquid outlet 7004, and/or between the liquid inlet 7003 and the liquid outlet 7004, and can be switched between a first turn-on state and a second turn-on state to control the first liquid path 7001 and the second liquid path 7002 to be selectively connected.

In the first turn-on state, a first external liquid of the detection assembly 70 can flow into the first liquid path 7001 from the liquid inlet 7003 and can flow out of the first liquid path 7001 from the liquid outlet 7004.

In the second turn-on state, a second external liquid of the detection assembly 70 can flow into the second liquid path 7002 from the liquid inlet 7003 and can flow out of the second liquid path 7002 from the liquid outlet 7004.

The first liquid path 7001 includes a first inlet 7001a and a first outlet 7001b, the second liquid path 7002 includes a second inlet 7002a and a second outlet 7002b, and the valve assembly 7005 includes a first valve 7005a and a second valve 7005b; the first inlet 7001a and the second inlet 7002a are configured to be in communication with the liquid inlet 7003, and the first outlet 7001b and the second outlet 7002b are configured to be in communication with the liquid outlet 7004; the first valve 7005a is arranged between the first inlet 7001a and the first outlet 7001b to control the connection and disconnection of the first liquid path 7001, and the second valve 7005b is arranged between the second inlet 7002a and the second outlet 7002b to control the connection and disconnection of the second liquid path 7002; either the first valve 7005a or the second valve 7005b may be selectively turned on.

The valve assembly 7005 further includes a third valve 7005c, which is arranged between the liquid inlet 7003 and the second inlet 7002a and is configured to control the opening and closing of the liquid inlet 7003 and the second inlet 7002a; either the third valve 7005c or the first valve 7005a may be selectively turned on.

The first liquid path 7001 includes a first liquid inlet section 7001c and a first liquid outlet section 7001d, the first liquid inlet section 7001c being in communication with the liquid inlet 7003 and the first liquid outlet section 7001d being in communication with the liquid outlet 7004; the first valve 7005a is arranged between the first liquid inlet section 7001c and the first liquid outlet section 7001d to control the connection and disconnection of the first liquid inlet section 7001c and the first liquid outlet section 7001d, and the third valve 7005c is arranged between the second inlet 7002a and the first liquid inlet section 7001c to control the opening and closing of the second inlet 7002a and the connection and disconnection of the first liquid inlet section 7001c.

The detection assembly 70 includes a housing 7006 and a plate 7007; where the plate defines a first through opening 7007a and a second through opening 7007b, and the housing 7006 includes a cavity 7006a; a cavity wall of the cavity 7006a defines a first fluid slot 7006r and a second fluid slot 7006s; the plate 7007 is disposed in the cavity 7006a; the first through opening 7007a is in communication with a part of the first fluid slot 7006r, and the second through opening 7007b is in communication with a part of the second fluid slot 7006s. The plate 7007 encloses and seals another part of the first fluid slot 7006r and another part of the second fluid slot 7006s to form the first liquid path 7001 and the second liquid path 7002. The liquid inlet 7003 and the liquid outlet 7004 are respectively in communication with the cavity 7006a.

The valve assembly 7005 is arranged outside the cavity 7006a, and the first valve 7005a is configured to control the connection and disconnection of the first liquid path 7001, and the second valve 7005b is configured to control the connection and disconnection of the second liquid path 7002.

The housing 7006 includes a first housing 7006b and a second housing 7006c surrounding the first housing 7006b to define the cavity 7006a, and the first housing 7006b and the second housing 7006c cooperate to clamp the plate 7007 to form the first liquid path 7001 and the second liquid path 7002.

The first housing 7006b defines a first through hole 7006u and a second through hole 7006v, the liquid inlet 7003 and the first inlet 7001a are arranged on both ends of the first through hole 7006u of the first housing 7006b, or the liquid inlet 7003 and the second inlet 7002a are arranged on both ends of the first through hole 7006u of the first housing 7006b; and/or, the first outlet 7001b and the liquid outlet 7004 are arranged on both ends of the second through hole 7006v of the first housing 7006b, or the second outlet 7002b and the liquid outlet 7004 are arranged on both ends of the second through hole 7006v of the first housing 7006b.

The second housing 7006c includes a receiving cavity 7006d, the first housing 7006b is at least partially embedded in the receiving cavity 7006d and cooperates with the second housing 7006c to define the cavity 7006a, and the plate 7007 is disposed between the first housing 7006b and the second housing 7006c.

A first groove 7006e is defined on the housing 7006, and the housing 7006 is arranged with a first liquid inlet channel 7006g and a first liquid outlet channel 7006h that are in communication with the first groove 7006e. A second groove 7006f is defined on the housing 7006, and the housing 7006 is arranged with a second liquid inlet channel 7006i and a second liquid outlet channel 7006j that are in communication with the second groove 7006f.

The first liquid path 7001 between the first inlet 7001a and the first outlet 7001b includes two first sub-liquid paths that are discontinuous, an end of the first liquid inlet channel 7006g being in communication with the first inlet 7001a, and an end of the first liquid outlet channel 7006h being in communication with the first outlet 7001b. The two first sub-liquid paths are in communication through the first groove 7006e, the first liquid inlet channel 7006g, and the first liquid outlet channel 7006h.

The second liquid path 7002 between the second inlet 7002a and the second outlet 7002b includes two second sub-liquid paths that are discontinuous, an end of the second liquid inlet channel 7006i being in communication with the second inlet 7002a, and an end of the second liquid outlet channel 7006j being in communication with the second outlet 7002b. The two second sub-liquid paths are in communication through the second groove 7006f, the second liquid inlet channel 7006i, and the second liquid outlet channel 7006j.

The first valve 7005a is an elastomer and disposed on the housing 7006, and can abut against the first groove 7006e under the action of an external force to block the first liquid inlet channel 7006g and the first liquid outlet channel 7006h, for disconnecting the first liquid path 7001; the second valve 7005b is an elastomer and disposed on the housing 7006, and can abut against the second groove 7006f under the action of an external force to block the second liquid inlet channel 7006i and the second liquid outlet channel 7006j, for disconnecting the second liquid path 7002.

The housing 7006 is arranged with a third groove 7006m, a third valve 7005c, and a third liquid inlet channel 7006p and a third liquid outlet channel 7006q that are respectively in communication with the third groove 7006m; the first liquid path 7001 and the second liquid path 7002 are discontinuous; an end of the third liquid inlet channel 7006p is in communication with the first inlet 7001a, and an end of the third liquid outlet channel 7006q is in communication with the second inlet 7002a; the first liquid path 7001 and the second liquid path 7002 are in communication through the third groove 7006m, the third liquid inlet channel 7006p, and the third liquid channel 7006q; the third valve 7005c is an elastomer that can abut against the third groove 7006m under the action of an external force to block the third liquid inlet channel 7006p and the third liquid outlet channel 7006q, for disconnecting the first liquid path 7001 from the second liquid path 7002.

The first valve 7005a, the second valve 7005b, and the third valve 7005c form an integrated structure.

In the detection assembly provided in the embodiments of the present disclosure, the connection of either the first liquid path or the second liquid path is controlled through the valve assembly. When the second liquid path is connected, external liquid in the detection assembly, such as reference liquid, can enter the second liquid path. When the first liquid path is connected, external liquid in the detection assembly, such as a detection sample, can enter the first liquid path. That is, it is not necessary to prepare auxiliary measurement liquids in advance, such as reference liquid, etc., inside the detection assembly upon completion of assembly. Instead, the auxiliary measurement liquid, such as reference liquid, etc., can be injected into the second liquid path by controlling the connection of the second liquid path by the valve assembly when in use, which may reduce the volume of the detection assembly upon completion of assembly and avoid the problem of the auxiliary measurement liquid such as reference liquid, etc., expiring due to the detection assembly not being used for a long period of time while the auxiliary measurement liquid such as reference liquid, etc., is placed inside the detection assembly.

It should be noted that the terms "including" and "having" and any of their derivatives are intended to cover non-exclusive inclusion. For example, a process, method, system, product, or device that includes a series of steps or units is not limited to the listed steps or units, but may optionally further include unlisted steps or units, or may optionally further include other steps or units that are inherent to the process, method, product, or device.

The above is some embodiments of the present disclosure. It should be noted that for those skilled in the art, it is possible to make a number of improvements and refinements without departing from the principles of the present disclosure, and these improvements and refinements are also considered to be within the scope of the present disclosure.

## Claims

1. A blood gas analysis device, comprising:
a first detection assembly, comprising a detection element, a first liquid path, and a first port, wherein the first liquid path and the first port are in communication, and the detection element is configured to perform a blood gas detection on liquid in the first fluid path; and
a reagent kit, comprising a cavity, a second fluid path, and a second port, wherein the cavity is configured to hold a liquid pack; an end of the second fluid path is configured to be in communication with the liquid pack, and another end of the second fluid path is configured to be in communication with the second port;
wherein the first detection assembly and the reagent kit are capable of being docked and of being separated, and the first port and the second port are caused to be in communication or separated; in condition of the first detection assembly and the reagent kit being docked with each other, the first liquid path is in communication with the second liquid path, and reagent in the liquid pack is configured to reach the first liquid path; in condition of the reagent kit being moved into a mounting bracket of the blood gas analysis device, the first port and the second port are in communication.

2. The blood gas analysis device according to claim 1, wherein the reagent kit is arranged with a sampling element, a sample inlet of the sampling element being configured to selectively collect the reagent from the liquid pack or liquid external to the reagent kit, and a sample outlet of the sampling element being configured to be in communication with the second port; in condition of that it is required to collect the reagent from the liquid pack, the sampling element forms a part of the second liquid path.

3. The blood gas analysis device according to claim 2, wherein the reagent kit comprises a first connecting pipe, an end of the first connecting pipe being in communication with the liquid pack and another end of the first connecting pipe being in communication with the sample inlet of the sampling element; the first connecting pipe forms another part of the second liquid path.

4. The blood gas analysis device according to claim 1, wherein the first detection assembly comprises a third port spaced apart from the first port, and the third port is in communication with the first liquid path; the reagent kit comprises a fourth port and a second connecting pipe, and the fourth port is spaced apart from the second port; an end of the second connecting pipe is in communication with the fourth port, and another end of the second connecting pipe is in communication with the cavity; in condition of the first port and the second port being in communication, the third port and the fourth port are in communication.

5. The blood gas analysis device according to claim 4, wherein a docking base is arranged on an outer side of a cavity wall of the cavity, the second port and the fourth port are arranged on the docking base, and the second connecting pipe is partially arranged between the cavity wall of the cavity and the docking base.

6. The blood gas analysis device according to claim 4, wherein the liquid pack comprises a reagent pack and a recovery pack that are disposed in the cavity; an end of the first connecting pipe is configured to be in communication with the reagent pack, and an end of the second connecting pipe is configured to be in communication with the recovery pack.

7. The blood gas analysis device according to claim 1, wherein the reagent kit is arranged with a liquid channel exposed from the cavity, and the liquid channel forms another part of the second liquid path; liquid flowing out of the first liquid path is configured to pass through the liquid channel and reach the cavity;
the blood gas analysis device further comprises a second detection assembly arranged on the mounting bracket, the second detection assembly being configured to detect liquid in the liquid channel exposed from the cavity, wherein the first detection assembly is configured to perform the blood gas detection, and the second detection assembly is configured to perform a blood oxygen detection.

8. A detection assembly, comprising:
a housing, a detection element and a first liquid path that are disposed in the housing, and a first port and a connection terminal that are disposed on the housing, wherein the connection terminal is electrically connected to the detection element;
wherein the first liquid path is in communication with the first port, and the detection element is configured to perform a blood gas detection on liquid in the first liquid path; the first port is capable of being docked with and of being separated from a second port of a reagent kit; in condition of the first port being docked with the second port, reagent in the reagent kit is obtainable through the first port; in condition of the first port being docked with the second port, reagent in the reagent kit is obtainable through the first port, the connection terminal is configured to be electrically connected to a processing circuit of a blood gas analysis device.

9. The detection assembly according to claim 8, wherein the housing is arranged with a positioning member for positioning a bracket, of the blood gas analysis device, for mounting the detection assembly.

10. The detection assembly according to claim 8, wherein an end of the first port that is configured to be docked with the second port comprises a tapered recess or tapered protrusion to guide the second port to be docked with the first port.

11. A reagent kit, comprising:
a cavity, a second liquid path, and a second port;
wherein the cavity is configured to hold a liquid pack; an end of the second liquid path is in communication with the liquid pack, and another end of the second liquid path is in communication with to the second port; the second port is capable of being docked with and of being separated from a first port of a first detection assembly; in condition of the first port being docked with the second port, reagent in the liquid pack is configured to be delivered to the first detection assembly through the second port; the reagent kit is capable of being moved into and of being moved out of a mounting bracket of a blood gas analysis device, and in condition of the reagent kit being moved into the mounting bracket, the first port and the second port are in communication.

12. The reagent kit according to claim 11, wherein the reagent kit is arranged with a sampling element, a sample inlet of the sampling element being configured to selectively collect the reagent from the liquid pack or liquid external to the reagent kit, and a sample outlet of the sampling element being configured to be in communication with the second port; in condition of that it is required to collect the reagent from the liquid pack, the sampling element forms a part of the second liquid path.

13. The reagent kit according to claim 11, wherein the reagent kit is arranged with a liquid channel exposed from the cavity, and the liquid channel forms another part of the second liquid path; liquid flowing out of the first detection assembly is configured to pass through the liquid channel and reach the cavity;
the blood gas analysis device further comprises a second detection assembly arranged on the mounting bracket, the second detection assembly being configured to detect liquid in the liquid channel exposed from the cavity, wherein the first detection assembly is configured to perform a blood gas detection, and the second detection assembly is configured to perform a blood oxygen detection.

14. The reagent kit according to claim 11, wherein the first detection assembly comprises a third port spaced apart from the first port; the reagent kit comprises a fourth port spaced apart from the second port; in condition of the first port and the second port being in communication, the third port and the fourth port are in communication.

15. The blood gas analysis device according to claim 14, wherein a docking base is arranged on an outer side of a cavity wall of the cavity, and the second port and the fourth port are arranged on the docking base.

16. A blood gas analysis device, comprising:
a mounting bracket, comprising a first mounting position and a second mounting position, wherein the first mounting position is configured to detachably mount a first detection assembly, and the second mounting position is configured to hold a reagent kit;
wherein the first detection assembly and the reagent kit are capable of being docked and of being separated, and the first detection assembly and the reagent kit are caused to be in communication or separated; in condition of the first detection assembly and the reagent kit being docked with each other, reagent in the reagent kit is capable of reaching the first detection assembly.

17. The blood gas analysis device according to claim 16, wherein the mounting bracket further comprises a third mounting position, and the third mounting position is configured to detachably mount a second detection assembly; the reagent kit is arranged with a liquid channel exposed from the cavity from the third mounting position, and liquid flowing out of the first detection assembly is configured to pass through the liquid channel and reach the cavity; the second detection assembly is configured to detect liquid in the liquid channel exposed from the cavity; the first detection assembly is configured to perform a blood gas detection, and the second detection assembly is configured to perform a blood oxygen detection.

18. The blood gas analysis device according to claim 16, wherein the mounting bracket comprises a fixed bracket and a movable bracket; the fixed bracket comprises a cavity, the cavity forming the second mounting position; the movable bracket comprises a receiving cavity, the receiving cavity forming the first mounting position; in condition of the reagent kit being moved into the cavity, the movable bracket is movable relative to the fixed bracket, and the first detection assembly is configured to be docked with the reagent kit.

19. The blood gas analysis device according to claim 18, wherein the movable bracket is movable relative to the fixed bracket in a first direction or a second direction;
in condition of the movable bracket moving in the first direction relative to the fixed bracket, the first detection assembly is capable of being moved into and of being moved out of the movable bracket;
in condition of the movable bracket moving in the second direction relative to the fixed bracket, the first detection assembly is capable of being docked with and of being separated from the reagent kit.

20. A regulating device, applied to a liquid sample detection apparatus and comprising:
a first adjusting component and a second adjusting component;
wherein the first adjusting component is configured to adjust a rotation angle of a sampling element relative to the liquid sample detection apparatus and switch between a first fluid path and a second fluid path for conveying liquid;
the second adjusting component is configured to adjust an axial position of a sleeve sleeved on the sampling element and switch between a first position and a second position of the sleeve;
wherein in condition of the sleeve being in the first position, the sampling element is capable of achieving a connection of the first fluid path;
in condition of the sleeve being in the second position, the first adjusting component is capable of adjusting the rotation angle of the sampling element and achieving a connection of the second fluid path.

21. The regulating device according to claim 20, wherein the first adjusting component comprises an adjusting seat arranged on the liquid sample detection apparatus, a first power member arranged on the adjusting seat, and an adjusting bracket; the first power member is configured to drive the adjusting bracket to rotate; the adjusting bracket is configured to be assembled with the sampling element to adjust the rotation angle of the sampling element relative to the liquid sample detection apparatus.

22. The regulating device according to claim 21, wherein the first adjusting component further comprises a rotary member arranged between the adjusting seat and the adjusting bracket, the rotary member being connected to the adjusting bracket, and the first power member being capable of driving the rotary member to rotate and thereby driving the adjusting bracket to rotate.

23. The regulating device according to claim 22, wherein the adjusting seat defines a rotating groove, and the rotary member is accommodated at least partially in the rotating groove and rotatable relative to the rotating groove;
the rotating groove is arranged with a shaft hole, the adjusting bracket is arranged with a rotating shaft, and the rotating shaft passes through the rotary member and is inserted into the shaft hole.

24. The regulating device according to claim 23, wherein the first power member is arranged on a side of the adjusting seat away from the rotary member, and a rotating shaft of the first power member passes through the adjusting seat and is transmission-connected to the rotary member.

25. The regulating device according to claim 23, wherein a bottom wall of the rotating groove defines a curved slot extending in a rotation direction of the rotary member; a first positioning member is arranged on the curved slot, and the rotary member is arranged with a slider that is slidable along the curved slot; the slider is configured to cooperate with the first positioning member to obtain a rotation angle of the rotary member relative to the adjusting seat.

26. The regulating device according to claim 21, wherein the adjusting bracket is assembled with the second adjusting component; in condition of the first power member driving the adjusting bracket to rotate, the second adjusting component is rotatable synchronously with the adjusting bracket.

27. The regulating device according to claim 26, wherein the second adjusting component comprises a second power member arranged on the adjusting bracket, and a movable bracket connected to the second power member; the second power member is configured to drive the movable bracket to move relative to the adjusting bracket; in condition of the liquid sample detection apparatus being docked with the reagent kit, the sampling element is nested on a side of the movable bracket, and the movable bracket is connected to the sleeve of the sampling element and capable of driving the sleeve to switch between the first position and the second position.

28. The regulating device according to claim 27, wherein the movable bracket comprises a first movable bracket and a second movable bracket; the first movable bracket is connected to the second power member, and the second movable bracket is elastically connected to the first movable bracket; the second movable bracket is connected to the sleeve in condition of the liquid sample detection apparatus being docked with the reagent kit.

29. A liquid sample detection apparatus, comprising:
a body, comprising a first mounting position; and
a regulating device, arranged on the first mounting position;
wherein the regulating device comprises:
a first adjusting component and a second adjusting component;
wherein the first adjusting component is configured to adjust a rotation angle of a sampling element relative to the liquid sample detection apparatus and switch between a first fluid path and a second fluid path for conveying liquid;
the second adjusting component is configured to adjust an axial position of a sleeve sleeved on the sampling element and switch between a first position and a second position of the sleeve;
wherein in condition of the sleeve being in the first position, the sampling element is capable of achieving a connection of the first fluid path;
in condition of the sleeve being in the second position, the first adjusting component is capable of adjusting the rotation angle of the sampling element and achieving a connection of the second fluid path.

30. The liquid sample detection apparatus according to claim 29, wherein the first adjusting component comprises an adjusting seat arranged on the liquid sample detection apparatus, a first power member arranged on the adjusting seat, and an adjusting bracket; the first power member is configured to drive the adjusting bracket to rotate; the adjusting bracket is configured to be assembled with the sampling element to adjust the rotation angle of the sampling element relative to the liquid sample detection apparatus.

31. The liquid sample detection apparatus according to claim 30, wherein the second adjusting component comprises a second power member arranged on the adjusting bracket, and a movable bracket connected to the second power member; the second power member is configured to drive the movable bracket to move relative to the adjusting bracket; in condition of the liquid sample detection apparatus being docked with the reagent kit, the sampling element is nested on a side of the movable bracket, and the movable bracket is connected to the sleeve of the sampling element and capable of driving the sleeve to switch between the first position and the second position.

32. A biological tissue detection frame, comprising:
a plurality of plates that are interconnected to form a cavity and a first mounting position, wherein the first mounting position is configured to mount a biological tissue measurement platform, and the cavity is configured to contain an auxiliary consumable;
wherein an avoidance passage is arranged between the cavity and the first mounting position, the avoidance passage being configured to avoid mechanical docking operations and consumable delivery between the biological tissue measurement platform and the auxiliary consumable; the consumable delivery is a passage connecting a first pipe of the biological tissue measurement platform and a second pipe of the auxiliary consumable.

33. The biological tissue detection frame according to claim 32, wherein the plurality of plates comprise a top plate and a bottom plate disposed opposite each other, the cavity being defined between the top plate and the bottom plate, the avoidance passage passing through the top plate, and the first mounting position being disposed on a side of the top plate away from the bottom plate.

34. The biological tissue detection frame according to claim 33, wherein the cavity comprises a pick-up and placement opening, and the auxiliary consumable is capable of being moved into and of being moved out of the cavity through the pick-up and placement opening; the side of the top plate away from the bottom plate is arranged with a limit member, and the limit member spans the avoidance passage; the avoidance passage is configured to limit a height of the auxiliary consumable protruding from the top plate.

35. The biological tissue detection frame according to claim 33, wherein the plurality of plates further comprise a side plate disposed between the top plate and the bottom plate; the side plate is arranged opposite the pick-up and placement opening; the side plate comprises a first docking port and a second mounting position, wherein the first docking port is in communication with the cavity, and the second mounting position is configured to mount a regulating device; in condition of the auxiliary consumable being moved into the cavity, a sampling element on the auxiliary consumable is exposed through the first docking port to dock with the regulating device.

36. The biological tissue detection frame according to claim 35, wherein the side plate further comprises a second docking port and a third mounting position; the second docking port is in communication with the cavity, and the third mounting position is configured to mount a valve apparatus; in condition of the auxiliary consumable being moved into the cavity, a liquid pack connector in the auxiliary consumable is exposed through the second docking port to dock with the valve apparatus.

37. The biological tissue detection frame according to claim 35, wherein the side plate further comprises a third docking port and a fourth mounting position; the third docking port is in communication with the cavity, and the fourth mounting position is configured to mount a drive apparatus; in condition of the auxiliary consumable being moved into the cavity, the drive apparatus extends into the cavity from the third docking port and docks with the auxiliary consumable to drive liquid in the auxiliary consumable to flow.

38. The biological tissue detection frame according to claim 33, wherein the avoidance passage passes through the first mounting position, and a positioning member is arranged on the first mounting position for positioning the biological tissue measurement platform on the top plate.

39. A biological tissue detection device, comprising:
a biological tissue detection frame, comprising a cavity and a first mounting position;
a biological tissue measurement platform, detachably arranged on the first mounting position and comprising a first pipe; and
an auxiliary consumable, capable of being moved into and of being moved out of the cavity, and comprising a second pipe;
wherein an avoidance passage is arranged between the cavity and the first mounting position, the avoidance passage being configured to avoid mechanical docking operations and consumable delivery between the biological tissue measurement platform and the auxiliary consumable; the consumable delivery is a passage connecting the first pipe and the second pipe.

40. The biological tissue detection device according to claim 39, wherein the biological tissue detection frame comprises a plurality of plates, and the plurality of plates comprise a top plate and a bottom plate disposed opposite to each other, as well as a side plate disposed between the top plate and the bottom plate; the top plate, the bottom plate, and the side plate enclose to define the cavity with a pick-up and placement opening, and the auxiliary consumable capable of being moved into and of being moved out of the cavity through the pick-up and placement opening; the avoidance passage runs through the top plate, and the first mounting position is arranged on a side of the top plate away from the bottom plate.

41. The biological tissue detection device according to claim 40, wherein the auxiliary consumable comprises a sampling element, and the biological tissue detection device comprises a regulating device; the regulating device is configured to adjust a sampling posture of the sampling element; the side plate comprises a first docking port and a second mounting position; the first docking port is in communication with the cavity, and the second mounting position is configured to mount the regulating device; the sampling element is docked with the regulating device in condition of the auxiliary consumable being moved into the cavity.

42. The biological tissue detection device according to claim 40, wherein the auxiliary consumable comprises a receiving cavity for accommodating a liquid pack, and a cavity wall of the receiving cavity is arranged with the liquid pack connector; the biological tissue detection device further comprises a valve apparatus, the valve apparatus being configured to be docked with the liquid pack connector to control liquid in the liquid pack to flow out; the side plate comprises a second docking port and a third mounting position; the second docking port is in communication with the cavity, and the third mounting position is configured to mount the valve apparatus; the liquid pack connector is docked with the valve apparatus in condition of the auxiliary consumable being moved into the cavity.

43. The biological tissue detection device according to claim 40, wherein the auxiliary consumable comprises a receiving cavity for accommodating a liquid pack, and a cavity wall of the receiving cavity is arranged with a first drive member; the biological tissue detection device further comprises a drive apparatus, the drive apparatus being configured to be docked with the first drive member to drive liquid in the liquid pack to flow out; the side plate comprises a third docking port and a fourth mounting position; the third docking port is in communication with the cavity, and the fourth mounting position is configured to mount the drive apparatus; the drive apparatus is docked with the first drive member in condition of the auxiliary consumable being moved into the cavity.

44. A sample analysis apparatus, comprising:
a fixing seat and a mounting seat, wherein a guide rod is arranged between the fixing seat and the mounting seat;
a conveying assembly, disposed between the fixing seat and the mounting seat, wherein the conveying assembly is sleeved on the guide rod and is slidable along the guide rod; and
a testing assembly, disposed between fixing seat and mounting seat, wherein the testing assembly is sleeved on the guide rod and is slidable along the guide rod;
wherein the testing assembly is arranged on a side of the conveying assembly away from the fixing seat, and a first elastic member is arranged between the conveying assembly and the fixing seat; the conveying assembly and the fixing seat are configured to be separated by the first elastic member; a second elastic member is arranged between the conveying assembly and the testing assembly, and the conveying assembly and the testing assembly are configured to be separated by the second elastic member.

45. The sample analysis apparatus according to claim 44, wherein the conveying assembly comprises a carrier seat that is sleeved on and slidable along the guide rod, a telescopic seat slidably connected to the carrier seat, and a first drive member disposed on the carrier seat; the first elastic member is disposed between the carrier seat and the fixing seat, and the first drive member is capable of driving the telescopic seat to move relative to the carrier seat.

46. The sample analysis apparatus according to claim 45, wherein the carrier seat is arranged with a channel extending along a moving direction of the telescopic seat, and the telescopic seat is partially disposed in the channel and is movable along the channel relative to the carrier seat.

47. The sample analysis apparatus according to claim 46, wherein the telescopic seat defines a clamping slot for placing a detection assembly, and the clamping slot is configured to switch between a first position and a second position in condition of the telescopic seat moving relative to the carrier seat;
in condition of the clamping slot being in the first position, the detection assembly is capable of being moved into and of being moved out of the clamping slot;
in condition of the clamping slot being in the second position, the clamping slot is accommodated in the channel.

48. The sample analysis apparatus according to claim 47, wherein the carrier seat comprises a top wall and a bottom wall disposed opposite each other, the bottom wall being disposed on a side of the carrier seat close to the fixing seat, the top wall being disposed on a side of the carrier seat close to the testing assembly, and the channel being formed between the top wall and the bottom wall; the top wall defines a first avoidance hole, and the bottom wall defines a second avoidance hole; in condition of the clamping slot being in the second position, a detection element of the detection assembly placed in the clamping slot is exposed from the first avoidance hole, the testing assembly is capable of being docked with the detection element of the detection assembly through the first avoidance hole, and a liquid hole of the detection assembly is exposed from the second avoidance hole.

49. The sample analysis apparatus according to claim 46, wherein the telescopic seat is arranged with a transmission member, and the transmission member is assembled with the first drive member to drive the telescopic seat to move by means of the transmission member under drive of the first drive member.

50. The sample analysis apparatus according to claim 49, wherein the telescopic seat defines an avoidance slot, the transmission member is disposed on a slot wall of the avoidance slot, and an output shaft of the first drive member extends into the avoidance slot and is transmission-connected to the transmission member.

51. The sample analysis apparatus according to claim 48, wherein the testing assembly comprises a testing seat that is sleeved on the guide rod and slidable along the guide rod, and a testing plate arranged on the testing seat; the testing plate is arranged with a test head, and the test head contacts the detection element of the detection assembly through the first avoidance hole in condition of the testing seat and the carrier seat being docked.

52. The sample analysis apparatus according to claim 51, wherein the testing assembly further comprises a supporting component arranged on the testing seat, and a second elastic member arranged between the supporting component and the testing seat; in condition of the testing seat and the carrier seat being docked such that the testing plate contacts the detection element of the detection assembly, the second elastic member is compressed to generate an elastic force, and the elastic force is configured to cause the supporting component and the testing seat to separate.

53. The sample analysis apparatus according to claim 52, wherein the testing seat comprises a first mounting position and a second mounting position on a same side; the testing plate is arranged on the first mounting position, and the supporting component is arranged on the second mounting position; before the second elastic member is compressed, a height of the supporting component protruding from the testing seat is greater than or equal to a height of the test head protruding from the testing seat.

54. The sample analysis apparatus according to claim 53, wherein the supporting component comprises a body and a limit member; the limit member is fixedly disposed on the second mounting position, and the second elastic member is disposed between the body and the second mounting position; the body and the limit member are slidably connected, and the limit member is configured to limit a sliding travel of the body under an action of an elastic force.

55. The sample analysis apparatus according to claim 51, wherein the mounting seat is arranged with a second drive member, and the second drive member is configured to drive the testing seat to slide along the guide rod.

56. A biological detection device, comprising:
a first base and a second base;
a guide member, disposed between the first base and the second base and extending in an arrangement direction of the first base and the second base;
a testing assembly, arranged on the guide member and movable along the guide member; and
a transmission assembly, arranged on the first base and comprising a drive member;
wherein the testing assembly is arranged with a supporting member on a side close to the first base, and the drive member is in rolling fit with the supporting member to drive the testing assembly to move along the guide member.

57. The biological detection device according to claim 56, wherein the transmission assembly comprises a power member arranged on the first base and a support shaft passing through the drive member; the power member and the support shaft are transmission-assembled to drive the support shaft to rotate, and a rotation of the support shaft drives the drive member to rotate synchronously.

58. The biological detection device according to claim 57, wherein the biological detection device further comprises a mounting bracket and a drive bracket that are spaced apart and arranged on the first base, the support shaft being arranged between the mounting bracket and the drive bracket, and the power member being arranged on the drive bracket.

59. The biological detection device according to claim 58, wherein the first base defines an avoidance hole, and the avoidance hole is configured to avoid the drive member; the mounting bracket is arranged on a side of the first base close to the testing assembly, a spacer is arranged on at least one of the first base and the testing assembly, and the spacer is arranged between the first base and the testing assembly to prevent the mounting bracket from interfering with the rolling fit between the drive member and the supporting member.

60. The biological detection device according to claim 58, wherein the mounting bracket comprises a first bracket and a second bracket spaced apart; the second bracket is arranged between the first bracket and the drive bracket, and the support shaft is arranged between the first bracket and the drive bracket; the drive member is arranged between the first bracket and the second bracket and faces the avoidance hole, and the power member and the supporting shaft are transmission-assembled between the second bracket and the drive bracket.

61. The biological detection device according to claim 60, wherein the first bracket is arranged with a first positioning member, and the drive member or the supporting shaft is arranged with a second positioning member; the second positioning member is configured to rotate synchronously with the drive member or the supporting shaft and cooperate with the first positioning member to obtain a rotation angle of the drive member or the supporting shaft.

62. The biological detection device according to claim 60, wherein the power member is arranged on a side of the drive bracket away from the second bracket, and an output shaft of the power member passes through the drive bracket and is transmission-assembled with the support shaft.

63. The biological detection device according to claim 62, wherein the transmission assembly comprises a first transmission assembly disposed between the drive bracket and the second bracket; an end of the output shaft of the power member passing through the drive bracket and extending into between the drive bracket and the second bracket is arranged with a first transmission wheel, and a portion of the support shaft disposed between the drive bracket and the second bracket is arranged with a second transmission wheel, the first transmission assembly being transmission-assembled with the first transmission wheel and the second transmission wheel, respectively.

64. The biological detection device according to claim 63, wherein the first transmission assembly comprises a first fixed shaft arranged between the drive bracket and the second bracket, and a first driving wheel and a first driven wheel that are arranged on the first fixed shaft; the first driving wheel is transmission-assembled with the first transmission wheel, and the first driven wheel is transmission-assembled with the second transmission wheel;
wherein a diameter of the first driving wheel is greater than a diameter of the first driven wheel, an axial thickness of the first driving wheel is less than an axial thickness of the first transmission wheel, the diameter of the first driven wheel is less than a diameter of the second transmission wheel, and an axial thickness of the second transmission wheel is less than an axial thickness of the first driven wheel.

65. The biological detection device according to claim 64, wherein the transmission assembly further comprises a second transmission assembly disposed between the first transmission assembly and the second transmission wheel, and the second transmission assembly is transmission-assembled with the first transmission assembly and the second transmission wheel, respectively.

66. The biological detection device according to claim 65, wherein the second transmission assembly comprises a second fixed shaft arranged between the drive bracket and the second bracket, and a second driving wheel and a second driven wheel that are arranged on the second fixed shaft; the second driving wheel is transmission-assembled with the first driven wheel, and the second driven wheel is transmission-assembled with the second transmission wheel;
wherein a diameter of the second driving wheel is greater than a diameter of the second driven wheel, an axial thickness of the second driving wheel is less than the axial thickness of the first driven wheel, the diameter of the second driven wheel is less than the diameter of the second transmission wheel, and the axial thickness of the second transmission wheel is less than an axial thickness of the second driven wheel.

67. The biological detection device according to claim 60, wherein a limit block is arranged on one of the first bracket and the testing assembly, and the other of the first bracket and the testing assembly defines a limit groove; the limit block and the limit groove cooperate to limit a spacing between the first base and the testing assembly.

68. The biological detection device according to one of claims 56-67, wherein the drive member is a turbine, and the supporting member is a roller.

69. A blood sample analysis platform, comprising:
a blood sample analyzer, comprising a non-contact detection element and an avoidance slot; and
an auxiliary liquid box, comprising a cavity and a liquid path, wherein the cavity is configured to hold an auxiliary liquid and a waste liquid pack; an end of the liquid path is configured to introduce the auxiliary liquid, and another end of the liquid path is configured to communicate with the waste liquid pack;
wherein the liquid path has a portion that is exposed from the auxiliary liquid box, and the non-contact detection element is configured to detect a portion of the liquid path entering the avoidance slot.

70. The blood sample analysis platform according to claim 69, wherein the blood sample analysis platform comprises a mounting bracket, the mounting bracket comprising a receiving cavity and a first mounting position; the auxiliary liquid box is capable of being moved into and of being moved out of the receiving cavity, and the first mounting position is configured to mount the blood sample analyzer; an avoidance passage is arranged between the receiving cavity and the first mounting position, and in condition of the auxiliary liquid box being moved into the receiving cavity, the portion of the liquid path enters the avoidance slot through the avoidance passage.

71. The blood sample analysis platform according to claim 70, wherein the auxiliary liquid box is arranged with a sampling seat, and the liquid path comprises a liquid channel arranged in the sampling seat; in condition of the auxiliary liquid box being moved into the receiving cavity, the sampling seat is moved into the avoidance slot, and the non-contact detection element is configured to detect liquid in the liquid channel.

72. The blood sample analysis platform according to claim 71, wherein the liquid channel comprises a liquid inlet section, a liquid outlet section, and a detection section connecting the liquid inlet section and the liquid outlet section; the liquid inlet section is configured to introduce the auxiliary liquid, and the liquid outlet section is configured to be in communication with the waste liquid pack; in condition of the sampling seat being moved into the avoidance slot, the non-contact detection element is capable of detecting liquid in the detection section.

73. The blood sample analysis platform according to claim 72, wherein the sampling seat is arranged with a light-transmitting region corresponding to the detection section, and light emitted from the blood sample analyzer is configured to be directed to the liquid in the detection section through the light-transmitting region for detection.

74. The blood sample analysis platform according to claim 73, wherein the blood sample analyzer further comprises a testing seat and a testing plate, the testing seat being arranged on the first mounting position and the testing plate being arranged on the testing seat; the avoidance slot is defined on the testing seat, and the non-contact detection element comprises a light source and a light sensor device arranged on the testing plate; the light emitted by the light source is configured to pass through the light-transmitting region to reach the light sensor device to detect the liquid in the detection section.

75. The blood sample analysis platform according to claim 74, wherein the blood sample analyzer further comprises an ultrasonic sounder arranged on the testing seat, for ultrasonic processing of the liquid in the liquid channel entering the avoidance slot.

76. A blood sample analyzer, comprising:
a testing seat and a non-contact detection element, wherein the testing seat defines an avoidance slot, and the non-contact detection element is connected to the testing seat for detecting liquid entering the avoidance slot;
wherein the testing seat is capable of being docked with and of being separated from an auxiliary liquid box; a liquid path of the auxiliary liquid box comprises a bent part that protrudes from the auxiliary liquid box; in condition of the testing seat being docked with the auxiliary liquid box, the bent part enters the avoidance slot to be detected by the non-contact detection element.

77. The blood sample analyzer according to claim 76, wherein the blood sample analyzer further comprises a testing seat and a testing plate, the testing seat being arranged on the first mounting position and the testing plate being arranged on the testing seat; the avoidance slot is defined on the testing seat, and the non-contact detection element comprises a light source and a light sensor device arranged on the testing plate; the light emitted by the light source is configured to pass through the light-transmitting region to reach the light sensor device to detect the liquid in the detection section.

78. The blood sample analyzer according to claim 77, wherein the blood sample analyzer further comprises an ultrasonic sounder arranged on the testing seat, for ultrasonic processing of the liquid in the liquid channel entering the avoidance slot.

79. An auxiliary liquid box, comprising:
a cavity and a liquid path;
wherein the cavity is configured to hold an auxiliary liquid and a waste liquid pack; an end of the liquid path is configured to introduce the auxiliary liquid, and another end of the liquid path is configured to communicate with the waste liquid pack; the liquid path has a portion that is exposed from the auxiliary liquid box, and in condition of the auxiliary liquid box being docked with a blood sample analyzer, the portion of the liquid path enters an avoidance slot of the blood sample analyzer to be detected by a non-contact detection element of the blood sample analyzer.

80. The auxiliary liquid box according to claim 79, wherein the auxiliary liquid box is arranged with a sampling seat, and the liquid path comprises a liquid channel arranged in the sampling seat; in condition of the auxiliary liquid box being docked with the blood sample analyzer, the sampling seat is moved into the avoidance slot, and the non-contact detection element is configured to detect liquid in the liquid channel.

81. The auxiliary liquid box according to claim 80, wherein the liquid channel comprises a liquid inlet section, a liquid outlet section, and a detection section connecting the liquid inlet section and the liquid outlet section; the liquid inlet section is configured to introduce the auxiliary liquid, and the liquid outlet section is configured to be in communication with the waste liquid pack; in condition of the sampling seat being moved into the avoidance slot, the non-contact detection element is capable of detecting liquid in the detection section.

82. The auxiliary liquid box according to claim 81, wherein the sampling seat is arranged with a light-transmitting region corresponding to the detection section, and light emitted from the blood sample analyzer is configured to be directed to the liquid in the detection section through the light-transmitting region for detection.

83. A fluid detection instrument, comprising:
a valve assembly, comprising a control element, a first pipe, a plurality of inlets, and an outlet, wherein the control element is configured to control one of the plurality of inlets to be in communication with an end of the first pipe, and another end of the first pipe is in communication with the outlet; and
a fluid kit, comprising: a cavity for accommodating a fluid pack, and a plurality of ports, wherein an end of each port is configured to be in communication with the fluid pack, and the other end of the port is configured to be in communication with a corresponding inlet;
wherein the valve assembly is capable of being docked with and of being separated from the fluid kit, and the inlet and the port are configured to be docked or separated; in condition of the inlet and the port being docked, the first pipe is capable of being in communication with the port under control of the control element, and fluid in the fluid pack is configured to reach the first pipe.

84. The fluid detection instrument according to claim 83, wherein the valve assembly comprises a vent, and an end of the first pipe is selectively in communication with the inlet or the vent under the control of the control element.

85. The fluid detection instrument according to claim 83, wherein the fluid kit defines a docking groove configured to dock with a sampling element, and the docking groove is arranged with a through hole and a connecting pipe passing through the through hole; an end of the connecting pipe is in communication with the outlet, and the other end of the connecting pipe is configured to be in communication with a sample inlet of the sampling element; the connecting pipe is in sealed contact with a hole wall of the through hole.

86. The fluid detection instrument according to claim 85, wherein the sampling element comprises a sampling needle, a sleeve sleeved on the sampling needle, and a docking member arranged on an end of the sleeve; an outer diameter of the sleeve is less than or equal to an inner diameter of the docking groove; the docking member defines another through hole; the docking member is movable along an axial direction of the sampling needle following the sleeve, and the sampling needle is configured to be inserted into an end of the through hole on the docking member, or pass through the through hole on the docking member and be exposed on a side of the docking member;
wherein in condition of the sleeve moving along the axial direction of the sampling needle and towards the connecting pipe such that the docking member is inserted into the docking groove, the sampling needle and the connecting pipe are inserted into both ends of the through hole on the docking member to achieve connection.

87. A valve assembly, comprising:
a control element, a first pipe, a plurality of inlets, and an outlet;
wherein the control element is configured to control one of the plurality of inlets to be in communication with an end of the first pipe, and another end of the first pipe is in communication with the outlet; each inlet is capable of being docked with and of being separated from a port of a fluid kit, to obtain fluid of the fluid kit by controlling the inlet through the control element in condition of the inlet being docked with the port.

88. The fluid detection instrument according to claim 87, wherein the valve assembly comprises a vent, and an end of the first pipe is selectively in communication with the inlet or the vent under control of the control element.

89. A fluid kit, comprising:
a cavity for accommodating a fluid pack, and a plurality of ports;
wherein an end of each port is configured to be in communication with the fluid pack, and the other end of the port is configured to be in communication with a corresponding inlet of a valve assembly; the valve assembly is capable of being docked with and of being separated from the fluid kit, and in condition of the valve assembly and the fluid kit being docked, fluid in the fluid kit is configured to be delivered to the valve assembly through the port.

90. The fluid kit according to claim 89, wherein the fluid kit defines a docking groove configured to dock with a sampling element, and the docking groove is arranged with a through hole and a connecting pipe passing through the through hole; an end of the connecting pipe is in communication with the outlet, and the other end of the connecting pipe is configured to be in communication with a sample inlet of the sampling element; the connecting pipe is in sealed contact with a hole wall of the through hole.

91. The fluid kit according to claim 90, wherein the sampling element comprises a sampling needle, a sleeve sleeved on the sampling needle, and a docking member arranged on an end of the sleeve; an outer diameter of the sleeve is less than or equal to an inner diameter of the docking groove; the docking member defines another through hole; the docking member is movable along an axial direction of the sampling needle following the sleeve, and the sampling needle is configured to be inserted into an end of the through hole on the docking member, or pass through the through hole on the docking member and be exposed on a side of the docking member;
wherein in condition of the sleeve moving along the axial direction of the sampling needle and towards the connecting pipe such that the docking member is inserted into the docking groove, the sampling needle and the connecting pipe are inserted into both ends of the through hole on the docking member to achieve connection.

92. An integrated reagent kit, comprising:
a case, defining a storage space, wherein the storage space is configured to store a reagent pack and a recovery pack;
a sampling apparatus, arranged on the case and disposed outside the storage space; and
a first pipe and a second pipe that are arranged on the case, wherein an input end of the sampling apparatus is capable of being in communication with the reagent pack through the first pipe, and an output end of the sampling apparatus is in communication with the recovery pack through the second pipe; the sampling apparatus is rotatable relative to the case, and the input end of the sampling apparatus is configured to collect liquid from the reagent pack or an external container.

93. The integrated reagent kit according to claim 92, wherein the case comprises a first port and a second port arranged at intervals; the first port is in communication with the output end of the sampling apparatus, and the second port is in communication with the recovery pack through the second pipe; the first port and the second port are configured to dock with a testing assembly, and liquid collected by the sampling apparatus is configured to reach the testing assembly.

94. The integrated reagent kit according to claim 93, wherein the case is arranged with a sample introduction seat disposed outside the storage space, the first port and the second port being arranged on the sample introduction seat, and a first connecting pipe and the second connecting pipe are arranged in the sample introduction seat; an end of the first connecting pipe is in communication with the first port, and another end of the first connecting pipe is in communication with the output end of the sampling apparatus; an end of the second connecting pipe is in communication with the second port, and another end of the second connecting pipe is configured to be in communication with the recovery pack.

95. The integrated reagent kit according to claim 94, wherein the output end of the sampling apparatus is inserted into the sample introduction seat and in communication with the first connecting pipe.

96. The integrated reagent kit according to claim 95, wherein the sample introduction seat comprises a seat body and a pipe body arranged on an end of the seat body; the first port and the second port are arranged on the seat body; an end of the first connecting pipe is inserted into the pipe body, and another end of the first connecting pipe is in communication with the first port; the output end of the sampling apparatus is inserted into the pipe body to be in communication with the first connecting pipe.

97. The integrated reagent kit according to claim 96, wherein the sampling apparatus comprises a sampling element, a sleeve sleeved on an input end of the sampling element, and a swivel sleeved on an output end of the sampling element; the swivel is rotatably assembled with the pipe body, and the swivel is configured to be rotated relative to the pipe body; a rotation of the swivel is configured to drive the sleeve to rotate, and the input end of the sampling element is configured to collect the liquid from the reagent pack or the external container.

98. The integrated reagent kit according to claim 97, wherein the pipe body is arranged with a first hole segment and a second hole segment that are in communication, the first hole segment being configured to be rotatably connected to the swivel, and the output end of the sampling element and the first connecting pipe are in communication through the second hole segment; a hole diameter of the first hole segment is greater than a hole diameter of the second hole segment.

99. The integrated reagent kit according to claim 97, wherein the swivel comprises a first swivel and a second swivel clamped to the sleeve, the first swivel being rotatably assembled with the pipe body, and the second swivel being rotatable relative to the pipe body under an action of an external force, for driving the sleeve to rotate.

100. The integrated reagent kit according to claim 99, wherein the sleeve is movable axially along the sample inlet of the sampling element under an external force to expose or cover the sample inlet of the sampling element; the sleeve defines an avoidance hole to avoid the sampling element when the sleeve is moved axially.

101. The integrated reagent kit according to claim 92, wherein the case is arranged with a docking base, the docking base comprising a port; the port is configured to be docked with the input end of the sampling apparatus; an end of the first pipe is in communication with the port, and another end of the first pipe is configured to be in communication with the reagent pack.

102. A reagent storage apparatus, comprising:
a storage box, and a sampling assembly and a docking assembly that are disposed on the storage box and outside the storage box;
wherein an outlet end of the sampling assembly is in communication with a first position in the storage box, the docking assembly defines a sampling groove, and the sampling groove is in communication with a second position in the storage box; the sampling assembly is capable of being connected with and of being separated from the docking assembly; in condition of the sampling assembly and the docking assembly being connected, an inlet end of the sampling assembly is inserted into the sampling groove to sample from the second position in storage box; in condition of the sampling assembly and the docking assembly being separated, the inlet end of sampling assembly is capable of sampling from outside the storage box.

103. The reagent storage apparatus according to claim 102, wherein the docking assembly comprises a docking base arranged on the storage box, the sampling groove is defined on the docking base, and a connector pipe is disposed in the sampling groove; an end of the connector pipe is configured to be in communication with the inlet end of the sampling assembly, and another end of the connector pipe is configured to be in communication with the second position in the storage box; the inlet end of the sampling assembly is arranged with a connector, and in condition of the sampling assembly being connected to the docking assembly, the connector pipe is inserted into the connector.

104. The reagent storage apparatus according to claim 103, wherein the sampling assembly comprises a sampling element capable of being inserted into or passing through the connector; in condition of the sampling assembly and the docking assembly being connected, a sampling end of the sampling element is inserted into the connector, and the connector pipe is arranged on the connector to be in communication with the sampling end of the sampling element.

105. The reagent storage apparatus according to claim 103, wherein the docking assembly further comprises a sealing member embedded in the sampling groove, and the connector pipe passes through the sealing member; in condition of the connector pipe being inserted into the connector, the sealing member abuts against the connector.

106. The reagent storage apparatus according to claim 105, wherein an end of the connector close to the sealing member is tapered and concave to guide the connector pipe to be inserted into the connector.

107. The reagent storage apparatus according to claim 103, wherein the docking base defines a matching groove opened facing the storage box; an end of the connector pipe extends into the matching groove to be in communication with the second position in the storage box through a connecting pipe, and another end of the connector pipe extends into sampling groove to be inserted in the connector in condition of the sampling assembly being connected to the docking assembly.

108. The reagent storage apparatus according to claim 103, wherein the sampling assembly comprises a sleeve sleeved on the sampling element, and the connector is embedded in an end portion of the sleeve; a clamping member is sleeved on the connector at the end portion of the sleeve, and the clamping member is connected to the sleeve; a radial width of the clamping member is greater than a radial width of the sealing member, and the radial width of the clamping member is less than or equal to a radial width of the sampling groove.

109. The reagent storage apparatus according to claim 103, wherein the docking assembly further comprises a sliding member that is slidably connected to the docking base, and the sliding member is capable of abutting against the sampling assembly to stabilize a sampling state of the sampling assembly.

110. The reagent storage apparatus according to claim 109, wherein the docking base defines a mating hole, the sliding member passes through the mating hole and is elastically connected to the storage box, and the sliding member is configured to slide relative to the docking base.

111. The reagent storage apparatus according to claim 110, wherein the mating hole comprises a first mating section and a second mating section that are in communication; the first mating section has a cross-sectional area that is smaller than a cross-sectional area of the second mating section, and the first mating section is arranged between the second mating section and the storage box; the sliding member comprises a sliding portion and a limit portion, the sliding portion being slidable relative to the first mating portion, and the limit portion being slidable relative to the second mating portion and stopped by the second mating portion.

112. The reagent storage apparatus according to claim 110, wherein the docking assembly further comprises an elastic member arranged between the storage box and the sliding member; an end of the elastic member is connected to the sliding member, and another end of the elastic member is connected to the storage box.

113. The reagent storage apparatus according to claim 103, wherein the docking base defines an avoidance slot that passes through a side wall of the sampling groove; the inlet end of the sampling assembly is capable of being unscrewed out of the sampling groove through the avoidance slot, and the inlet end of the sampling assembly is capable of sampling from outside the storage box.

114. A liquid parameter measurement platform, comprising:
a liquid loading box, comprising a liquid taking assembly and an adjusting assembly, wherein the adjusting assembly is configured to be driven under an external force to drive the liquid taking assembly to rotate relative to the liquid loading box; and
a rotatable assembly and a movable assembly, wherein the rotatable assembly is rotatable to drive the movable assembly to rotate synchronously, and the movable assembly is movable relative to the rotatable assembly;
wherein the adjusting assembly is capable of being docked with and of being separated from the rotatable assembly; in condition of the adjusting assembly and the rotatable assembly being docked, the rotatable assembly is capable of driving the adjusting assembly to rotate relative to the liquid loading box, thereby driving the liquid taking assembly to rotate relative to the liquid loading box;
the liquid taking assembly and the movable assembly are configured to follow docking or separation of the adjusting assembly and the rotatable assembly to achieve corresponding docking or separation;
in condition of the liquid taking assembly and the movable assembly being docked, the movable assembly is configured to adjust an axial position of at least part of the liquid taking assembly, and the rotatable assembly is configured to drive the liquid taking assembly to switch between a first attitude and a second attitude;
wherein the first attitude corresponds to that the liquid taking assembly is limited to collect liquid from inside the liquid loading box, and the second attitude corresponds to that the liquid taking assembly is limited to collect liquid from outside the liquid loading box.

115. The liquid parameter measurement platform according to claim 114, wherein the liquid taking assembly comprises a liquid taking element and a sleeve; a liquid taking end of the liquid taking element is configured to collect the liquid inside the liquid loading box or the liquid outside the liquid loading box; liquid collected by the liquid taking element is configured to flow to an interior of liquid loading box or a liquid recovery container through a liquid outlet end of the liquid taking element;
the sleeve is sleeved on the liquid taking end of the liquid taking element and is capable of being docked with the movable assembly, and an axial position of the sleeve relative to the liquid taking element is adjustable under drive of the movable assembly.

116. The liquid parameter measurement platform according to claim 115, wherein the adjusting assembly comprises a swivel clamped to the sleeve; an end of the swivel is sleeved on the liquid outlet end of the liquid taking element, and another end of the swivel is capable of being docked with and of being separated from the rotatable assembly; in condition of the rotatable assembly being docked with the swivel, the rotatable assembly is capable of driving the swivel to rotate, thereby driving the sleeve and the liquid taking element to rotate.

117. The liquid parameter measurement platform according to claim 116, wherein the rotatable assembly comprises an adjusting seat to be arranged on the liquid parameter measurement platform, a first power member arranged on the adjusting seat, and an adjusting bracket; the first power member is configured to drive the adjusting bracket to rotate; the adjusting bracket is configured to be assembled with the swivel to adjust a rotation angle of the swivel relative to the liquid loading box.

118. The liquid parameter measurement platform according to claim 117, wherein the rotatable assembly further comprises a rotary member disposed between the adjusting seat and the adjusting bracket, the rotary member being connected to the adjusting bracket for synchronous rotation, and the first power member being capable of driving the rotary member to rotate.

119. The liquid parameter measurement platform according to claim 118, wherein the first power member is arranged on a side of the adjusting seat away from the rotary member, and an output shaft of the first power member passes through the adjusting seat and is transmission-connected to the rotary member.

120. The liquid parameter measurement platform according to claim 118, wherein the adjusting seat defines a rotating groove, the rotary member is at least partially embedded in the rotating groove, and an output shaft of the first power member is transmission-connected to the rotatory member to drive the rotary member to rotate relative to the rotating groove;
a shaft hole is defined on the rotating groove, a rotating shaft is arranged on the adjusting bracket, and the rotating shaft passes through the rotary member and is inserted into the shaft hole.

121. The liquid parameter measurement platform according to claim 120, wherein a bottom wall of the rotating groove defines a curved slot extending in a rotation direction of the rotary member, the curved slot is arranged with a first positioning member, and the rotary member is arranged with a slider that is slidable along the curved slot; the slider is configured to cooperate with the first positioning member to obtain a rotation angle of the rotary member relative to the adjusting seat.

122. The liquid parameter measurement platform according to claim 117, wherein the adjusting bracket is assembled with the movable assembly; in condition of the first power member driving the adjusting bracket to rotate, the movable assembly is rotatable synchronously with the adjusting bracket; the adjusting bracket defines an adjusting slot, and the movable assembly is embedded in the adjusting slot to rotate synchronously with the adjusting bracket.

123. The liquid parameter measurement platform according to claim 122, wherein the movable assembly comprises a second power member arranged on the adjusting bracket, and a movable bracket connected to the second power member; the second power member is configured to drive the movable bracket to move relative to the adjusting bracket;
in condition of the adjusting assembly being docked with the rotatable assembly, the sleeve of the liquid taking assembly is nested on a side of the movable bracket, and the movable bracket is connected to the sleeve and capable of driving the sleeve to switch between a first position and a second position.

124. The liquid parameter measurement platform according to claim 123, wherein the movable assembly further comprises a fixed bracket embedded in the adjusting slot, and an end of the output shaft of the second power member passes through the fixed bracket to connect with the movable bracket.

125. The liquid parameter measurement platform according to claim 124, wherein an elastic member is arranged between the fixed bracket and the movable bracket, and the movable bracket is configured to move relative to the fixed bracket under drive of the second power member, causing the elastic member to generate elasticity; in condition of the drive being cancelled, the elasticity causes the movable bracket to reset.

126. The liquid parameter measurement platform according to claim 123, wherein the movable bracket comprises a first engagement portion, and the sleeve comprises a second engagement portion; in condition of the adjusting assembly being docked with the rotatable assembly, the first engagement portion and the second engagement portion are engaged and connected.

127. A liquid loading box, applied to a liquid parameter measurement platform and comprising:
a liquid taking assembly and an adjusting assembly, wherein the adjusting assembly is configured to, under an external force, drive the liquid taking assembly to rotate relative to the liquid loading box;
wherein the adjusting assembly is capable of being docked with and of being separated from a rotatable assembly, and in condition of the adjusting assembly being docked with the rotatable assembly, the rotatable assembly is capable of driving the adjusting assembly to rotate relative to the liquid loading box, thereby driving the liquid taking assembly to rotate relative to the liquid loading box;
the liquid taking assembly is capable of being docked with and of being separated from a movable assembly, and the liquid taking assembly and the movable assembly are configured to follow docking or separation of the adjusting assembly and the rotatable assembly to achieve corresponding docking or separation; in condition of the liquid taking assembly and the movable assembly being docked, the movable assembly is configured to adjust an axial position of at least part of the liquid taking assembly, and the rotatable assembly is configured to drive the liquid taking assembly to switch between a first attitude and a second attitude;
wherein the first attitude corresponds to that the liquid taking assembly is limited to collect liquid from inside the liquid loading box, and the second attitude corresponds to that the liquid taking assembly is limited to collect liquid from outside the liquid loading box.

128. The liquid loading box according to claim 127, wherein the liquid taking assembly comprises a liquid taking element and a sleeve; a liquid taking end of the liquid taking element is configured to collect the liquid inside the liquid loading box or the liquid outside the liquid loading box; liquid collected by the liquid taking element is configured to flow to an interior of liquid loading box or a liquid recovery container through a liquid outlet end of the liquid taking element;
the sleeve is sleeved on the liquid taking end of the liquid taking element and is capable of being docked with the movable assembly, and an axial position of the sleeve relative to the liquid taking element is adjustable under drive of the movable assembly.

129. The liquid loading box according to claim 128, wherein the adjusting assembly comprises a swivel clamped to the sleeve; an end of the swivel is sleeved on the liquid outlet end of the liquid taking element, and another end of the swivel is capable of being docked with and of being separated from the rotatable assembly; in condition of the rotatable assembly being docked with the swivel, the rotatable assembly is capable of driving the swivel to rotate, thereby driving the sleeve and the liquid taking element to rotate.

130. A motion apparatus, applied to a liquid parameter measurement platform and comprising:
a rotatable assembly and a movable assembly, wherein the rotatable assembly is rotatable to drive the movable assembly to rotate synchronously, and the movable assembly is movable relative to the rotatable assembly;
wherein the rotatable assembly is capable of being docked with and of being separated from an adjusting assembly of a liquid loading box; in condition of the adjusting assembly and the rotatable assembly being docked, the rotatable assembly is capable of driving the adjusting assembly to rotate relative to the liquid loading box, thereby driving a liquid taking assembly of the liquid loading box to rotate relative to the liquid loading box;
wherein the movable assembly is capable of being docked with and of being separated from the liquid taking assembly; the liquid taking assembly and the movable assembly are configured to follow docking or separation of the adjusting assembly and the rotatable assembly to achieve corresponding docking or separation;
in condition of the liquid taking assembly and the movable assembly being docked, the movable assembly is configured to adjust an axial position of at least part of the liquid taking assembly, and the rotatable assembly is configured to drive the liquid taking assembly to switch between a first attitude and a second attitude;
wherein the first attitude corresponds to that the liquid taking assembly is limited to collect liquid from inside the liquid loading box, and the second attitude corresponds to that the liquid taking assembly is limited to collect liquid from outside the liquid loading box.

131. The motion apparatus according to claim 130, wherein the rotatable assembly comprises an adjusting seat to be arranged on the liquid parameter measurement platform, a first power member arranged on the adjusting seat, and an adjusting bracket; the first power member is configured to drive the adjusting bracket to rotate; the adjusting bracket is configured to be assembled with the swivel to adjust a rotation angle of the swivel relative to the liquid loading box.

132. The motion apparatus according to claim 131, wherein the adjusting bracket is assembled with the movable assembly; in condition of the first power member driving the adjusting bracket to rotate, the movable assembly is rotatable synchronously with the adjusting bracket; the adjusting bracket defines an adjusting slot, and the movable assembly is embedded in the adjusting slot to rotate synchronously with the adjusting bracket.

133. The liquid parameter measurement platform according to claim 132, wherein the movable assembly comprises a second power member arranged on the adjusting bracket, and a movable bracket connected to the second power member; the second power member is configured to drive the movable bracket to move relative to the adjusting bracket;
in condition of the adjusting assembly being docked with the rotatable assembly, the sleeve of the liquid taking assembly is nested on a side of the movable bracket, and the movable bracket is connected to the sleeve and capable of driving the sleeve to switch between a first position and a second position.

134. A biological sample analysis device, comprising:
a drive assembly, comprising an output shaft; and
a reagent storage apparatus, comprising a receiving cavity, a connecting pipe, and a squeezing assembly, wherein the receiving cavity is configured to hold a reagent pack and a recovery pack, and the connecting pipe is partially embedded in the squeezing assembly; an end of the connecting pipe is configured to be in communication with the reagent pack, and another end of the connecting pipe is configured to be in communication with the recovery pack;
wherein the drive assembly is capable of being docked with and of being separated from the reagent storage apparatus, causing the output shaft to be docked with or separated from the squeezing assembly; in condition of the drive assembly and the reagent storage apparatus being docked, the output shaft is capable of driving the squeezing assembly to squeeze the connecting pipe to cause liquid in the connecting pipe to flow.

135. The biological sample analysis device according to claim 134, wherein the biological sample analysis device further comprises a mounting bracket; the mounting bracket comprises a cavity and a first mounting position; the reagent storage apparatus is capable of being moved into and of being moved out of the cavity, and the drive assembly is arranged on the first mounting position; the first mounting position is arranged on an end of a travel of the reagent storage apparatus moving into the cavity.

136. The biological sample analysis device according to claim 135, wherein the drive assembly is arranged outside the cavity, and an avoidance hole is defined between the cavity and the first mounting position, with the output shaft of the drive assembly extending into the cavity through the avoidance hole; in condition of the reagent storage apparatus being moved into the cavity, the output shaft of the drive assembly is transmission-assembled with the squeezing assembly.

137. The biological sample analysis device according to claim 135, wherein the squeezing assembly comprises a first squeezing member and a second squeezing member, the second squeezing member being arranged coaxially with the first squeezing member and being embedded in the first squeezing member, and a part of the connecting pipe being arranged between the first squeezing member and the second squeezing member; the second squeezing member is configured to be transmission-assembled with the output shaft, and the second squeezing member is configured to be driven by the output shaft to rotate relative to the first squeezing member and thereby squeeze the connecting pipe.

138. The biological sample analysis device according to claim 137, wherein the first squeezing member is annular in shape, and the second squeezing member comprises a rotating portion and a squeezing portion; the rotating portion is configured to be docked with the output shaft, and the squeezing portion is disposed on a peripheral side of the rotating portion and rotatable synchronously with the rotating portion;
the squeezing portion is arranged spaced apart from the first squeezing portion, and the connecting pipe is partially arranged between the squeezing portion and the first squeezing portion.

139. The biological sample analysis device according to claim 138, wherein the rotating portion defines a shaft hole, and an end of the output shaft is assembled with the shaft hole; the squeezing portion is sleeved with a shaft sleeve, the shaft sleeve being configured to abut against the connecting pipe.

140. The biological sample analysis device according to claim 135, wherein a cavity wall of the receiving cavity defines a storage groove, the squeezing assembly is embedded in the storage groove and arranged coaxially with the storage groove, and the connecting pipe is partially arranged between the storage groove and the squeezing assembly; the squeezing assembly is configured to be transmission-assembled with the output shaft, and the squeezing assembly is configured to be driven by the output shaft to rotate relative to the storage groove and thereby squeeze the connecting pipe.

141. A reagent storage apparatus, comprising:
a receiving cavity, a connecting pipe, and a squeezing assembly, wherein the receiving cavity is configured to hold a reagent pack and a recovery pack, and the connecting pipe is partially embedded in the squeezing assembly; an end of the connecting pipe is configured to be in communication with the reagent pack, and another end of the connecting pipe is configured to be in communication with the recovery pack;
wherein the squeezing assembly is capable of being docked with and of being separated from an output shaft of a drive assembly; in condition of the squeezing assembly and the output shaft being docked, the output shaft of the drive assembly is configured to drive the squeezing assembly to squeeze the connecting pipe to cause liquid in the connecting pipe to flow.

142. The reagent storage apparatus according to claim 141, wherein the squeezing assembly comprises a first squeezing member and a second squeezing member, the second squeezing member being arranged coaxially with the first squeezing member and being embedded in the first squeezing member, and a part of the connecting pipe being arranged between the first squeezing member and the second squeezing member; the second squeezing member is configured to be transmission-assembled with the output shaft, and the second squeezing member is configured to be driven by the output shaft to rotate relative to the first squeezing member and thereby squeeze the connecting pipe.

143. The reagent storage apparatus according to claim 142, wherein the first squeezing member is annular in shape, and the second squeezing member comprises a rotating portion and a squeezing portion; the rotating portion is configured to be docked with the output shaft, and the squeezing portion is disposed on a peripheral side of the rotating portion and rotatable synchronously with the rotating portion;
the squeezing portion is arranged spaced apart from the first squeezing portion, and the connecting pipe is partially arranged between the squeezing portion and the first squeezing portion.

144. The reagent storage apparatus according to claim 143, wherein the rotating portion defines a shaft hole, and an end of the output shaft is assembled with the shaft hole; the squeezing portion is sleeved with a shaft sleeve, the shaft sleeve being configured to abut against the connecting pipe.

145. The reagent storage apparatus according to claim 141, wherein a cavity wall of the receiving cavity defines a storage groove, the squeezing assembly is embedded in the storage groove and arranged coaxially with the storage groove, and the connecting pipe is partially arranged between the storage groove and the squeezing assembly; the squeezing assembly is configured to be transmission-assembled with the output shaft, and the squeezing assembly is configured to be driven by the output shaft to rotate relative to the storage groove and thereby squeeze the connecting pipe.

146. A liquid drive apparatus, comprising:
a power assembly, a rotatable assembly, and a liquid pipe;
wherein one of the power assembly and the rotatable assembly comprises a rotating shaft, and the other of the power assembly and the rotatable assembly comprises a mating portion into which the rotating shaft is configured to be inserted;
one of the rotating shaft and the mating portion is arranged with a snap fastener, and the other of the rotating shaft and the mating portion defines a snap groove;
the liquid pipe is arranged in the rotatable assembly, and the rotatable assembly is rotatable to squeeze the liquid pipe;
wherein the power assembly is capable of being docked with and of being separated from the rotatable assembly, and the rotating shaft is configured to be inserted into the mating portion or pulled out of the mating portion; in condition of the rotating shaft being inserted into the mating portion, the rotating shaft is rotatable to engage the snap fastener and the snap groove to achieve a locking limit.

147. The liquid drive apparatus according to claim 146, wherein the snap fastener is arranged on the rotating shaft, the snap groove is defined on the mating portion, and the snap fastener protrudes from a peripheral side of the rotating shaft in condition of no external force being applied; in condition of the rotating shaft being inserted into the mating portion, the snap fastener is restrained by the mating portion and is retracted in the rotating shaft; in condition of the rotating shaft rotating relative to the mating portion until the snap fastener is opposite the snap groove, the snap fastener protrudes from the peripheral side of the rotating shaft and snaps into the snap groove.

148. The liquid drive apparatus according to claim 146, wherein the snap fastener is arranged on the mating portion, the snap groove is defined on the rotating shaft, and the snap fastener protrudes from the mating portion in condition of no external force being applied; in condition of the rotating shaft being inserted into the mating portion, the snap fastener is restrained by the rotating shaft and is retracted in the mating portion; in condition of the rotating shaft rotating relative to the mating portion until the snap fastener is opposite the snap groove, the snap fastener protrudes from the mating portion and snaps into the snap groove.

149. The liquid drive apparatus according to claim 147 or 148, wherein an end of the snap fastener that engages with the snap groove comprises a guide ramp, and the guide ramp is configured to provide resistance in condition of the rotating shaft being inserted into the mating portion, causing the snap fastener to be retracted.

150. The liquid drive apparatus according to claim 149, wherein an end of the rotating shaft comprises a chamfer, and the chamfer is configured to guide the rotating shaft to be inserted into the mating portion.

151. The liquid drive apparatus according to claim 149, wherein a mounting hole is defined on the rotating shaft or the mating portion, the snap fastener is assembled in the mounting hole, and an elastic member that abuts against the snap fastener is arranged in the mounting hole; the elastic member is configured to generate an elastic force in condition of the snap fastener being retracted; in condition of the snap fastener facing the snap groove, the elastic force causes the snap fastener to extend to snap with the snap groove.

152. The liquid drive apparatus according to claim 151, wherein a first limit portion is arranged in the mounting hole, and the snap fastener is arranged with a second limit portion; the first limit portion and the second limit portion are configured to cooperate to limit an extension travel of the snap fastener.

153. The liquid drive apparatus according to claim 146, wherein a first gear is sleeved on the rotating shaft, and the power assembly comprises a drive member and a second gear, the second gear being transmission-assembled with an output shaft of the drive member; the first gear is connected to the second gear by meshing, and a thickness of the first gear along an axial direction of the rotating shaft is less than a thickness of the second gear along the axial direction of the rotating shaft.

154. The liquid drive apparatus according to claim 153, wherein the first gear is spaced from the drive member along the axial direction of the rotating shaft.

155. The liquid drive apparatus according to claim 146, wherein the rotatable assembly comprises a holding space for holding the liquid pipe and a window communicating with the holding space, with two ends of the liquid pipe extending out of the holding space through the window.

156. A reagent kit, comprising:
a cavity, a liquid pipe, and a rotatable assembly;
wherein the cavity is configured to hold a reagent pack and a recovery pack, the liquid pipe is arranged in the rotatable assembly, and the rotatable assembly is rotatable to squeeze the liquid pipe, causing liquid in the reagent pack to pass through the liquid pipe and reach the recovery pack;
the rotatable assembly is configured to capable of being docked with and of being separated from a power assembly; one of the power assembly and the rotatable assembly comprises a rotating shaft, and the other of the power assembly and the rotatable assembly comprises a mating portion into which the rotating shaft is configured to be inserted; one of the rotating shaft and the mating portion is arranged with a snap fastener, and the other of the rotating shaft and the mating portion defines a snap groove;
in condition of the rotatable assembly being docked with the power assembly, the rotating shaft is inserted into the mating portion, and the rotating shaft is rotatable to engage the snap fastener and the snap groove to achieve a locking limit.

157. A sample analysis apparatus, comprising:
a mounting bracket, comprising a first mounting position and a second mounting position, wherein the first mounting position is configured to mount a reagent kit, and the second mounting position is configured to mount a power assembly;
wherein the power assembly is capable of being docked with and of being separated from a rotatable assembly of the reagent kit; one of the power assembly and the rotatable assembly comprises a rotating shaft, and the other of the power assembly and the rotatable assembly comprises a mating portion into which the rotating shaft is configured to be inserted; one of the rotating shaft and the mating portion is arranged with a snap fastener, and the other of the rotating shaft and the mating portion defines a snap groove;
in condition of the rotatable assembly being docked with the power assembly, the rotating shaft is inserted into the mating portion, and the rotating shaft is rotatable to engage the snap fastener and the snap groove to achieve a locking limit.

158. A medical detection device, comprising:
a detection assembly, comprising a detection element, a first liquid path, and a first port, wherein the first liquid path is in communication with the first port, and the detection element is configured to detect liquid in the first liquid path to obtain a detection signal;
a processing circuit, detachably connected to the detection element of the detection assembly and configured to receive the detection signal in condition of being connected to the detection element; and
a reagent kit, comprising a cavity, a second liquid path, and a second port, wherein the cavity is configured to hold a liquid pack; an end of the second liquid path is configured to be in communication with the liquid pack, and another end of the second liquid path is in communication with the second port;
wherein the detection assembly and the reagent kit are capable of being docked and of being separated, causing the first port and the second port to be connected or separated; in condition of the first port and the second port being connected, the first liquid path is in communication with the second liquid path, causing reagent in the liquid pack to reach the first liquid path; in condition of the detection assembly and the reagent kit being separated to replace the reagent kit, the processing circuit keeps electrically connected to the detection element of the detection assembly.

159. The medical detection device according to claim 158, wherein the medical detection device comprises a mounting bracket, the mounting bracket comprising a first mounting position, a second mounting position, and a third mounting position; the first mounting position is configured to mount the detection assembly, the second mounting position is configured to place the reagent kit, and the processing circuit is arranged in the third mounting position;
the detection assembly and the processing circuit are movable synchronously relative to the reagent kit while maintaining an electrical connection, causing the detection assembly to be docked with or separated from the reagent kit; the reagent kit is replaceable in condition of the detection assembly and the reagent kit being separated.

160. The medical detection device according to claim 159, wherein the mounting bracket comprises a fixed bracket, a first drive mechanism, a first movable bracket, and a second movable bracket; the first movable bracket is arranged on the fixed bracket, and the first movable bracket is connected to the second movable bracket; the first movable bracket is configured to hold the detection assembly, and the second movable bracket is configured to assemble the processing circuit; the detection assembly and the processing circuit are configured to undergo a relative movement driven by the first drive mechanism, causing the detection element of the detection assembly to maintain electrically connected to or be disconnected from the processing circuit; the first drive mechanism is configured to drive the first movable bracket to move, causing the detection assembly to be docked with or separated from the reagent kit.

161. The medical detection device according to claim 160, wherein the first movable bracket defines a storage groove for accommodating the detection assembly, the detection assembly is capable of being moved into and of being moved out of the storage groove, and the second movable bracket is elastically connected to the first movable bracket; in condition of the detection assembly being moved into the storage groove, the processing circuit is capable of moving towards the detection assembly and being electrically connected under drive of the first drive mechanism.

162. The medical detection device according to claim 160, wherein the first movable bracket is elastically connected to the mounting bracket; under drive of the first drive mechanism, the detection assembly is movable towards the reagent kit to generate an elastic force and achieve communication between the first port and the second port; the detection assembly is movable away from the reagent kit by means of an elastic force to achieve separation between the first port and the second port.

163. The medical detection device according to claim 160, wherein the mounting bracket comprises a second drive mechanism and a liquid path selector; the second drive mechanism is arranged on the second movable bracket and configured to drive the liquid path selector to move; in condition of the detection assembly being docked with the reagent kit, the liquid path selector is driven by the second drive mechanism to enable the detection assembly to realize different liquid path circulations.

164. The medical detection device according to claim 160, wherein the fixed bracket comprises a cavity for accommodating the reagent kit, and a cavity wall of the cavity defines an avoidance opening; the detection assembly is arranged outside the cavity; in condition of the reagent kit being moved into the cavity, the second port of the reagent kit is exposed from the avoidance opening to achieve connection or separation with the first port of the detection assembly.

165. The medical detection device according to claim 164, wherein a region in which the second port of the reagent kit is located forms a concave portion, the shape of the concave portion being adapted to the shape of the detection assembly.

166. The medical detection device according to claim 158, wherein the reagent kit is arranged with a sampling element, a sample inlet of the sampling element being configured to selectively collect reagent from the liquid pack or liquid outside the reagent kit, and a sample outlet of the sampling element being configured to be in communication with the second port; in condition of the reagent from the liquid pack being is required to be collected, the sampling element forms a part of the second liquid path.

167. The medical detection device according to claim 166, wherein the reagent kit further comprises a first connecting pipe, an end of the first connecting pipe being in communication with the liquid pack and another end of the first connecting pipe being in communication with the sample inlet of the sampling element; the first connecting pipe forms another part of the second liquid path.

168. The medical detection device according to claim 158, wherein the detection assembly further comprises a third port spaced apart from the first port, and the third port is in communication with the first liquid path; the reagent kit further comprises a fourth port and a second connecting pipe, and the fourth port is spaced apart from the second port; an end of the second connecting pipe is in communication with the fourth port, and another end of the second connecting pipe is in communication with the cavity; in condition of the first port and the second port being in communication, the third port and the fourth port are in communication.

169. A detection assembly, comprising:
a housing, a detection element and a first liquid path that are disposed in the housing, and a first port and a connection terminal that are disposed on the housing, wherein the connection terminal is electrically connected to the detection element;
wherein the first liquid path is in communication with the first port, and the detection element is configured to perform a blood gas detection on liquid in the first liquid path to obtain a detection signal; the first port is capable of being docked with and of being separated from a second port of a reagent kit for obtaining reagent of the reagent kit through the first port in condition of the first port being docked with the second port; in condition of the detection assembly and the reagent kit being separated to replace the reagent kit, the connection terminal remains electrically connected to a processing circuit of a medical detection device.

170. The detection assembly according to claim 169, wherein the housing is arranged with a first positioning member to position a bracket for mounting the detection assembly on the medical detection device.

171. The detection assembly according to claim 169, wherein an end of the first port that is docked with the second port comprises a tapered recess or tapered protrusion for guiding the second port to dock with the first port.

172. A reagent kit, comprising:
a cavity, a second liquid path, and a second port;
wherein the cavity is configured to hold a liquid pack; an end of the second liquid path is configured to be in communication with the liquid pack, and another end of the second liquid path is in communication with the second port; the second port is configured to be docked with a first port of a detection assembly in condition of a detection being performed using the reagent kit, and reagent in the liquid pack is configured to be delivered to the detection assembly through the second port; the second port is separated from the detection assembly before the detection is performed using the reagent kit; the reagent kit is capable of being moved into and of being moved out of a mounting bracket of a medical detection device, and in condition of the reagent kit being moved in, the first port and the second port are in communication.

173. The reagent kit according to claim 172, wherein a region in which the second port of the reagent kit is located forms a concave portion, the shape of the concave portion being adapted to the shape of the detection assembly.

174. The detection assembly according to claim 173, wherein the concave portion is arranged with a second positioning member for positioning the detection assembly.

175. The reagent kit according to claim 172, wherein the reagent kit is arranged with a sampling element, a sample inlet of the sampling element being configured to selectively collect reagent from the liquid pack or liquid outside the reagent kit, and a sample outlet of the sampling element being configured to be in communication with the second port; in condition of the reagent from the liquid pack being required to be collected, the sampling element forms a part of the second liquid path.

176. The reagent kit according to claim 172, wherein the detection assembly comprises a third port spaced apart from the first port, and the reagent kit further comprises a fourth port spaced apart from the second port; in condition of the first port and the second port being in communication, the third port and the fourth port are in communication.

177. A medical detection device, comprising:
a mounting bracket, comprising a first mounting position, a second mounting position, and a third mounting position, wherein the first mounting position is configured to mount a detection assembly, and the second mounting position is configured to mount a reagent kit; and
a processing circuit, arranged on the third mounting position and detachably connected to the detection assembly;
wherein the detection assembly and the reagent kit are capable of being docked and of being separated, and the detection assembly and the reagent kit are caused to be in communication or separated; in condition of the detection assembly and the reagent kit being in communication, reagent in the reagent kit is capable of reaching the detection assembly; in condition of the detection assembly and the reagent kit being separated to replace the reagent kit, the processing circuit remains electrically connected to the detection assembly.

178. The medical detection device according to claim 177, wherein a processing circuit is arranged on the mounting bracket, the mounting bracket comprising a first mounting position, a second mounting position, and a third mounting position, wherein the first mounting position is configured to mount the detection assembly, the second mounting position is configured to hold the reagent kit, and the processing circuit is arranged on the third mounting position;
wherein the detection assembly and the processing circuit is movable synchronously relative to the reagent kit while maintaining an electrical connection, causing the detection assembly to be capable of being docked with and of being separated from the reagent kit; the reagent kit is replaceable in condition of the detection assembly being separated from the reagent kit.

179. The medical detection device according to claim 178, wherein the mounting bracket comprises a fixed bracket, a first drive mechanism, a first movable bracket, and a second movable bracket; the first movable bracket is arranged on the fixed bracket, and the first movable bracket is connected to the second movable bracket; the first movable bracket is configured to hold the detection assembly, and the second movable bracket is configured to assemble the processing circuit; the detection assembly and the processing circuit are configured to undergo a relative movement driven by the first drive mechanism, causing the detection element of the detection assembly to maintain electrically connected to or be disconnected from the processing circuit; the detection assembly is configured to drive the first movable bracket to move under drive of the first drive mechanism, causing the detection assembly to be capable of being docked with and of being separated from the reagent kit.

180. The medical detection device according to claim 179, wherein the first movable bracket defines a storage groove for accommodating the detection assembly, the detection assembly is capable of being moved into and of being moved out of the storage groove, and the second movable bracket is elastically connected to the first movable bracket; in condition of the detection assembly being moved into the storage groove, the processing circuit is capable of moving towards the detection assembly and being electrically connected under the drive of the first drive mechanism.

181. The medical detection device according to claim 180, wherein the first movable bracket is elastically connected to the mounting bracket; under the drive of the first drive mechanism, the detection assembly is movable towards the reagent kit to generate an elastic force and achieve communication between a first port of the detection assembly and a second port of the reagent kit; the detection assembly is movable away from the reagent kit by means of an elastic force to achieve separation between the first port and the second port.

182. The medical detection device according to claim 179, wherein the mounting bracket comprises a second drive mechanism and a liquid path selector; the second drive mechanism is arranged on the second movable bracket and configured to drive the liquid path selector to move; in condition of the detection assembly being docked with the reagent kit, the liquid path selector is driven by the second drive mechanism to enable the detection assembly to realize different liquid path circulations.

183. A medical detection device, comprising:
a detection assembly, comprising a detection element, a first liquid path, and a first port, wherein the first liquid path is in communication with the first port, and the detection element is configured to detect liquid in the first liquid path to obtain a detection signal;
a reagent kit, comprising a cavity, a second liquid path, and a second port, wherein the cavity is configured to hold a liquid pack; an end of the second liquid path is configured to be in communication with the liquid pack, and another end of the second liquid path is in communication with the second port; and
a processing circuit, detachably connected to the detection element of the detection assembly for receiving the detection signal in condition of being connected to the detection element, wherein in condition of the processing circuit and the detection element being disassembled, the detection assembly is capable of being disassembled synchronously with the reagent kit;
wherein the detection assembly is detachably arranged on the reagent kit, and the first port and the second port are caused to be in communication or separated; in condition of the first port and the second port being in communication, the first liquid path is in communication with the second liquid path, and reagent in the liquid pack is capable of reaching the first liquid path.

184. The medical detection device according to claim 183, wherein the medical detection device further comprises a mounting bracket, and the mounting bracket comprises a first mounting position for positioning the reagent kit; the reagent kit comprises a second mounting position for mounting the detection assembly;
wherein the mounting bracket is arranged with an avoidance channel that avoids the second mounting position, and the detection element of the detection assembly is capable of protruding from the avoidance channel and being connected to the processing circuit.

185. The medical detection device according to claim 184, wherein the mounting bracket further comprises a fixed bracket, and a movable bracket and a drive mechanism that are arranged on the fixed bracket; the movable bracket is configured to place the processing circuit, and the detection assembly and the processing circuit are configured to undergo a relative movement under drive of the drive mechanism, causing the detection element of the detection assembly to come into contact or break contact with the processing circuit; in condition of the detection element breaking contact with the processing circuit, the detection assembly is capable of being disassembled synchronously with the reagent kit.

186. The medical detection device according to claim 184, wherein a region in which the second port of the reagent kit located forms a concave portion, the shape of the concave portion being adapted to the shape of the detection assembly.

187. The medical detection device according to claim 186, wherein the depth of the concave portion is greater than or equal to the thickness of the detection assembly.

188. The medical detection device according to claim 186, wherein a side of the concave portion defines an avoidance notch, and the avoidance notch is configured to remove the detection assembly disposed on the concave portion.

189. The medical detection device according to claim 186, wherein a peripheral portion of the concave portion defines a positioning groove in communication with the concave portion, and the positioning groove is configured to guide the processing circuit into contact with the detection element of the detection assembly.

190. The medical detection device according to claim 184, wherein a region in which the second port of the reagent kit is located in formed with a first positioning portion, and the first positioning portion is configured to guide the detection assembly to be mounted on the second mounting position, thereby guiding the first port and the second port to dock with each other.

191. The medical detection device according to claim 183, wherein the reagent kit is arranged with a sampling element, a sample inlet of the sampling element being configured to selectively collect reagent from the liquid pack or liquid outside the reagent kit, and a sample outlet of the sampling element being configured to be in communication with the second port; in condition of the reagent from the liquid pack being required to be collected, the sampling element forms a part of the second liquid path.

192. The medical detection device according to claim 191, wherein the reagent kit further comprises a first connecting pipe, an end of the first connecting pipe being in communication with the liquid pack and another end of the first connecting pipe being in communication with the sample inlet of the sampling element; the first connecting pipe forms another part of the second liquid path.

193. The medical detection device according to claim 183, wherein the detection assembly comprises a third port spaced apart from the first port, and the third port is in communication with the first liquid path; the reagent kit further comprises a fourth port and a second connecting pipe, and the fourth port is spaced apart from the second port; an end of the second connecting pipe is in communication with the fourth port, and another end of the second connecting pipe is in communication with the; in condition of the first port and the second port being in communication, the third port and the fourth port are in communication.

194. A detection assembly, comprising:
a housing, a detection element and a first liquid path that are disposed in the housing, and a first port and a connection terminal that are disposed on the housing, wherein the connection terminal is electrically connected to the detection element;
wherein the first liquid path is in communication with the first port, and the detection element is configured to perform a detection on liquid in the first liquid path to obtain a detection signal; the first port is capable of being docked with and of being separated from a second port of a reagent kit for obtaining reagent of the reagent kit through the first port in condition of the first port being docked with the second port; the detection assembly is configured to be arranged on the reagent kit, and the connection terminal is configured to be electrically connected to a processing circuit of a medical detection device.

195. The detection assembly according to claim 194, wherein a region in which the second port of the reagent kit is located in formed with a first positioning portion, and the housing is arranged with a second positioning portion; the first positioning portion and the second positioning portion are configured to cooperate to guide the detection assembly to be mounted on the reagent kit.

196. The detection assembly according to claim 194, wherein a region in which the second port of the reagent kit is located defines a positioning groove, and the positioning groove is configured to guide the processing circuit into contact with the detection element of the detection assembly.

197. The detection assembly according to claim 194, wherein an end of the first port that is docked with the second port comprises a tapered recess or tapered protrusion to guide the second port to dock with the first port.

198. A reagent kit, comprising:
a cavity, a second liquid path, and a second port;
wherein the cavity is configured to hold a liquid pack; an end of the second liquid path is configured to be in communication with the liquid pack, and another end of the second liquid path is in communication with the second port; the second port is configured to be docked with a first port of a detection assembly, and reagent in the liquid pack is capable of being delivered to the detection assembly through the second port in condition of the first port and the second port being docked; the detection assembly is detachably arranged on the reagent kit, and in condition of the reagent kit being disassembled, the detection assembly is capable of being disassembled simultaneously with the reagent kit.

199. The reagent kit according to claim 198, wherein a region in which the second port of the reagent kit is located forms a concave portion, the shape of the concave portion being adapted to the shape of the detection assembly.

200. The reagent kit according to claim 199, wherein the concave portion is arranged with a first positioning portion for positioning the detection assembly.

201. The reagent kit according to claim 198, wherein the reagent kit is arranged with a sampling element, a sample inlet of the sampling element being configured to selectively collect reagent from the liquid pack or liquid outside the reagent kit, and a sample outlet of the sampling element being configured to be in communication with the second port; in condition of the reagent from the liquid pack being required to be collected, the sampling element forms a part of the second liquid path.

202. The reagent kit according to claim 198, wherein the detection assembly comprises a third port spaced apart from the first port, and the reagent kit further comprises a fourth port spaced apart from the second port; in condition of the first port and the second port being in communication, the third port and the fourth port are in communication.

203. A medical detection device, comprising:
a mounting bracket, comprising a first mounting position for detachably mounting a reagent kit, wherein the reagent kit is arranged with a second mounting position for detachably mounting a detection assembly;
wherein the detection assembly and the reagent kit are capable of being docked and of being separated, and the detection assembly and the reagent kit are caused to be in communication or separated; in condition of the detection assembly and the reagent kit being in communication, reagent in the reagent kit is capable of reaching the detection assembly; in condition of the reagent kit being disassembled, the detection assembly is capable of being disassembled synchronously with the reagent kit.

204. The medical detection device according to claim 203, wherein the medical detection device further comprises a processing circuit; the mounting bracket is arranged with an avoidance passage that avoids the second mounting position, and a detection element of the detection assembly is capable of protruding from the avoidance passage and thereby achieving an electrical connection to the processing circuit.

205. The medical detection device according to claim 204, wherein the mounting bracket further comprises a fixed bracket, and a movable bracket and a drive mechanism that are arranged on the fixed bracket; the movable bracket is configured to place the processing circuit, and the detection assembly and the processing circuit are configured to undergo a relative movement under drive of the drive mechanism, causing the detection element of the detection assembly to maintain electrically connected to or be electrically disconnected from the processing circuit; in condition of the detection element being electrically disconnected from the processing circuit, the detection assembly is capable of being disassembled synchronously with the reagent kit.

206. A biological tissue detection apparatus, comprising:
a bracket, comprising a storage space and a first mounting position, wherein the first mounting position is configured to mount a sample measuring device, and a reagent kit is capable of being moved into and of being moved out of the storage space; the reagent kit is configured to convey liquid to the sample measuring device to assist the sample measuring device in sample measurement, and the sample measuring device is configured to measure the liquid flowing into the sample measuring device;
wherein the dimension of the receiving space in a moving direction of the reagent kit is larger than an intended placement dimension of the reagent kit, and the bracket is arranged with a limit portion and an elastic portion; the limit portion is configured to position the reagent kit, and the elastic member is configured to provide an elastic force in condition of the reagent kit being moved into the storage space; and the reagent kit is capable of retracting part of a moving travel and being restrained by the limit portion after being moved into the storage space.

207. The biological tissue detection apparatus according to claim 206, wherein the bracket comprises a plurality of plates that are interconnected to form the storage space and the first mounting position;
an avoidance window is arranged between the storage space and the first mounting position, and the avoidance window is configured to avoid mechanical docking operations and a liquid delivery path between the sample measuring device and the reagent kit, the liquid delivery path being a path connecting a first pipe of the sample measuring device and a second pipe of the reagent kit; the first pipe and the second pipe are delivery pipes for the liquid.

208. The biological tissue detection apparatus according to claim 207, wherein the plurality of plates comprise a frame plate connected end to end and a mounting plate disposed on a side of the frame plate, the frame plate and the mounting plate are configured to cooperate to enclose for defining the storage space, and the first mounting position and the avoidance window are arranged on the frame plate; a side of the frame plate away from the mounting plate defines a pick-up and placement opening for the reagent kit to move in or out.

209. The biological tissue detection apparatus according to claim 208, wherein the frame plate is arranged with at least one guide member, the at least one guide member being configured to guide the reagent kit to move into or out of the storage space.

210. The biological tissue detection apparatus according to claim 208, wherein the elastic member is arranged on the mounting plate or the frame plate, and in condition of the reagent kit being moved into the storage space, the elastic member is compressed or stretched to generate an elastic force in a direction of the reagent kit moving out of the storage space.

211. The biological tissue detection apparatus according to claim 210, wherein the limit portion is arranged on the frame plate and is arranged adjacent to the pick-up and placement opening in the direction of the reagent kit moving out of the storage space; the reagent kit is arranged with an engagement portion corresponding to the limit portion, and the engagement portion is configured to cooperate with the limit portion to position the reagent kit in condition of the reagent kit retracting part of the moving travel after being moved into the storage space.

212. The biological tissue detection apparatus according to claim 208, wherein the mounting plate comprises a first docking port and a second mounting position; the first docking port is in communication with the storage space, and the second mounting position is configured to mount a regulating device; in condition of the reagent kit being moved into the storage space, a sampling element on the reagent kit is exposed via the first docking port to dock with the regulating device, and the sampling element is capable of obtaining liquid inside or outside the reagent kit and causing the liquid to enter the second pipe, under regulation of the regulating device.

213. The biological tissue detection apparatus according to claim 212, wherein the mounting plate further comprises a second docking port and a third mounting position; the second docking port is in communication with the storage space, and the third mounting position is configured to mount a valve apparatus; in condition of the reagent kit being moved into the storage space, a liquid pack connector within the reagent kit is exposed via the second docking port to dock with the valve apparatus, and the valve apparatus is capable of controlling the liquid in the reagent kit to flow into the second pipe; in condition of the sampling element being docked with the reagent kit, the sampling element is in communication with a liquid outlet of the valve apparatus, and the sampling element is capable of obtaining the liquid in the reagent kit and causing the liquid to enter the second pipe.

214. The biological tissue detection apparatus according to claim 212, wherein the mounting plate further comprises a third docking port and a fourth mounting position; the third docking port is in communication with the storage space, and the fourth mounting position is configured to mount a drive apparatus; in condition of the reagent kit being moved into the storage space, the drive apparatus is docked with the reagent kit via the third docking port to drive the liquid in the second pipe to flow.

215. The biological tissue detection apparatus according to claim 208, wherein the avoidance window extends through the first mounting position, and the first mounting position comprises a positioning member for positioning the sample measuring device on the frame plate.

216. A biological tissue detection apparatus, comprising:
a bracket, comprising a storage space and a first mounting position;
a sample measuring device, detachably arranged on the first mounting position and comprising a first pipe; and
a reagent kit, capable of being moved into and of being moved out the storage space, wherein the reagent kit comprises a second pipe;
wherein a dimension of the storage space in a moving direction of the reagent kit is larger than an intended placement dimension of the reagent kit; the bracket is arranged with a limit portion and an elastic member, the limit portion being configured to position the reagent kit, and the elastic member being configured to provide an elastic force for the reagent kit to move into the storage space, causing the reagent kit to retract a part of a movement travel and be restrained by the limit portion after being moved into the storage space.

217. The biological tissue detection apparatus according to claim 216, wherein an avoidance window is arranged between the storage space and the first mounting position, and the avoidance window is configured to avoid mechanical docking operations and a liquid delivery path between the sample measuring device and the reagent kit, the liquid delivery path being a path connecting a first pipe of the sample measuring device and a second pipe of the reagent kit.

218. The biological tissue detection apparatus according to claim 216, wherein the plurality of plates comprise a frame plate connected end to end and a mounting plate disposed on a side of the frame plate, the frame plate and the mounting plate are configured to cooperate to enclose for defining the storage space, and the first mounting position and the avoidance window are arranged on the frame plate; a side of the frame plate away from the mounting plate defines a pick-up and placement opening for the reagent kit to move in or out.

219. The biological tissue detection apparatus according to claim 218, wherein the reagent kit is arranged with a sampling element, the biological tissue detection apparatus comprises an regulating device, and the regulating device is configured to adjust a sampling posture of the sampling element; the mounting plate comprises a first docking port and a second mounting position, the first docking port is in communication with the storage space, and the second mounting position is configured to mount the regulating device; in condition of the reagent kit being moved into the storage space, the sampling element is docked with the regulating device, and the sampling element is capable of obtaining liquid inside or outside the reagent kit and causing the liquid to enter the second pipe, under regulation of the regulating device.

220. The biological tissue detection apparatus according to claim 218, wherein the reagent kit comprises a receiving cavity for accommodating a liquid pack, and a cavity wall of the receiving cavity is arranged with a liquid pack connector; the biological tissue detection apparatus comprises a valve apparatus for docking with the liquid pack connector to control an outflow of liquid from the liquid pack; the mounting plate comprises a second docking port and a third mounting position; the second docking port is in communication with the storage space, and the third mounting position is configured to mount the valve apparatus; in condition of the reagent kit being moved into the storage space, the liquid pack connector is docked with the valve apparatus, and the valve apparatus is capable of controlling the liquid in the reagent kit to flow into the second pipe; in condition of the sampling element being docked with the reagent kit, the sampling element is in communication with a liquid outlet of the valve apparatus, and the sampling element is capable of obtaining the liquid in the reagent kit and causing the liquid to enter the second pipe.

221. The biological tissue detection apparatus according to claim 218, wherein the reagent kit comprises a receiving cavity for accommodating a liquid pack, and a cavity wall of the receiving cavity is arranged with a first drive member; the biological tissue detection apparatus comprises a drive apparatus configured to be docked with the first drive member to drive an outflow of liquid from the liquid pack; the mounting plate comprises a third docking port and a fourth mounting position; the third docking port is in communication with the storage space, and the fourth mounting position is configured to mount the drive apparatus; in condition of the reagent kit being moved into the storage space, the drive apparatus is docked with the first drive member.

222. A sample analysis apparatus, comprising:
a seat body, defining a storage groove and a pick-up and placement opening in communication with the storage groove, wherein a detection card is capable of being moved into and of being moved out of the storage groove from the pick-up and placement opening; at least one of the storage groove and detection card is arranged with a locking member, the locking member being configured to lock the detection card in the seat body in condition of the detection card being moved into the storage groove; at least one of the seat body and the detection card is arranged with an elastic member;
wherein in condition of the detection card being moved into the storage groove, the detection card squeezes or stretches the elastic member, causing the elastic member to deform and generate an elastic force; in condition of the locking member releasing locking and fixing of the detection card, the detection card pops up from the pick-up and placement opening under an action of the elastic force.

223. The sample analysis apparatus according to claim 222, wherein an inner wall of the pick-up and placement opening is arranged with a damping portion, and the damping portion is configured to limit a pop-up travel of the detection card.

224. The sample analysis apparatus according to claim 222, wherein the seat body is arranged with a drive member, and the drive member is configured to drive the locking member to release the locking and fixing of the detection card.

225. The sample analysis apparatus according to claim 224, wherein the seat body defines a receiving groove, the receiving groove and the storage groove are arranged at intervals in a moving direction of the detection card, and the drive member is arranged in the receiving groove; an end of the locking member is assembled and connected to the drive member, and another end of the locking member is disposed in the storage groove to lock and fix the detection card in the storage groove.

226. The sample analysis apparatus according to claim 225, wherein the seat body defines an avoidance space in communication with the storage groove and the receiving groove, and the locking member is at least partially disposed in the avoidance space; the locking member is rotatably connected to the seat body to rotate under drive of the drive member and thereby release the locking and fixing of the detection card.

227. The sample analysis apparatus according to claim 225, wherein the elastic member is arranged in the receiving groove, and the seat body is arranged with a supporting member; an end of the supporting member is assembled and connected to the elastic member, and another end of the supporting member is configured to support the detection card; in condition of the detection card being moved into the storage groove, the detection card abuts against the supporting member, causing the elastic member to deform and generate the elastic force.

228. The sample analysis apparatus according to claim 227, wherein the receiving groove is arranged with a limit portion, and the limit portion is configured to limit a movement travel of the supporting member.

229. The sample analysis apparatus according to claim 228, wherein the elastic member is sleeved on the supporting member, and in condition of the detection card being moved into the storage groove, the detection card abuts against the supporting member, causing the elastic member to abut against the limit portion and generate the elastic force.

230. The sample analysis apparatus according to claim 227, wherein a side of the detection card is arranged with a first port, a second port, and a snap-fit portion, and the storage groove comprises an avoidance opening; in condition of the detection card being moved into the storage groove, the first port and the second port are exposed from the avoidance opening for communication to an external liquid path, and the snap-fit portion is locked and fixed with the locking member.

231. The sample analysis apparatus according to claim 227, wherein the storage groove is arranged with at least one elastic tab in a region adjacent to the pick-up and placement opening, and the at least one elastic tab is configured to position the detection card in condition of the detection card being moved into the storage groove.

232. A fluid detection instrument, comprising:
a valve assembly, comprising a control element, a first pipe, a first inlet, and a first outlet, wherein the control element is configured to control the first inlet to be in communication with an end of the first pipe, and another end of the first pipe is in communication with the first outlet; and
a reagent kit, comprising a cavity, a second pipe, and a first port, wherein the cavity is configured to hold a reagent pack and a recovery pack, the first port is configured to connect the reagent pack to the first inlet, and the first outlet is in communication with a docking groove defined on the reagent kit; the docking groove is configured to be docked with a sampling element, an end of the second pipe being in communication with the docking groove, and another end of the second pipe being in communication with the recovery pack;
wherein the valve assembly and the reagent kit are capable of being docked and of being separated, and the first inlet and the first port are caused to be in communication or separated; in condition of the first inlet and the first port being in communication, the first pipe is configured to be in communication with the docking groove under control of the control element, and liquid in the reagent pack is capable of reaching the docking groove, and liquid in the docking groove is capable of reaching the recovery pack through the second pipe.

233. The fluid detection instrument according to claim 232, wherein the docking groove is arranged with a liquid inlet hole and a liquid outlet hole; the liquid inlet hole is configured to be in communication with the first outlet, and the liquid outlet hole is configured to be in communication with the second pipe.

234. The fluid detection instrument according to claim 233, wherein the docking groove is arranged with an isolation portion disposed between the liquid inlet hole and the liquid outlet hole, and a flow channel in communication with the liquid inlet hole and the liquid outlet hole and bypassing the isolation portion.

235. The fluid detection instrument according to claim 234, wherein the sampling element comprises a sampling needle, a sleeve sleeved on the sampling needle, and a docking member arranged on an end of the sleeve; an outer diameter of the sleeve is less than or equal to an inner diameter of the docking groove, and the docking member defines a through hole; the docking member is movable along an axial direction of the sampling needle following the sleeve, causing the sampling needle to be inserted into an end of the through hole, or pass through the through hole and be exposed on a side of the docking member.

236. The fluid detection instrument according to claim 233, wherein the valve assembly further comprises a third pipe, a third inlet, and a third outlet; the control element is configured to control the third inlet to be in communication with an end of the third pipe, and another end of the third pipe is in communication with the third outlet;
the reagent kit further comprises a fourth pipe and a second port, the second port being configured to connect the third inlet to the reagent pack, and the third outlet being configured to be in communication with a sample inlet of the sampling element; an end of the fourth pipe is configured to be in communication with a sample outlet of the sampling element, and another end of the fourth pipe is configured to be in communication with a detection assembly of the fluid detection instrument.

237. The fluid detection instrument according to claim 236, wherein the docking groove is arranged with a through hole and a connecting pipe passing through the through hole; an end of the connecting pipe is in communication with the third outlet, and another end of the connecting pipe is configured to be in communication with the sample inlet of the sampling element; the connecting pipe is in sealed contact with a hole wall of the through hole; in condition of the sleeve moving along the axial direction of the sampling needle and towards the connecting pipe such that the docking member is inserted into the docking groove, the sampling needle and the connecting pipe are respectively inserted into both ends of the through hole to achieve communication.

238. The fluid detection instrument according to claim 236, wherein the valve assembly defines a first air hole and a second air hole; an end of the first pipe is selectively connected to the first inlet or the first air hole under control of the control element; an end of the third pipe is selectively connected to the third inlet or the second air hole under the control of the control element.

239. A valve assembly, comprising:
a control element, a first pipe, a first inlet, and a first outlet;
wherein the control element is configured to control the first inlet to be in communication with an end of the first pipe, and another end of the first pipe is in communication with the first outlet; the first inlet is capable of being docked with and of being separated from a first port of a reagent kit; in condition of the first port being docked with the first inlet, fluid in the reagent kit is capable of being obtained by controlling the first inlet through the control element; the first outlet is configured to be in communication with a docking groove of the reagent kit; in condition of the first outlet being docked with the docking groove, the liquid in the reagent kit is capable of passing through the first outlet to reach the docking groove, and liquid in the docking groove is capable of passing through a second pipe of the reagent kit to reach a recovery pack of the reagent kit.

240. The valve assembly according to claim 239, wherein the valve assembly further comprises a third pipe, a third inlet, and a third outlet; the control element is configured to control the third inlet to be in communication with an end of the third pipe, and another end of the third pipe is in communication with the third outlet;
the third inlet is capable of being docked with and of being separated from a second port of the reagent kit, and the third outlet is configured to be in communication with a sample inlet of a sampling element; in condition of the third outlet being docked with the sample inlet, the liquid in the reagent kit is capable of passing through the third outlet to reach the sampling element.

241. The valve assembly according to claim 240, wherein the valve assembly defines a first air hole and a second air hole; an end of the first pipe is selectively connected to the first inlet or the first air hole under control of the control element; an end of the third pipe is selectively connected to the third inlet or the second air hole under the control of the control element.

242. A reagent kit, comprising:
a cavity, a second pipe, a first port, and a docking groove;
wherein the cavity is configured to hold a reagent pack and a recovery pack; an end of the second pipe is in communication with the docking groove, and another end of the second pipe is in communication with the recovery pack; the first port is capable of being docked with and of being separated from a first inlet of a valve assembly, and the docking groove is configured to be in communication with a first outlet of the valve assembly; in condition of the first port being docked with the first inlet, liquid in the reagent pack is capable of being delivered to the valve assembly through the first port, and liquid in the docking groove is capable of reaching the recovery pack through the second pipe.

243. The reagent kit according to claim 242, wherein the docking groove is arranged with a liquid inlet hole and a liquid outlet hole; the liquid inlet hole is configured to be in communication with the first outlet, and the liquid outlet hole is configured to be in communication with the second pipe.

244. The reagent kit according to claim 243, wherein the docking groove is arranged with an isolation portion disposed between the liquid inlet hole and the liquid outlet hole, and a flow channel in communication with the liquid inlet hole and the liquid outlet hole and bypassing the isolation portion.

245. The reagent kit according to claim 243, wherein the reagent kit further comprises a fourth pipe and a second port, the second port being configured to connect a third inlet of the valve assembly to the reagent kit, and the third outlet being configured to be in communication with a sample inlet of a sampling element; an end of the fourth pipe is configured to be in communication with a sample outlet of the sampling element, and another end of the fourth pipe is configured to be in communication with a detection assembly of a fluid detection instrument.

246. The fluid detection instrument according to claim 245, wherein the docking groove is arranged with a through hole and a connecting pipe passing through the through hole; an end of the connecting pipe is in communication with the third outlet, and another end of the connecting pipe is configured to be in communication with the sample inlet of the sampling element; the connecting pipe is in sealed contact with a hole wall of the through hole.

247. The fluid detection instrument according to claim 246, wherein the sampling element comprises a sampling needle, a sleeve sleeved on the sampling needle, and a docking member arranged on an end of the sleeve; an outer diameter of the sleeve is less than or equal to an inner diameter of the docking groove, and the docking member defines another through hole; the docking member is movable along an axial direction of the sampling needle following the sleeve, causing the sampling needle to be inserted into an end of the through hole on the docking member, or pass through the through hole on the docking member and be exposed on a side of the docking member;
in condition of the sleeve moving along the axial direction of the sampling needle and towards the connecting pipe such that the docking member is inserted into the docking groove, the sampling needle and the connecting pipe are respectively inserted into both ends of the through hole to achieve communication.

248. A liquid pack, comprising a body and a connector, wherein the body is configured to contain liquid, and the connector is configured to control the liquid to flow from the liquid pack through the connector or to seal the body;
wherein the connector comprises a pipe in communication with an internal space of the body, and a seal disposed in the pipe; the seal is movable in the pipe under an external force to open the pipe, and return to an initial position to seal the pipe in a case where the external force is removed.

249. The liquid pack according to claim 248, wherein a first abutting portion is arranged in the pipe, and the seal is arranged with a second abutting portion; in condition of the seal being in the initial position, the first abutting portion abuts against the second abutting portion to seal the pipe; the seal is configured to move within the pipe under the external force to separate the first abutting portion and the second abutting portion and open the pipe.

250. The liquid pack according to claim 249, wherein the pipe comprises a first pipe and a second pipe that are in communication; an end of the first pipe is in communication with the body, and another end of the first pipe is in communication with the second pipe, an inner diameter of the first pipe being greater than an inner diameter of the second pipe to form the first abutting portion, that is stepped in shape, at a connection between the first pipe and the second pipe; a gap exists between the seal and the first pipe, and the second abutting portion is configured to seal an end of the second pipe connected to the first pipe.

251. The liquid pack according to claim 250, wherein an outer diameter of the seal is less than the inner diameter of the first pipe, and an outer diameter of the seal is greater than the inner diameter of the second pipe; the seal in the initial position is disposed in the first pipe, and the second abutting portion is formed by an end of the seal near the second pipe to abut against the first abutting portion.

252. The liquid pack according to claim 250, wherein the seal comprises a sealing portion and a guiding portion; an outer diameter of the sealing portion is greater than an outer diameter of the guiding portion, the outer diameter of the sealing portion is less than the inner diameter of first pipe, and the outer diameter of the sealing portion is greater than the inner diameter of the second pipe; in condition of the seal being in the initial position, the sealing portion is disposed in the first pipe, and an end of the seal close to the second pipe forms the second abutting portion to abut against the first abutting portion, and the guiding portion is disposed in the second pipe and has a gap with the second pipe; the guiding portion is configured to receive the external force.

253. The liquid pack according to claim 252, wherein an end of the first pipe away from the second pipe comprises a limit portion, and the limit portion is configured to limit entry of the sealing portion into the body.

254. The liquid pack according to claim 252, wherein the connector comprises a docking port, the docking port being arranged on an end of the second pipe away from the first pipe and communicating with the second pipe; the docking port is configured to guide an external connector into the pipe to exert a force on the seal.

255. The liquid pack according to claim 254, wherein a sealing ring, which is annular in shape, is arranged in the docking port; an end of the guiding portion is at least partially exposed from the liquid pack from an annular hollow portion of the sealing ring; the external connector is capable of entering the pipe from the annular hollow portion of the sealing ring to exert the force on the seal; in condition of the external connector abutting against the guiding portion and pushing the guiding portion to move towards the first pipe, the sealing ring seals a gap between the external connector and the docking port.

256. The liquid pack according to claim 255, wherein the sealing ring comprises a first sealing ring and a second sealing ring, the first sealing ring being arranged between the guiding portion and the second sealing ring; the external connector is configured to exert a force on the guiding portion through the first sealing ring and the second sealing ring.

257. The liquid pack according to claim 256, wherein the first sealing ring and the second sealing ring are arranged coaxially; an outer diameter of the first sealing ring is less than an outer diameter of the second sealing ring.

258. The liquid pack according to claim 257, wherein the first sealing ring and the second sealing ring are both elastomers, and the external connector is configured to enter annular hollow portions of the first sealing ring and the second sealing ring to exert the force on the seal;
the external connector is sealed and assembled with at least one of the first sealing ring and the second sealing ring when exerting the force on the seal.

259. The liquid pack according to claim 257, wherein an end of the second sealing ring away from the first sealing ring comprises a guide ramp, and the guide ramp is configured to guide the external connector into the seal.

260. An integrated reagent kit, comprising:
a case, and a sampling assembly disposed on the case;
wherein the sampling assembly comprises a rotary member rotatably connected to the case and a sampling member connected to the rotary member;
the rotary member is capable of changing to a state under an action of a first external force to drive the sampling member to rotate synchronously relative to the case, and of returning to an original state in a case where the first external force is removed;
wherein the sampling member comprises a sampling needle and a sleeve sleeved on the sampling needle; the sleeve is capable of changing to a state under an action of a second external force to move relative to the rotary member to expose the sampling needle, and of returning to an original state to cover the sampling needle in a case where the second external force is removed.

261. The integrated reagent kit according to claim 260, wherein the rotary member comprises a first rotary member and a second rotary member connected to each other, the first rotary member being movably connected to the sleeve in an axial direction of the sleeve, the second rotary member being rotatably connected to the case, and the first rotary member being configured to take the first external force.

262. The integrated reagent kit according to claim 261, wherein the integrated reagent kit further comprises a first elastic member connecting the first rotary member and the sleeve; the first elastic member changes a state to generate an elastic force in condition of the sleeve being moved under force, and in a case where the force is removed from the sleeve, the first elastic member restores the state under an action of an elastic force to restore the sleeve to the original state.

263. The integrated reagent kit according to claim 262, wherein the sampling needle comprises a sampling portion and a sample discharge portion connected by a bend; the sleeve is arranged on the sampling portion and movable along an axial direction of the sampling portion; the sample discharge portion extends from an end of the sampling portion to the second rotary member or extends and passes through the second rotary member; the sleeve defines an avoidance slot to avoid the sample discharge portion in condition of the sleeve being moved axially.

264. The integrated reagent kit according to claim 263, wherein an end of the sleeve adjacent to a sample inlet of the sampling portion comprises a bulge; the bulge is pushed to move the sleeve under the action of the second external force exerted by an external container, in condition of the sampling portion obtaining liquid from the external container.

265. The integrated reagent kit according to claim 262, wherein the sleeve passes through the first rotary portion, and an end of the sleeve is connected to the first rotary portion through the first elastic member.

266. The integrated reagent kit according to claim 261, wherein the integrated reagent kit further comprises a second elastic member arranged between the second rotary member and the case, and the rotary member changes a state of the second elastic member to generate an elastic force in condition of the rotary member rotating relative to the case under the action of the first external force; in a case where the first external force is removed, the second elastic member restores the state of the rotary member under an action of the elastic force.

267. The integrated reagent kit according to claim 266, wherein the case defines a rotating groove, the second rotary member is inserted in the rotating groove, the second elastic member is sleeved on the second rotary member, and an end of the second elastic member abuts against the rotating groove; the sampling needle is capable of being in communication with a pipe on the case through the rotating groove.

268. The integrated reagent kit according to claim 267, wherein the case is arranged with an assembly portion, the assembly portion being arranged in a corner region of the case; the rotating groove is defined on the assembly portion, and the second rotary member is inserted into the rotating groove; in condition of the sampling assembly being rotated, a sample inlet of the sampling assembly is capable of being unscrewed out of the corner region of the case.

269. A reagent kit that is easy to clean, comprising:
a case, a first pipe, a second pipe, and a docking groove, wherein a reagent pack and a recovery pack are arranged in the case; an end of the first pipe is in communication with the docking groove, and another end of the first pipe is in communication with the reagent pack; an end of the second pipe is in communication with the docking groove, and another end of the second pipe is in communication with the recovery pack;
wherein the docking groove is capable of being docked with and of being separated from a sampling element; in condition of the docking groove being docked with the sampling element, liquid in the reagent pack is capable of reaching the docking groove through the first pipe for cleaning a sample inlet of the sampling element, and liquid after the cleaning is capable of reaching the recovery pack through the second pipe.

270. The reagent kit according to claim 269, wherein the docking groove comprises a liquid inlet in communication with the first pipe and a liquid outlet in communication with the second pipe; in condition of the docking groove being docked with the sampling element, the sample inlet of the sampling element seals the docking groove and cooperates with the docking groove to define a cavity, and the liquid inlet and the liquid outlet are respectively in communication with the cavity.

271. The reagent kit according to claim 270, wherein the docking groove comprises a first groove section and a second groove section that are in communication; the first groove section is away from a bottom of the docking groove relative to the second groove section; the first groove section is capable of being docked with and of being separated from the sampling element, and the second groove section comprises the liquid inlet and the liquid outlet; an inner diameter of the first groove section is greater than an inner diameter of the second groove section, the inner diameter of the first groove section is greater than an outer diameter of the sample inlet of the sampling element, and the inner diameter of the second groove section is less than the outer diameter of the sample inlet of the sampling element.

272. The reagent kit according to claim 270, wherein the sampling element comprises a sleeve, and a sampling needle and a docking head that are arranged in the sleeve, the docking head defining a through hole; the docking head is embedded in an end of the sleeve, and a sample inlet of the sampling needle is inserted in the through hole; in condition of the docking groove being docked with the sampling element, the sleeve abuts against the first groove section, and the docking head abuts against the second groove section, the cavity being defined between the docking head and the second groove section.

273. The reagent kit according to claim 272, wherein the second groove section comprises an open end and a narrow end that are oppose to each other, an inner diameter of the open end being greater than an inner diameter of the narrow end; the inner diameter of the open end is greater than an outer diameter of the docking head and less than an outer diameter of the sleeve.

274. The reagent kit according to claim 273, wherein the inner diameter of the narrow end is less than or equal to an inner diameter of the sleeve.

275. The reagent kit according to claim 272, wherein the reagent kit further comprises a third pipe partially disposed in the docking groove; in condition of the docking groove being docked with the sampling element, the docking head is inserted into the second groove section, and the third pipe is inserted into the docking head to communicate with the sampling needle.

276. The reagent kit according to claim 275, wherein an end of the docking head abutting against the second groove section comprises a groove, and the through hole is in communication with a bottom wall of the groove; the docking head is in sealed contact with a sidewall of the second groove section, and the groove and the second groove section enclose to define the cavity where the sample inlet is configured to be cleaned.

277. The reagent kit according to claim 276, wherein the second groove section is arranged with an isolation portion, and the liquid inlet and liquid outlet are disposed on opposite sides of the isolation portion and are respectively in communication with the cavity; the liquid in the reagent pack is capable of reaching the cavity from the liquid inlet and cleaning the sample inlet of the sampling element; the liquid after the cleaning is configured to flow out of the cavity from the liquid outlet and reach the recovery pack through the second pipe.

278. The reagent kit according to claim 269, wherein the sampling element comprises a sleeve, and a sampling needle and a docking connector that are arranged in the sleeve, the docking head defining a through hole; the docking head is embedded in an end of the sleeve, and a sample inlet of the sampling needle is inserted in the through hole; an inner wall of the sleeve comprises a step portion, and the docking head comprises an engagement portion; the engagement portion is disposed on a side of the step portion away from a bottom wall of the docking groove and abuts against the step portion, and the docking head is movable axially along with the sleeve.

279. A sample analysis device, comprising:
a reagent kit, comprising a sampling assembly, a regulating assembly, and a movable assembly, wherein the regulating assembly is configured to drive the sampling assembly to rotate relative to the reagent kit; the movable assembly is arranged facing the sampling assembly; the movable assembly is capable of being docked with and of being separated from the sampling assembly;
wherein the movable assembly is movable between a first position and a second position relative to the sampling assembly; the movable assembly is docked with the sampling assembly in the first position, and the movable assembly is separated from the sampling assembly in the second position;
the first position corresponds to that the sampling assembly is subjected to positional restriction such that the sampling assembly is capable of collecting liquid inside the reagent kit; the second position corresponds to that positional restriction on the sampling assembly is cancelled such that the regulating assembly is capable of driving the sampling assembly to rotate, and the sampling assembly is capable of collecting liquid outside the reagent kit.

280. The sample analysis device according to claim 279, wherein the reagent kit further comprises a case for holding a liquid pack, and the case is arranged with a first port and a second port; the first port is configured to be docked with a detection assembly, and the second port is configured to be docked with the liquid pack; the movable assembly comprises a third port; an end of the sampling assembly is in communication with the first port through a first liquid path, and another end of the sampling assembly is capable of being docked with and of being separated from the third port, the second port being in communication with the third port through a second liquid path.

281. The sample analysis device according to claim 280, wherein the regulating assembly comprises a seat body and a shaft portion disposed on the seat body; the sampling assembly comprises a sampling element; an end of the sampling element is at least partially disposed in the seat body and is in communication with the first port through the first liquid path, and another end of the sampling element is disposed outside the seat body for selectively docking with or separating from the third port; the shaft portion is docked with the case and is rotatable relative to the case.

282. The sample analysis device according to claim 281, wherein the sampling assembly further comprises a sleeve sleeved on the sampling element; the sleeve is movable along an axial direction of the sampling element; the sleeve defines an avoidance slot to avoid the first liquid path or the sampling element in condition of the sleeve being moved.

283. The sample analysis device according to claim 282, wherein the sleeve comprises a bulge on an end adjacent to the movable assembly, and the bulge is pushed to move the sleeve under an action of a force exerted by an external container in condition of the sampling element obtaining liquid from the external container.

284. The sample analysis device according to claim 283, wherein the sampling assembly further comprises a first elastic member assembled between the sleeve and the seat body; the first elastic member changes a state to generate an elastic force in condition of the sleeve being moved under the action of the force; in a case where the force on the sleeve is removed, the first elastic member restores the state under an action of the elastic force, causing the sleeve to reset and cover a sample inlet of the sampling element.

285. The sample analysis device according to claim 283, wherein the sampling assembly further comprises a second elastic member assembled between the regulating assembly and the case, and the regulating assembly changes a state of the second elastic member to generate an elastic force in condition of rotating relative to the case under an action of a force; in condition of the force on the regulating assembly being removed, the second elastic member returns to an original state under an action of the elastic force to reset the regulating assembly.

286. The sample analysis device according to claim 281, wherein the seat body is arranged with a docking portion configured to be docked with a first drive component of the sample analysis device for transmission fit; the movable assembly is capable of being docked with and of being separated from the sampling element under drive of a second drive component of the sample analysis device, in condition of the movable assembly being docked with the second drive component.

287. The sample analysis device according to claim 286, wherein the sample analysis device further comprises a mounting bracket; the mounting bracket comprises a cavity, a first mounting position, and a second mounting position; the reagent kit is capable of being moved into and of being moved out of the cavity, the first mounting position is configured to mount the first drive component, and the second mounting position is configured to mount the second drive component;
wherein in condition of the reagent kit being moved into the cavity, the docking portion is docked with the first drive component and the movable assembly is docked with the second drive component.

288. The sample analysis device according to claim 287, wherein the first drive component comprises a first power member arranged on the first mounting position, and the docking portion of the seat body defines a shaft hole; in condition of the docking portion and the first drive component being docked, an output shaft of the first drive component cooperates with the shaft hole to achieve a transmission fit between the docking portion and the first drive component.

289. The sample analysis device according to claim 287, wherein the movable assembly comprises a docking seat that is movable relative to the case; an end of the docking seat forms the third port, and another end of the docking seat is configured to be docked with the second drive component; in condition of the reagent kit being moved into the cavity, the docking base is docked with the second drive component to move under drive of the second drive component.

290. The sample analysis device according to claim 289, wherein the second drive component comprises a second power member arranged on the second mounting position, and a clamping member cooperating with an output shaft of the second power member, and the second power member is configured to drive the clamping member to move;
wherein in condition of the reagent kit being moved into the cavity, the clamping member is clamped and connected with the docking base, causing the second power member to synchronously drive the docking base to move while driving the clamping member to move.

291. A biological sample analysis device, comprising:
a drive assembly, comprising an output shaft; and
a reagent storage apparatus, comprising a receiving cavity, a first connecting pipe, a second connecting pipe, and a squeezing assembly, wherein the receiving cavity is configured to hold a reagent pack, a cleaning pack, and a recovery pack; the first connecting pipe and the second connecting pipe are partially embedded in the squeezing assembly; an end of the first connecting pipe is configured to be in communication with the reagent pack, and another end of the first connecting pipe is configured to be in communication with the recovery pack; an end of the second connecting pipe is configured to be in communication with the cleaning pack, and another end of the second connecting pipe is configured to be in communication with the recovery pack;
wherein the drive assembly and the reagent storage apparatus are capable of being docked and of being separated, and the output shaft is caused to be docked with or separated from the squeezing assembly; in condition of the output shaft being docked with the squeezing assembly, the output shaft is capable of driving the squeezing assembly to synchronously squeeze the first connecting pipe and the second connecting pipe, and liquid in the first connecting pipe or the second connecting pipe is capable of flowing.

292. The biological sample analysis device according to claim 291, wherein the biological sample analysis device further comprises a mounting bracket, the mounting bracket comprising a cavity and a first mounting position; the reagent storage apparatus is capable of being moved into and of being moved out of the cavity, and the drive assembly is arranged on the first mounting position; the first mounting position is arranged on an end of a travel of the reagent storage apparatus moving into the cavity.

293. The biological sample analysis device according to claim 292, wherein the drive assembly is arranged outside the cavity; an avoidance hole is defined between the cavity and the first mounting position, and the output shaft of the drive assembly extends from the avoidance hole into the cavity; in condition of the reagent storage apparatus being moved into the cavity, the output shaft of the drive assembly is transmission-assembled with the squeezing assembly.

294. The biological sample analysis device according to claim 292, wherein the squeezing assembly comprises a first squeezing member and a second squeezing member, the second squeezing member being embedded in the first squeezing member and arranged coaxially with the first squeezing member; the first connecting pipe and the second connecting pipe are partially arranged between the first squeezing member and the second squeezing member; the second squeezing member is configured to be transmission-assembled with the output shaft and rotate relative to the first squeezing member under drive of the output shaft and thereby squeeze the first connecting pipe and the second connecting pipe.

295. The biological sample analysis device according to claim 294, wherein the first squeezing member is annular in shape, and the second squeezing member comprises a rotary portion and a squeezing portion; the rotary portion is configured to be docked with the output shaft, and the squeezing portion is arranged on a peripheral side of the rotary portion and rotatable synchronously with the rotary portion;
wherein the squeezing portion is arranged spaced apart from the first squeezing member, and the first connecting pipe and the second connecting pipe are partially arranged between the squeezing portion and the first squeezing member.

296. The biological sample analysis device according to claim 295, wherein the rotary portion defines a shaft hole; an end of the output shaft is assembled with the shaft hole, and the first connecting pipe and the second connecting pipe are arranged along an axial direction of the shaft hole on a position between the squeezing portion and the first squeezing member.

297. The biological sample analysis device according to claim 295, wherein the squeezing portion is arranged with a bushing, the bushing is arranged with a spacer, and the spacer is disposed between the first connecting pipe and the second connecting pipe.

298. The biological sample analysis device according to claim 292, wherein a cavity wall of the receiving cavity defines a receiving groove, the squeezing assembly is embedded in the receiving groove and arranged coaxially with the receiving groove, and the first connecting pipe and the second connecting pipe are partially arranged between the receiving groove and the squeezing assembly; the squeezing assembly is configured to be transmission-assembled with the output shaft and rotate relative to the storage groove under drive of the output shaft and thereby squeeze the first connecting pipe and the second connecting pipe.

299. A reagent storage apparatus, comprising:
a receiving cavity, a first connecting pipe, a second connecting pipe, and a squeezing assembly;
wherein the receiving cavity is configured to hold a reagent pack, a cleaning pack, and a recovery pack; the first connecting pipe and the second connecting pipe are partially embedded in the squeezing assembly; an end of the first connecting pipe is configured to be in communication with the reagent pack, and another end of the first connecting pipe is configured to be in communication with the recovery pack; an end of the second connecting pipe is configured to be in communication with the cleaning pack, and another end of the second connecting pipe is configured to be in communication with the recovery pack;
the squeezing assembly is capable of being docked with and of being separated from the output shaft of the drive assembly; in condition of the squeezing assembly being docked with the output shaft, the squeezing assembly is driven by the output shaft to synchronously squeeze the first connecting pipe and the second connecting pipe, and liquid in the first connecting pipe or the second connecting pipe is capable of flowing.

300. The reagent storage apparatus according to claim 299, wherein the squeezing assembly comprises a first squeezing member and a second squeezing member, the second squeezing member being embedded in the first squeezing member and arranged coaxially with the first squeezing member; the first connecting pipe and the second connecting pipe are partially arranged between the first squeezing member and the second squeezing member; the second squeezing member is configured to be transmission-assembled with the output shaft and rotate relative to the first squeezing member under drive of the output shaft and thereby squeeze the first connecting pipe and the second connecting pipe.

301. The reagent storage apparatus according to claim 300, wherein the first squeezing member is annular in shape, and the second squeezing member comprises a rotary portion and a squeezing portion; the rotary portion is configured to be docked with the output shaft, and the squeezing portion is arranged on a peripheral side of the rotary portion and rotatable synchronously with the rotary portion;
wherein the squeezing portion is arranged spaced apart from the first squeezing member, and the first connecting pipe and the second connecting pipe are partially arranged between the squeezing portion and the first squeezing member.

302. The reagent storage apparatus according to claim 301, wherein the rotary portion defines a shaft hole; an end of the output shaft is assembled with the shaft hole, and the first connecting pipe and the second connecting pipe are arranged along an axial direction of the shaft hole on a position between the squeezing portion and the first squeezing member.

303. The reagent storage apparatus according to claim 301, wherein the squeezing portion is arranged with a bushing, the bushing is arranged with a spacer, and the spacer is disposed between the first connecting pipe and the second connecting pipe.

304. The reagent storage apparatus according to claim 299, wherein a cavity wall of the receiving cavity defines a receiving groove, the squeezing assembly is embedded in the receiving groove and arranged coaxially with the receiving groove, and the first connecting pipe and the second connecting pipe are partially arranged between the receiving groove and the squeezing assembly; the squeezing assembly is configured to be transmission-assembled with the output shaft and rotate relative to the storage groove under drive of the output shaft and thereby squeeze the first connecting pipe and the second connecting pipe.

305. A biological parameter analysis device, comprising:
a housing, arranged with a window and a door capable of covering the window;
a reagent kit, capable of being moved into and of being moved out of the housing, wherein the reagent kit comprises a sampling assembly and a docking assembly; in condition of the reagent kit being arranged within the housing, the sampling assembly is capable of being unscrewed from the window to collect liquid outside the housing; the docking assembly is capable of being docked with the sampling assembly to enable the sampling assembly to collect liquid inside the reagent kit; and
a linkage assembly, coupled to the docking assembly and the door, wherein the door covers the window in condition of the sampling assembly being docked with the docking assembly, and the door opens the window in condition of the sampling assembly being separated from the docking assembly.

306. The biological parameter analysis device according to claim 305, wherein the housing comprises a shell and a bracket arranged in the shell; the window is arranged on the shell, and the door is arranged on an inner side of the housing; the bracket comprises a cavity and a first mounting position, and the reagent kit is capable of being moved into and of being moved out of the cavity, the first mounting position being arranged on an end of a travel of the reagent kit moving into the cavity; the docking assembly is arranged facing the first mounting position in condition of the reagent kit being moved into the cavity; a part of the linkage assembly is assembled on the first mounting position, and another part of the linkage assembly is assembled on the shell.

307. The biological parameter analysis device according to claim 306, wherein the bracket comprises a frame plate and a mounting plate arranged on a side of the frame plate, and the frame plate and the mounting plate cooperate to define the cavity; an avoidance opening is defined between the frame plate and the mounting plate to avoid the linkage assembly and the sampling assembly; the avoidance opening is arranged opposite the door, and the linkage assembly is configured to drive the sampling assembly to be separated from the docking assembly while driving the door to open the window; a sample inlet of the sampling assembly is capable of being unscrewed from the window.

308. The biological parameter analysis device according to claim 307, wherein the linkage assembly comprises a drive member and a first linkage member; the drive member is arranged on the first mounting position, and the first linkage member is disposed on an output end of the drive member; the first linkage member is docked with the docking assembly in condition of the reagent kit being moved into the cavity; the drive member is configured to drive the first linkage member to move the docking assembly and to drive the first linkage member to move the door.

309. The biological parameter analysis device according to claim 308, wherein the drive member and the first linkage member are arranged on opposite sides of the frame plate, and the drive member is arranged outside the cavity.

310. The biological parameter analysis device according to claim 308, wherein the first linkage member comprises a first docking portion for docking with the docking assembly and a first linkage portion for linkage with the door.

311. The biological parameter analysis device according to claim 310, wherein the linkage assembly comprises a second linkage assembly arranged on the shell; an end of the second linkage assembly is docked with the first linkage member, and another end of the second linkage assembly is docked with the door.

312. The biological parameter analysis device according to claim 311, wherein the second linkage assembly comprises a second linkage member and a transmission component that is transmission-connected to the second linkage member; the second linkage member is docked with the first linkage member to move relative to the shell under an action of the first linkage member; a moving direction of the second linkage member is perpendicular to a direction of opening or closing of the door.

313. The biological parameter analysis device according to claim 312, wherein the transmission component comprises a first transmission member which is transmission-connected to the second linkage member, a second transmission member which is transmission-connected to the first transmission member, a third transmission member which is transmission-connected to the second transmission member, and a fourth transmission member which is transmission-connected to the third transmission member; the fourth transmission member is assembled with the door;
wherein the second linkage member is configured to move to drive the first transmission member to rotate, and the second transmission member rotates synchronously following a rotation of the first transmission member; a rotation of the second transmission member drives the third transmission member to move, and the third transmission member moves to drive the fourth transmission member to rotate, causing the door to rotate synchronously with the fourth transmission member; a moving direction of the third transmission member is perpendicular to a moving direction of the second linkage member.

314. The biological parameter analysis device according to claim 313, wherein the linkage assembly comprises a seat body arranged on the shell, and the second linkage member is slidably connected to the seat body; one of the second linkage member and the seat body defines a travel groove, and the other of the second linkage member and the seat body is arranged with a limit block; the limit block is slidable in the travel groove to limit a sliding travel of the second linkage member relative to the seat body.

315. The biological parameter analysis device according to claim 314, wherein a first elastic member is arranged between the second linkage member and the seat body; in condition of the second linkage member moving relative to the housing under an action of the first linkage member, the first elastic member changes a state to generate an elastic force; in condition of the action of the first linkage member being cancelled, the first elastic member returns to an original state, causing the second linkage member to return to an original position.

316. The biological parameter analysis device according to claim 314, wherein the fourth transmission member is rotatably connected to the seat body, and a second elastic member is arranged between the fourth transmission member and the seat body; in condition of the third transmission member driving the fourth transmission member to rotate and thereby driving the door to open the window, the second elastic member changes a state to generate an elastic force; in condition of a force exerted by the third transmission member on the fourth transmission member being cancelled, the fourth transmission member drives the door to cover the window under an action of the elastic force.

317. A sample parameter analysis apparatus, comprising:
a housing, arranged with a window and a door that is capable of covering the window, wherein the housing comprises a first side and a second side arranged opposite each other, and the door is arranged on the first side and is rotatably connected to the housing; and
a connecting assembly, arranged on the second side of the housing to connect the housing and the door in condition of the door covering the window, wherein the connecting assembly comprises a clamping member, a drive member, and a first elastic member; an end of the first elastic 6402c is connected to a middle portion of the clamping member, and another end of the first elastic member is connected to the housing; an end of the clamping member is connected to the drive member, and another end of the clamping member is configured to limit a position of the door in condition of the door covering the window;
wherein the drive member is configured to drive the clamping member to release a restriction on the door, and the door is rotatable relative to the housing to open the window; the first elastic member deforms to generate an elastic force in condition of the clamping member moving, and the elastic force is configured to act on the clamping member to cause the clamping member to return to an original position in condition of the drive member cancelling a drive on the clamping member.

318. The sample parameter analysis apparatus according to claim 317, wherein the second side of the housing defines an engagement groove, and a side of the door close to the housing is arranged with an engagement portion; the engagement portion is capable of being moved into and of being moved out of the engagement groove; in condition of the engagement portion being moved into the engagement groove, the clamping member abuts against the engagement portion to limit the position of the door.

319. The sample parameter analysis apparatus according to claim 318, wherein an end of the clamping member is arranged with a protrusion, and the engagement portion defines a groove; in condition of the engagement portion being moved into the engagement groove, the protrusion is inserted into the groove to limit the position of the door.

320. The sample parameter analysis apparatus according to claim 319, wherein the engagement groove comprises a first notch and a second notch that are disposed on adjacent sides of the engagement groove; the engagement portion is capable of being moved into and of being moved out of the first notch, and the protrusion is capable of being moved into and of being moved out of the second notch;
wherein in condition of the engagement portion moving from the first notch into the engagement groove, the engagement portion pushes the protrusion out of the second notch; the protrusion is capable of being moved into the engagement groove or the groove under an action of the elastic force of the first elastic member.

321. The sample parameter analysis apparatus according to claim 320, wherein at least one of the engagement portion and the protrusion is arranged with a guide ramp; the guide ramp is configured to guide the engagement portion to push the protrusion out of the second notch in condition of the engagement portion being moved into the engagement groove.

322. The sample parameter analysis apparatus according to claim 320, wherein the engagement groove comprises a first side wall and a second side wall arranged at intervals, the first side wall and the second side wall being respectively connected to the housing; the clamping member is rotatable around the first side wall, and the protrusion is caused to be capable of being moved into and of being moved out of a space between the first side wall and the second side wall;
the clamping member is rotatable around the first side wall; an end of the clamping member that is arranged with the protrusion and another end of the clamping member that is connected to the drive member are arranged on both sides of the first side wall, and an end of the first elastic member is connected between the ends of the clamping member.

323. The sample parameter analysis apparatus according to claim 318, wherein the connecting assembly further comprises a second elastic member arranged on the engagement groove; in condition of the engagement portion being moved into the engagement groove, the engagement portion squeezes or stretches the second elastic member, causing the second elastic member to deform and generate an elastic force; in condition of the drive member driving the clamping member to release the restriction on the door, the elastic force acts on the door to cause the door to rotate relative to the housing and open the window.

324. The sample parameter analysis apparatus according to claim 323, wherein the engagement groove comprises a third side wall spaced apart from the housing, the connecting assembly further comprises a supporting member passing through the third side wall, and the second elastic member acts between the supporting member and the third side wall; the engagement portion pushes the supporting member to move in condition of the engagement portion being moved into the engagement groove to change a state of the second elastic member to generate the elastic force.

325. The sample parameter analysis apparatus according to claim 324, wherein the third side wall defines a storage groove, and the supporting member is arranged with a limit portion; an end of the supporting member is inserted into the storage groove to be connected to the second elastic member, and the limit portion is disposed in the storage groove to limit a movement travel of the limit portion in condition of the engagement portion pushing the supporting member to move.

326. The sample parameter analysis apparatus according to claim 318, wherein the connecting assembly further comprises a limit member disposed on the second side of the housing, and the clamping member is disposed between the limit member and the housing.

327. A sample analysis box, comprising:
a seat body, comprising a first cavity, a second cavity, and an isolation member, wherein the isolation member is configured to cause the first cavity and the second cavity to communicate or isolate with each other, and in condition of the first cavity and the second cavity being in communication, liquid in the first cavity is capable of flowing to the second cavity;
wherein the isolation member is capable of changing to a state under an action of an external force to enable the first cavity and the second cavity to be in communication, and in condition of the external force being removed, the isolation member returns to another state where the first cavity and the second cavity are isolated.

328. The sample analysis box according to claim 327, wherein the seat body comprises a first liquid port for communicating the first cavity with the second cavity; the isolation member is arranged on a cavity wall of the first cavity corresponding to the first liquid port and is configured to open or cover the first liquid port; the isolation member covers the first liquid port to isolate the first cavity from the second cavity in condition of no external force being applied to the isolation member; in condition of the external force being applied to the isolation member, the first liquid port is opened to allow the first cavity and the second cavity to be in communication through the first liquid port.

329. The sample analysis box according to claim 328, wherein the cavity wall of the first cavity is arranged with a squeezing member; the squeezing member abuts against the isolation member and is configured to apply a force to the isolation member, causing the isolation member to open the first liquid port.

330. The sample analysis box according to claim 329, wherein the first cavity comprises a squeezing opening, and the squeezing member is sealed and assembled with the squeezing opening; the squeezing member extends into the first cavity from the squeezing opening to abut against the isolation member and apply the force to the isolation member.

331. The sample analysis box according to claim 329, wherein the isolation member comprises a connecting portion and an isolation portion; the connecting portion is connected to the cavity wall of the first cavity; an end of the isolation portion is connected to the connecting portion, and another end of the isolation portion is configured to abut against the squeezing member; the isolation portion is movable relative to the connecting portion under the action of the external force to open the first liquid port, and in condition of the external force being removed, the isolation portion returns to a state that covers the first liquid port.

332. The sample analysis box according to claim 331, wherein at least one of the isolation portion and the connecting portion is arranged with an elastic member, the elastic member being disposed between the connecting portion and the isolation portion; the isolation portion is deformed under the action of the external force to deform the elastic member to generate an elastic force; in condition of the external force being removed, the elastic force causes the isolation portion to return to an original state.

333. The sample analysis box according to claim 331, wherein the isolation portion is arranged with a seal on a side close to the first liquid port; in condition of the isolation portion returning to an original state to cover the first liquid port, the seal is sealed and assembled with the first liquid port.

334. The sample analysis box according to claim 331, wherein the connecting portion is fixedly arranged on the cavity wall of the first cavity, and the isolation portion is rotatably connected to the connecting portion through a rotating shaft.

335. The sample analysis box according to claim 331, wherein the squeezing member comprises an assembly portion and a squeezing portion, the assembly portion being configured to mount the squeezing member to the squeezing opening; the assembly portion is annular in shape, and the squeezing portion is arranged in an annular hollow region of the assembly portion and is elastically connected to the assembly portion; the assembly portion is sealed and assembled with the squeezing opening.

336. The sample analysis box according to claim 327, wherein the seat body comprises a third cavity and a salt bridge; an end of the salt bridge is exposed from the second cavity, and another end of the salt bridge is exposed from the third cavity and connected to an electrode terminal in the third cavity.

337. A detection assembly, comprising:
a first liquid path, a second liquid path, a liquid inlet, a liquid outlet, and a valve assembly;
wherein an end of the first liquid path is configured to be in communication with the liquid inlet, and another end of the first liquid path is configured to be in communication with the liquid outlet; an end of the second liquid path is configured to be in communication with the liquid inlet, and another end of the second liquid path is configured to be in communication with the liquid outlet;
the valve assembly is arranged on the liquid inlet, and/or on the liquid outlet, and/or between the liquid inlet and the liquid outlet, and is capable of being switched between a first turn-on state and a second turn-on state to control the first liquid path and the second liquid path to be selectively connected;
in the first turn-on state, a first external liquid of the detection assembly is capable of flowing into the first liquid path from the liquid inlet and flowing out of the first liquid path from the liquid outlet;
in the second turn-on state, a second external liquid of the detection assembly is capable of flowing into the second liquid path from the liquid inlet and flowing out of the second liquid path from the liquid outlet.

338. The detection assembly according to claim 337, wherein the first liquid path comprises a first inlet and a first outlet, the second liquid path comprises a second inlet and a second outlet, and the valve assembly comprises a first valve and a second valve; the first inlet and the second inlet are configured to be in communication with the liquid inlet, and the first outlet and the second outlet are configured to be in communication with the liquid outlet; the first valve is arranged between the first inlet and the first outlet to control connection and disconnection of the first liquid path, and the second valve is arranged between the second inlet and the second outlet to control connection and disconnection of the second liquid path;
either the first valve or the second valve is selectively turned on.

339. The detection assembly according to claim 338, wherein the valve assembly further comprises a third valve, which is arranged between the liquid inlet and the second inlet and is configured to control opening and closing of the liquid inlet and the second inlet; either the third valve or the first valve is selectively turned on.

340. The detection assembly according to claim 339, wherein the first liquid path comprises a first liquid inlet section and a first liquid outlet section, the first liquid inlet section being in communication with the liquid inlet and the first liquid outlet section being in communication with the liquid outlet; the first valve is arranged between the first liquid inlet section and the first liquid outlet section to control connection and disconnection of the first liquid inlet section and the first liquid outlet section, and the third valve is arranged between the second inlet and the first liquid inlet section to control the second inlet to be communicated or dis-communicated with the first liquid inlet section.

341. The detection assembly according to claim 339, wherein the detection assembly further comprises a housing and a plate; the plate defines a first through opening and a second through opening, and the housing comprises a cavity; a cavity wall of the cavity defines a first fluid slot and a second fluid slot; the plate is disposed in the cavity; the first through opening is in communication with a part of the first fluid slot, and the second through opening is in communication with a part of the second fluid slot; the plate encloses and seals another part of the first fluid slot to form the first liquid path, and encloses and seals another part of the second fluid slot to form the second liquid path; the liquid inlet and the liquid outlet are respectively in communication with the cavity; the valve assembly is arranged outside the cavity, and the first valve is configured to control the connection and disconnection of the first liquid path, and the second valve is configured to control the connection and disconnection of the second liquid path.

342. The detection assembly according to claim 341, wherein the housing further comprises a first housing and a second housing enclosing cooperatively to define the cavity, and the first housing and the second housing cooperate to clamp the plate to form the first liquid path and the second liquid path;
the first housing defines a first through hole and a second through hole;
wherein the liquid inlet and the first inlet are arranged on both ends of the first through hole of the first housing, or the liquid inlet and the second inlet are arranged on both ends of the first through hole of the first housing; and/or,
wherein the first outlet and the liquid outlet are arranged on both ends of the second through hole of the first housing, or the second outlet and the liquid outlet are arranged on both ends of the second through hole of the first housing.

343. The detection assembly according to claim 342, wherein the second housing comprises a receiving cavity, the first housing is at least partially embedded in the receiving cavity and cooperates with the second housing to define the cavity, and the plate is disposed between the first housing and the second housing.

344. The detection assembly according to claim 342, wherein a first groove is defined on the housing, and the housing is arranged with a first liquid inlet channel and a first liquid outlet channel that are in communication with the first groove; a second groove is defined on the housing, and the housing is arranged with a second liquid inlet channel and a second liquid outlet channel that are in communication with the second groove;
the first liquid path between the first inlet and the first outlet comprises two first sub-liquid paths that are discontinuous, an end of the first liquid inlet channel being in communication with the first inlet, and an end of the first liquid outlet channel being in communication with the first outlet; the two first sub-liquid paths are in communication through the first groove, the first liquid inlet channel, and the first liquid outlet channel;
the second liquid path between the second inlet and the second outlet comprises two second sub-liquid paths that are discontinuous, an end of the second liquid inlet channel being in communication with the second inlet, and an end of the second liquid outlet channel being in communication with the second outlet;
the two second sub-liquid paths are in communication through the second groove, the second liquid inlet channel, and the second liquid outlet channel;
the first valve is an elastomer and disposed on the housing, and capable of abutting against the first groove under an action of an external force to block the first liquid inlet channel and the first liquid outlet channel, for disconnecting the first liquid path; the second valve is an elastomer and disposed on the housing, and capable of abutting against the second groove under an action of an external force to block the second liquid inlet channel and the second liquid outlet channel, for disconnecting the second liquid path.

345. The detection assembly according to claim 344, wherein the housing is arranged with a third groove, the third valve, and a third liquid inlet channel and a third liquid outlet channel that are respectively in communication with the third groove;
the first liquid path and the second liquid path are discontinuous; an end of the third liquid inlet channel is in communication with the first inlet, and an end of the third liquid outlet channel is in communication with the second inlet; the first liquid path and the second liquid path are in communication through the third groove, the third liquid inlet channel, and the third liquid outlet channel;
the third valve is an elastomer and capable of abutting against the third groove under an action of an external force to block the third liquid inlet channel and the third liquid outlet channel, for disconnecting the first liquid path from the second liquid path.

346. The detection assembly according to claim 345, wherein the first valve, the second valve, and the third valve form an integrated structure.
